# EUROPÄISCHE PATENTSCHRIFT

(11) **EP 2 773 614 B1**
(45) Veröffentlichungstag und Bekanntmachung des Hinweises auf die Patenterteilung: **30.11.2016**
(21) Anmeldenummer: 12778354.6
(22) Anmeldetag: 29.10.2012
(51) Int. Cl.: A01N 43/40, C07D 213/24

(54) **SUBSTITUIERTE 4-CYAN-3-PHENYL-4-(PYRIDIN-3-YL)BUTANOATE, VERFAHREN ZU DEREN HERSTELLUNG UND DEREN VERWENDUNG ALS HERBIZIDE UND PFLANZENWACHSTUMSREGULATOREN**
SUBSTITUTED 4.CYAN-3-(PHENYL-4-(PYRIDIN-3-YL) BUTANOATES, METHOD FOR THEIR PRODUCTION AND THEIR USE AS HERBICIDES AND PLANT GROWTH REGULATORS
4-CYAN-3-PHÉNYLE-4-(PYRIDINE-3-YL)BUTANOATES SUBSTITUÉS, LEUR PROCÉDÉ DE FABRICATION ET D'UTILISATION EN TANT QU'HERBICIDES ET RÉGULATEURS DE CROISSANCE DE PLANTES

(30) Priorität: 31.10.2011 EP 11187228
(43) Veröffentlichungstag der Anmeldung: 10.09.2014
(73) Patentinhaber: Bayer Intellectual Property GmbH, 40789 Monheim (DE)
(72) Erfinder: JAKOBI, Harald, 60598 Frankfurt (DE); MOSRIN, Marc, 65933 Frankfurt am Main (DE); DIETRICH, Hansjörg, 65835 Liederbach am Taunus (DE); GATZWEILER, Elmar, 61231 Bad Nauheim (DE); HÄUSER-HAHN, Isolde, 51375 Leverkusen (DE); HEINEMANN, Ines, 65719 Hofheim (DE); ROSINGER, Christopher, Hugh, 65719 Hofheim (DE)
(74) Vertreter: BIP Patents
(86) Internationale Anmeldenummer: PCT/EP2012/071391
(87) Internationale Veröffentlichungsnummer: WO 2013/064462

(56) Entgegenhaltungen:
- EP-A- 0 005 341

## Beschreibung

Die Erfindung betrifft das technische Gebiet der Herbizide und Pflanzenwachstumsregulatoren, vorzugsweise der Herbizide zur Bekämpfung von unerwünschtem Pflanzenwuchs oder der Herbizide zur selektiven Bekämpfung von Unkräutern und Ungräsern in Nutzpflanzenkulturen, oder der Pflanzenwachstumsregulatoren, welche zur Beeinflussung des Wachstums von Kulturpflanzen eingesetzt werden können.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es, dass sie (a) keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen und/ oder (d) ein toxikologisch ungünstiges Profil besitzen. Weiterhin führen manche Wirkstoffe, die als Pflanzenwachstumsregulatoren bei einigen Nutzpflanzen eingesetzt werden können, bei anderen Nutzpflanzen zu unerwünscht verminderten Ernteerträgen oder sind mit der Kulturpflanze nicht oder nur in einem engen Aufwandmengenbereich verträglich. Einige der bekannten Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab.

Aus den veröffentlichten Patentanmeldungen EP-A-5341, EP-A-266725, EP-A-270830, JP-04/297454, JP-04/297455, JP-05/058979, WO 2011/003775, WO 2011/003776, WO 2011/042378, WO 2011/073143 und WO2011/098417 sind herbizide Cyanobutyrate bekannt.

EP-A-5341 beschreibt herbizide Ester und Amide von 4-Cyano-3,4-diaryl-butansäuren. Als Arylreste werden gegebenenfalls substituierte Phenyl-reste und unsubstituiertes Pyridyl oder Thienyl genannt. In den konkreten Beispielen überwiegen die 4-Cyano-3,4-diphenyl-butansäuren und -säureester. Zu den Verbindungen mit Pyridyl gibt es zwei konkrete Beispiele, und zwar 4-Cyano-4-phenyl-4-(pyridin-3-yl)-butansäure und der entsprechende Ethylester.

Gemäß EP-A-5341 eignen sich die threo-Isomere allgemein zur nicht-selektiven Bekämpfung von Schadpflanzen, während die erythro-threo-Isomerengemische für die selektive Bekämpfung von Schadpflanzen in einigen Nutzpflanzenkulturen in Betracht kommen. In EP-A-5341 wird außerdem erwähnt, dass die 2 Enantiomere, die zur threo-Form gehören, unterschiedlich wirksam sind, was am Beispiel der unterschiedlichen Wirkungen der Enantiomere des Enantiomerenpaares der in den Phenylresten unsubstituierten 4-Cyano-3,4-diphenyl-butansäure untersucht worden ist. Aus EP-A-266725 sind einige erythro-threo-Isomerengemische von 4-Cyano-3,4-diphenyl-butansäuren und -säurederivaten bekannt, die selektiv zur Unkrautkontrolle in Reiskulturen eingesetzt werden können.

EP-A-270830 beschreibt, dass threo-Isomere und erythro-threo-Isomerengemische von 4-Cyano-3,4-diaryl-butansäure(-estern) als Pflanzenregulatoren verwendet werden können und die Ausbildung eines Fruchtstands bei verschiedenen Schadgräsern verhindern können. Als Arylreste werden gegebenenfalls substituierte Phenyl-reste und unsubstituiertes Pyridyl oder halogensubstituiertes Pyridyl genannt. Konkrete Beispiele betreffen überwiegend (substituierte) 4-Cyano-3,4-diphenyl-butansäure(-ester). Darüber hinaus sind auch 4-Cyano-3-pyridyl-4-phenyl-butansäureester, wie die Verbindung 4-Cyano-3-pyridyl-4-phenyl-butansäure-ethylester, oder 4-Cyano-3-phenyl-4-pyridyl-butansäure-ester, wie die Verbindung 4-Cyano-3-(4-chlorphenyl)-4-(6-fluor-pyridin-3-yl)-butansäuremethylester, beschrieben.

Aus JP-04/297455 sind herbizide 4-Cyano-3-phenyl-4-heteroaryl-butansäure(-ester) bekannt, wobei die heterocyclischen Reste aus der Gruppe 1,2,4-Triazolyl, 1-Pyrazolyl, 1-Imidazolyl, 2- und 3-Thienyl, 2- und 3-Pyridyl, 1-Methyl-pyrrol-2-yl, 2-Quinolinyl, 2-Methyl-3-trifluormethylpyrazol-5-yl und 5-Trifluormethylpyridin-2-yl ausgewählt sind. Konkret Verbindungen aus der 3-Pyridyl-Reihe enhalten nur unsubstituierte Pyridin-3-ylreste, z. B. die Verbindungen 4-Cyano-3-phenyl-4-(pyridin-3-yl)-butansäurepropargyl und -allylester.
Aus WO 2011/003775 sind spezielle Ester von 4-Cyano-3,4-diphenyl-butansäuren bekannt, die als wirksame Herbizide, vorzugsweise auch in Nutzpflanzenkulturen eingesetzt werden können.
Aus WO 2011/003776, WO 2011/042378, WO 2011/073143 und WO2011/098417 sind 4-Cyano-3,4-diphenyl-butansäuren und -säureester bekannt, die an den Phenylresten spezifisch substituiert sind und als wirksame Herbizide, vorzugsweise auch in Nutzpflanzenkulturen eingesetzt werden können.
Die herbizide Wirkung der bekannten Verbindungen der genannten Stoffklasse, insbesondere bei niedrigen Aufwandmengen, bzw. deren Verträglichkeit gegenüber Kulturpflanzen bleiben verbesserungswürdig.

Aus den genannten Gründen besteht weiterhin ein Bedarf nach alternativen wirkungsstarken Herbiziden für die selektive Anwendung in Pflanzenkulturen oder die Anwendung auf Nichtkulturland. Auch ist es wünschenswert, alternative chemische Wirkstoffe bereitzustellen, die gegebenenfalls mit Vorteilen als Herbizide oder Pflanzenwachstumsregulatoren eingesetzt werden können.
Gewünscht sind auch Verbindungen mit herbizider Wirkung, die bereits bei relativ niedrigen Aufwandmengen gegen wirtschaftlich wichtige Schadpflanzen hochwirksam sind und vorzugsweise bei guter Wirksamkeit gegen Schadpflanzen selektiv in Kulturpflanzen eingesetzt werden können.

Überraschenderweise wurde nun gefunden, dass Verbindungen bestimmter heterocyclisch substituierter Cyanobutyrate besondere herbizide Wirkungen aufweisen und vorzugsweise in einigen Kulturen selektiv zur Schadpflanzenkontrolle eingesetzt werden können.

Gegenstand der vorliegenden Erfindung sind Verbindungen der Formel (I) oder deren Salze, 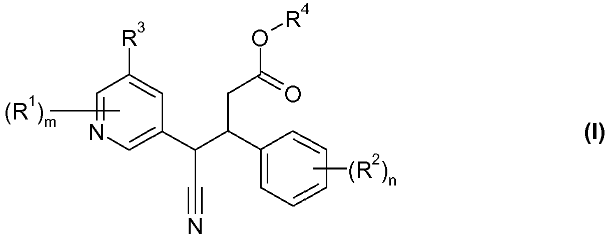 in welcher
(R¹)ₘ m Substituenten R¹ bedeutet,
   wobei R¹, wenn m = 1, oder jeder der Substituenten R¹, wenn m größer als 1 ist, unabhängig voneinander Halogen, Cyano, Nitro, Hydroxy, (C₁-C₈)Alkyl, (C₂-C₆)Alkenyl, (C₂-C₆)Alkinyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Haloalkenyl, (C₂-C₆)Haloalkinyl, (C₁-C₆)Alkoxy-(C₁-C₄)alkyl, (C₁-C₆)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR⁵, C(O)NR⁶R⁷, C(O)-Het¹, NR⁸R⁹ oder Het² bedeutet oder wobei jeweils zwei am Ring ortho-ständige Gruppen R¹ oder R¹ und R³ gemeinsam eine Gruppe der Formel -Z¹-A*-Z² bedeuten, in welcher
   A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
   Z¹ für eine direkte Bindung, O oder S steht und
   Z² für eine direkte Bindung, O oder S steht,
   wobei die Gruppe -Z¹-A*-Z² zusammen mit den an die Gruppe gebundenen C-Atomen des Phenylrings einen ankondensierten 5 oder 6 Ring bilden,
(R²)ₙ n Substituenten R² bedeutet,
   wobei R², wenn n = 1, oder jeder der Substituenten R², wenn n größer als 1 ist, unabhängig voneinander Halogen, Cyano, Nitro, Hydroxy, (C₁-C₈)Alkyl, (C₂-C₆)Alkenyl, (C₂-C₆)Alkinyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Haloalkenyl, (C₂-C₆)Haloalkinyl, (C₁-C₆)Alkoxy-(C₁-C₄)alkyl, (C₁-C₆)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR¹⁰, C(O)NR¹¹R¹², C(O)-Het³, NR¹3¹⁴ oder Het⁴ bedeutet oder wobei jeweils zwei am Ring ortho-ständige Gruppen R² gemeinsam eine Gruppe der Formel -Z³ -A**-Z⁴ bedeuten, in welcher
   A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
   Z³ für eine direkte Bindung, O oder S steht und
   Z⁴ für eine direkte Bindung, O oder S steht,
   wobei die Gruppe -Z³-A**-Z⁴ zusammen mit den an die Gruppe gebundenen C-Atomen des Phenylrings einen ankondensierten 5 oder 6 Ring bilden,
R³ Halogen, Cyano, Nitro, Hydroxy, (C₁-C₈)Alkyl, (C₂-C₆)Alkenyl, (C₂-C₆)Alkinyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Haloalkenyl, (C₂-C₆)Haloalkinyl, (C₁-C₆)Alkoxy-(C₁-C₄)alkyl, (C₁-C₆)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR¹⁵, C(O)NR¹⁶R¹⁷, C(O)-Het⁵, NR¹⁸R¹⁹ oder Het⁶ bedeutet,
R⁴ Wasserstoff oder einen hydrolysierbaren Rest bedeutet, vorzugsweise
   R⁴ Wasserstoff oder einen gegebenenfalls substituierten Kohlenwasserstoffrest oder einen gegebenenfalls substituierten Heterocyclylrest, wobei jeder der beiden letztgenannten kohlenstoffhaltigen Reste inklusive Substituenten 1 bis 30 C-Atome, vorzugsweise 1 bis 24 C-Atome, insbesondere 1 bis 20 C-Atome aufweist, bedeutet
      oder
   R⁴ einen Rest der Formel SiR^{a}R^{b}R^{c}, -NR^{a}R^{b} oder -N=CR^{c}R^{d} bedeutet, wobei in den letztgenannten 3 Formeln jeder der Reste R^{a}, R^{b}, R^{c} und R^{d} unabhängig voneinander Wasserstoff oder einen gegebenenfalls substituierten Kohlenwasserstoffrest bedeutet, dabei aber SiH₃ für SiR^{a}R^{b}R^{c} ausgenommen ist, oder R^{a} und R^{b} zusammen mit dem N-Atom der Gruppe -NR^{a}R^{b} einen 3- bis 9-gliedrigen Heterocyclus, welcher zusätzlich zum N-Atom ein oder zwei weitere Heteroringatome aus der Gruppe N, O und S enthalten kann und welcher unsubstituiert oder substituiert ist, bedeuten oder R^{c} und R^{d} zusammen mit dem C-Atom der Gruppe -N=CR^{c}R^{d} einen 3- bis 9-gliedrigen carbocyclischen Rest oder einen heterocyclischen Rest, welcher 1 bis 3 Heteroringatome aus der Gruppe N, O und S enthalten kann, wobei der carbocyclische oder heterocyclischen Rest unsubstituiert oder substituiert ist, bedeuten, wobei jeder der Reste R^{a}, R^{b}, R^{c} und R^{d} inklusive Substituenten bis 30 C-Atome, vorzugsweise bis 24 C-Atome, insbesondere bis 20 C-Atome aufweist,
      oder
R⁴ einen Rest der Formel -C(=O)-R^{e} oder -P(=O)(R^{f})₂ bedeutet, wobei R^{e} und die R^{f} unabhängig voneinander jeweils Wasserstoff, OH, (C₁-C₈)Alkyl, (C₁-C₄)Haloalkyl, (C₂-C₈)Alkenyl, (C₂-C₈)Alkinyl, (C₁-C₆)Alkoxy, (C₁-C₆)Alkoxy-(C₁-C₈)alkyl, (C₁-C₄)Haloalkoxy, (C₁-C₄)Haloal koxy-(C₁-C₈)alkyl, (C₃-C₈)Alkenyloxy, (C₃-C₈)Alkenyloxy-(C₁-C₈)alkyl, (C₃-C₈)Alkinyloxy, (C₃-C₈)Alkinyloxy-(C₁-C₈)alkyl, -NR*R**, wobei R^{*} und R^{**} weiter unten definiert sind, Tri-[(C₁-C₄)alkyl]-silyl, Tri-[(C₁-C₄)alkyl]-silyl-(C₁-C₈)alkyl, (C₃-C₆)Cycloalkyl, (C₃-C₆)Cycloalkyl-(C₁-C₈)alkyl, (C₅-C₆)Cycloalkenyl, (C₅-C₆)Cycloalkenyl-(C₁-C₈)alkyl, (C₅-C₆)Cycloalkinyl, (C₅-C₆)Cycloalkinyl-(C₁-C₈)alkyl, Phenyl, Phenyl-(C₁-C₈)alkyl, Phenoxy, Phenoxy-(C₁-C₈)alkyl, Phenylamino, Phenylamino-(C₁-C₈)alkyl, einen Rest Het⁷, Het⁷-(C₁-C₆)alkyl oder Het⁷-O-(C₁-C₆)alkyl bedeuten,
   wobei jeder der letztgenannten 15 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist,
R⁵, R¹⁰ und R¹⁵ jeweils unabhängig voneinander Wasserstoff, (C₁-C₆)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die unten genannte Gruppe M, vorzugsweise (C₁-C₄)Alkyl bedeuten,
R⁶, R⁷ R⁸, R⁹, R¹¹, R¹², R¹³, R¹⁴, R¹⁶, R¹⁷, R¹⁸ und R¹⁹ jeweils unabhängig voneinander Wasserstoff, (C₁-C₆)Alkyl, (C₂-C₆)Alkenyl oder (C₂-C₆)Alkinyl, wobei jeder der letztgenannten 3 Reste jeweils unabhängig voneinander unsubstituiert oder durch einen oder mehrere Reste aus der Gruppe Halogen, Nitro, Cyano und Phenyl, das gegebenenfalls substituiert ist, vorzugweise unsubstitutiert oder durch einen oder mehrere Reste aus der Gruppe Halogen, Nitro, Cyano, (C₁-C₄)Alkyl und (C₁-C₄)Haloalkyl substituiert ist, substituiert ist, oder (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, Nitro, Cyano, (C₁-C₄)Alkyl, (C₁-C₄)Haloalkyl, Phenyl und Benzyl, wobei jeder der letztgenannten 2 Reste gegebenenfalls substituiert ist, vorzugsweise unsubstituiert oder durch einen oder mehrere Reste aus der Gruppe Halogen, Nitro, Cyano, (C₁-C₄)Alkyl und (C₁-C₄)Haloalkyl substituiert ist, substituiert ist, bedeuten,
Het¹, Het², Het³, Het⁴, Het⁵ und Het⁶ jeweils unabhängig voneinander einen gesättigten oder teilungesättigten Rest eines Heterocyclus mit 3 bis 9 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, (C₁-C₄)Alkoxy, (C₁-C₄)Haloalkoxy, (C₁-C₄)Alkylthio und Oxo substituiert ist, vorzugsweise den Rest eines gesättigten Heterocyclus der genannten Art, insbesondere eine Morpholino- Piperidino- oder Pyrrolidinogruppe bedeuten,
Het⁷ jeweils unabhängig voneinander einen gesättigten, teilweise ungesättigten oder heteroaromatischen monocyclischen Heterocyclylrest mit 3 bis 9 Ringatomen oder einen 9- oder 10-gliedrigen bicyclischen Heterocyclus, jeweils enthaltend 1, 2, 3 oder 4 Heteroatome, ausgewählt aus der Gruppe bestehend aus O, N und S, bedeutet,
R*, R** jeweils unabhängig voneinander (unabhängig auch von anderen Resten NR*R**) H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus gegebenenfalls durch einen oder mehrere gleiche oder verschiedene Reste aus der Gruppe Halogen, (C₁-C₄)Alkyl, (C₁-C₄)Haloalkyl, (C₁-C₄)Alkoxy oder (C₁-C₄)Haloalkoxy oder im Fall gesättigter oder teilungesättigter cyclischer Basisgruppen auch Oxo substituiert ist, bedeuten oder
R* und R** zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten,
R^{A} Halogen, Cyano, Hydroxy oder (C₁-C₆)Alkoxy bedeutet,
R^{B} Halogen, Cyano, Hydroxy, Oxo, Nitro, (C₁-C₈)Alkyl, (C₁-C₆)Haloalkyl, Cyano-(C₁-C₆)alkyl, Hydroxy-(C₁-C₆)alkyl, Nitro-(C₁-C₆)alkyl, (C₂-C₈)Alkenyl, (C₂-C₈)Haloalkenyl, (C₂-C₈)Alkinyl, (C₂-C₈)Haloalkinyl, (C₁-C₈)Alkoxy, (C₂-C₈)Alkenyloxy, (C₂-C₈)Alkinyloxy, (C₁-C₈)Haloalkoxy, (C₁-C₆)Alkoxy-(C₁-C₆)alkyl, (C₁-C₆)Alkoxy-(C₁-C₄)alkoxy, (C₁-C₆)Haloalkoxy-(C₁-C₄)alkyl, (C₁-C₆)Haloalkoxy-(C₁-C₄)alkoxy, (C₁-C₈)Alkylthio, (C₂-C₆)Alkenylthio, (C₂-C₆)Alkinylthio, (C₁-C₈)Alkylsulfinyl , (C₁-C₆)Haloalkylsulfinyl, (C₁-C₈)Alkylsulfonyl, (C₁-C₆)Haloalkylsulfonyl, einen Rest der Formel R^{aa}-C(=O)-, R^{aa}-C(=O)-(C₁-C₆)alkyl, wobei die R^{aa} weiter unten definiert sind, -NR*R**, wobei R^{*} und R** weiter unten definiert sind, Tri-[(C₁-C₄)alkyl]-silyl, Tri-[(C₁-C₄)alkyl]-silyl-(C₁-C₆)alkyl, (C₃-C₈)Cycloalkyl, (C₃-C₈)Cycloalkoxy, (C₃-C₈)Cy_{C}loalkyl-(C₁-C₈)alkyl, (C₃-C₈)Cycloalkyl-(C₁-C₈)alkoxy, Phenyl, Phenyl-(C₁-C₈)alkyl, Phenoxy, Phenoxy-(C₁-C₈)alkyl, Phenylamino, Phenylamino-(C₁-C₈)alkyl oder einen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, wobei jeder der letztgenannten 11 Reste im cyclischen Teil gegebenenfalls durch einen oder mehrere gleiche oder verschiedene Reste R^{bb} substituiert ist, bedeutet,
R^{aa} jeweils unabhängig voneinander Wasserstoff, OH, (C₁-C₈)Alkyl, (C₁-C₆)Haloalkyl, (C₂-C₈)Alkenyl, (C₂-C₈)Alkinyl, (C₁-C₈)Alkoxy, (C₁-C₆)Alkoxy-(C₁-C₆)alkyl, (C₁-C₆)Alkoxy-(C₁-C₆)alkyloxy, (C₁-C₆)Haloalkoxy, (C₁-C₆)Haloalkoxy-(C₁-C₆)alkyl, (C₁-C₆)Haloalkoxy-(C₁-C₆)alkoxy, (C₃-C₈)Alkenyloxy, (C₃-C₈)Alkenyloxy-(C₁-C₆)alkyl, (C₃-C₈)Alkenyloxy-(C₁-C₆)alkoxy, (C₃-C₈)Alkinyloxy, (C₃-C₈)Alkinyloxy-(C₁-C₆)alkyl, (C₃-C₈)Alkinyloxy-(C₁-C₆)alkoxy, -NR*R*, wobei R^{*} und R^{**} wie oben definiert sind, Tri-[(C₁-C₄)alkyl]-silyl, Tri-[(C₁-C₄)alkyl]-silyl-(C₁-C₆)alkyl, Tri-[(C₁-C₄)alkyl]-silyl-(C₁-C₆)alkoxy, (C₃-C₈)Cycloalkyl, (C₃-C₈)Cycloalkoxy, (C₃-C₈)Cycloalkyl-(C₁-C₈)alkyl, (C₃-C₈)Cycloalkyl-(C₁-C₈)alkoxy, (C₅-C₈)Cycloalkenyl, (C₅-C₈)Cycloalkenyl-(C₁-C₆)alkyl, (C₅-C₈)Cycloalkenyloxy, (C₅-C₈)Cycloalkinyl, (C₅-C₈)Cycloalkinyl-(C₁-C₆)alkyl, (C₅-C₈)Cycloalkinyl-(C₁-C₆)alkoxy, Phenyl, Phenyl-(C₁-C₈)alkyl, Phenyl-(C₁-C₈)alkoxy, Phenoxy, Phenoxy-(C₁-C₈)alkyl, Phenoxy-(C₁-C₈)alkoxy, Phenylamino, Phenylamino-(C₁-C₈)alkyl, Phenylamino-(C₁-C₈)alkoxy oder einen gegebenenfalls über eine Alkylengruppe oder eine Alkoxygruppe gebundenen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, bedeutet, wobei jeder der letztgenannten 20 Reste im cyclischen Teil gegebenenfalls durch einen oder mehrere gleiche oder verschiedene Reste R^{cc} substituiert ist, bedeutet und
R^{bb} und R^{cc} jeweils unabhängig voneinander Halogen, (C₁-C₄)Alkyl, (C₁-C₄)Haloalkyl, (C₁-C₄)Alkoxy oder (C₁-C₄)Haloalkoxy oder im Fall gesättigter oder teilungesättigter cyclischer Basisgruppen auch Oxo bedeutet und
M ein Äquivalent eines Kations,
   vorzugsweise ein Metallionenäquivalent, ein Ammoniumion, dass gegebenenfalls durch 1 bis 4 gleiche oder verschiedene Reste aus der Gruppe (C₁-C₆)Alkyl, (C₂-C₆)Alkenyl, (C₂-C₆)Alkinyl, (C₃-C₆)Cycloalkyl, (C₅-C₆)Cycloalkenyl, (C₅-C₆)Cycloalkinyl, Phenyl, (C₃-C₆)Cycloalkyl-(C₁-C₄)alkyl, (C₅-C₆)Cycloalkenyl-(C₁-C₄)alkyl, Phenyl(C₁-C₄)alkyl substituiert ist, oder ein tertiäres Sulfoniumion, das vorzugsweise durch 3 gleiche oder verschiedene Reste aus der Gruppe (C₁-C₆)Alkyl, (C₂-C₆)Alkenyl, (C₂-C₆)Alkinyl, (C₃-C₆)Cycloalkyl, (C₅-C₆)Cycloalkenyl, (C₅-C₆)Cycloalkinyl, Phenyl, (C₃-C₆)Cycloalkyl-(C₁-C₄)alkyl, (C₅-C₆)Cycloalkenyl-(C₁-C₄)alkyl, Phenyl(C₁-C₄)alkyl, insbesondere (C₁-C₄)Alkyl substituiert ist, bedeutet,
m 0, 1, 2 oder 3, vorzugsweise 0, 1 oder 2, insbesondere 0 oder 1, und
n 0, 1, 2, 3, 4 oder 5, vorzugsweise 0, 1, 2, oder 3 bedeuten.

In der Formel (I) bedeutet die Formel "(R¹)ₘ" m Reste R¹, die als Substituenten am betreffenden Pyridylring gebunden sind, wobei die Reste im Falle m größer 1 gleich oder verschieden sein können und die jeweils näher genannte Bedeutung haben. Im Fall m = 0 ist der betreffende Pyridylring nicht durch Substituenten R¹ substituiert, d. h. alle Ring-C-Atome des Pyridylrings in den Positionen 2, 4 und 6 des Pyridin-3-yl-restes sind mit einem Wasserstoffatom verbunden.

In der Formel (I) bedeutet die Formel "(R²)ₙ" n Reste R², die als Substituenten am betreffenden Phenylring gebunden sind, wobei die Reste im Falle n größer 1 gleich oder verschieden sein können und die jeweils näher genannte Bedeutung haben. Im Fall n = 0 ist der betreffende Phenylring nicht durch Substituenten R² substituiert, d. h. alle Ring-C-Atome des Phenylrings in den Positionen 2 bis 6 des Phenylrestes sind mit einem Wasserstoffatom verbunden.

Die erfindungsgemäßen Verbindungen der Formel (I) umfassen alle Stereoisomeren, welche aufgrund der Asymmetriezentren oder Doppelbindungen im Molekül, deren Konfiguration in der Formel nicht speziell bezeichnet oder die nicht speziell angegeben sind, auftreten können, und deren Mischung, inklusive der racemischen Verbindungen und der teilweise mit bestimmten Stereoisomeren angereicherten Mischungen. Die Erfindung umfasst auch alle Tautomeren, wie Keto- und Enol-Tautomeren, und deren Mischungen und Salze, wenn entsprechende funktionelle Gruppen vorhanden sind.

Die Verbindungen der Formel (I) enthalten in Position 3 und 4 des substituierten Butansäuregerüstes zwei Chiralitätszentren und treten deshalb in mindestens vier Stereoisomeren auf und deren Gemischen, d. h. 2 enantiomere Erythro-Isomere und 2 enantiomere Threo-Isomere. Je nach Substituenten (R¹)ₘ und (R²)ₙ R³ und R⁴ können ein oder mehrere weitere Chiralitätszentren enthalten sein.

Gegenstand der Erfindung sind daher auch Erythro-Threo-Gemische (Diastereomerengemische) der Verbindungen der Formel (I).

Gegenstand der Erfindung sind auch die racemischen Erythro-Isomere oder die racemischen Threo-Isomere der Verbindungen der Formel (I).
Gegenstand der Erfindung sind auch die optisch aktiven (3R, 4S)- und (3S, 4R)-Erythro-Isomere und deren Gemische mit einem Überschuss an einem Enantiomeren.

Gegenstand der Erfindung sind auch die optisch aktiven (3R, 4R)- und (3S, 4S)-Threo-Isomere und deren Gemische mit einem Überschuss an einem Enantiomeren.

Aufgrund der zwei Chiralitätszentren in Position 3 und 4 existieren Verbindungen derselben chemischen Konstitution als 4 stereoisomere Konfigurationen, und zwar zwei Erythro-Enantiomere mit den Konfigurationen (3S,4R) [= Erythro-1 bzw. (3R,4S) [= Erythro-2] und zwei Threo-Enantiomere mit den Konfigurationen (3S,4S) [= Threo-1] bzw. (3R,4R) [= Threo-2]; siehe nachfolgendes Schema:

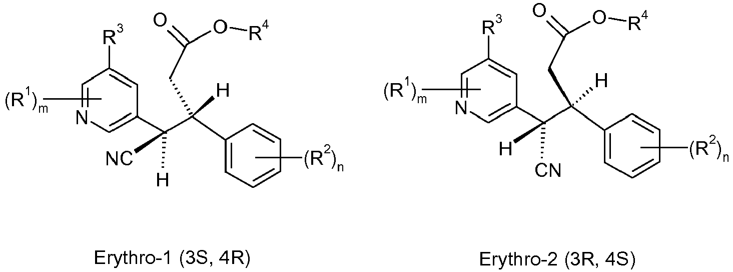

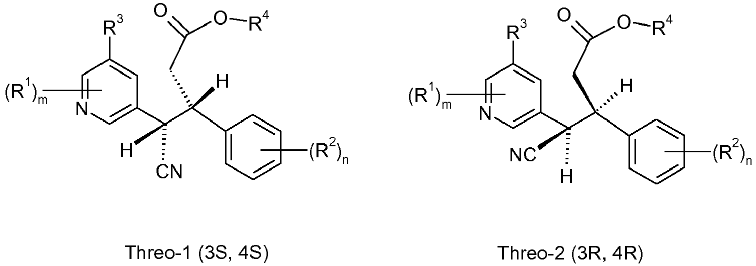

Die erfindungsgemäßen Verbindungen (I) stellen Diastereomerengemische der 4 Stereoisomeren dar, umfassen aber auch die getrennten diastereomeren Erythro- oder Threo-Formen, jeweils als racemisches Gemisch der Erythro-Enantiomere oder Threo-Enantiomere oder als reine oder stereochemisch angereicherte oben genannte Enantiomere Erythro-1, Erythro-2, Threo-1 oder Threo-2.

Bevorzugt sind die Diastereomerengemische der Formel (I) (Erythro-Threo-Gemische).

Bevorzugt sind auch die racemischen Erytho-Gemische der Formel (I) aus den genannten Enantiomeren Erythro-1 und Erythro-2 im Verhältnis 50:50.

Weiterhin bevorzugt sind die racemischen Threo-Gemische der Formel (I) aus den genannten Enantiomeren Threo-1 und Threo-2 im Verhältnis 50:50.

Weiter bevorzugt sind die (3R, 4R)-Enantiomere Threo-2 der Formel (Ia) oder deren Salze, 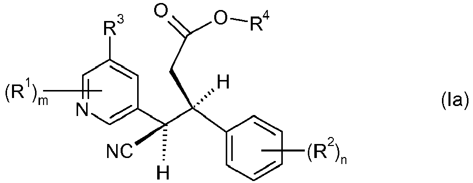
worin (R¹)ₘ und (R²)ₙ wie in Formel (I) definiert sind,
wobei die stereochemische Konfiguration am C-Atom in Position 3 des Butansäurederivats eine stereochemische Reinheit von 60 bis 100 % (R), vorzugsweise 70 bis 100 % (R), mehr bevorzugt 80 bis 100 % (R), insbesondere 90 bis 100 % (R), bezogen auf das vorliegende Gemisch der threo-Enantiomeren aufweist und
die stereochemische Konfiguration am C-Atom in Position 4 des Butansäurederivats eine stereochemische Reinheit von 60 bis 100 % (R), vorzugsweise 70 bis 100 % (*R*)*,* mehr bevorzugt 80 bis 100 % (R), insbesondere 90 bis 100 % (R), bezogen auf das vorliegende Gemisch der threo-Enantiomeren aufweist.

Die Verbindungen der Formel (I) können im Falle R⁴ = H oder im Falle geeigneter saurer Substituenten durch Umsetzung mit Basen Salze bilden, wobei der saure Wasserstoff durch ein für die Landwirtschaft geeignetes Kation ersetzt wird.
Die Verbindungen der Formel (I) können durch Anlagerung einer geeigneten anorganischen oder organischen Säure, an eine basische Gruppe, wie z.B. Amino oder Alkylamino oder auch das Stickstoffatom im Pyridylring, Salze bilden. Geeignete vorhandene saure Gruppen, wie z.B. Carbonsäuregruppen, können innere Salze mit ihrerseits protonierbaren Gruppen, wie Aminogruppen bilden.

Die Verbindungen der Formel (I) können vorzugsweise in Form landwirtschaftlich einsetzbarer Salze vorliegen, wobei es ansonsten auf die Art des Salzes in der Regel nicht ankommt. Im Allgemeinen kommen dabei die Salze derjenigen Kationen oder die Säureadditionssalze derjenigen Säuren in Betracht, deren Kationen, beziehungsweise Anionen, die herbizide Wirkung der Verbindungen (I) nicht negativ beeinträchtigen.
Es kommen als Kationen insbesondere Ionen der Alkalimetalle, vorzugsweise Lithium, Natrium oder Kalium, der Erdalkalimetalle, vorzugsweise Calcium oder Magnesium, und der Übergangsmetalle, vorzugsweise Mangan, Kupfer, Zink oder Eisen in Betracht. Ebenso kann als Kation Ammonium oder substituiertes Ammonium verwendet werden, wobei hier ein bis vier Wasserstoffatome durch (C₁-C₄)Alkyl, Hydroxy-(C₁-C₄)alkyl, (C₁-C₄)Alkoxy-(C₁-C₄)alkyl, Hydroxy-(C₁-C₄)alkoxy-(C₁-C₄)alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium. Des Weiteren kommen Phosphoniumionen, Sulfoniumionen, vorzugsweise Tri(C₁-C₄)alkylsulfonium, insbesondere Trimethylsulfonium, oder Sulfoxoniumionen, vorzugsweise Tri(C₁-C₄) alkylsulfoxonium, insbesondere Trimethyl-sulfoxonium, in Betracht.
Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von (C₁-C₄)Alkansäuren, vorzugsweise Formiat, Acetat, Propionat, Butyrat oder Trifluoracetat.

In der Formel (I) und allen nachfolgenden Formeln werden Bezeichnungen für chemische Reste verwendet, die Sammelbegriffe für die Aufzählung individueller Gruppenmitglieder darstellen oder individuelle chemische Reste spezifisch bezeichnen. In der Regel werden Bezeichnungen verwendet, die dem Fachmann geläufig sind und/oder insbesondere die nachstehend erläuterten Bedeutungen haben.

Ein hydrolysierbarer Rest (siehe Definition von R⁴) bedeutet einen unter den Anwendungsbedingungen hydrolysierbaren Rest, beispielsweise einen schon in der Spritzbrühe oder insbesondere unter den physiologischen Bedingungen in Pflanzen hydrolysierbaren Rest, wobei eine Verbindung der Formel (I) mit der Carbonsäureestergruppe -CO-OR⁴ (R⁴ ungleich Wasserstoff) zur Verbindung der Formel (I) mit der Carbonsäuregruppe -CO-OH (d. h. Verbindung (I) mit R⁴ = H) hydrolysiert wird. In der Definition der hydrolysierbaren Reste sind auch ausdrücklich die Reste mit R⁴ = Kohlenwasserstoffrest oder Heterocyclylrest, wobei die beiden letztgenannten Reste unsubstituiert oder substituiert sind, umfasst, auch wenn sie teilweise vergleichsweise langsam hydrolysierbar sind.

Ein Kohlenwasserstoffrest ist ein aliphatischer, cycloaliphatischer oder aromatischer monocyclischer oder, im Falle eines gegebenenfalls substituierten Kohlenwasserstoffrestes, auch ein bicyclischer oder polycyclischer organischer Rest auf Basis der Elemente Kohlenstoff und Wasserstoff, beispielsweise umfassend die Reste Alkyl, Alkenyl, Alkinyl, Cycloalkyl, Cycloalkenyl, Aryl, Phenyl, Naphthyl, Indanyl, Indenyl, etc.; Entsprechendes gilt für Kohlenwasserstoffreste in zusammengesetzte Bedeutungen wie Kohlenwasserstoffoxyresten oder anderen über Heteroatomgruppen gebundene Kohlenwasserstoffresten.
Wenn nicht näher definiert, weisen die Kohlenwasserstoffreste vorzugsweise 1 bis 20 C-Atome, weiter bevorzugt 1 bis 16 C-Atome, insbesondere 1 bis 12 C-Atome auf. Die Kohlenwasserstoffreste, auch in den speziellen Resten Alkyl, Alkoxy, Haloalkyl, Haloalkoxy, Alkylamino und Alkylthio, sowie die entsprechenden ungesättigten und/oder substituierten Reste können im Kohlenstoffgerüst jeweils geradkettig oder verzweigt sein.
Der Ausdruck "(C₁-C₄)Alkyl" bedeutet eine Kurzschreibweise für Alkyl mit einem bis 4 Kohlenstoffatomen, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl" umfassen entsprechend auch gradkettige oder verzweigte Alkylreste mit einer größeren Zahl von C-Atomen, d. h. gemäß Beispiel auch die Alkylreste mit 5 und 6 C-Atomen.
Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl group, vorzugsweise Allyl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl.
Alkenyl schließt insbesondere auch geradkettige oder verweigte Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl;
Alkinyl bedeutet z.B. Propargyl, But-2-in-1-yl, But-3-in-1-yl, 1-Methyl-but-3-in-1-yl. Alkinyl schließt insbesondere auch geradkettige oder verweigte Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl.
Ein 3- bis 9-gliedriger carbocyclischer Ring bedeutet (C₃-C₉)Cycloalkyl oder (C₅-C₉)Cycloalkenyl.
(C₃-C₉)Cycloalkyl bedeutet ein carbocyclisches, gesättigtes Ringsystem mit vorzugsweise 3-9 C-Atomen, z.B. Cyclopropyl, Cyclobutyl, Cyclopentyl, Cyclohexyl, Cycloheptyl, Cyclooctyl oder Cyclononyl. Im Falle von substituiertem Cycloalkyl werden cyclische Systeme mit Substituenten umfasst, wobei die Substituenten auch mit einer Doppelbindung am Cycloalkylrest, z. B. eine Alkylidengruppe wie Methyliden, gebunden sein können.
(C₅-C₉)Cycloalkenyl bedeutet ein carbocyclisches, nicht aromatisches, partiell ungesättigtes Ringsystem mit 5-9 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend
Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.
Halogen bedeutet beispielsweise Fluor, Chlor, Brom oder lod. Haloalkyl, -alkenyl und -alkinyl, gegebenenfalls auch als Halogenalkyl, Halogenalkenyl bzw. Halogenalkinyl bezeichnet, bedeuten durch gleiche oder verschiedene Halogenatome, vorzugsweise aus der Gruppe Fluor, Chlor, Brom und lod, insbesondere aus der Gruppe Fluor, Chlor und Brom, ganz besonders aus der Gruppe Fluor und Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste wie beispielsweise Halocycloalkyl (= Halogencycloalkyl).

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.
Im Falle gegebenenfalls substituiertes Aryl sind auch mehrcyclische Systeme, wie Tetrahydronaphthyl, Indenyl, Indanyl, Fluorenyl, Biphenylyl, umfasst, wobei die Bindungsstelle am aromatischen System ist.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P, B, Si, Se) der gesättigt, ungesättigt oder heteroaromatisch ist und dabei unsubstituiert oder substituiert sein kann, wobei die Bindungsstelle an einem Ringatom lokalisiert ist. Wenn nicht anders definiert, enthält er vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält.
Ist der Heterocyclylrest oder der heterocyclische Ring gegebenenfalls substituiert, kann er mit anderen carbocyclischen oder heterocyclischen Ringen annelliert sein. Bevorzugt sind benzokondensierte (benzoannellierte) heterocyclische bzw. heteroaromatische Ringe.
Im Falle von gegebenenfalls substituiertem Heterocyclyl werden auch mehrcyclische Systeme umfasst, wie beispielsweise 8-Aza-bicyclo[3.2.1]octanyl oder 1-Aza-bicyclo[2.2.1]heptyl.
Im Falle von gegebenenfalls substituiertem Heterocyclyl werden auch spirocyclische Systeme umfasst, wie beispielsweise 1-Oxa-5-aza-spiro[2.3]hexyl.
Vorzugsweise ist er ein Rest eines heteroaromatischen Rings mit einem Heteroatom aus der Gruppe N, O und S, beispielsweise der Rest eines Fünf- oder Sechsrings, wie Pyridyl, Pyrrolyl, Thienyl oder Furyl;
weiterhin bevorzugt ist er ein Rest eines entsprechenden heteroaromatischen Rings mit 2, 3 oder 4 Heteroatomen, z. B. Pyrimidinyl, Pyridazinyl, Pyrazinyl, Triazinyl, Tetrazinyl, Thiazolyl, Thiadiazolyl, Oxazolyl, Isoxazolyl, Pyrazolyl, Imidazolyl oder Triazolyl oder Tetrazolyl.
Bevorzugt ist er dabei ein Rest eines heteroaromatischen Fünf- oder Sechsrings mit 1 bis 4 Heteroatomen, wie z. B. 1,2,3-Triazolyl, 1,2,4-Triazolyl, Tetrazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Tetrazolyl, 1,2,3-Triazinyl, 1,2,4-Triazinyl, 1,3,5-Triazinyl, 1,2,3,4-Tetrazinyl, 1,2,3,5-Tetrazinyl, 1,2,4,5-Tetrazinyl, Thiazolyl, Isothiazolyl, Oxazolyl, Isoxazolyl, Pyrazolyl, Imidazolyl.
Weiter bevorzugt sind dabei heteroaromatische Reste von Fünfring-Heterocyclen mit 3 N-Atomen, wie 1,2,3-Triazol-1-yl, 1,2,3-Triazol-4-yl, 1,2,3-Triazol-5-yl, 1,2,5-Triazol-1-yl, 1,2,5-Triazol-3-yl, 1,3,4-Triazol-1-yl, 1,3,4-Triazol-2-yl, 1,2,4-Triazol-3-yl, 1,2,4-Triazol-5-yl;
weiter bevorzugt sind auch heteroaromatische Reste von Sechsring-Heterocyclen mit 3 N-Atomen, wie 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl;
weiter bevorzugt sind auch heteroaromatische Reste von Fünfring-Heterocyclen mit zwei N-Atomen und einem O-Atom, wie 1,2,4-Oxadiazol-3-yl; 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, weiter bevorzugt sind auch heteroaromatische Reste von Fünfring-Heterocyclen mit zwei N-Atomen und einem S-Atom, wie 1,2,4-Thiadiazol-3-yl, 1,2,4-Thiadiazol-5-yl, 1,3,4-Thiadiazol-2-yl, 1,2,3-Thiadiazol-4-yl, 1,2,3-Thiadiazol-5-yl, 1,2,5-Thiadiazol-3-yl; weiter bevorzugt sind auch heteroaromatische Reste von Fünfring-Heterocyclen mit vier N-Atomen, wie 1,2,3,4-Tetrazol-1-yl, 1,2,3,4-Tetrazol-5-yl, 1,2,3,5-Tetrazol-1-yl, 1,2,3,5-Tetrazol-4-yl, 2*H*-1,2,3,4-Tetrazol-5-yl, 1*H*-1,2,3,4-Tetrazol-5-yl,
weiter bevorzugt sind auch heteroaromatische Reste von Sechsring-Heterocyclen, wie 1,2,4,5-Tetrazin-3-yl;
weiter bevorzugt sind auch heteroaromatische Reste von Fünfring-Heterocyclen mit drei N-Atomen und einem O- oder S-Atom, wie 1,2,3,4-Oxatriazol-5-yl; 1,2,3,5-Oxatriazol-4-yl; 1,2,3,4-Thiatriazol-5-yl;1,2,3,5-Thiatriazol-4-yl;
weiter bevorzugt sind auch heteroaromatische Reste von Sechsring-Heterocyclen, wie beispielsweise 1,2,4,6-Thiatriazin-1-yl; 1,2,4,6-Thiatriazin-3-yl; 1,2,4,6-Thiatriazin-5-yl. Weiterhin bevorzugt ist der heterocyclische Rest oder Ring ein partiell oder vollständig hydrierter heterocyclischer Rest mit einem Heteroatom aus der Gruppe N, O und S, beispielsweise Oxiranyl, Oxetanyl, Oxolanyl (= Tetrahydrofuryl), Oxanyl, Pyrrolinyl, Pyrrolidyl oder Piperidyl,
Weiterhin bevorzugt ist er auch ein partiell oder vollständig hydrierter heterocyclischer Rest mit 2 Heteroatomen aus der Gruppe N, O und S, beispielsweise Piperazinyl, Dioxolanyl, Oxazolinyl, Isoxazolinyl, Oxazolidinyl, Isoxazolidinyl und Morpholinyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.
Bevorzugte Beispiele für Heterocyclyl sind ein heterocyclischer Rest mit 3 bis 6 Ringatomen aus der Gruppe Pyridyl, Thienyl, Furyl, Pyrrolyl, Oxiranyl, 2-Oxetanyl, 3-Oxetanyl, Oxolanyl (= Tetrahydrofuryl), Pyrrolidyl, Piperidyl, insbesondere Oxiranyl, 2-Oxetanyl, 3-Oxetanyl oder Oxolanyl, oder ist ein heterocyclischer Rest mit zwei oder drei Heteroatomen, beispielsweise Pyrimidinyl, Pyridazinyl, Pyrazinyl, Triazinyl, Thiazolyl, Thiadiazolyl, Oxazolyl, Isoxazolyl, Pyrazolyl, Triazolyl, Piperazinyl, Dioxolanyl, Oxazolinyl, Isoxazolinyl, Oxazolidinyl, Isoxazolidinyl oder Morpholinyl. Bevorzugte heterocyclische Reste sind auch benzokondensierte oder benzoannellierte heteroaromatische Ringe, beispielsweise Benzofuryl, Benzisofuryl, Benzothiophenyl, Benzisothiophenyl, Isobenzothiophenyl, Indolyl, Isoindolyl, Indazolyl, Benzimidazolyl, Benztriazolyl, Benzoxazolyl, 1,2-Benzisoxazolyl, 2,1-Benzisoxazolyl, Benzothiazolyl, 1,2-Benzisothiazolyl, 2,1-Benzoisothiazolyl, 1,2,3-Benzoxadiazolyl, 2,1,3-Benzoxadiazolyl, 1,2,3-Benzothiadiazolyl, 2,1,3-Benzothiadiazolyl, Chinolyl (Chinolinyl), Isochinolyl (Isochinolinyl), Chinnolinyl, Phtalazinyl, Chinazolinyl, Chinoxalinyl, Naphthyridinyl, Benzotriazinyl, Purinyl, Pteridinyl, Indolizinyl, Benzo-1,3-dioxylyl, 4H-Benzo-1,3-dioxinyl, und 4H-Benzo-1,4-dioxinyl, und wo es möglich ist, N-Oxide und Salze davon.
Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.
Substituierte Reste, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Alkylsulfonyl und, im Falle cyclischer Reste, auch Alkyl, Haloalkyl, Alkylthio-alkyl, Alkoxy-alkyl, gegebenfalls substituiertes Mono- und Dialkyl-aminoalkyl und Hydroxyalkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfasst, die mit einer Doppelbindung am Ring gebunden sind, z. B. mit einer Alkylidengruppe wie Methyliden oder Ethyliden substituiert sind.

Der Ausdruck "Reste aus der Gruppe (gefolgt von der Gruppe = Liste der Substituenten)", wo immer verwendet, soll gleichbedeutend sein mit "Reste ausgewählt aus der Gruppe (...)" oder "Reste ausgewählt aus der Gruppe bestehend aus (...)". Der Ausdruck "ein oder mehrere Reste aus der Gruppe (gefolgt von der Gruppe = Liste der Substituenten)", wo immer verwendet, soll gleichbedeutend sein mit "ein oder mehrere gleiche oder verschiedene Reste ausgewählt aus der Gruppe (...)" oder "Reste ausgewählt aus der Gruppe bestehend aus (...)".

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.
Mit "Basisrest" wird der jeweilige Grundkörper eines Restes bezeichnet, an dem Substituenten einer Substituentenebene gebunden sind.
Bevorzugte Substituenten für die Substituentenebenen sind beispielsweise Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfinyl, Alkylsulfonyl, Monoalkyl-aminosulfonyl, Dialkyl-aminosulfonyl, N-Alkyl-aminocarbonyl, N,N-Dialkylaminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino und Benzylamino.
Zwei Substituenten können auch gemeinsam eine gesättigte oder ungesättigte Kohlenwasserstoff-Brücke oder eine entsprechende Brücke, in denen C-Atome, CH-Gruppen oder CH₂-Gruppen durch Heteroatome ersetzt sind, bilden und damit einen ankondensierten oder annellierten Cyclus bilden. Bevorzugt werden dabei benzokondensierte Systeme auf Basis der Grundkörper gebildet.

Gegebenenfalls substituiertes Phenyl bedeutet vorzugsweise Phenyl oder Phenyl, das unsubstituiert oder durch einen oder mehrere Reste aus der Gruppe Halogen, Cyano, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkoxy-(C₁-C₄)alkoxy, (C₁-C₄)Alkylthio und Nitro substituiert ist, insbesondere Phenyl, das gegebenenfalls durch einen oder mehrere Reste aus der Gruppe Halogen, (C₁-C₄)Alkyl, (C₁-C₄)Haloalkyl und (C₁-C₄)Alkoxy substituiert ist.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind der Rest -CO-R einer Carbonsäure HO-CO-R und Reste davon abgeleiteter Säuren wie der Thiocarbonsäure, gegebenenfalls N-substituierten Iminocarbonsäuren oder der Rest von Kohlensäuremonoestern, N-substituierter Carbaminsäure, Sulfonsäuren, Sulfinsäuren, N-substituierter Sulfonamidsäuren, Phosphonsäuren oder Phosphinsäuren.
Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch einen oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.

Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Formyl, Alkylcarbonyl wie Acetyl oder [(C₁-C₄)Alkyl]-carbonyl, Phenylcarbonyl, Alkylsulfonyl, Alkylsulfinyl und andere Reste von organischen Säuren.
Weiter bevorzugt bedeutet Acyl einen Alkanoylrest mit 1 bis 6 C-Atomen, insbesondere 1 bis 4 C-Atomen. (C₁-C₄)Alkanoyl bedeutet dabei den Rest einer Alkansäure mit 1 bis 4 C-Atomen nach Abtrennen der OH-Gruppe der Säuregruppe, d.h. Formyl, Acetyl, n-Propionyl, i-Propionyl oder n-, i-, sec. oder tert.-Butanoyl.
Die "yl-Position" eines Restes bezeichnet das C-Atom mit der freien Bindung. Erfindungsgemäße bzw. erfindungsgemäß verwendete Verbindungen der Formel (I) und/oder deren Salze werden abgekürzt auch als "Verbindungen (I)" bezeichnet. Gegenstand der Erfindung sind auch alle Stereoisomeren, die von Formel (I) umfasst sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfasst und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Gegenstand der Erfindung sind auch alle Tautomeren der Verbindungen der Formel (I), die durch Verschiebung eines Wasserstoffatoms entstehen können (z. B. Keto-Enol-Tautomere). Die Tautomeren sind ebenfalls von der Verbindung der Formel (I) umfasst, auch wenn die Formel (I) nur eines der jeweiligen im Gleichgewicht stehenden bzw. ineinander umwandelbaren Tautomeren formal richtig beschreibt. Die Verbindungen der Formel (I) umfassen auch alle physikalischen Formen, in denen diese in Reinsubstanz oder gegebenenfalls in Mischung mit anderen Stoffen auftreten können, insbesondere auch polymorphe Kristallformen der Verbindungen der Formel (I) oder deren Salze oder Lösungsmitteladditionsverbindungen (z. B. Hydrate).

Vor allem aus den Gründen der höheren herbiziden Wirkung, besseren Selektivität, besseren Herstellbarkeit, besseren Formulierbarkeit und/oder anderer relevanter Eigenschaften, wie sind erfindungsgemäße Verbindungen der genannten Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.
Unabhängig von den jeweils anderen Resten gemäß den Symbolen (R¹)ₘ, (R²)ₙ, R³, R⁴ und den Definitionen für m und n in Formel (I) sowie den Definitionen für die Reste (oder chemische Gruppen) gemäß den Symbolen R⁵ bis R¹⁹, Het¹ bis Het⁷, M, R^{*} und R**, R^{A}, R^{B}, R^{aa}, R^{bb} und R^{cc} in den entsprechenden Unterbedeutungen zu Resten in der Formel (I) sind erfindungsgemäße Verbindungen der Formel (I) bzw. deren erfindungsgemäßen Verwendungen mit nachfolgend aufgeführten bevorzugten Bedeutungen der betreffenden Symbole bzw. chemischen Reste oder chemischen Gruppen von besonderem Interesse.

Bevorzugt sind Verbindungen (I), worin
(R¹)ₘ m Substituenten R¹ bedeutet,
wobei R¹, wenn m = 1, oder jeder der Substituenten R¹, wenn m größer als 1, unabhängig voneinander Halogen, Cyano, Nitro, Hydroxy, (C₁-C₆)Alkyl, (C₂-C₄)Alkenyl, (C₂-C₄)Alkinyl, (C₁-C₆)Alkoxy, (C₁-C₆)Alkylthio, (C₁-C₆)Alkylsulfinyl, (C₁-C₆)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Haloalkenyl, (C₂-C₄)Haloalkinyl, (C₁-C₄)Alkoxy-(C₁-C₄)alkyl, (C₁-C₄)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR⁵, C(O)NR⁶R⁷, C(O)-Het¹, NR⁸R⁹ oder Het² bedeutet oder wobei jeweils zwei am Ring ortho-ständige Gruppen R¹ oder R¹ und R³ gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht,
wobei die Gruppe -Z¹-A*-Z² zusammen mit den an die Gruppe gebundenen C-Atomen des Phenylrings einen ankondensierten 5 oder 6 Ring bilden,
R⁵ Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die genannte Gruppe M bedeutet, vorzugsweise Wasserstoff, (C₁-C₄)Alkyl oder die genannte Gruppe M bedeutet,
R⁶, R⁷, R⁸, R⁹, Het¹ und Het², die genannten Bedeutungen haben, vorzugsweise
R⁶, R⁷, R⁸ und R⁹ jeweils unabhängig voneinander Wasserstoff oder (C₁-C₄)Alkyl, das unsubstituiert oder durch einen oder mehrere Reste aus der Gruppe Halogen, Nitro, Cyano und Phenyl substituiert ist, oder
(C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, (C₁-C₄)Alkyl, (C₁-C₄)Haloalkyl, Phenyl und Benzyl substituiert ist, insbesondere Wasserstoff, (C₁-C₄)Alkyl oder (C₁-C₄)Haloalkyl bedeuten,
Het¹ und Het² jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, und Oxo substituiert ist, vorzugsweise den Rest eines gesättigten Heterocyclus der genannten Art, insbesondere eine Morpholino- Piperidino- oder Pyrrolidinogruppe bedeuten und
m 0, 1, 2 oder 3, vorzugsweise 0, 1 oder 2, insbesondere 0 oder 1 bedeutet.

Weiter bevorzugt sind Verbindungen (I), worin
(R¹)ₘ m Substituenten R¹ bedeutet,
wobei R¹, wenn m = 1, oder jeder der Substituenten R¹, wenn m größer als 1, unabhängig voneinander Halogen, Cyano, Nitro, Hydroxy, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₁-C₄)Alkoxy-(C₁-C₄)alkyl, (C₃-C₆)Cycloalkyl, oder einen Rest der Formel C(O)OR⁵, C(O)NR⁶R⁷, C(O)-Het¹, NR⁸R⁹ oder Het² bedeutet,
oder wobei jeweils zwei am Ring ortho-ständige Gruppen R¹ oder R¹ und R³ gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht,
wobei die Gruppe -Z¹-A*-Z² zusammen mit den an die Gruppe gebundenen C-Atomen des Phenylrings einen ankondensierten 5 oder 6 Ring bilden,
R⁵ Wasserstoff, (C₁-C₄)Alkyl oder die genannte Gruppe M bedeutet,
R⁶, R⁷, R⁸ und R⁹ jeweils unabhängig voneinander Wasserstoff, (C₁-C₄)Alkyl, (C₁-C₄)Haloalkyl, Benzyl, (C₃-C₆)Cycloalkyl oder Phenyl bedeuten, insbesondere Wasserstoff, Methyl oder Ethyl bedeuten,
Het¹ und Het² jeweils unabhängig voneinandereine Morpholino- Piperidino- oder Pyrrolidinogruppe bedeuten und
m 0, 1, 2 oder 3, vorzugsweise 0, 1 oder 2, insbesondere 0 oder 1 bedeutet.

Weiter bevorzugt sind dabei Verbindungen (I), worin
(R¹), m Substituenten R¹ bedeutet,
wobei R¹, wenn m = 1, oder jeder der Substituenten R¹, wenn m größer als 1 ist, unabhängig voneinander Halogen, Cyano, Nitro, Methyl, Ethyl, Methoxy, Ethoxy, Methylthio, Ethylthio, (C₁-C₂)Alkylsulfinyl, (C₁-C₂)Alkylsulfonyl, (C₁-C₂)Haloalkyl, (C₁-C₂)Haloalkoxy, (C₁-C₂)Haloalkylthio, (C₁-C₂)Haloalkylsulfinyl, (C₁-C₂)Haloalkylsulfonyl oder (C₁-C₂)Alkoxy-(C₁-C₂)alkyl bedeutet,
insbesondere jeder der Substituenten R¹ unabhängig voneinander Halogen, wie Fluor, Chlor, Brom oder Iod, oder Cyano, Nitro, Methyl, Methoxy, Methylthio, Methylsulfinyl, Methylsulfonyl, Trifluormethyl, Trifluormethoxy, Difluormethoxy, Trifluoralkylthio, Trifluormethylsulfinyl oder Trifluormethylsulfonyl bedeutet, insbesondere Cyano oder Halogen wie Fluor, Chlor oder Brom bedeutet, und
m 0, 1, 2 oder 3, vorzugsweise 0, 1 oder 2, insbesondere 0 oder 1 bedeutet.

Weiter bevorzugt sind Verbindungen der Formel (I) oder deren Salze, worin
m 0 (= die Zahl Null, d. h. keine Substituenten R¹ vorhanden, d. h. betreffenden freien Bindungen am Ring sind mit Wasserstoff besetzt) bedeutet oder vorzugsweise
(R¹)ₘ 2-Brom, 4-Brom, 6-Brom, 2-Chlor, 4-Chlor, 6-Chlor, 2-Fluor, 4-Fluor, 6-Fluor, 2-Cyano, 4-Cyano, 6-Cyano, 2-Methyl, 4-Methyl, 6-Methyl, 2-Ethyl, 4-Ethyl, 6-Ethyl, 2-CF₃, 4-CF₃, 6-CF₃, 2-Methoxy, 4-Methoxy, 6-Methoxy, 2-Ethoxy, 4-Ethoxy, 6-Ethoxy, 2-Trifluormethoxy, 4-Trifluormethoxy, 6-Trifluormethoxy, 2-Difluormethoxy, 4-Difluormethoxy, 6-Difluormethoxy, 2-Methylthio, 4-Methylthio, 6-Methylthio, 2-Methylsulfinyl, 4-Methylsulfinyl, 6-Methylsulfonyl, 2-Methylsulfonyl, 4-Methylsulfonyl, 6-Methylsulfonyl, 2-Nitro, 4-Nitro, 6-Nitro, 2,4-Dimethyl, 2,6-Dimethyl, 4,6-Dimethyl, 2,4-Difluor, 2,6-Difluor, 4,6-Difluor, 2,4-Dichlor, 2,6-Dichlor, 4,6-Dichlor, (2-CI-4-F), (2-CI-6-F), (4-CI-6-F), (2-F-4-Cl), (2-F-6-CI), (4-F-6-Cl), 2,4,6-Trifluor oder 2,4,6-Trichlor bedeutet, wobei die Bezifferung der Reste sich auf die Position des Restes am Pyridin-3-yl-Rest bezieht, in dem das N-Atom die Position 1 hat und das C-Atom, das am Buttersäure-Grundkörper gebunden ist, die 3-Position im Ring hat.

Bevorzugt sind auch Verbindungen (I), worin
(R²)ₙ n Substituenten R² bedeutet,
wobei R², wenn n = 1, oder jeder der Substituenten R², wenn n größer als 1 ist, unabhängig voneinander Halogen, Cyano, Nitro, Hydroxy, (C₁-C₆)Alkyl, (C₂-C₄)Alkenyl, (C₂-C₄)Alkinyl, (C₁-C₆)Alkoxy, (C₁-C₆)Alkylthio, (C₁-C₆)Alkylsulfinyl, (C₁-C₆)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Haloalkenyl, (C₂-C₄)Haloalkinyl, (C₁-C₄)Alkoxy-(C₁-C₄)alkyl, (C₁-C₄)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR¹⁰, C(O)NR¹¹R¹², C(O)-Het³, NR¹³R¹⁴ oder Het⁴ bedeutet oder wobei jeweils zwei am Ring ortho-ständige Gruppen R² gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴ zusammen mit den an die Gruppe gebundenen C-Atomen des Phenylrings einen ankondensierten 5 oder 6 Ring bilden,
R¹⁰ Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die genannte Gruppe M bedeutet,
R¹¹, R¹², R¹³, R¹⁴, Het³ und Het⁴, die genannten Bedeutungen haben, vorzugsweise
R¹¹, R¹², R¹³ und R¹⁴ jeweils unabhängig voneinander Wasserstoff oder (C₁-C₄)Alkyl, das unsubstituiert oder durch einen oder mehrere Reste aus der Gruppe Halogen, Nitro, Cyano und Phenyl substituiert ist, oder
(C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, (C₁-C₄)Alkyl, (C₁-C₄)Haloalkyl, Phenyl und Benzyl substituiert ist, bedeuten,
Het³ und Het⁴ jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl und Oxo substituiert ist, vorzugsweise den Rest eines gesättigten Heterocyclus der genannten Art, insbesondere eine Morpholino- Piperidino- oder Pyrrolidinogruppe bedeuten und
n 0, 1, 2, 3, 4 oder 5, vorzugsweise 0, 1, 2 oder 3 bedeutet.

Weiter bevorzugt sind dabei Verbindungen (I), worin
(R²)ₙ n Substituenten R² bedeutet,
wobei R², wenn n = 1, oder jeder der Substituenten R², wenn n größer als 1 ist, unabhängig voneinander Halogen, Cyano, Nitro, Hydroxy, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₁-C₄)Alkoxy-(C₁-C₄)alkyl, (C₃-C₆)Cycloalkyl oder einen Rest der Formel C(O)OR¹⁰, C(O)NR¹¹R¹², C(O)-Het³, NR¹³R¹⁴ oder Het⁴ bedeutet
oder wobei jeweils zwei am Ring ortho-ständige Gruppen R² gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴ zusammen mit den an die Gruppe gebundenen C-Atomen des Phenylrings einen ankondensierten 5 oder 6 Ring bilden,
R¹⁰ Wasserstoff, (C₁-C₄)Alkyl oder die genannte Gruppe M bedeutet,
R¹¹, R¹², R¹³ und R¹⁴, Het³ und Het⁴, die genannten Bedeutungen haben, vorzugsweise
R¹¹, R¹² , R¹³ und R¹⁴ jeweils unabhängig voneinander Wasserstoff oder (C₁-C₄)Alkyl, (C₁-C₄)Haloalkyl, Benzyl, (C₃-C₆)Cycloalkyl oder Phenyl bedeuten,
Het³ und Het⁴ jeweils unabhängig voneinander eine Morpholino- Piperidino- oder Pyrrolidinogruppe bedeuten und
n 0, 1, 2, 3, 4 oder 5, vorzugsweise 0, 1, 2, 3 oder 4, insbesondere 0, 1, 2 oder 3 bedeutet.

Weiter bevorzugt sind dabei Verbindungen (I), worin
(R²)ₙ n Substituenten R² bedeutet,
wobei R², wenn n = 1, oder jeder der Substituenten R², wenn n größer als 1 ist, unabhängig voneinander Halogen, Cyano, Nitro, Methyl, Ethyl, Methoxy, Ethoxy, Methylthio, Ethylthio, (C₁-C₂)Alkylsulfinyl, (C₁-C₂)Alkylsulfonyl, (C₁-C₂)Haloalkyl, (C₁-C₂)Haloalkoxy, (C₁-C₂)Haloalkylthio, (C₁-C₂)Haloalkylsulfinyl, (C₁-C₂)Haloalkylsulfonyl oder (C₁-C₂)Alkoxy-(C₁-C₂)alkyl bedeutet,
insbesondere jeder der Substituenten R² unabhängig voneinander Halogen, wie Fluor, Chlor, Brom oder Iod, oder Cyano, Nitro, Methyl, Methoxy, Methylthio, Methylsulfinyl, Methylsulfonyl, Trifluormethyl, Trifluormethoxy, Difluormethoxy, Trifluoralkylthio, Trifluormethylsulfinyl oder Trifluormethylsulfonyl bedeutet, insbesondere Cyano, Nitro oder Halogen wie Fluor, Chlor oder Brom bedeutet, und
n 0, 1, 2, 3, 4 oder 5, vorzugsweise 0, 1, 2, 3 oder 4, insbesondere 0, 1, 2 oder 3 bedeutet.

Weiter bevorzugt sind Verbindungen der Formel (I) oder deren Salze, worin
n 0 (= die Zahl Null, d. h. keine Substituenten R² vorhanden, d. h. alle freien Bindungen am Phenylring sind mit Wasserstoff besetzt) bedeutet oder vorzugsweise
(R²)ₙ 2-Brom, 3-Brom, 4-Brom, 2-Chlor, 3-Chlor, 4-Chlor, 2-Fluor, 3-Fluor, 4-Fluor, 2-Cyano, 3-Cyano, 4-Cyano, 2-Methyl, 3-Methyl, 4-Methyl, 2-Ethyl, 3-Ethyl, 4-Ethyl, 2-CF₃, 3-CF₃, 4-CF₃, 2-Methoxy, 3-Methoxy, 4-Methoxy, 2-Ethoxy, 3-Ethoxy, 4-Ethoxy, 2-Trifluormethoxy, 3-Trifluormethoxy, 4-Trifluormethoxy, 2-Difluormethoxy, 3-Difluormethoxy, 4-Difluormethoxy, 2-Methylthio, 3-Methylthio, 4-Methylthio, 2-Methylsulfinyl, 3-Methylsulfinyl, 4-Methylsulfonyl, 2-Methylsulfonyl, 3-Methylsulfonyl, 4-Methylsulfonyl, 2-Nitro, 3-Nitro, 4-Nitro, 2,3-Dimethyl, 2,4-Dimethyl, 2,5-Dimethyl, 2,6-Dimethyl, 3,4-Dimethyl, 3,5-Dimethyl, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, (2-CI-3-F), (2-CI-4-F), (2-CI-5-F), (2-CI-6-F), (3-CI-2-F), (3-CI-4-F), (3-CI-5-F), (3-CI-6-F), (4-CI-2-F), (4-CI-3-F), (4-Br-2-F), (4-Br-3-F), (4-CN-3-F), (4-NO₂-3-F), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor, 3,4,5-Trichlor oder auch (2,6-Difluor-4-Cl), 2,5-Dicyano, 2,6-Dicyano, (4-Methoxy-3-F), (3-CN-4-F), (3-Nitro-4-F), (3-CN-4-Cl), (3-Nitro-4-Cl) oder (5-CN-2-F) bedeutet, wobei die Bezifferung der Reste sich auf die Position des Restes am Phenyl-1-yl-Rest bezieht, in dem das C-Atom, das in 3-Position am Buttersäure-Grundkörper gebunden ist, die 1-Position im Ring hat.

Weiter bevorzugt sind Verbindungen der Formel (I) oder deren Salze, worin
(R²)ₙ 2-Cyano, 3-Cyano, 4-Cyano, 2-Brom, 3-Brom, 4-Brom, 2-Chlor, 3-Chlor, 4-Chlor, 2-Fluor, 3-Fluor, 4-Fluor, 2-Nitro, 3-Nitro, 4-Nitro, 2-Methoxy, 3-Methoxy, 4-Methoxy, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, (2-CI-3-F), (2-CI-4-F), (2-CI-5-F), (2-CI-6-F), (3-CI-2-F), (3-CI-4-F), (3-CI-5-F), (3-CI-6-F), (4-CI-2-F), (4-CI-3-F), (4-Br-2-F), (4-Br-3-F), (4-CN-3-F), (4-NO₂-3-F), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor, 3,4,5-Trichlor oder auch (2,6-Difluor-4-Cl), 2,5-Dicyano, 2,6-Dicyano, (4-Methoxy-3-F), (3-CN-4-F), (3-Nitro-4-F), (3-CN-4-Cl), (3-Nitro-4-Cl) oder (5-CN-2-F) bedeutet.

Weiter bevorzugt sind dabei auch Verbindungen der Formel (I) oder deren Salze, worin
(R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, 3,4-Dichlor, 3,5-Dichlor, (3-CI-2-F), (3-CI-4-F), (3-CI-5-F), (3-CI-6-F), (4-CI-2-F), (4-CI-3-F) oder auch (2,6-Difluor-4-Cl), bedeutet.
Dabei sind insbesondere bevorzugt:
Verbindungen der Formel (I) oder deren Salze, worin (R²)ₙ 3-Chlor bedeutet.
Verbindungen der Formel (I) oder deren Salze, worin (R²)ₙ 4-Chlor bedeutet.
Verbindungen der Formel (I) oder deren Salze, worin (R²)ₙ 2-Fluor bedeutet.
Verbindungen der Formel (I) oder deren Salze, worin (R²)ₙ 3-Fluor bedeutet.
Verbindungen der Formel (I) oder deren Salze, worin (R²)ₙ 4-Fluor bedeutet.
Verbindungen der Formel (I) oder deren Salze, worin (R²)ₙ 2,3-Difluor bedeutet.
Verbindungen der Formel (I) oder deren Salze, worin (R²)ₙ 2,4-Difluor bedeutet.
Verbindungen der Formel (I) oder deren Salze, worin (R²)ₙ 2,5-Difluor bedeutet.
Verbindungen der Formel (I) oder deren Salze, worin (R²)ₙ 2,6-Difluor bedeutet.
Verbindungen der Formel (I) oder deren Salze, worin (R²)ₙ 3,4-Difluor bedeutet.
Verbindungen der Formel (I) oder deren Salze, worin (R²)ₙ 3,5-Difluor bedeutet.
Verbindungen der Formel (I) oder deren Salze, worin (R²)ₙ (3-CI-2-F) bedeutet.
Verbindungen der Formel (I) oder deren Salze, worin (R²)ₙ (3-CI-4-F) bedeutet.
Verbindungen der Formel (I) oder deren Salze, worin (R²)ₙ (3-CI-5-F) bedeutet.
Verbindungen der Formel (I) oder deren Salze, worin (R²)ₙ (3-CI-6-F) bedeutet.
Verbindungen der Formel (I) oder deren Salze, worin (R²)ₙ (4-CI-2-F) bedeutet.
Verbindungen der Formel (I) oder deren Salze, worin (R²)ₙ (4-CI-3-F) bedeutet.
Verbindungen der Formel (I) oder deren Salze, worin (R²)ₙ (2,6-Difluor-4-Cl) bedeutet.

Bevorzugt sind auch Verbindungen (I), worin
R³ Halogen, Cyano, Nitro, Hydroxy, (C₁-C₆)Alkyl, (C₂-C₄)Alkenyl, (C₂-C₄)Alkinyl, (C₁-C₆)Alkoxy, (C₁-C₆)Alkylthio, (C₁-C₆)Alkylsulfinyl, (C₁-C₆)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Haloalkenyl, (C₂-C₄)Haloalkinyl, (C₁-C₄)Alkoxy-(C₁-C₄)alkyl, (C₁-C₄)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR¹⁵, C(O)NR¹⁶R¹⁷, C(O)-Het⁵, NR¹⁸R¹⁹ oder Het⁶ bedeutet,
R¹⁵ Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die genannte Gruppe M bedeutet, vorzugsweise Wasserstoff, (C₁-C₄)Alkyl oder die genannte Gruppe M bedeutet,
R¹⁶, R¹⁷, R¹⁸, R¹⁹, Het⁵ und Het⁶, die genannten Bedeutungen haben, vorzugsweise
R¹⁶, R¹⁷, R¹⁸ und R¹⁹ jeweils unabhängig voneinander Wasserstoff oder (C₁-C₄)Alkyl, das unsubstituiert oder durch einen oder mehrere Reste aus der Gruppe Halogen, Nitro, Cyano und Phenyl substituiert ist, oder (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, (C₁-C₄)Alkyl, (C₁-C₄)Haloalkyl, Phenyl und Benzyl substituiert ist, insbesondere Wasserstoff, (C₁-C₄)Alkyl oder (C₁-C₄)Haloalkyl bedeuten,
Het⁵ und Het⁶ jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, und Oxo substituiert ist, vorzugsweise den Rest eines gesättigten Heterocyclus der genannten Art, insbesondere eine Morpholino- Piperidino- oder Pyrrolidinogruppe bedeuten.

Weiter bevorzugt sind Verbindungen (I), worin
R³ Halogen, Cyano, Nitro, Hydroxy, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C4)Haloalkylthio, (C₁-C₄)Haloalkylsulfinyl, (C₁-C₄)Haloalkylsulfonyl, (C₁-C₄)Alkoxy-(C₁-C₄)alkyl, (C₃-C₆)Cycloalkyl oder einen Rest der Formel C(O)OR¹⁵, C(O)NR¹⁶R¹⁷, C(O)-Het⁵, NR¹⁸R¹⁹ oder Het⁶ bedeutet,
R¹⁵ Wasserstoff, (C₁-C₄)Alkyl oder die genannte Gruppe M bedeutet,
R¹⁶, R¹⁷, R¹⁸ und R¹⁹ jeweils unabhängig voneinander Wasserstoff, (C₁-C₄)Alkyl, (C₁-C₄)Haloalkyl, Benzyl, (C₃-C₆)Cycloalkyl oder Phenyl bedeuten, insbesondere Wasserstoff, Methyl oder Ethyl bedeuten,
Het⁵ und Het⁶ jeweils unabhängig voneinandereine Morpholino- Piperidino- oder Pyrrolidinogruppe bedeuten.

Weiter bevorzugt sind dabei Verbindungen (I), worin
R³ Halogen, Cyano, Nitro, Methyl, Ethyl, Methoxy, Ethoxy, Methylthio, Ethylthio, (C₁-C₂)Alkylsulfinyl, (C₁-C₂)Alkylsulfonyl, (C₁-C₂)Haloalkyl, (C₁-C₂)Haloalkoxy, (C₁-C₂)Haloalkylthio, (C₁-C₂)Haloalkylsulfinyl, (C₁-C₂)Haloalkylsulfonyl, (C₁-C₂)Alkoxy-(C₁-C₂)alkyl oder [(C₁-C₄)Alkoxy]-carbonyl bedeutet, insbesondere R³ Halogen, wie Fluor, Chlor, Brom oder lod, oder Cyano, Nitro, Methyl, Methoxy, Methylthio, Methylsulfinyl, Methylsulfonyl, Trifluormethyl, Trifluormethoxy, Difluormethoxy, Trifluoralkylthio, Trifluormethylsulfinyl, Trifluormethylsulfonyl, Methoxycarbonyl oder Ethoxycarbonyl bedeutet, insbesondere Halogen wie Fluor, Chlor oder Brom bedeutet, und
m 0, 1, 2 oder 3, vorzugsweise 0, 1 oder 2, insbesondere 0 oder 1 bedeutet.

Weiter bevorzugt sind Verbindungen der Formel (I) oder deren Salze, worin
R³ Brom, Chlor, Fluor, Cyano, Methyl, Nitro, Methoxycarbonyl oder Ethoxycarbonyl bedeutet.

Weiter bevorzugt sind Verbindungen der Formel (I) oder deren Salze, worin
(R¹)ₘ und R³ gemeinsam 5-Brom, 5-Chlor, 5-Fluor, 5-Cyano, 5-Methyl, 5-Ethyl, 5-CF₃, 5-Methoxy, 5-Ethoxy, 5-Trifluormethoxy, 5-Difluormethoxy, 5-Methylthio, 5-Methylsulfinyl, 5-Methylsulfonyl, 5-Nitro, 5-Methoxycarbonyl, 5-Ethoxycarbonyl, 2,5-Difluor, 4,5-Difluor, 5,6-Difluor, 2,5-Dichlor, 4,5-Dichlor, 5,6-Dichlor, 2,5-Dibrom, 4,5-Dibrom, 5,6-Dibrom, 2,5-Dimethyl, 4,5-Dimethyl, 5,6-Dimethyl, 2,5-Dicyano, 4,5-Dicyano, 5,6-Dicyano, (2-CI-5-F), (4-CI-5-F), (6-CI-5-F), (2-Br-5-F), (4-Br-5-F), (6-Br-5-F), (2-F-5-Cl), (4-F-5-Cl), (6-F-5-Cl), (2-F-5-Br), (4-F-5-Br), (6-F-5-Br), (2-Br-5-Cl), (4-Br-5-Cl), (6-Br-5-Cl), (2-CI-5-Br), (4-CI-5-Br), (6-CI-5-Br), (2-CN-5-F), (4-CN-5-F), (6-CN-5-F), (2-CH₃-5-F), (4-CH₃-5-F), (6-CH₃-5-F), (2-OCH₃-5-F), (4-OCH₃-5-F), (6-OCH₃-5-F), (2-OCHF₂-5-F), (4-OCHF₂-5-F), (6-OCHF₂-5-F), (2-CN-5-Cl), (4-CN-5-Cl), (6-CN-5-Cl), (2-CH₃-5-Cl), (4-CH₃-5-Cl), (6-CH₃-5-Cl), (2-OCH₃-5-Cl), (4-OCH₃-5-Cl), (6-OCH₃-5-Cl), (2-OCHF₂-5-Cl), (4-OCHF₂-5-Cl), (6-OCHF₂-5-Cl),
(2-CN-5-Br), (4-CN-5-Br), (6-CN-5-Br), (2-CH₃-5-Br), (4-CH₃-5-Br), (6-CH₃-5-Br), (2-OCH₃-5-Br), (4-OCH₃-5-Br), (6-OCH₃-5-Br), (2-OCHF₂-5-Br), (4-OCHF₂-5-Br), (6-OCHF₂-5-Br),
(2-CN-5-CH₃), (4-CN-5-CH₃), (6-CN-5-CH₃), (2-F-5-CH₃), (4-F-5-CH₃), (6-F-5-CH₃), (2-Cl-5-CH₃), (4-Cl-5-CH₃), (6-Cl-5-CH₃), (2-Br-5-CH₃), (4-Br-5-CH₃), (6-Br-5-CH₃), (2-OCH₃-5-CH₃), (4-OCH₃-5-CH₃), (6-OCH₃-5-CH₃), (2-OCHF₂-5-CH₃), (4-OCHF₂-5-CH₃), (6-OCHF₂-5-CH₃), (2-F-5-CN), (4-F-5-CN), (6-F-5-CN), (2-Cl-5-CN), (4-Cl-5-CN), (6-Cl-5-CN), (2-Br-5-CN), (4-Br-5-CN), (6-Br-5-CN), (2-CH₃-5-CN), (4-CH₃-5-CN), (6-CH₃-5-CN), (2-OCH₃-5-CN), (4-OCH₃-5-CN), (6-OCH₃-5-CN), (2-OCHF₂-5-CN), (4-OCHF₂-5-CN), (6-OCHF₂-5-CN), (2-F-5-NO₂), (4-F-5-NO₂), (6-F-5-NO₂), (2-Cl-5-NO₂), (4-Cl-5-NO₂), (6-Cl-5-NO₂), (2-Br-5-NO₂), (4-Br-5-NO₂), (6-Br-5-NO₂), (2-CN-5-NO₂), (4-CN-5-NO₂), (6-CN-5-NO₂), (2-CH₃-5-NO₂), (4-CH₃-5-NO₂), (6-CH₃-5-NO₂), (2-OCH₃-5-NO₂), (4-OCH₃-5-NO₂), (6-OCH₃-5-NO₂), (2-OCHF₂-5-NO₂), (4-OCHF₂-5-NO₂), (6-OCHF₂-5-NO₂), (2-F-5-CO₂CH₃), (4-F-5-CO₂CH₃), (6-F-5-CO₂CH₃), (2-CI-5-CO₂CH₃), (4-Cl-5-CO₂CH₃), (6-Cl-5-CO₂CH₃), (2-Br-5-CO₂CH₃), (4-Br-5-CO₂CH₃), (6-Br-5-CO₂CH₃), (2-CN-5-CO₂CH₃), (4-CN-5-CO₂CH₃), (6-CN-5-CO₂CH₃), (2-CH₃-5-CO₂CH₃), (4-CH₃-5-CO₂CH₃), (6-CH₃-5-CO₂CH₃), (2-OCH₃-5-CO₂CH₃), (4-OCH₃-5-CO₂CH₃), (6-OCH₃-5-CO₂CH₃), (2-OCHF₂-5-CO₂CH₃), (4-OCHF₂-5-CO₂CH₃) oder (6-OCHF₂-5-CO₂CH₃) bedeutet.

Weiter bevorzugt sind dabei Verbindungen der Formel (I) oder deren Salze, worin (R¹)ₘ und R³ gemeinsam 5-Brom, 5-Chlor, 5-Fluor, 5-Cyano, 5-Methyl, 5-Ethyl, 5-CF₃, 5-Methoxy, 5-Nitro, 5-Methoxycarbonyl, 5-Ethoxycarbonyl, 2,5-Difluor, 4,5-Difluor, 5,6-Difluor, 5,6-Dichlor, 5,6-Dibrom, 5,6-Dimethyl, 5,6-Dicyano, (6-CI-5-F), (6-Br-5-F), (2-F-5-Cl), (4-F-5-Cl), (6-F-5-Cl), (2-F-5-Br), (4-F-5-Br), (6-F-5-Br), (6-Br-5-Cl), (6-CI-5-Br), (6-CN-5-F), (6-CH₃-5-F), (6-OCH₃-5-F), (6-OCHF₂-5-F), (6-CN-5-Cl), (6-CH₃-5-Cl), (6-OCH₃-5-Cl), (6-OCHF₂-5-Cl), (6-CN-5-Br), (6-CH₃-5-Br), (6-OCH₃-5-Br), (6-OCHF₂-5-Br), (6-CN-5-CH₃), (2-F-5-CH₃), (4-F-5-CH₃), (6-F-5-CH₃), (6-Cl-5-CH₃), (6-Br-5-CH₃), (6-OCH₃-5-CH₃), (6-OCHF₂-5-CH₃), (2-F-5-CN), (4-F-5-CN), (6-F-5-CN), (6-Cl-5-CN), (6-Br-5-CN), (6-CH₃-5-CN), (6-OCH₃-5-CN), (6-OCHF₂-5-CN), (2-F-5-NO₂), (4-F-5-NO₂), (6-F-5-NO₂), (6-Cl-5-NO₂), (6-Br-5-NO₂), (6-CN-5-NO₂), (6-CH₃-5-NO₂), (6-OCH₃-5-NO₂), (6-OCHF₂-5-NO₂), (2-F-5-CO₂CH₃), (4-F-5-CO₂CH₃), (6-F-5-CO₂CH₃), (6-Cl-5-CO₂CH₃), (6-Br-5-CO₂CH₃), (6-CN-5-CO₂CH₃), (6-CH₃-5-CO₂CH₃), (6-OCH₃-5-CO₂CH₃) oder (6-OCHF₂-5-CO₂CH₃)
bedeutet.

Weiter bevorzugt sind:
Verbindungen der Formel (I) oder deren Salze, worin m = 0 und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-CI-2-F), (3-CI-4-F), (3-CI-5-F), (3-CI-6-F), (4-CI-2-F) oder (4-CI-3-F) oder auch (2,6-Difluor-4-Cl), 3-Cyano, 4-Cyano, 3-Nitro oder 4-Nitro und R³ Fluor, Chlor, Brom, Methyl, Cyano, Nitro oder Methoxycarbonyl bedeuten.
Verbindungen der Formel (I) oder deren Salze, worin (R¹)ₘ = 2-F, 2-Cl, 4-F, 4-Cl, 6-F, 6-Cl, 2-Br, 4-Br, 6-Br, 2-CN, 4-CN, 6-CN, 2-Methyl, 4-Methyl, 6-Methyl, 2-Ethyl, 4-Ethyl, 6-Ethyl, 2-Methoxy, 4-Methoxy, 6-Methoxy, 2-Ethoxy, 4-Ethoxy, 6-Ethoxy, 2-Difluormethoxy, 4-Difluormethoxy, 6-Difluormethoxy, 2-Trifluormethoxy, 4-Trifluormethoxy oder 6-Trifluormethoxy und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-CI-2-F), (3-CI-4-F), (3-CI-5-F), (3-CI-6-F), (4-CI-2-F) oder (4-CI-3-F) oder auch (2,6-Difluor-4-CI), 3-Cyano, 4-Cyano, 3-Nitro oder 4-Nitro und R³ Fluor, Chlor, Brom, Methyl, Cyano, Nitro, Methoxycarbonyl oder Ethoxycarbonyl bedeuten.
Verbindungen der Formel (I) oder deren Salze, worin (R¹)ₘ = 2-F, 2-Cl, 4-F, 4-Cl, 6-F, 6-Cl, 6-Br, 6-CN, 6-Methyl, 6-Methoxy oder 6-Difluormethoxy und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-CI-2-F), (3-CI-4-F), (3-CI-5-F), (3-CI-6-F), (4-CI-2-F) oder (4-CI-3-F) oder auch (2,6-Difluor-4-Cl), 3-Cyano, 4-Cyano, 3-Nitro oder 4-Nitro und R³ Fluor, Chlor, Brom, Methyl, Cyano, Nitro oder Methoxycarbonyl bedeuten.

Generell sind unter den Verbindungen mit den obengenannten Bedeutungen für einzelne Gruppen oder Kombinationen von Gruppen (R¹)ₘ, (R²)ₙ und R³ solche bevorzugt, in denen die übrigen Gruppen oder Kombinationen von Gruppen in den Verbindungen gemäß bevorzugt genannten Bedeutungen definiert sind.

Bevorzugt sind auch Verbindungen (I), worin
M ein Äquivalent eines Kations, vorzugsweise ein Metallionenäquivalent, ein Ammoniumion, dass gegebenenfalls durch 1 bis 4 gleiche oder verschiedene Reste aus der Reste aus der Gruppe (C₁-C₄)Alkyl, (C₃-C₆)Cycloalkyl, Phenyl, (C₃-C₆)CyCloalkyl-(C₁-C₄)alkyl und Phenyl(C₁-C₄)alkyl, insbesondere (C₁-C₄)Alkyl substituiert ist, oder ein tertiäres Sulfoniumion, das vorzugsweise durch 3 gleiche oder verschiedene Reste aus der Gruppe (C₁-C₄)Alkyl, (C₃-C₆)Cycloalkyl, Phenyl, (C₃-C₆)CyCloalkyl-(C₁-C₄)alkyl und Phenyl(C₁-C₄)alkyl, insbesondere (C₁-C₄)Alkyl substituiert ist, bedeutet.

Bevorzugt sind die erfindungsgemäßen Verbindungen der Formel (I), vorzugsweise der Formel (Ia), oder deren Salze, in welchen
R⁴ Wasserstoff, Alkyl, Alkenyl oder Alkinyl, wobei jeder der letztgenannten 3 Reste unsubstituiert oder substituiert ist und inklusive Substituenten bis zu 30 C-Atome, vorzugsweise bis 24 C-Atome, insbesondere bis 20 C-Atome aufweist, oder
Cycloalkyl, Cycloalkenyl, Cycloalkinyl oder Aryl, wobei jeder der letztgenannten 4 Reste unsubstituiert oder substituiert ist und inklusive Substituenten bis zu 30 C-Atome, vorzugsweise bis 24 C-Atome, insbesondere bis 20 C-Atome aufweist, oder
einen Heterocyclylrest mit 3 bis 9 Ringatomen, der 1 bis 4 Heteroatome aus der Gruppe N, O und S enthält und der unsubstituiert oder substituiert ist und der inklusive Substituenten 1 bis 30 C-Atome, vorzugsweise 1 bis 24 C-Atome, insbesondere 1 bis 20 C-Atome aufweist, bedeutet.

Weiter bevorzugt sind dabei auch Verbindungen (I), vorzugsweise der Formel (la), oder deren Salze, worin R⁴ Wasserstoff bedeutet.

Weiter bevorzugt sind dabei auch Verbindungen (I), vorzugsweise der Formel (la), oder deren Salze, worin
R⁴ H, (C₁-C₁₈)Alkyl, (C₂-C₁₈)Alkenyl oder (C₂-C₁₈)Alkinyl, wobei jeder der letztgenannten 3 Reste unsubstituiert oder substituiert ist und inklusive Substituenten bis zu 30 C-Atome, vorzugsweise bis 24 C-Atome, insbesondere bis 20 C-Atome aufweist, oder
(C₃-C₉)Cycloalkyl, (C₅-C₉)Cycloalkenyl, (C₅-C₉)Cycloalkinyl oder Phenyl, wobei jeder der letztgenannten 4 Reste unsubstituiert oder substituiert ist und inklusive Substituenten bis zu 30 C-Atome, vorzugsweise bis 24 C-Atome, insbesondere bis 20 C-Atome aufweist, bedeutet.

Weiter bevorzugt sind dabei auch Verbindungen (I), vorzugsweise der Formel (la), oder deren Salze, worin
R⁴ Wasserstoff, (C₁-C₁₈)Alkyl, (C₂-C₁₈)Alkenyl oder (C₂-C₁₈)Alkinyl,
wobei jeder der letztgenannten 3 Reste unsubstituiert oder durch einen oder mehrere Reste aus der Gruppe, die aus den Resten [Untergruppen (a)-(d)]
(a) Halogen, Cyano, Thio, Nitro, Hydroxy, Carboxy, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio, (C₁-C₈)Haloalkylthio, (C₂-C₈)Haloalkenylthio, (C₂-C₈)Haloalkinylthio, (C₁-C₈)Alkylsulfinyl, (C₂-C₈)Alkenylsulfinyl, (C₂-C₈)Alkinylsulfinyl, (C₁-C₈)Haloalkylsulfinyl, (C₂-C₈)Haloalkenylsulfinyl, (C₂-C₈)Haloalkinylsulfinyl, (C₁-C₈)Alkylsulfonyl, (C₂-C₈)Alkenylsulfonyl, (C₂-C₈)Alkinylsulfonyl, (C₁-C₈)Haloalkylsulfonyl, (C₂-C₈)Haloalkenylsulfonyl, (C₂-C₈)Haloalkinylsulfonyl, Reste der Formel -NR*R**, wobei R* und R** wie oben oder weiter unten definiert sind, und
   (C₃-C₈)Cycloalkyl, (C₅-C₈)CyCloalkenyl, (C₅-C₈)CyCloalkinyl, (C₃-C₈)Cycloalkyl-(C₁-C₆)alkoxy, (C3-C8)Cycloalkyl-(C₁-C₆)alkyl-S(O)ₚ-, (C₅-C₈)Cycloalkenyl-(C₁-C₆)alkoxy, (C₅-C₈)Cycloalkenyl-(C₁-C₆)alkyl-S(O)ₚ-, (C₅-C₈)Cycloalkinyl-(C₁-C₆)alkoxy, (C₅-C₈)Cycloalkinyl-(C₁-C₆)alkyl-S(O)ₚ-, (C₃-C₈)Cycloalkoxy, (C₃-C₈)Cycloalkyl-S(O)ₚ-, (C₅-C₈)CyCloalkenyloxy, (C₅-C₈)Cycloalkenyl-S(O)ₚ-, (C₅-C₈)CyCloalkinyloxy, (C₅-C₈)Cycloalkinyl-S(O)ₚ-, (C₃-C₈)Cycloalkoxy-(C₁-C₆)alkoxy, (C₃-C₈)CyCloalkoxy-(C₁-C₆)alkyl-S(O)ₚ-, Phenyl, Phenyl-(C₁-C₆)alkoxy, Phenoxy, Phenyl-S(O)ₚ-, Phenyl-(C₁-C₆)alkyl-S(O)ₚ-, Phenoxy-(C₁-C₆)alkoxy, Phenoxy-(C₁-C₆)alkyl-S(O)ₚ-, einen Rest Het⁷, Het⁷-S(O)ₚ-, Het⁷-(C₁-C₆)alkoxy, Het⁷-O-, Het⁷-O-(C₁-C₆)alkoxy, wobei der heterocyclische Rest Het⁷ wie weiter oben oder weiter unten definiert ist,
   wobei jeder der letztgenannten 29 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist und p jeweils unabhängig voneinander 0, 1 oder 2 bedeutet,
   und
   vorzugsweise die Reste (a)
   Halogen, Cyano, Nitro, Hydroxy, Carboxy, (C₁-C₈)Alkoxy, (C₁-C₈)Haloalkoxy, (C₁-C₄)Alkoxy-(C₁-C₄)alkoxy, (C₁-C₆)Alkylthio, (C₁-C₆)Haloalkylthio, (C₁-C₆)Alkylsulfinyl, (C₁-C₆)Haloalkylsulfinyl, (C₁-C₆)Alkylsulfonyl, (C₁-C₈)Haloalkylsulfonyl,
   (C₃-C₈)Cycloalkyl,
   (C₅-C₈)Cycloalkenyl,
   (C₅-C₈)Cycloalkinyl,
   (C₃-C₈)Cycloalkyl-(C₁-C₆)alkoxy,
   (C₅-C₈)Cycloalkenyl-(C₁-C₆)alkoxy,
   (C₅-C₈)Cycloalkinyl-(C₁-C₆)alkoxy,
   (C₃-C₈)Cycloalkoxy, (C₃-C₈)Cycloalkylthio,
   (C₃-C₈)Cycloalkylsulfinyl, (C₃-C₈)Cycloalkylsulfonyl, (C₃-C₈)Cycloalkoxy-(C₁-C₆)alkoxy,
   Phenyl, Phenoxy, Phenylthio, Phenylsulfinyl, Phenylsulfonyl, Phenyl-(C₁-C₆)alkoxy, Phenyl-(C₁-C₆)alkylthio, Phenyl-(C₁-C₆)alkylsulfinyl, Phenyl-(C₁-C₆)alkylsulfonyl, Phenoxy-(C₁-C₆)alkoxy, Phenoxy-(C₁-C₆)alkylthio, Phenoxy-(C₁-C₆)alkylsulfinyl und Phenoxy-(C₁-C₆)alkylsulfonyl,
   wobei jeder der genannten Reste mit cyclischen Teilen im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist,
(b) Reste der Formeln -C(=O)-R^{C}, -C(=O)-O-R^{C}, -O-C(=O)-R^{C}, -O-C(=O)-O-R^{C}, -C(=O)-S-R^{C}, -C(=S)-S-R^{C}, -C(=S)-S-R^{C}, -C(=O)-N R*R**, -C(=O)-O-NR*R**, -O-C(=O)-NR*R**, -N(R*)-C(=O)-R^{C}, -N(R*)-C (=O)-NR*R**, -N(R*)-C(=O)-O-R^{C}, -P(=O)(R^{C})(R^{D}), -P(=O)(OR^{C})(R^{D}), -P( =O)(OR^{C})(OR^{D}) oder -O-P(=O)(OR^{C})(OR^{D}), vorzugsweise einen Rest der Formel -C(=O)-R^{C}, -C(=O)-O-R^{C}, -O-C(=O)-R^{C} und -O-C(=O)-O-R^{C}, insbesondere einen Rest der Formel -C(=O)-O-R^{C}, -O-C(=O)-R^{C} und -O-C(=O)-O-R^{C},
   wobei R*, R**, R^{C} und R^{D} wie weiter unten definiert sind, vorzugsweise die Reste (b1)
   [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]-carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₈)Alkoxy]-carbonyloxy, [(C₂-C₈)Alkenyloxy]-carbonyloxy und [(C₂-C₈)Alkinyloxy]-carbonyloxy,
   wobei jeder der letztgenannten 23 Reste unsubstituiert oder durch einen oder mehrere Reste aus der Gruppe Halogen, NO₂, (C₁-C₄)Alkoxy und gegebenenfalls durch Halogen, CN, NO₂, (C₁-C₄)Alkyl, (C₁-C₄)Alkoxy und (C₁-C₄)Alkylthio substituiertes Phenyl substituiert ist, und vorzugsweise die Reste (b2)
   (C₃-C₈)Cycloalkylcarbonyl,
   (C₃-C₈)Cycloalkyl-[(C₁-C₆)alkyl]-carbonyl, (C₃-C₈)Cycloalkyl-[(C₁-C₆)alkoxy]-carbonyl,
   (C₃-C₈)Cycloalkoxycarbonyl,
   (C₃-C₈)Cycloalkoxy-[(C₁-C₆)alkyl]-carbonyl, (C₃-C₈)Cycloalkoxy-[(C₁-C₆)alkoxy]-carbonyl, (C₃-C₈)Cycloalkylcarbonyloxy, (C₃-C₈)Cycloalkyl-[(C₁-C₆)alkyl]-carbonyloxy, (C₅-C₈)Cycloalkenyl-[(C₁-C₆)alkyl]-carbonyloxy, (C₅-C₈)Cycloalkinyl-[(C₁-C₆)alkyl]-carbonyloxy, (C₃-C₈)Cycloalkyl-[(C₁-C₆)alkoxy]-carbonyloxy, (C₅-C₈)Cycloalkenyl-[(C₁-C₆)alkoxy]-carbonyloxy, (C₅-C₈)Cycloalkinyl-[(C₁-C₆)alkoxy]-carbonyloxy, (C₃-C₈)Cycloalkoxycarbonyloxy, (C₃-C₈)Cycloalkoxy-[(C₁-C₆)alkyl]-carbonyloxy, (C₃-C₈)Cycloalkoxy-[(C₁-C₆)alkoxy]-carbonyloxy, (C₃-C₈)Cycloalkylcarbonylamino, (C₃-C₈)Cycloalkyl-[(C₁-C₆)alkyl]-carbonylamino, (C₅-C₈)Cycloalkenyl-[(C₁-C₆)alkyl]-carbonylamino, (C₅-C₈)Cycloalkinyl-[(C₁-C₆)alkyl]-carbonylamino, (C₃-C₈)Cycloalkyl-[(C₁-C₆)alkoxy]-carbonylamino, (C₃-C₈)Cycloalkoxycarbonylamino, (C₃-C₈)Cycloalkoxy-[(C₁-C₆)alkyl]-carbonylamino und (C₃-C₈)Cycloalkoxy-[(C₁-C₆)alkoxy]-carbonylamino,
   Phenylcarbonyl,
   Phenyl-[(C₁-C₆)alkyl]-carbonyl,
   Phenyl-[(C₁-C₆)alkoxy]-carbonyl,
   Phenoxycarbonyl,
   Phenoxy-[(C₁-C₆)alkyl]-carbonyl,
   Phenoxy-[(C₁-C₆)alkoxy]-carbonyl,
   Phenylcarbonyloxy,
   Phenyl-[(C₁-C₆)alkyl]-carbonyloxy,
   Phenyl-[(C₁-C₆)alkoxy]-carbonyloxy,
   Phenoxycarbonyloxy,
   Phenoxy-[(C₁-C₆)alkyl]-carbonyloxy,
   Phenoxy-[(C₁-C₆)alkoxy]-carbonyloxy,
   Phenylcarbonylamino,
   Phenyl-[(C₁-C₆)alkyl]-carbonylamino,
   Phenyl-[(C₁-C₆)alkoxy]-carbonylamino,
   Phenoxycarbonylamino,
   Phenoxy-[(C₁-C₆)alkyl]-carbonylamino,
   Phenoxy-[(C₁-C₆)alkoxy]-carbonylamino,
   wobei jeder der letztgenannten 42 Reste im cyclischen Teil gegebenenfalls mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise benzokondensiert ist, und im Ring oder im mehrcyclischen System unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist, und
(c) Reste der Formeln -SiR'₃, -O-SiR'₃,
   (R')₃Si-(C₁-C₆)alkoxy, -CO-O-NR'₂, -O-N=CR'₂, -N=CR'₂, -O-NR'₂,-CH(OR')₂ und -O-(CH₂)_{q}-CH(OR')₂,
   in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und q eine ganze Zahl von 0 bis 6 bedeuten, und
(d) Reste der Formel R"O-CHR"'CH(OR")-(C₁-C₆)alkoxy,
   in welcher jeder der Reste R" unabhängig voneinander H oder (C₁-C₄)Alkyl oder gemeinsam eine (C₁-C₆)Alkylengruppe bedeuten und R"' H oder (C₁-C₄)Alkyl bedeuten,
   besteht, substituiert ist,
   oder
R⁴ (C₃-C₉)Cycloalkyl, (C₅-C₉)Cycloalkenyl, (C₅-C₉)Cycloalkinyl oder Phenyl, wobei jeder der letztgenannten 4 Reste unsubstituiert oder durch einen oder mehrere Reste aus der Gruppe, die aus den Resten [Untergruppen (a')-(e')]
(a') Halogen, Cyano, Thio, Nitro, Hydroxy, Carboxy, (C₁-C₈)Alkyl, (C₁-C₈)Haloalkyl, (C₁-C₄)Alkoxy-(C₁-C₄)alkyl, (C₂-C₈)Alkenyl, (C₂-C₈)Haloalkenyl, (C₂-C₈)Alkinyl, (C₂-C₈)Haloalkinyl, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio und Reste der Formeln -NR*R**, wobei die Reste R^{*} und R^{**} weiter unten definiert sind,
(b') Reste der Formeln -C(=O)-R^{C}, -C(=O)-O-R^{C}, -O-C(=O)-R^{C}, -O-C(=O)-O-R^{C}, -C(=O)-S-R^{C}, -C(=S)-S-R^{C}, -C(=S)-S-R^{C}, -C(=O)-N R*R**, -C(=O)-O-NR*R**, -O-C(=O)-NR*R**, -N(R*)-C(=O)-R^{C}, -N(R*)-C (=O)-NR*R**, -N(R*)-C(=O)-O-R^{C}, -P(=O)(R^{C})(R^{D}), -P(=O)(OR^{C})(R^{D}), -P( =O)(OR^{C})(OR^{D}) oder -O-P(=O)(OR^{C})(OR^{D}), vorzugsweise einen Rest der Formel -C(=O)-R^{C}, -C(=O)-O-R^{C}, -O-C(=O)-R^{C} und -O-C(=O)-O-R^{C}, insbesondere einen Rest der Formel -C(=O)-O-R^{C}, -O-C(=O)-R^{C} und -O-C(=O)-O-R^{C},
   wobei R^{*}, R^{**}, R^{C} und R^{D} wie weiter unten definiert sind,
   und vorzugsweise die Reste (b1')
   [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]-carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, (C₁-C₄)Alkylimino, (C₁-C₄)Alkoxyimino, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₈)Alkoxy]-carbonyloxy, [(C₂-C₈)Alkenyloxy]-carbonyloxy, [(C₂-C₈)Alkinyloxy]-carbonyloxy, (C₁-C₈)Alkylsulfinyl und (C₁-C₈)Alkylsulfonyl,
   wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist, und vorzugsweise die Reste (b2')
   (C₃-C₈)Cycloal kylcarbonyl,
   (C₃-C₈)Cycloalkyl-[(C₁-C₆)alkyl]-carbonyl, (C₃-C₈)Cycloalkyl-[(C₁-C₈)alkoxy]-carbonyl,
   (C₃-C₈)Cycloaloxycarbonyl, (C₃-C₈)Cycloalkoxy-[(C₁-C₆)alkyl]-carbonyl, (C₃-C₈)Cycloalkoxy-[(C₁-C₆)alkoxy]-carbonyl,
   (C₃-C₈)Cycloalkylcarbonyloxy, (C₃-C₈)Cycloalkyl-[(C₁-C₆)alkyl]-carbonyloxy, (C₅-C₈)Cycloalkenyl-[(C₁-C₆)alkyl]-carbonyloxy, (C₅-C₈)Cycloalkinyl-[(C₁-C₆)alkyl]-carbonyloxy, (C₃-C₈)Cycloalkyl-[(C₁-C₆)alkoxy]-carbonyloxy, (C₅-C₈)Cycloalkenyl-[(C₁-C₆)alkoxy]-carbonyloxy, (C₅-C₈)Cycloalkinyl-[(C₁-C₆)alkoxy]-carbonyloxy, (C₃-C₈)Cycloalkoxycarbonyloxy, (C₃-C₈)Cycloalkoxy-[(C₁-C₆)alkyl]-carbonyloxy, (C₃-C₈)Cycloalkoxy-[(C₁-C₆)alkoxy]-carbonyloxy,
   (C₃-C₈)Cycloalkylcarbonylamino, (C₃-C₈)Cycloalkyl-[(C₁-C₆)alkyl]-carbonylamino, (C₅-C₈)Cycloalkenyl-[(C₁-C₆)alkyl]-carbonylamino, (C₅-C₈)Cycloalkinyl-[(C₁-C₆)alkyl]-carbonylamino, (C₃-C₈)Cycloal kyl-[(C₁-C₆)alkoxy]-carbonylamino, (C₃-C₈)Cycloalkoxycarbonylamino, (C₃-C₈)Cycloalkoxy-[(C₁-C₆)alkyl]-carbonylamino und (C₃-C₈)Cycloalkoxy-[(C₁-C₆)alkoxy]-carbonylamino,
   Phenylcarbonyl,
   Phenyl-[(C₁-C₆)alkyl]-carbonyl,
   Phenyl-[(C₁-C₆)alkoxy]-carbonyl,
   Phenoxycarbonyl,
   Phenoxy-[(C₁-C₆)alkyl]-carbonyl, Phenoxy-[(C₁-C₆)alkoxy]-carbonyl,
   Phenylcarbonyloxy,
   Phenyl-[(C₁-C₆)alkyl]-carbonyloxy,
   Phenyl-[(C₁-C₆)alkoxy]-carbonyloxy,
   Phenoxycarbonyloxy,
   Phenoxy-[(C₁-C₆)alkyl]-carbonyloxy,
   Phenoxy-[(C₁-C₆)alkoxy]-carbonyloxy,
   Phenylcarbonylamino,
   Phenyl-[(C₁-C₆)alkyl]-carbonylamino,
   Phenyl-[(C₁-C₆)alkoxy]-carbonylamino,
   Phenoxycarbonylamino,
   Phenoxy-[(C₁-C₆)alkyl]-carbonylamino,
   Phenoxy-[(C₁-C₆)alkoxy]-carbonylamino,
   wobei jeder der letztgenannten 42 Reste im cyclischen Teil gegebenenfalls mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise benzokondensiert ist, und im Ring oder im mehrcyclischen System unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist, und
(c') Reste der Formeln -SiR'₃, -O-SiR'₃,
   (R')₃Si-(C₁-C₆)alkoxy, -CO-O-NR'₂, -O-N=CR'₂, -N=CR'₂, -O-NR'₂,-CH(OR')₂ und -O-(CH₂)_{q}-CH(OR')₂,
   in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und q eine ganze Zahl von 0 bis 6 bedeuten, und
(d') Reste der Formel R"O-CHR"'CH(OR")-(C₁-C₆)alkoxy,
   in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten, und
(e') ein Rest der Formel Het⁷, der unsubstituiert oder durch einen oder mehrere gleiche oder verschiedene Reste R^{B} substituiert ist,
   besteht, substituiert ist,
   oder
R⁴ einen mehrcyclischen Rest auf Basis von (C₃-C₉)Cycloalkyl, (C₅-C₉)Cycloalkenyl, (C₅-C₉)Cycloalkinyl oder Phenyl, wobei der Basisring mit einem carbocyclischen oder heterocyclischen Ring, vorzugsweise einem 5-oder 6-gliedrigen Ring mit 0 oder 1 bis 3 Heteroringatomen aus der Gruppe N, O und S kondensiert ist, vorzugsweise benzokondensiert ist, und wobei der Basisring oder das mehrcyclische System unsubstituiert oder durch einen oder mehrere gleiche oder verschiedene Reste R^{B} substituiert ist, vorzugsweise unsubstitutiert oder durch einen oder mehrere Reste aus der Gruppe Halogen, Cyano, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)Alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist, oder
R⁴ einen heterocyclischen Rest Het⁷, der im Ring oder im mehrcyclischen System unsubstituiert oder durch einen oder mehrere gleiche oder verschiedene Reste R^{B} substituiert ist, vorzugsweise unsubstituiert oder durch einen oder mehrere Reste aus der Gruppe Halogen, Cyano, Thio, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
bedeutet,
wobei in den obengenannten und den nachfolgenden Resten
Het⁷ jeweils unabhängig voneinander einen gesättigten, teilweise ungesättigten oder heteroaromatischen monocyclischen Heterocyclylrest mit 3 bis 9 Ringatomen, vorzugsweise mit 5 oder 6 Ringatomen, oder 9- oder 10-gliedrigen bicyclischen Heterocyclus, enthaltend 1, 2, 3 oder 4 Heteroatome ausgewählt aus der Gruppe bestehend aus O, N und S, vorzugsweise einen 5- oder 6-gliedrigen Heterocyclus mit 1 bis 3 Heteroringatomen aus der Gruppe N, O und S, der gegebenenfalls auch mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise gegebenenfalls benzokondensiert ist, bedeutet,
R^{*}, R^{**} jeweils unabhängig voneinander (d. h. auch von anderen Gruppen NR*R**) H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus gegebenenfalls durch einen oder mehrere gleiche oder verschiedene Reste R^{bb} substituiert ist, bedeuten oder
R* und R** zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten,
R^{A} Halogen, Cyano, Hydroxy oder (C₁-C₆)Alkoxy bedeutet,
R^{B} Halogen, Cyano, Hydroxy, Oxo, Nitro, (C₁-C₆)Alkyl, (C₁-C₄)Haloalkyl, Cyano-(C₁-C₄)alkyl, Hydroxy-(C₁-C₄)alkyl, Nitro-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkyl, (C₁-C₄)Alkoxy-(C₁-C₄)Alkoxy, (C₁-C₄)Haloalkoxy-(C₁-C₄)alkyl, (C₁-C₄)Haloalkoxy-(C₁-C₄)alkoxy, (C₁-C₆)Alkylthio, (C₂-C₆)Alkenylthio, (C₂-C₆)Alkinylthio, (C₁-C₆)Alkylsulfinyl, (C₁-C₆)Haloalkylsulfinyl, (C₁-C₆)Alkylsulfonyl, (C₁-C₆)Haloalkylsulfonyl, ein Rest der Formel R^{aa}-C(=O)-oder R^{aa}-C(=O)-(C₁-C₆)alkyl, wobei die R^{aa} weiter unten definiert sind, -NR*R**, wobei R^{*} und R^{**} weiter unten definiert sind, Tri-[(C₁-C₄)alkyl]-silyl, Tri-[(C₁-C₄)alkyl]-silyl-(C₁-C₆)alkyl, (C₃-C₆)Cycloalkyl, (C₃-C₆)Cycloalkoxy, (C₃-C₆)Cycloalkyl-(C₁-C₄)alkyl, (C₃-C₆)Cycloalkyl-(C₁-C₈)alkoxy, Phenyl, Phenyl-(C₁-C₆)alkyl, Phenoxy, Phenoxy-(C₁-C₆)alkyl, Phenylamino, Phenylamino-(C₁-C₆)alkyl oder einen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, wobei jeder der letztgenannten 11 Reste im cyclischen Teil gegebenenfalls durch einen oder mehrere gleiche oder verschiedene Reste R^{bb} substituiert ist, substituiert ist, bedeutet,
R^{C}, R^{D} jeweils unabhängig voneinander (auch unabhängig von Resten R^{C}, R^{D} in anderen Gruppen)
Wasserstoff, (C₁-C₈)Alkyl, (C₂-C₈)Alkenyl oder (C₂-C₈)Alkinyl bedeutet,
wobei jeder der letztgenannten 3 Reste unsubstituiert oder durch einen oder mehrere Reste aus der Gruppe Halogen, Cyano, Nitro, Hydroxy, (C₁-C₆)Alkoxy, (C₂-C₆)Alkenyloxy, (C₂-C₆)Alkinyloxy, (C₁-C₈)Haloalkoxy, (C₁-C₄)Alkoxy-(C₁-C₄)alkoxy, (C₁-C₈)Alkylthio, (C₁-C₈)Haloalkylthio, (C₁-C₈)Alkylsulfinyl, (C₁-C₈)Haloalkylsulfinyl, (C₁-C₈)Alkylsulfonyl, (C₁-C₈)Haloalkylsulfonyl und Tri-[(C₁-C₄)alkyl]-silyl substituiert ist,
oder
(C₃-C₈)Cycloalkyl, (C₅-C₈)Cycloalkenyl, (C₅-C₈)Cycloalkinyl, Phenyl, (C₃-C₈)Cycloalkyl-(C₁-C₆)alkyl, (C₅-C₈)Cycloalkenyl-(C₁-C₆)alkyl, (C₅-C₈)Cycloalkinyl-(C₁-C₆)alkyl, Phenyl-(C₁-C₆)alkyl, (C₃-C₈)Cycloalkyloxy-(C₁-C₆)alkyl, (C₃-C₈)Cycloalkyl-S(O)ₚ-(C₁-C₆)alkyl, (C₅-C₈)Cycloalkenyloxy-(C₁-C₆)alkyl, (C₅-C₈)Cycloalkinyloxy-(C₁-C₆)alkyl, Phenoxy-(C₁-C₆)alkyl, Phenyl-S(O)ₚ-(C₁-C₆)alkyl, (C₃-C₈)Cycloalkylamino-(C₁-C₆)alkyl, (C₅-C₈)Cycloalkenylamino-(C₁-C₆)alkyl, (C₅-C₈₎Cycloalkinylamino-(C₁-C₆)alkyl, Phenylamino-(C₁-C₆)alkyl, Het⁷, Het⁷-(C₁-C₆)alkyl, Het⁷-O-(C₁-C₆)alkyl oder Het⁷-S(O)ₚ-(C₁-C₆)alkyl, wobei Het⁷ die genannte Bedeutung hat,
wobei jeder der letztgenannten 22 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist und p jeweils unabhängig voneinander 0, 1 oder 2 bedeutet,
bedeuten,
R^{aa} jeweils unabhängig voneinander Wasserstoff, OH, (C₁-C₆)Alkyl, (C₁-C₄)Haloalkyl, (C₂-C₆)Alkenyl, (C₂-C₆)Alkinyl, (C₁-C₆)Alkoxy, (C₁-C₆)Alkoxy-(C₁-C₆)alkyl, (C₁-C₆)Alkoxy-(C₁-C₆)alkyloxy, (C₁-C₄)Haloalkoxy, (C₁-C₄)Haloalkoxy-(C₁-C₆)alkyl, (C₁-C₄)Haloalkoxy-(C₁-C₆)alkoxy, (C₃-C₆)Alkenyloxy, (C₃-C₆)Alkenyloxy-(C₁-C₆)alkyl, (C₃-C₆)Alkenyloxy-(C₁-C₆)alkoxy, (C₃-C₆)Alkinyloxy, (C₃-C₆)Alkinyloxy-(C₁-C₆)alkyl, (C₃-C₆)Alkinyloxy-(C₁-C₆)alkoxy, -NR*R*, wobei R* und R** wie oben definiert sind, Tri-[(C₁-C₄)alkyl]-silyl, Tri-[(C₁-C₄)alkyl]-silyl-(C₁-C₆)alkyl, Tri-[(C₁-C₄)alkyl]-silyl-(C₁-C₆)alkoxy, (C₃-C₆)Cycloalkyl, (C₃-C₆)Cycloalkoxy, (C₃-C₆)Cycloalkyl-(C₁-C₈)alkyl, (C₃-C₆)Cycloalkyl-(C₁-C₈)alkoxy, (C₅-C₆)Cycloalkenyl, (C₅-C₆)Cycloalkenyl-(C₁-C₆)alkyl, (C₅-C₆)Cycloalkenyloxy,(C₅-C₆)Cycloalkinyl, (C₅-C₆)Cycloalkinyl-(C₁-C₆)alkyl, (C₅-C₆)Cycloalkinyl-(C₁-C₆)alkoxy, Phenyl, Phenyl-(C₁-C₆)alkyl, Phenyl-(C₁-C₆)alkoxy, Phenoxy, Phenoxy-(C₁-C₆)alkyl, Phenoxy-(C₁-C₆)alkoxy, Phenylthio, Phenyl-S(O)ₚ-(C₁-C₆)alkyl, Phenyl-S(O)ₚ-(C₁-C₆)alkoxy, wobei p jeweils unabhängig voneinander 0, 1 oder 2 bedeutet, Phenylamino, Phenylamino-(C₁-C₆)alkyl, Phenylamino-(C₁-C₆)alkoxy oder einen gegebenenfalls über eine Alkylengruppe oder eine Alkoxygruppe gebundenen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, bedeutet, wobei jeder der letztgenannten 20 Reste im cyclischen Teil gegebenenfalls durch einen oder mehrere gleiche oder verschiedene Reste R^{cc} substituiert ist, bedeutet und
R^{bb} und R^{cc} jeweils unabhängig voneinander Halogen, (C₁-C₄)Alkyl, (C₁-C₄)Haloalkyl, (C₁-C₄)Alkoxy oder (C₁-C₄)Haloalkoxy bedeutet.

Weiter bevorzugt sind dabei auch Verbindungen (I), vorzugsweise der Formel (la), oder deren Salze, worin
R⁴ Wasserstoff, (C₁-C₁₈)Alkyl, (C₂-C₁₈)Alkenyl oder (C₂-C₁₈)Alkinyl, vorzugsweise H, (C₁-C₁₂)Alkyl, (C₂-C₁₂)Alkenyl oder (C₂-C₁₂)Alkinyl, insbesondere H, (C₁-C₈)Alkyl, (C₂-C₈)Alkenyl oder (C₂-C₈)Alkinyl, weiter bevorzugt H oder (C₁-C₆)Alkyl, (C₂-C₆)Alkenyl oder (C₂-C₆)Alkinyl, weiter bevorzugt (C₁-C₄)Alkyl, wobei jeder der letztgenannten 13 Reste, die C-Atome enthalten, unsubstituiert oder durch einen oder mehrere Reste aus der Gruppe, die aus den Resten [Untergruppen (a)-(d)]
(a) Halogen, Cyano, Thio, Nitro, Hydroxy, Carboxy, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₁-C₆)Haloalkylthio, (C₂-C₆)Haloalkenylthio, (C₂-C₆)Haloalkinylthio, (C₁-C₆)Alkylsulfinyl, (C₂-C₆)Alkenylsulfinyl, (C₂-C₆)Alkinylsulfinyl, (C₁-C₆)Haloalkylsulfinyl, (C₂-C₆)Haloalkenylsulfinyl, (C₂-C₆)Haloalkinylsulfinyl, (C₁-C₆)Alkylsulfonyl, (C₂-C₆)Alkenylsulfonyl, (C₂-C₆)Alkinylsulfonyl, (C₁-C₆)Haloalkylsulfonyl, (C₂-C₆)Haloalkenylsulfonyl, (C₂-C₆)Haloalkinylsulfonyl, Reste der Formel -NR*R**, wobei R* und R** weiter unten definiert sind, und (C₃-C₆)Cycloalkyl, (C₅-C₆)Cycloalkenyl, (C₅-C₆)Cycloalkinyl, (C₃-C₆)Cycloalkyl-(C₁-C₄)alkoxy, (C₅-C₆)Cycloalkenyl-(C₁-C₄)alkoxy, (C₅-C₆)Cycloalkinyl-(C₁-C₄)alkoxy, (C₃-C₆)Cycloalkoxy, (C₅-C₆)Cycloalkenyloxy, (C₅-C₆)Cycloalkinyloxy, (C₃-C₆)Cycloalkoxy-(C₁-C₄)alkoxy, Phenyl, Phenyl-(C₁-C₆)alkoxy, Phenoxy, Phenoxy-(C₁-C₄)alkoxy, Phenyl-S(O)ₚ-, Phenyl-(C₁-C₆)alkyl-S(O)ₚ-, Phenyloxy-(C₁-C₆)alkyl-S(O)ₚ-, einen Rest Het⁷, Het⁷-(C₁-C₆)alkoxy, Het⁷-O-, Het⁷-O-(C₁-C₄)alkoxy, Het⁷-(C₁-C₆)alkoxy, Het⁷-S(O)ₚ-, Het⁷-O-(C₁-C₄)alkyl-S(O)ₚ-, wobei der heterocyclische Rest Het⁷ wie weiter oben oder unten definiert ist,
   wobei jeder der letztgenannten 24 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert und p jeweils unabhängig voneinander 0, 1 oder 2 bedeutet,
   und
   vorzugsweise die Reste (a1)
   Halogen, Cyano, Nitro, Hydroxy, Carboxy, (C₁-C₆)Alkoxy, (C₁-C₆)Haloalkoxy, (C₁-C₄)Alkoxy-(C₁-C₄)alkoxy, (C₁-C₆)Alkylthio, (C₁-C₆)Haloalkylthio, (C₁-C₆)Alkylsulfonyl, (C₁-C₆)Haloalkylsulfonyl, (C₃-C₆)Cycloalkyl, (C₅-C₆)Cycloalkenyl, (C₅-C₆)Cycloalkinyl, (C₃-C₆)Cycloalkyl-(C₁-C₄)alkoxy, (C₅-C₄)Cycloalkenyl-(C₁-C₄)alkoxy, (C₅-C₄)Cycloalkinyl-(C₁-C₄)alkoxy, (C₃-C₄)Cycloalkoxy, (C₃-C₄)Cycloalkoxy-(C₁-C₄)alkoxy, Phenyl, Phenyl-(C₁-C₄)alkoxy, Phenoxy und Phenoxy-(C₁-C₄)alkoxy, Phenylthio, Phenylsulfinyl, Phenylsulfonyl,
   wobei jeder der Reste (a1) im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist,
(b) Reste der Formeln -C(=O)-R^{C}, -C(=O)-O-R^{C}, -O-C(=O)-R^{C}, -O-C(=O)-O-R^{C}, -C(=O)-S-R^{C}, -C(=S)-S-R^{C}, -C(=S)-S-R^{C}, -C(=O)-N R*R**, -C(=O)-O-NR*R**, -O-C(=O)-NR*R**, -N(R*)-C(=O)-R^{C}, -N(R*)-C (=O)-NR*R**, -N(R*)-C(=O)-O-R^{C}, -P(=O)(R^{C})(R^{D}), -P(=O)(OR^{C})(R^{D}), -P( =O)(OR^{C})(OR^{D}) oder -O-P(=O)(OR^{C})(OR^{D}), vorzugsweise einen Rest der Formel -C(=O)-R^{C}, -C(=O)-O-R^{C}, -O-C(=O)-R^{C} und -O-C(=O)-O-R^{C}, insbesondere einen Rest der Formel -C(=O)-O-R^{C}, -O-C(=O)-R^{C}
   und -O-C(=O)-O-R^{C},
   wobei R*, R**, R^{C} und R^{D} wie weiter unten definiert sind,
   vorzugsweise die Reste (b1)
   [(C₁-C₆)Alkoxy]-carbonyl, [(C₁-C₆)Alkoxy]-thiocarbonyl, [(C₂-C₆)Alkenyloxy]-carbonyl, [(C₂-C₈)Alkinyloxy]-carbonyl, [(C₁-C₆)Alkylthio]-carbonyl, [(C₂-C₆)Alkenylthio]-carbonyl, [(C₂-C₆)Alkinylthio]-carbonyl, (C₁-C₆)Alkanoyl, [(C₂-C₆)Alkenyl]-carbonyl, [(C₂-C₆)Alkinyl]-carbonyl, [(C₁-C₆)Alkyl]-carbonylamino, [(C₂-C₆)Alkenyl]-carbonylamino, [(C₂-C₆)Alkinyl]-carbonylamino, [(C₁-C₆)Alkoxy]-carbonylamino, [(C₂-C₆)Alkenyloxy]-carbonylamino, [(C₂-C₆)Alkinyloxy]-carbonylamino, [(C₁-C₆)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₆)Alkoxy]-carbonyloxy, [(C₂-C₆)Alkenyloxy]-carbonyloxy und [(C₂-C₆)Alkinyloxy]-carbonyloxy,
   wobei jeder der letztgenannten 23 Reste unsubstituiert oder durch einen oder mehrere Reste aus der Gruppe Halogen, NO₂, (C₁-C₄)Alkoxy und gegebenenfalls durch Halogen, CN, NO₂, (C₁-C₄)Alkyl, (C₁-C₄)Alkoxy und (C₁-C₄)Alkylthio substituiertes Phenyl substituiert ist, und
   vorzugsweise die Reste (b2)
   (C₃-C₆)Cycloalkylcarbonyl,
   (C₃-C₆)Cycloalkyl-[(C₁-C₄)alkyl]-carbonyl,
   (C₃-C₆)Cycloalkyl-[(C₁-C₄)alkoxy]-carbonyl,
   (C₃-C₆)Cycloal koxycarbonyl,
   (C₃-C₆)Cycloalkoxy-[(C₁-C₄)alkyl]-carbonyl, (C₃-C₆)Cycloalkoxy-[(C₁-C₄)alkoxy]-carbonyl, (C₃-C₆)Cycloalkylcarbonyloxy,
   (C₃-C₆)Cycloalkyl-[(C₁-C₄)alkyl]-carbonyloxy, (C₅-C₆)Cycloalkenyl-[(C₁-C₄)alkyl]-carbonyloxy, (C₅-C₆)Cycloalkinyl-[(C₁-C₄)alkyl]-carbonyloxy, (C₃-C₆)Cycloalkyl-[(C₁-C₄)alkoxy]-carbonyloxy, (C₅-C₆)Cycloalkenyl-[(C₁-C₄)alkoxy]-carbonyloxy, (C₅-C₆)Cycloalkinyl-[(C₁-C₄)alkoxy]-carbonyloxy, (C₃-C₆)Cycloalkoxycarbonyloxy, (C₃-C₆)Cycloalkoxy-[(C₁-C₄)alkyl]-carbonyloxy, (C₃-C₆)Cycloalkoxy-[(C₁-C₄)alkoxy]-carbonyloxy, (C₃-C₆)Cycloalkylcarbonylamino, (C₃-C₆)Cycloalkyl-[(C₁-C₄)alkyl]-carbonylamino, (C₅-C₆)Cycloalkenyl-[(C₁-C₄)alkyl]-carbonylamino, (C₅-C₆)Cycloalkinyl-[(C₁-C₄)alkyl]-carbonylamino, (C₃-C₆)Cycloalkyl-[(C₁-C₄)alkoxy]-carbonylamino, (C₃-C₆)Cycloalkoxycarbonylamino, (C₃-C₆)Cycloalkoxy-[(C₁-C₄)alkyl]-carbonylamino und (C₃-C₆)Cycloalkoxy-[(C₁-C₄)alkoxy]-carbonylamino, Phenylcarbonyl,
   Phenyl-[(C₁-C₄)alkyl]-carbonyl, Phenyl-[(C₁-C₄)alkoxy]-carbonyl,
   Phenoxycarbonyl,
   Phenoxy-[(C₁-C₄)alkyl]-carbonyl, Phenoxy-[(C₁-C₄)alkoxy]-carbonyl, Phenylcarbonyloxy,
   Phenyl-[(C₁-C₄)alkyl]-carbonyloxy, Phenyl-[(C₁-C₄)alkoxy]-carbonyloxy, Phenoxycarbonyloxy, Phenoxy-[(C₁-C₄)alkyl]-carbonyloxy, Phenoxy-[(C₁-C₄)alkoxy]-carbonyloxy, Phenylcarbonylamino,
   Phenyl-[(C₁-C₄)alkyl]-carbonylamino,
   Phenyl-[(C₁-C₄)alkoxy]-carbonylamino,
   Phenoxycarbonylamino,
   Phenoxy-[(C₁-C₄)alkyl]-carbonylamino,
   Phenoxy-[(C₁-C₄)alkoxy]-carbonylamino,
   wobei jeder der letztgenannten 42 Reste im cyclischen Teil gegebenenfalls mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise benzokondensiert ist, und im Ring oder im mehrcyclischen System unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist, und
(c) Reste der Formeln -SiR'₃, -O-SiR'₃,
   (R')₃Si-(C₁-C₄)alkoxy, -CO-O-NR'₂, -O-N=CR'₂, -N=CR'₂, -O-NR'₂, - CH(OR')₂ und -O-(CH₂)_{q}-CH(OR')₂,
   in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)AlkylenBrücke substituiert ist, und q eine ganze Zahl von 0 bis 6 bedeuten, und
(d) Reste der Formel R"O-CHR"'CH(OR")-(C₁-C₆)alkoxy,
   in welcher jeder der Reste R" unabhängig voneinander H oder (C₁-C₄)Alkyl oder gemeinsam eine (C₁-C₆)Alkylengruppe bedeuten und R"' H oder (C₁-C₄)Alkyl bedeuten,
besteht, substituiert ist,
oder
R⁴ (C₃-C₆)Cycloalkyl, (C₅-C₆)Cycloalkenyl, (C₅-C₆)Cycloalkinyl oder Phenyl, wobei jeder der letztgenannten 4 Reste unsubstituiert oder durch einen oder mehrere Reste aus der Gruppe, die aus den Resten [Untergruppen (a')-(e')]
(a') Halogen, Cyano, Thio, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio und Reste der Formeln -NR*R**, wobei die Reste R* und R**wie oben oder weiter unten definiert sind,
(b') Reste der Formeln -C(=O)-R^{C}, -C(=O)-O-R^{C}, -O-C(=O)-R^{C}, -O-C(=O)-O-R^{C}, -C(=O)-S-R^{C}, -C(=S)-S-R^{C}, -C(=S)-S-R^{C}, -C(=O)-N R*R**, -C(=O)-O-NR*R**, -O-C(=O)-NR*R**, -N(R*)-C(=O)-R^{C}, -N(R*)-C (=O)-NR*R**, -N(R*)-C(=O)-O-R^{C}, -P(=O)(R^{C})(R^{D}), -P(=O)(OR^{C})(R^{D}), -P( =O)(OR^{C})(OR^{D}) oder -O-P(=O)(O^{C})(OR^{D}), vorzugsweise einen Rest der Formel -C(=O)-R^{C}, -C(=O)-O-R^{C}, -O-C(=O)-R^{C} und -O-C(=O)-O-R^{C}, insbesondere einen Rest der Formel -C(=O)-O-R^{C}, -O-C(=O)-R^{C} und -O-C(=O)-O-R^{C},
   wobei R*, R**, R^{C} und R^{D} wie weiter unten definiert sind,
   und vorzugsweise die Reste (b1') [(C₁-C₆)Alkoxy]-carbonyl, [(C₁-C₆)Alkoxy]-thiocarbonyl, [(C₂-C₆)Alkenyloxy]-carbonyl, [(C₂-C₆)Alkinyloxy]-carbonyl, [(C₁-C₆)Alkylthio]-carbonyl, [(C₂-C₆)Alkenylthio]-carbonyl, [(C₂-C₆)Alkinylthio]-carbonyl, (C₁-C₈)Alkanoyl, [(C₂-C₆)Alkenyl]-carbonyl, [(C₂-C₆)Alkinyl]-carbonyl, (C₁-C₄)Alkylimino, (C₁-C₄)Alkoxyimino, [(C₁-C₆)Alkyl]-carbonylamino, [(C₂-C₆)Alkenyl]-carbonylamino, [(C₂-C₆)Alkinyl]-carbonylamino, [(C₁-C₆)Alkoxy]-carbonylamino, [(C₂-C₆)Alkenyloxy]-carbonylamino, [(C₂-C₆)Alkinyloxy]-carbonylamino, [(C₁-C₆)Alkylamino]-carbonylamino, [(C₁-C₄)Alkyl]-carbonyloxy, [(C₂-C₄)Alkenyl]-carbonyloxy, [(C₂-C₄)Alkinyl]-carbonyloxy, [(C₁-C₆)Alkoxy]-carbonyloxy, [(C₂-C₆)Alkenyloxy]-carbonyloxy, [(C₂-C₆)Alkinyloxy]-carbonyloxy, (C₁-C₆)Alkylsulfinyl und (C₁-C₆)Alkylsulfonyl,
   wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist, und vorzugsweise die Reste (b2')
   (C₃-C₆)Cycloal kylcarbonyl,
   (C₃-C₆)Cycloalkyl-[(C₁-C₄)alkyl]-carbonyl,
   (C₃-C₆)Cycloalkyl-[(C₁-C₄)alkoxy]-carbonyl, (C₃-C₆)Cycloal koxycarbonyl,
   (C₃-C₆)Cycloalkoxy-[(C₁-C₄)alkyl]-carbonyl, (C₃-C₆)Cycloalkoxy-[(C₁-C₄)alkoxy]-carbonyl, (C₃-C₆)Cycloalkylcarbonyloxy,
   (C₃-C₆)Cycloalkyl-[(C₁-C₄)alkyl]-carbonyloxy, (C₅-C₆)Cycloalkenyl-[(C₁-C₄)alkyl]-carbonyloxy, (C₅-C₆)Cycloalkinyl-[(C₁-C₄)alkyl]-carbonyloxy, (C₃-C₆)Cycloalkyl-[(C₁-C₄)alkoxy]-carbonyloxy, (C₅-C₆)Cycloalkenyl-[(C₁-C₄)alkoxy]-carbonyloxy, (C₅-C₆)Cycloalkinyl-[(C₁-C₄)alkoxy]-carbonyloxy, (C₃-C₆)Cycloalkoxycarbonyloxy, (C₃-C₆)Cycloalkoxy-[(C₁-C₄)alkyl]-carbonyloxy, (C₃-C₆)Cycloalkoxy-[(C₁-C₄)alkoxy]-carbonyloxy, (C₃-C₆)Cycloalkylcarbonylamino, (C₃-C₆)Cycloalkyl-[(C₁-C₄)alkyl]-carbonylamino, (C₅-C₆)Cycloalkenyl-[(C₁-C₄)alkyl]-carbonylamino, (C₅-C₆)Cycloalkinyl-[(C₁-C₄)alkyl]-carbonylamino, (C₃-C₆)Cycloalkyl-[(C₁-C₄)alkoxy]-carbonylamino, (C₃-C₆)Cycloalkoxycarbonylamino, (C₃-C₆)Cycloalkoxy-[(C₁-C₄)alkyl]-carbonylamino und (C₃-C₆)Cycloalkoxy-[(C₁-C₄)alkoxy]-carbonylamino, Phenylcarbonyl,
   Phenyl-[(C₁-C₄)alkyl]-carbonyl, Phenyl-[(C₁-C₄)alkoxy]-carbonyl,
   Phenoxycarbonyl,
   Phenoxy-[(C₁-C₄)alkyl]-carbonyl, Phenoxy-[(C₁-C₄)alkoxy]-carbonyl, Phenylcarbonyloxy,
   Phenyl-[(C₁-C₄)alkyl]-carbonyloxy, Phenyl-[(C₁-C₄)alkoxy]-carbonyloxy, Phenoxycarbonyloxy,
   Phenoxy-[(C₁-C₄)alkyl]-carbonyloxy, Phenoxy-[(C₁-C₄)alkoxy]-carbonyloxy,
   Phenylcarbonylamino,
   Phenyl-[(C₁-C₄)alkyl]-carbonylamino,
   Phenyl-[(C₁-C₄)alkoxy]-carbonylamino,
   Phenoxycarbonylamino,
   Phenoxy-[(C₁-C₄)alkyl]-carbonylamino,
   Phenoxy-[(C₁-C₄)alkoxy]-carbonylamino,
   wobei jeder der letztgenannten 42 Reste im cyclischen Teil gegebenenfalls mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise benzokondensiert ist, und im Ring oder im mehrcyclischen System unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist, und
(c') Reste der Formeln -SiR'₃, -O-SiR'₃,
   (R')₃Si-(C₁-C₆)alkoxy, -CO-O-NR'₂, -O-N=CR'₂, -N=CR'₂, -O-NR'₂, - CH(OR')₂ und -O-(CH₂)_{q}-CH(OR')₂,
   in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)AlkylenBrücke substituiert ist, und q eine ganze Zahl von 0 bis 6 bedeuten, und
(d') Reste der Formel R"O-CHR"'CH(OR")-(C₁-C₆)alkoxy,
   in welcher jeder der Reste R" unabhängig voneinander H oder (C₁-C₄)Alkyl oder gemeinsam eine (C₁-C₆)Alkylengruppe bedeuten und R"' H oder (C₁-C₄)Alkyl bedeuten, und
(e') ein Rest der Formel Het⁷, der unsubstituiert oder durch einen oder
   mehrere gleiche oder verschiedene Reste R^{B} substituiert ist,
besteht, substituiert ist,
oder
R⁴ einen mehrcyclischen Rest auf Basis von (C₃-C₆)Cycloalkyl, (C₅-C₆)Cycloalkenyl, (C₅-C₆)Cycloalkinyl oder Phenyl, wobei der Basisring mit einem carbocyclischen oder heterocyclischen Ring, vorzugsweise einem 5- oder 6-gliedrigen Ring mit 0 oder 1 bis 3 Heteroringatomen aus der Gruppe N, O und S kondensiert ist, vorzugsweise benzokondensiert ist, und wobei der Basisring oder das mehrcyclische System unsubstituiert oder durch einen oder mehrere gleiche oder verschiedene Reste R^{B} substituiert ist, vorzugsweise unsubstitutiert oder durch einen oder mehrere Reste aus der Gruppe Halogen, Cyano, Nitro, Hydroxy, Carboxy, (C₁-C₄)Alkyl, (C₁-C₄)Haloalkyl, (C₁-C₄)Alkoxy-(C₁-C₄)alkyl, (C₂-C₄)Alkenyl, (C₂-C₄)Haloalkenyl, (C₂-C₄)Alkinyl, (C₂-C₄)Haloalkinyl, (C₁-C₄)Alkoxy, (C₂-C₄)Alkenyloxy, (C₂-C₄)Alkinyloxy, (C₁-C₄)Haloalkoxy, (C₁-C₄)Alkoxy-(C₁-C₄)alkoxy, (C₁-C₄)Alkylthio, (C₂-C₄)Alkenylthio, (C₂-C₄)Alkinylthio, (C₃-C₆)Cycloalkyl, (C₃-C₆)Cycloalkoxy, [(C₁-C₄)Alkoxy]-carbonyl, [(C₁-C₄)Haloalkoxy]-carbonyl und Oxo substituiert ist, oder
R⁴ einen heterocyclischen Rest Het⁷, der im Ring oder im mehrcyclischen System der unsubstituiert oder durch einen oder mehrere gleiche oder verschiedene Reste R^{B} substituiert ist, vorzugsweise unsubstituiert oder durch einen oder mehrere Reste aus der Gruppe Halogen, Cyano, Thio, Nitro, Hydroxy, Carboxy, (C₁-C₄)Alkyl, (C₁-C₄)Haloalkyl, (C₁-C₄)Alkoxy-(C₁-C₄)alkyl, (C₂-C₄)Alkenyl, (C₂-C₄)Haloalkenyl, (C₂-C₄)Alkinyl, (C₂-C₆)Haloalkinyl, (C₁-C₄)Alkoxy, (C₂-C₄)Alkenyloxy, (C₂-C₄)Alkinyloxy, (C₁-C₄)Haloalkoxy, (C₁-C₄)Alkoxy-(C₁-C₄)alkoxy, (C₁-C₄)Alkylthio, (C₂-C₄)Alkenylthio, (C₂-C₄)Alkinylthio, (C₃-C₆)Cycloalkyl, (C₃-C₆)Cycloalkoxy, [(C₁-C₄)Alkoxy]-carbonyl, [(C₁-C₄)Haloalkoxy]-carbonyl und Oxo substituiert ist,
bedeutet,
wobei Het⁷, R*, R**, R^{A}, R^{B}, R^{C}, R^{D}, R^{aa}, R^{bb} und R^{cc} die oben bereits genannten Bedeutungen haben, vorzugsweise
Het⁷ jeweils unabhängig voneinander einen gesättigten, teilweise ungesättigten oder heteroaromatischen monocyclischen Heterocyclylrest mit 3 bis 9 Ringatomen, vorzugsweise mit 5 oder 6 Ringatomen, oder 9- oder 10-gliedrigen bicyclischen Heterocyclus, enthaltend 1, 2, 3 oder 4 Heteroatome ausgewählt aus der Gruppe bestehend aus O, N und S, vorzugsweise einen 5- oder 6-gliedrigen Heterocyclus mit 1 bis 3 Heteroringatomen aus der Gruppe N, O und S, der gegebenenfalls auch mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise gegebenenfalls benzokondensiert ist, bedeutet,
R*, R** jeweils unabhängig voneinander (d. h. auch von anderen Gruppen NR*R**) H, (C₁-C₆)Alkyl, (C₂-C₆)Alkenyl, (C₂-C₆)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus gegebenenfalls durch einen oder mehrere gleiche oder verschiedene Reste R^{bb} substituiert ist, bedeuten oder vorzugsweise
H, (C₁-C₄)Alkyl, Allyl, Propargyl, (C₁-C₄)Alkoxy-(C₁-C₄)alkyl, Formyl, Acetyl, n-Propanoyl, i-Propanoyl, Trifluoracetyl, Trichloracetyl. Methoxycarbonyl, Ethoxycarbonyl, n- oder i-Propoxycarbonyl, n-, i-, sec-, t-Butoxycarbonyl, [(C₁-C₄)Haloalkoxy]-carbonyl, Cyclopropyl, Cyclobutyl, Cyclopentyl, Cyclohexyl, Cyclopropyl-methyl, Phenyl, Benzyl, 1- oder 2-Phenyl-ethyl bedeuten,
R* und R** zusammen mit dem N-Atom einen vorzugsweise gesättigten 5- bis 6-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten, vorzugsweise 1-Piperidin-, 1-Piperazin-, 1-Pyrrolidin-, 1-Pyrazolidin-, 1-Piperazolidin- oder 1-Morpholinrest bedeuten,
R^{A} Halogen, Cyano, Hydroxy oder (C₁-C₆)Alkoxy bedeutet,
R^{B} Halogen, Cyano, Hydroxy, Oxo, Nitro, (C₁-C₄)Alkyl, (C₁-C₄)Haloalkyl, Cyano-(C₁-C₄)alkyl, Hydroxy-(C₁-C₄)alkyl, Nitro-(C₁-C₄)alkyl, (C₂-C₄)Alkenyl, (C₂-C₄)Alkinyl, (C₁-C₄)Alkoxy, (C₂-C₄)Alkenyloxy, (C₂-C₄)Alkinyloxy, (C₁-C₄)Haloalkoxy, (C₁-C₄)Alkoxy-(C₁-C₄)alkyl, (C₁-C₄)Alkoxy-(C₁-C₄)alkoxy, (C₁-C₄)Alkylthio, (C₁-C₄)Alkylsulfonyl, (C₁-C₄)Haloalkylsulfonyl, ein Rest der Formel R^{aa}-C(=O)- oder R^{aa}-C(=O)-(C₁-C₆)alkyl, wobei die R^{aa} weiter unten definiert sind, -NR*R**, wobei R* und R** weiter unten definiert sind, Cyclopropyl, Cyclopropyl-methyl, Phenyl, Benzyl, 1- oder 2-Phenylethyl, Phenoxy, 2-Phenoxy-ethyl oder einen 5- oder 6-gliedrigen monocyclischen oder 9- oder 10-gliedrigen bicyclischen Heterocyclus, enthaltend 1, 2 oder 3 4 Heteroatome ausgewählt aus der Gruppe bestehend aus O, N und S, wobei jeder der letztgenannten 9 Reste im cyclischen Teil gegebenenfalls durch einen oder mehrere gleiche oder verschiedene Reste R^{bb} substituiert ist, substituiert ist, bedeutet,
R^{C}, R^{D} jeweils unabhängig voneinander (auch unabhängig von Resten R^{C}, R^{D} in anderen Gruppen)
Wasserstoff, (C₁-C₆)Alkyl, (C₂-C₆)Alkenyl oder (C₂-C₆)Alkinyl bedeutet,
wobei jeder der letztgenannten 3 Reste unsubstituiert oder durch einen oder mehrere Reste aus der Gruppe Halogen, Cyano, Nitro, Hydroxy, (C₁-C₄)Alkoxy, (C₂-C₄)Alkenyloxy, (C₂-C₄)Alkinyloxy, (C₁-C₄)Haloalkoxy, (C₁-C₄)Alkoxy-(C₁-C₄)alkoxy, (C₁-C₄)Alkylthio, (C₁-C₆)Alkylsulfonyl und (C₁-C₆)Haloalkylsulfonyl substituiert ist,
oder
(C₃-C₆)Cycloalkyl, (C₅-C₆)Cycloalkenyl, (C₅-C₆)Cycloalkinyl, Phenyl, (C₃-C₆)Cycloalkyl-(C₁-C₆)alkyl, Phenyl-(C₁-C₄)alkyl, Phenoxy-(C₁-C₄)alkyl, oder Phenylamino-(C₁-C₆)alkyl, Reste Het⁷, Het⁷-(C₁-C₆)alkyl, Het⁷-O-(C₁-C₆)alkyl, wobei Het⁷ die genannte Bedeutung hat,
wobei jeder der letztgenannten 12 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist,
bedeuten,
R^{aa} jeweils unabhängig voneinander Wasserstoff, OH, (C₁-C₆)Alkyl, (C₁-C₄)Haloalkyl, (C₂-C₄)Alkenyl, (C₂-C₄)Alkinyl, (C₁-C₆)Alkoxy, (C₁-C₄)Alkoxy-(C₁-C₄)alkyl, (C₁-C₄)Alkoxy-(C₁-C₄)alkyloxy, (C₁-C₄)Haloalkoxy, (C₁-C₄)Haloalkoxy-(C₁-C₄)alkyl, (C₁-C₄)Haloalkoxy-(C₁-C₄₎alkoxy, -NR*R*, wobei R* und R** wie oben definiert sind, (C₃-C₆)Cycloalkyl, (C₃-C₆)Cycloalkoxy, (C₃-C₆)Cycloalkyl-(C₁-C₆)alkyl, (C₃-C₆)Cycloalkyl-(C₁-C₆)alkoxy, Phenyl, Phenyl-(C₁-C₄)alkyl, Phenyl-(C₁-C₄)alkoxy, Phenoxy, Phenoxy-(C₁-C₄)alkyl, Phenoxy-(C₁-C₄)alkoxy, Phenylamino, Phenylamino-(C₁-C₄)alkyl, Phenylamino-(C₁-C₄)alkoxy oder einen gegebenenfalls über eine (C₁-C₄)Alkylengruppe oder eine (C₁-C₄)Alkoxygruppe gebundenen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, bedeutet, wobei jeder der letztgenannten 14 Reste im cyclischen Teil gegebenenfalls durch einen oder mehrere gleiche oder verschiedene Reste R^{cc} substituiert ist, bedeutet,
R^{bb} jeweils unabhängig voneinander Halogen, (C₁-C₄)Alkyl, (C₁-C₄)Haloalkyl, (C₁-C₄)Alkoxy oder (C₁-C₄)Haloalkoxy, vorzugsweise Halogen, Methyl, CF₃, CCl₃. Methoxy, Ethoxy, OCH₂F, OCF₂H oder OCF₃ bedeutet und
R^{cc} jeweils unabhängig voneinander Halogen, (C₁-C₄)Alkyl, (C₁-C₄)Haloalkyl, (C₁-C₄)Alkoxy oder (C₁-C₄)Haloalkoxy, vorzugsweise Halogen, Methyl, CF₃, CCl₃. Methoxy, Ethoxy, OCH₂F, OCF₂H oder OCF₃ bedeutet.

Weiter bevorzugt sind dabei auch Verbindungen (I), vorzugsweise der Formel (Ia), oder deren Salze, worin
R⁴ H, (C₁-C₆)Alkyl, (C₂-C₆)Alkenyl, (C₂-C₆)Alkinyl,
wobei jeder der letztgenannten 3 Reste unsubstituiert oder durch einen oder mehrere Reste aus der Gruppe Halogen, (C₁-C₄)Alkoxy, (C₁-C₄)Alkylthio, Alkylsulfinyl, Alkylsulfonyl, (C₃-C₆)Cycloalkyl, das unsubstituiert oder durch einen oder mehrere Reste aus der Gruppe Halogen und (C₁-C₄)Alkyl substituiert ist, und
Phenyl, Phenoxy, Phenylthio, Phenylsulfinyl, Phenylsulfonyl, wobei der Phenylring in den letztgenannten 5 Resten jeweils unsubstituiert oder durch einen oder mehrere Reste aus der Gruppe Halogen, (C₁-C₄)Alkyl, (C₁-C₄)Alkoxy und (C₁-C₄)Haloalkyl substituiert ist, und
einen Rest Het⁷, vorzugsweise einen gesättigten oder teilweise ungesättigten monocyclischen Heterocyclylrest mit 5 oder 6 Ringatomen, enthaltend 1, 2 oder 3 Heteroatome ausgewählt aus der Gruppe bestehend aus O, N und S, der unsubstitutiert oder durch einen oder mehrere Reste aus der Gruppe Halogen, (C₁-C₄)Alkyl, (C₁-C₄)Alkoxy und (C₁-C₄)Haloalkyl substituiert ist,
oder
R⁴ (C₃-C₆)Cycloalkyl, unsubstituiert oder durch einen oder mehrere Reste aus der Gruppe (C₁-C₄)Alkyl, (C₁-C₄)Alkoxy und (C₁-C₄)Haloalkyl substituiert ist,
oder
R⁴ Phenyl, das unsubstituiert oder durch einen oder mehrere Reste aus der Gruppe Halogen, (C₁-C₄)Alkyl, (C₁-C₄)Alkoxy und (C₁-C₄)Haloalkyl substituiert ist,
bedeutet.

Besonders bevorzugt sind dabei auch Verbindungen (I), vorzugsweise der Formel (Ia), oder deren Salze, worin
R⁴ H, (C₁-C₄)Alkyl, (C₂-C₄)Alkenyl oder (C₂-C₄)Alkinyl,
wobei jeder der letztgenannten 3 Reste unsubstituiert oder durch einen oder mehrere Reste aus der Gruppe Halogen, (C₁-C₄)Alkoxy, (C₁-C₄)Alkylthio, Cyclopropyl, Cyclobutyl, wobei jeder der letztgenannten beiden Reste unsubstituiert oder durch einen oder mehrere Reste aus der Gruppe Halogen und (C₁-C₄)Alkyl substituiert ist, und Phenyl, Phenylthio (= Phenylsulfanyl), Phenylsulfinyl, Phenylsulfonyl, wobei jeder der letztgenannten 4 Reste unsubstituiert oder durch einen oder mehrere Reste aus der Gruppe Halogen, (C₁-C₄)Alkyl, (C₁-C₄)Alkoxy und (C₁-C₄)Haloalkyl substituiert ist, substituiert ist,
bedeutet.

Weiter bevorzugt bedeutet
R⁴ auch einen mehrcyclischen Rest auf Basis von (C₃-C₉)Cycloalkyl, (C₅-C₉)Cycloalkenyl, (C₅-C₉)Cycloalkinyl oder Phenyl, wobei der Basisring mit einem carbocyclischen oder heterocyclischen Ring, vorzugsweise einem 5- oder 6-gliedrigen Ring mit 0 oder 1 bis 3 Heteroringatomen aus der Gruppe N, O und S kondensiert ist, vorzugsweise benzokondensiert ist, und wobei der Basisring oder das mehrcyclische System unsubstituiert oder durch einen oder mehrere Reste aus der Gruppe Halogen, Cyano, Nitro, (C₁-C₆)Alkyl, (C₁-C₆)Haloalkyl, (C₁-C₄)Alkoxy-(C₁-C₄)alkyl, (C₂-C₄)Alkenyl, (C₂-C₄)Alkinyl, (C₁-C₄)Alkoxy, (C₁-C₄)Haloalkoxy, (C₁-C₄)Alkoxy-(C₁-C₄)alkoxy, (C₁-C₄)Alkylthio, (C₃-C₆)Cycloalkyl, (C₃-C₆)Cycloalkoxy, [(C₁-C₄)Alkoxy]-carbonyl und [(C₁-C₄)Haloalkoxy]-carbonyl substituiert ist.

Bevorzugt sind auch Verbindungen (I), vorzugsweise der Formel (Ia), oder deren Salze, worin
R⁴ einen gesättigten, teilweise ungesättigten oder heteroaromatischen Heterocyclylrest mit 3 bis 9 Ringatomen, vorzugsweise mit 5 oder 6 Ringatomen, der 1 bis 4 Heteroatome, vorzugsweise 1 bis 3 Heteroringatome aus der Gruppe N, O und S enthält und der unsubstituiert oder durch einen oder mehrere Reste aus der Gruppe Halogen, Cyano, Thio, Nitro, Hydroxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆ )Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
bedeutet.

Bevorzugt sind auch Verbindungen (I), vorzugsweise der Formel (Ia), oder deren Salze, worin
R⁴ einen Rest der Formel SiR^{a}R^{b}R^{c}, -NR^{a}R^{b} oder -N=CR^{c}R^{d}, vorzugsweise der Formel -NR^{a}R^{b} oder -N=CR^{c}R^{d},
wobei in den letztgenannten 5 Formeln jeder der Reste R^{a}, R^{b}, R^{c} und R^{d} unabhängig voneinander Wasserstoff, (C₁-C₄)Alkyl, (C₂-C₄)Alkenyl, (C₂-C₄)Alkinyl, Benzyl, substituiertes Benzyl, Phenyl oder substituiertes Phenyl bedeutet, dabei aber SiH₃ für SiR^{a}R^{b}R^{c} ausgenommen ist, oder R^{a} und R^{b} zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeuten oder R^{c} und R^{d} zusammen mit dem C-Atom einen 3- bis 8-gliedrigen carbocyclischen Rest oder heterocyclischen Rest, welcher 1 bis 3 Heteroringatome aus der Gruppe N, O und S enthalten kann, wobei der carbocyclische oder heterocyclischen Rest unsubstituiert oder durch einen oder mehrere Reste aus der Gruppe (C₁-C₄)Alkyl und (C₁-C₄)Haloalkyl substituiert ist, bedeuten,
bedeutet.

Besonders bevorzugt sind auch Verbindungen (I), vorzugsweise der Formel (Ia), oder deren Salze, worin
R⁴ H, Methyl, Ethyl, n-Propyl, i-Propyl, n-Butyl, s-Butyl, i-Butyl, t-Butyl, Allyl, Ethinyl, Propargyl (Prop-2-in-1-yl), Prop-1-in-1-yl, But-2-in-1-yl, But-3-in-1-yl, 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2-Methylprop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, 1- (2E)-1-methylbut-2-en-1-yl, (E)-Pent-3-en-2-yl oder (Z)-Pent-3-en-2-yl,
Phenyl, 2-Carboxy-phenyl, 2-Chlorphenyl, 3-Chlorphenyl, 4-Chlorphenyl, 2-Fluorphenyl, 3-Fluorphenyl, 4-Fluorphenyl, 3-Methoxyphenyl, 4-Methoxyphenyl, 2-Methoxyphenyl, 3-Methoxyphenyl, 4-Methoxyphenyl, Benzyl, 2-Fluoro-benzyl, 2-Fluoro-benzyl, 3-Fluoro-benzyl, 4-Fluoro-benzyl, 2,3-Difluoro-benzyl, 2,4-Difluoro-benzyl, 2,5-Difluoro-benzyl, 2,6-Difluoro-benzyl, 3,4-Difluoro-benzyl, 3,5-Difluoro-benzyl, 2-Phenyl-ethyl, 1-Phenyl-ethyl, (4-Chlorphenyl)-methyl [d. h. = CH₂(4-Cl-Ph) = 4-Chlorbenzyl], (4-Fluorphenyl)-methyl [d. h. = CH₂(4-F-Ph)], (4-Methoxyphenyl)-methyl [d. h. = CH₂(4-OMe-Ph)], 2-Phenoxy-ethyl, 2-Phenylthio-ethyl [= 2-(Phenylsulfanyl)-ethyl], 2-Phenylsulfinyl-ethyl, 2-Phenylsulfonyl-ethyl,
Trifluormethyl, Difluormethyl, Fluormethyl, Trichlormethyl, Dichlormethyl, Chlormethyl,
Methoxy-methyl, 2-Methoxyethyl, 2,2,2-Trifluorethyl, 1,1,1-Trifluorprop-2-yl, 2,2-Difluorethyl, 1,3-Difluorprop-2-yl, 2,3-Dimethoxypropyl, 2,3-Dimethoxyprop-2-yl, 2,2-Dimethoxy-eth-2-yl, 2-(2,2,2-Trifluorethoxy)-ethyl, 2-Fluorethyl, 2-Chlorethyl, 2-Bromethyl, 2-lodethyl, 2,2,3,3,3-Pentafluorpropyl, 1-Hydroxy-prop-2-yl, 2-Hydroxy-prop-2-yl, 2-Hydroxy-prop-1-yl, 3-Hydroxy-propyl, 3-Hydroxy-prop-2-yl, (2-Methoxyethoxy)-methyl; 2-(2-Methoxyethoxy)-ethyl; (2-Ethoxyethoxy)-methyl; 2-(2-Ethoxyethoxy)-ethyl,
(Acetoxy)-methyl, (Propanoyloxy)-methyl, (2-Methylpropanoyloxy)-methyl, (2,2-Dimethylpropanoyloxy)-methyl, 1-(Acetoxy)-ethyl, 2-(Acetoxy)-ethyl, 2-(Propanoyloxy)-ethyl, 1-(Propanoyloxy)-ethyl, 1-(2-Methylpropanoyloxy)-eth-1-yl, 2-(2-Methylpropanoyloxy)-eth-1-yl, 2-(2,2-Dimethylpropanoyloxy)-ethyl [d. h. 1-(t-Butylcarbonyloxy)-ethyl], 2-(2,2-Dimethylpropanoyloxy)-ethyl; 1-(2,2-Dimethylpropanoyloxy)-2-methylprop-1-yl, 1-(t-Butylcarbonyloxy)-2-methylprop-1-yl,
(Methoxycarbonyl)-methyl, (Ethoxycarbonyl)-methyl, (n-Propoxycarbonyl)-methyl, (i-Propoxycarbonyl)-methyl, (n-Butoxycarbonyl)-methyl, (s-Butoxycarbonyl)-methyl, (i-Butoxycarbonyl)-methyl, (t-Butoxycarbonyl)-methyl, 1-(Methoxycarbonyl)-ethyl, 2-(Methoxycarbonyl)-ethyl, 1-(Ethoxycarbonyl)-ethyl, 2-(Ethoxycarbonyl)-ethyl, 1-(n-Propoxycarbonyl)-ethyl, 2-(n-Propoxycarbonyl)-ethyl, 1-(i-Propoxycarbonyl)-ethyl, 2-(i-Propoxycarbonyl)-ethyl, 1-(n-Butoxycarbonyl)-ethyl, 2-(n-Butoxycarbonyl)-ethyl, 1-(s-Butoxycarbonyl)-ethyl, 2-(s-Butoxycarbonyl)-ethyl, 1-(i-Butoxycarbonyl)-ethyl, 2-(i-Butoxycarbonyl)-ethyl, 1-(t-Butoxycarbonyl)-ethyl, 2-(t-Butoxycarbonyl)-ethyl, (Methoxycarbonyloxy)-methyl, (Ethoxycarbonyloxy)-methyl, (n-Propoxycarbonyloxy)-methyl, (i-Propoxycarbonyloxy)-methyl, (n-Butoxycarbonyloxy)-methyl, (s-Butoxycarbonyloxy)-methyl, (i-Butoxycarbonyloxy)-methyl, (t-Butoxycarbonyloxy)-methyl, 1-(Methoxycarbonyloxy)-ethyl, 2-(Methoxycarbonyloxy)-ethyl, 1-(Ethoxycarbonyloxy)-ethyl, 2-(Ethoxycarbonyloxy)-ethyl, 1-(n-Propoxycarbonyloxy)-ethyl, 2-(n-Propoxycarbonyloxy)-ethyl, 1-(i-Propoxycarbonyloxy)-ethyl, 2-(i-Propoxycarbonyloxy)-ethyl, 1-(n-Butoxycarbonyloxy)-ethyl, 2-(n-Butoxycarbonyloxy)-ethyl, 1-(s-Butoxycarbonyloxy)-ethyl, 2-(s-Butoxycarbonyloxy)-ethyl, 1-(i-Butoxycarbonyloxy)-ethyl, 2-(i-Butoxycarbonyloxy)-ethyl, 1-(t-Butoxycarbonyloxy)-ethyl, 2-(t-Butoxycarbonyloxy)-ethyl, (Cyclohexoxycarbonyloxy)-methyl, 1-(Cyclohexoxycarbonyloxy)-eth-1-yl, 2-(Cyclohexoxycarbonyloxy)-eth-1-yl,
(Acetyl)-methyl, 1-(Acetyl)-ethyl, 2-(Acetyl)-ethyl, 1-(Acetyl)-propyl, 2-(Acetyl)-propyl, 3-(Acetyl)-propyl, (Propanoyl)-methyl, 1-(Propanoyl)-ethyl, 2-(Propanoyl)-ethyl, 1-(Propanoyl)-propyl, 2-(Propanoyl)-propyl, 3-(Propanoyl)-propyl, 1-(Propanoyl)-2-methyl-propyl,
2-Ethylthio-ethyl [= 2-(Ethylsulfanyl)-ethyl], 2-(Ethylsulfinyl)-ethyl, 2-(Ethylsulfonyl)-ethyl,
2-(Ethylidenaminooxy)-ethyl, 2-(Prop-2-ylidenaminooxy)-ethyl, 2-(But-2-ylidenaminooxy)-ethyl, 2-(Pent-3-ylidenaminooxy)-ethyl, (N,N-Dimethylamino)-methyl, 2-(N,N-Dimethylamino)-eth-1-yl, 1-(N,N-Dimethylamino)-eth-1-yl, 2-(N,N-Diethylamino)-eth-1-yl, 1-(N,N-Diethylamino)-eth-1-yl, (N,N-Diethylamino)-methyl,
(N,N-Dimethylaminocarbonyl)-methyl, 1-(N,N-Dimethylaminocarbonyl)-ethyl, 2-(N,N-Dimethylaminocarbonyl)-ethyl, (N,N-Diethylaminocarbonyl)-methyl, 1-(N,N-Diethylaminocarbonyl)-ethyl, 2-(N,N-Diethylaminocarbonyl)-ethyl, 1-(Dimethylamino)-prop-2-yl [d. h. 2-(Dimethylamino)-1-methyl-ethyl], 1-(Diethylamino)-prop-2-yl,
Trimethylsilyl-methyl, 1-(Trimethylsilyl)-ethyl, 2-(Trimethylsilyl)-ethyl, Triethylsilyl-methyl, 1-(Triethylsilyl)-ethyl, 2-(Triethylsilyl)-ethyl, Cyclopropyl, Cyclopropylmethyl, 1-Cyclopropyl-ethyl, 2-Cyclopropyl-ethyl, (1-Methyl-cyclopropyl)-methyl, 1-(1-Methyl-cyclopropyl)-ethyl, 2-(1-Methyl-cyclopropyl)-ethyl, (2,2-Dichlorcyclopropyl)-methyl, 1-(2,2-Dichlorcyclopropyl)-ethyl, 2-(2,2-Dichlorcyclopropyl)-ethyl, (2,2-Dimethyl-cyclopropyl)-methyl, 1-(2,2-Dimethyl-cyclopropyl)-ethyl, 2-(2,2-Dimethyl-cyclopropyl)-ethyl, Cyclobutylmethyl, Cyclopentyl-methyl, Cyclohexyl-methyl oder
Pyrid-2-yl, Pyrid-3-yl, Pyrid-4-yl, 2-Chlorpyrid-3-yl, 3-Chlorpyrid-2-yl, Thien-2-yl, Thien-3-yl, 2-Chlorthien-3-yl, 3-Chlorthien-2-yl, 4-Chlorthien-2-yl, Thietan-3-yl,
(1-Ethyl-5-methyl-1 H-pyrazol-4-yl)-methyl, 1-(1-Ethyl-5-methyl-1 H-pyrazol-4-yl)-ethyl, 2-(1-Ethyl-5-methyl-1 H-pyrazol-4-yl)-ethyl
(1-Ethyl-3-methyl-1 H-pyrazol-4-yl)-methyl, 1-(1-Ethyl-3-methyl-1 H-pyrazol-4-yl)-ethyl, 2-(1-Ethyl-3-methyl-1 H-pyrazol-4-yl)-ethyl,
Tetrahydrofuran-2-yl, Tetrahydrofuran-3-yl, Tetrahydrofuran-2-yl-methyl, Tetrahydrofuran-3-yl-methyl, (5-Methyl-2-oxo-1,3-dioxol-4-yl)methyl; Oxetan-3-yl, (Oxetan-3-yl)methyl, (Oxetan-2-yl)methyl, (1,3-Dioxolan-2-yl)-methyl, (1,3-Dioxolan-4-yl)-methyl, 5-Methyl-2-oxo-1,3-dioxolan-4-yl)methyl, (Morpholin-4-yl)-methyl; 1-(Morpholin-4-yl)-ethyl, 2-(Morpholin-4-yl)-ethyl, 2,3-Dihydro-1 H-inden-2-yl, Dihydro-1 H-inden-3-yl, Dihydro-1 H-inden-4-yl, Dihydro-1H-inden-5-yl,
1H-Inden-2-yl, 1H-Inden-3-yl, 1H-Inden-4-yl, 1H-Inden-5-yl, 1H-Inden-6-yl oder 1H-Inden-7-yl
bedeutet.

Ganz besonders bevorzugt sind dabei Verbindungen (I), vorzugsweise der Formel (Ia), und deren Salze, worin
R⁴ H, Methyl, Ethyl, n-Propyl, i-Propyl, n-Butyl, s-Butyl, i-Butyl, t-Butyl, Phenyl, Benzyl, CH₂(4-Cl-Ph), d. h. (4-Chlorphenyl)-methyl, CH₂(4-F-Ph), d. h. (4-Fluorphenyl)-methyl, CH₂(4-OMe-Ph), d. h. (4-Methoxyphenyl)-methyl, 2-Fluoro-benzyl, 2-Fluoro-benzyl, 3-Fluoro-benzyl, 4-Fluoro-benzyl, 2,3-Difluoro-benzyl, 2,4-Difluoro-benzyl, 2,5-Difluoro-benzyl, 2,6-Difluoro-benzyl, 3,4-Difluoro-benzyl, 3,5-Difluoro-benzyl, 2-Phenoxy-ethyl, 2-Ethylthio-ethyl, 2-Ethylsulfinylethyl, 2-Ethylsulfonyl-ethyl, 2-Phenylthio-ethyl, 2-Phenylsulfinyl-ethyl, 2-Phenylsulfonyl-ethyl, Methoxymethyl, 2-Methoxyethyl, Tetrahydrofuran-2-yl-methyl, 2-(Dimethylamino)ethyl, Oxetan-3-yl, (3-Methyloxetan-3-yl)methyl, Thietan-3-yl, Trifluormethyl, Difluor-methyl, 2,2,2-Trifluorethyl, 2,2-Difluorethyl, 2-Fluorethyl, 2,2,3,3,3-Pentafluorpropyl, Cyclopropylmethyl, 1-Cyclopropyl-ethyl, (1-Methyl-cyclopropyl)-methyl, (2,2-Dichlorcyclopropyl)-methyl, (2,2-Dimethyl-cyclopropyl)-methyl, Tetrahydrofuran-2-yl-methyl, Allyl, Ethinyl, Propargyl (= Prop-2-in-1-yl), Prop-1-in-1-yl, 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2-Methylprop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, 1- (2E)-1-methylbut-2-en-1-yl, (E)-Pent-3-en-2-yl oder (Z)-Pent-3-en-2-yl, Cyclobutylmethyl, Cyclopentylmethyl, Cyclohexylmethyl oder 1-Ethyl-5-methyl-1 H-pyrazol-4-methyl, d. h. (1-Ethyl-5-methyl-1 H-pyrazol-4-yl)-methyl,
bedeutet.

Ganz besonders bevorzugt sind dabei Verbindungen (I), vorzugsweise der Formel (Ia), und deren Salze, worin
R⁴ H, Methyl, Ethyl n-Butyl, s-Butyl, i-Butyl, t-Butyl, Allyl und Propargyl, insbesondere Methyl oder Ethyl bedeutet.

Die Erfindung umfasst auch alle Tautomeren, wie Keto- und Enol-Tautomeren, und deren Mischungen und Salze, wenn entsprechende funktionelle Gruppen vorhanden sind.

Gegenstand der vorliegenden Erfindung sind auch Verfahren zur Herstellung der Verbindungen der allgemeinen Formel (I) oder (Ia) und/oder deren Salze. Dazu gehören Verfahren, die analog bekannten Methoden durchgeführt werden können.

Zur Herstellung der erfindungsgemäßen Verbindungen (I) können zunächst die entsprechenden Diastereomerengemische in Form ihrer racemischen Gemische verwendet werden. Die Herstellung der Diastereomerengemische der Cyanobutyrate ist im Prinzip bekannt; siehe beispielsweise EP-A 5341, EP-A 266725, EP-A270 830, JP 04/297454, JP 04/297455, JP 05/058979, WO 2011/003776, WO 2011/003775, WO 2011/042378, WO 2011/073143, WO 2011/098417.
Analog den in den zitierten Schriften beschriebenen Syntheserouten lassen sich die Verbindungen nach Standardverfahren der organischen Chemie hergestellen.

Beispielsweise werden Diastereomerengemische der Verbindungen der Formel (I)] mit einem Gehalt an der herzustellenden Verbindung (I) erhalten, dadurch gekennzeichnet, dass man
(a) Verbindungen der Formel (II) ("Cyanomethylpyridine"/"Pyridylacetonitrile") 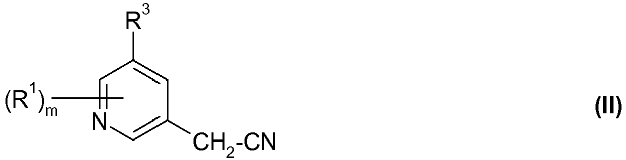 mit Verbindungen der Formel (III) (Zimtsäurederivaten) oder deren Salzen, 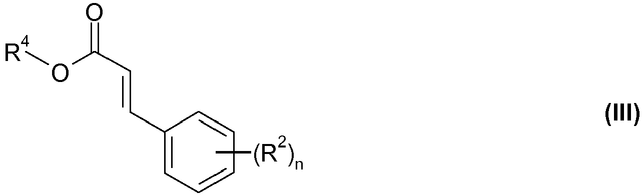 zu Verbindungen der Formel (I) (Diastereomere/racemisch) 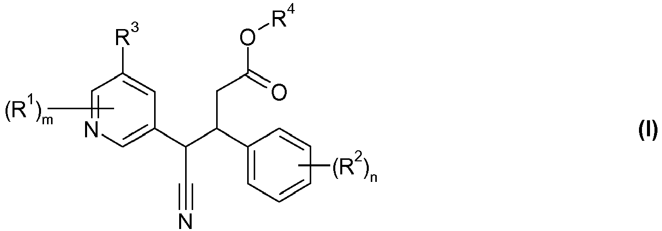 umsetzt, wobei R¹, R², R³, R⁴, m und n in den Verbindungen (II) und (III) wie in der jeweils herzustellenden Verbindung der Formel (I) definiert sind.

Im obengenannten und den nachfolgenden Verfahren werden partiell Lösemittel (gleichbedeutend mit "Lösungsmittel") verwendet. In diesem Zusammenhang bezeichnen "inerte Lösemittel" jeweils Lösemittel, die unter den jeweiligen Reaktionsbedingungen inert sind, jedoch nicht unter beliebigen Reaktionsbedingungen inert sein müssen.
Die jeweils beschriebenen Verfahren können in Apparaturen, wie sie im Labor, Technikum und in Anlagen für die Herstellung kommerzieller Mengen und großtechnischer Verfahren üblich sind oder alternativ auch in einem Mikrowellenofen durchgeführt werden.

Die für die Herstellung der Verbindungen (I) benötigten Ausgangsstoffe (III) sind gemäß der oben zitierten Literatur bekannt oder können analog der zitierten Literatur hergestellt werden.

Die für die Herstellung der Verbindungen (I) benötigten Ausgangsstoffe (II) sind kommerziell erhältlich oder in der Literatur beschrieben oder können analog in der Literatur beschriebener Verfahren hergestellt werden.

Die Umsetzung nach Variante (a) kann beispielsweise nach Methoden und unter Bedingungen, wie sie für Michael-Additionen bekannt sind, durchgeführt werden. Die Umsetzung erfolgt beispielsweise bei Temperaturen von -100°C bis 150°C, vorzugsweise -78°C bis 100°C, in einem organischen oder anorganischen Lösungsmittel, in der Regel in Gegenwart einer Base oder eines Katalysators oder beidem [vgl. J. Chem. Soc. (1945), S. 438].
Geeignete Lösungsmittel sind beispielsweise organische Lösungsmittel wie:
- aliphatische Kohlenwasserstoffe wie Pentan, Hexan, Cyclohexan oder Petrolether;
- aromatische Kohlenwasserstoffe wie Toluol, o-, m- oder p-Xylol,
- halogenierte Kohlenwasserstoffe wie Methylenchlorid, Chloroform oder Chlorbenzol,
- Ether wie Diethylether, Diisopropylether, tert.-Butylmethylether, Dioxan, Anisol und Tetrahydrofuran (THF),
- Nitrile wie Acetonitril oder Propionitril,
- Ketone wie Aceton, Methylethylketon, Diethylketon oder tert.-Butylmethylketon,
- Alkohole wie Methanol, Ethanol, n-Propanol, Isopropanol, n-Butanol und tert.-Butanol, sowie
- Dimethylsulfoxid, Dimethylformamid, Dimethylacetamid, Sulfolan,
- Gemische aus den genannten organischen Lösungsmitteln.

Geeignet sind in Einzelfällen auch anorganische Lösungsmittel wie Wasser oder Gemische organischer Lösungsmittel mit Wasser.
Bevorzugte Lösungsmittel sind THF, Toluol und Methanol und deren Gemische mit anderen organischen Lösungsmitteln.
Die Umsetzung nach Herstellungsvariante (a) erfolgt vorzugsweise in Gegenwart einer Base, beispielsweise aus der Gruppe anorganischer Verbindungen wie der Alkalimetall- und Erdalkalimetallhydroxide, zum Beispiel (z. B.) Lithiumhydroxid, Natriumhydroxid, Kaliumhydroxid oder Calziumhydroxid, der Alkalimetall- und Erdalkalimetalloxide, zum Beispiel Lithiumoxid, Natriumoxid, Calziumoxid oder Magnesiumoxid, der Alkalimetall- und Erdalkalimetallhydride, z. B. Lithiumhydrid, Natriumhydrid, Kaliumhydrid oder Calziumhydrid, der Alkalimetallamide, zum Beispiel Lithiumamid, Natriumamid oder Kaliumamid, der Alkalimetall- und Erdalkalimetallcarbonate, z. B. Lithiumcarbonat, Kaliumcarbonat oder Calziumcarbonat, der Alkalimetallhydrogencarbonate, z. B. Natriumhydrogencarbonat, oder der metallorganischen Verbindungen wie vorzugsweise der Alkalimetallalkyle, z. B. Methyllithium, Butyllithium oder Phenyllithium, der Alkylmagnesiumhalogenide, z. B. Methylmagnesiumchlorid, oder der Alkalimetall- und Erdalkalimetallalkoholate, z. B. Natriummethanolat, Natriumethanolat, Kaliumethanolat, Kalium- tert.Butanolat oder Dimethoxymagnesium.
Auch können als Basen organische Basen eingesetzt werden, beispielsweise aus der Gruppe der tertiären aliphatischen Amine, z. B. Trimethylamin, Triethylamin, Tributylamin, Di-isopropylethylamin oder N-Methylpiperidin, oder der aromatischen tertiären Amine, z. B. Pyridin oder substituierte Pyridine wie Collidin, Lutidin oder 4-Dimethylaminopyridin, oder der bicyclische Amine wie 7-Methyl-1,5,7-triazabicyclo[4.4.0]- dec-5-en oder 1,8-Diazabicyclo[5.4.0]undec-7en (DBU). Bevorzugte Basen sind beispielsweise Kalium-tert.Butanolat, Lithium-bis(trimethylsilyl)amid, DBU oder 7-Methyl-1,5,7-triazabicyclo[4.4.0]- dec-5-en.
Die Menge an Base kann im Allgemeinen breit variiert werden. Beispielsweise kann es sinnvoll sein, die Base in katalytischen Mengen, im Unterschuss, äquimolar oder im Überschuss einzusetzen. Eine vorzugsweise flüssige organische Base kann gegebenenfalls auch als Lösungsmittel verwendet werden.

Geeignete Katalysatoren für die Michael-Addition nach Variante (a) sind saure Katalysatoren, beispielsweise aus der Gruppe der anorganischen Säuren, z. B. Broensted-Säuren, wie Fluorwasserstoffsäure, Salzsäure, Bromwasserstoffsäure, Schwefelsäure oder Perchlorsäure, oder Lewis-Säuren wie Bortrifluorid, Aluminiumtrichlorid, Eisen-III-chlorid, Zinn-IV-chlorid, Titan-IV-chlorid, Scandium-IIItriflat oder Zink-II-chlorid, sowie der organischen Säuren, z. B. Ameisensäure, Essigsäure, Propionsäure, Oxalsäure, Toluolsulfonsäure, Benzolsulfonsäure, Camphersulfonsäure, Zitronensäure oder Trifluoressigsäure.

Die Menge an saurem Katalysator kann im Allgemeinen breit variiert werden. Beispielsweise kann es sinnvoll sein, die Säure in katalytischen Mengen, im Unterschuss, äquimolar oder im Überschuss einzusetzen. Eine vorzugsweise flüssige Säure kann gegebenenfalls auch als Lösungsmittel verwendet werden.

Variante (a1) zur Herstellung von Zwischenverbindungen der Formel (II): Verbindungen der Formel (II) und deren Salze, in denen R¹, R³, und m wie in der jeweils herzustellenden Verbindung der Formel (I) definiert sind, werden beispielsweise auch nach Verfahren [Variante (a1)] erhalten, dadurch gekennzeichnet, dass man eine Pyridincarbonsäure der Formel (IV) oder deren Ester der Formel (V), 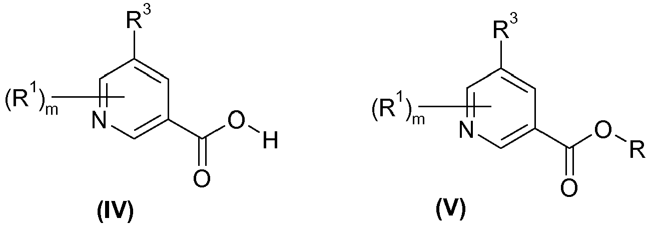 wobei in Formel (V) der Rest R einen Kohlenwasserstoffrest, vorzugsweise (C₁-C₆)Alkyl, (C₁-C₆)Haloalkyl oder (C₁-C₄)Alkoxy-(C₁-C₄)alkyl, insbesondere Methyl oder Ethyl bedeutet,
zur Verbindung der Formel (VI) reduziert (= Reduktion an der Säure- bzw. Estergruppe), 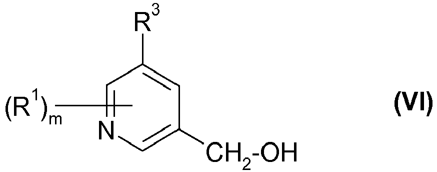 und die Verbindung (VI) zur Verbindung (VII) umsetzt, 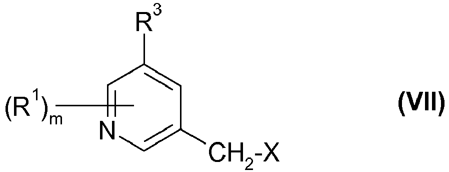 wobei X eine Abgangsgruppe darstellt, beispielsweise Halogen, wie Chlor, Brom oder lod, erhalten durch eine Halogenierungsreaktion, wie beispielsweise Substitution mit Halogenid, oder Sulfonate der Formel -O-SO₂-R (R = (C₁-C₆)Alkyl, (C₁-C₆)Haloalkyl, Phenyl, das gegebenfalls substituiert ist, beispielsweise durch Halogen, Nitro oder Alkyl substituiert ist), wie beispielsweise Mesylat (R = Methyl), Triflat (R = CF₃), Tosylat (R = p-Tolyl), Bromylat (R = p-Bromphenyl), Nosylat (R = p-Nitrophenyl), erhalten durch Veresterung eines entsprechenden Sulfonsäurechlorids mit Verbindungen der Formel (VI),
und die Verbindung (VII) zur Verbindung (II) durch Substitution mit Cyanid umsetzt, 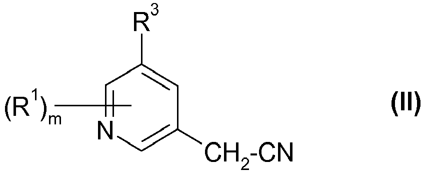 wobei R¹, R³ und m in den Formeln (IV), (V), (VI) und (VII) wie in Formel (II) bzw. Formel (I) definiert sind.

Die einzelnen Syntheseschritte nach Variante (a1) sind dem Fachmann grundsätzlich bekannt und beispielsweise in Acta Pharm. Suecica 1972, 9, 411-418; J. Org. Chem. 1999, 64 (23), 8576-8581; Synthesis 1998, 9, 1335-1338; J. Med. Chem. 2000, 43 (18) 3386-3399; US5225423 (Shell Research Limited 1993), US 4966902 (Hoechst AG, 1990); WO2007/045989 (Pfizer Limited, 2007); US2004/242572 (Boehringer Ingelheim International GmbH, 2004) und dort zitierter Literatur beschrieben.

Diastereomerengemische bzw. racemische Diastereomere der Verbindungen der Formel (I) können nach Variante (b) durch Umesterung erhalten werden, dadurch gekennzeichnet, dass man
(b) Verbindungen der Formel (I*), 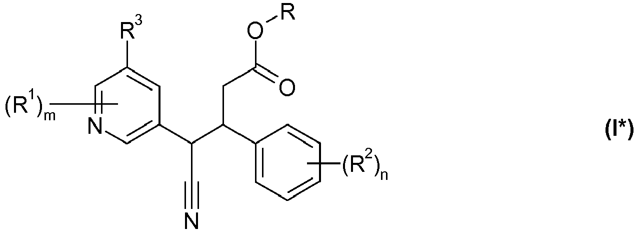 in denen R ein Rest aus der Gruppe der für R⁴ möglichen Reste bedeutet aber vom Rest R⁴ in der herzustellenden Verbindung (I) verschieden ist, mit einer Verbindung der Formel R⁴-OH, in der R⁴ wie in Formel (I) definiert ist, zur Verbindung (I) umsetzt, wobei R¹, R², R³, m und n in der Verbindung (I*) wie in der jeweils herzustellenden Verbindung der Formel (I) definiert sind.

In einer besonderen Ausführungsform lassen sich nach Variante (b1) auch stereochemisch angereicherte Verbindungen der oben genannten Formel (Ia) als Verbindungen (I) erhalten, dadurch gekennzeichnet, dass man
(b1) stereochemisch angereicherte Verbindungen der Formel (Ia*), die stereochemisch entsprechend [d. h. mindestens ebenso angereichert sind, wie in der gewünschten Verbindung (Ia)] 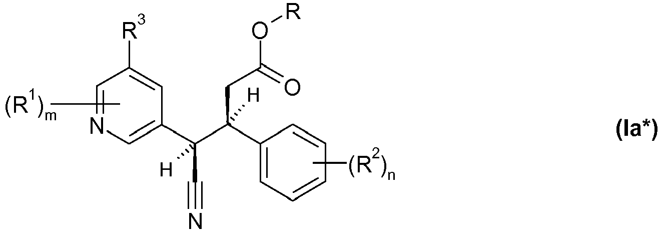 in denen R ein Rest aus der Gruppe der für R⁴ möglichen Reste bedeutet aber vom Rest R⁴ in der herzustellenden Verbindung (Ia) verschieden ist, mit einer Verbindung der Formel R⁴-OH, in der R⁴ wie in der herzustellenden Verbindung der Formel (Ia) definiert ist, umsetzt.

Die Umesterungen (b) und (b1) können beispielsweise mit einem geeigneten Alkohol R⁴-OH in Gegenwart eines Katalysators, gegebenenfalls in Gegenwart eines aprotischen Lösungsmittels erfolgen. Weiterhin sind in der Regel Bedingungen vorteilhaft, mit denen das chemische Gleichgewicht auf die Seite des gewünschten Produkts verschoben wird, beispielsweise mit einem großen Überschuss des Alkohols R⁴-OH unter praktisch wasserfreien Bedingungen, z. B. in Gegenwart eines Molekularsiebs.

Die Umsetzungen (Umesterungen) können in der Regel bei Temperaturen von 0°C bis180°C, vorzugsweise 20°C bis 100°C in Gegenwart einer Lewis- bzw. Broenstedt-Säure oder eines Enzyms durchgeführt werden [vgl. J. Org. Chem. 2002, 67, 431].

Geeignete Lösungsmittel sind z. B. organische aprotische Lösungsmittel wie:
- aliphatische Kohlenwasserstoffe wie Pentan, Hexan, Cyclohexan oder Petrolether;
- aromatische Kohlenwasserstoffe wie Toluol, o-, m- oder p-Xylol,
- halogenierte Kohlenwasserstoffe wie Methylenchlorid (Dichlormethan), Chloroform oder Chlorbenzol,
- Ether wie Diethylether, Diisopropylether, tert.-Butylmethylether, Dioxan, Anisol oder Tetrahydrofuran (THF),
- Nitrile wie Acetonitril oder Propionitril,
- Ketone wie Aceton, Methylethylketon, Diethylketon oder tert.-Butylmethylketon,
- Dimethylsulfoxid, Dimethylformamid, Dimethylacetamid oder Sulfolan oder
- Gemische aus den genannten organischen Lösungsmitteln.

Bevorzugtes Lösungsmittel ist der Alkohol R⁴-OH, der zugleich als Reaktant für die Umesterung verwendet wird, gegebenenfalls in Kombination mit einem der genannten aprotischen organischen Lösungsmittel.
Alternativ kann man den gewünschten Ester aus einem anderen Ester auch zweistufig durch saures oder basisches Verseifen des anderen Esters zur freien Säure, d. h. zu Verbindungen (I*) oder (Ia*), worin R jeweils H bedeutet, und nachfolgender Veresterung mit einem Alkohol R⁴-OH erhalten.

### Varianten (c) und (c1):

Die Herstellung von Diastereomerengemischen bzw. racemischen Diastereomeren der Formel (I) gemäß Variante (c) oder optisch aktiven Verbindungen (Ia) gemäß Variante (c1) ist demnach dadurch gekennzeichnet, dass man eine Verbindung der der genannten Formel (I*) bzw. Formel (Ia*), worin die Reste R jeweils Wasserstoff bedeuten (freie Carbonsäuren), mit einem Alkohol der Formel R⁴-OH nach üblichen Methoden verestert, gegebenenfalls kombiniert mit einer vorherigen Herstellung (c-1) bzw. (c1-1) der freien Säure aus einem anderen Ester der Formel (I*) bzw. Formel (Ia*), worin die Reste R jeweils ungleich Wasserstoff sind.

Die Veresterung aus der freien Säure der Formel (I*)/R = H bzw. (Ia*)/R = H kann beispielsweise analog üblicher Methoden erfolgen, beispielsweise bei Temperaturen von 0°C bis 120°C, vorzugsweise 20°C bis 50°C, gegebenenfalls in Gegenwart eines Katalysators, in einem weitgehend wasserfreien Medium bzw. unter Bedingungen, in denen Wasser inklusive das bei der Veresterung entstehende Wasser gebunden oder anderweitig entfernt wird. Als Katalysator kommen wasserfreie Säuren und Basen, vorzugsweise organische Säuren oder Basen in Frage; siehe Handbücher für chemische Verfahren zur Veresterung von Carbonsäuren; siehe auch z. B. J. Am. Chem. Soc. 2007,129 (43),13321; J. Org. Chem. 1984,49 (22), 4287.

Geeignete Lösungsmittel für die Veresterung sind die oben zu den Verfahrensvarianten (b) und (b1) genannten aprotischen organischen Lösungsmittel geeignet, inklusive des Alkohols R⁴-OH, der zugleich als Reaktant für die Veresterung verwendet wird, gegebenenfalls in Kombination mit einem der genannten aprotischen organischen Lösungsmittel.

Geeignete Katalysatoren für die Veresterung sind die zur genannten Verfahrensvariante (a) (Michael-Addition) erwähnten Basen oder sauren oder basischen Katalysatoren in wasserfreier Form oder mit möglichst geringem Wasseranteil. Bevorzugte Katalysatoren sind die Basen Lithiumhydroxid, Kaliumcarbonat oder organische Amine wie Pyridine, subsitutierte Pyridine und DBU.

Die der Veresterung gegebenenfalls vorgeschaltete Verseifung [Verfahrensvarianten (c-1) bzw. (c1-1)] anderer Ester der Formeln (I*) bzw. Formel (Ia*), jeweils mit R ungleich H, kann analog üblicher Methoden erfolgen, beispielsweise bei Temperaturen von 0°C bis 120°C, vorzugsweise 20°C bis 50°C, gegebenenfalls in Gegenwart eines Katalysators, in einem wasserhaltigen Medium/Lösungsmittel; siehe Handbücher für chemische Verfahren zur Verseifung von Carbonsäureestern; siehe auch z. B. J. Am. Chem. Soc. 2007,129 (43),13321; J. Org. Chem. 1984,49 (22), 4287.

Geeignete Lösungsmittel für die Verseifung [Verfahrensvarianten (c-1) bzw. (c1-1)] ist Wasser oder ein wasserhaltiges organisches Lösungsmittel, beispielsweise auf Basis der zur genannten Verfahrensvariante (a) (Michael-Addition) erwähnten organischen Lösungsmittel, vorzugsweise Wasser oder polare organische Lösungsmittel mit einem Wassergehalt wie THF.

Geeignete Katalysatoren für die Verseifung sind die zur genannten Verfahrensvariante (a) (Michael-Addition) erwähnten Säuren, Basen oder sauren oder basischen Katalysatoren, jeweils mit einem Wassergehalt. Bevorzugte Katalysatoren sind wässrige Säuren und Basen, insbesondere Basen wie Lithiumhydroxid, Natriumhydroxid, Kaliumcarbonat, Pyridine, substituierte Pyridine und DBU in Gegenwart von organischen Lösungsmitteln.

Die Katalysatoren für die Veresterung oder die Verseifung können im Allgemeinen in katalytischen Mengen eingesetzt werden. Eine Verwendung in größeren Mengen, auch äquimolar oder im molaren Überschuß ist in der Regel auch möglich. Eine Verwendung als Lösungsmittel kommt oftmals auch in Frage.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden.

Sofern die Zwischen- und Endprodukte als Feststoffe erhalten werden, kann die Reinigung auch durch Umkristallisieren oder Digerieren erfolgen. Sofern einzelne Verbindungen (I) bzw. (Ia) nicht auf den voranstehend beschriebenen Wegen zugänglich sind, können sie durch Derivatisierung anderer Verbindungen (I) bzw. (Ia) hergestellt werden.

Zur Herstellung der erfindungsgemäßen erythro-Verbindungen, threo-Verbindungen oder optisch aktiven erythro- oder threo-Verbindungen, vorzugsweise der threo-Verbindungen (Ia) aus den Diastereomerengemischen der Verbindungen (I) ist es erforderlich das jeweilige Diastereomer, z. B. das Threo-Isomer oder das Stereoisomer (Enantiomer) Erythro-1, Erythro-2, Threo-1 oder Threo-2, vorzugsweise Threo-2 aus dem Gemisch der Stereoisomeren entsprechend anzureichern. Ein zweckmäßiges Verfahren beinhaltet daher zunächst eine Isolierung der Erythro-1 und Erythro-2 bzw. Threo-Isomeren Threo-1 und Threo-2 aus dem Diastereomerengemisch der Verbindungen der Formel (I), welches noch die anderen Isomeren enthält, und gegebenenfalls anschließende Racematrennung mit Isolierung oder Anreicherung des gewünschten Enantiomeren aus dem Gemisch mit anderen Enantiomeren.

Die Isolierung der Diastereomeren als racemisches Gemisch kann analog der oben erwähnten üblichen Trenn- und Reinigungsverfahren erfolgen (Diastereomeren-Trennung).
Für die daran anschließende Herstellung von Verbindungen der optisch aktiven Verbindungen (I), vorzugsweise der Formel (Ia), kommen Racemattrennungsmethoden in Frage, die dem Fachmann aus analogen Fällen allgemein bekannt sind (vgl. Handbücher der Stereochemie), z. B. im Anschluss an Verfahren zur Trennung von Gemischen in Diastereomere, z. B. durch physikalische Verfahren wie Kristallisation, Chromatographieverfahren, vor allem Säulenchromatographie und Hochdruckflüssigchromatographie, Destillation, gegebenenfalls unter reduziertem Druck, Extraktion und andere Verfahren, können verbleibende Gemische von Enantiomeren in der Regel durch chromatographische Trennung an chiralen Festphasen getrennt werden. Für präparative Mengen oder im industriellen Maßstab kommen Verfahren wie die Kristallisation diastereomerer Salze, die aus den Diastereomerengemischen mit optisch aktiven Säuren und gegebenenfalls bei vorhandenen sauren Gruppen mit optisch aktiven Basen erhalten werden können, in Frage.

Zur Racemattrennung durch Kristallisation diastereomerer Salze kommen als optisch aktive Säure z. B. Camphersulfonsäure, Camphersäure, Bromcamphersulfonsäure, Chinasäure, Weinsäure, Dibenzoylweinsäure und andere analoge Säure in Betracht; als optisch aktive Basen kommen z. B. Chinin, Cinchonin, Chinidin, Brucin, 1-(S)- oder 1-(R)-Phenylethylamin und andere analoge Basen in Frage.
Die Kristallisationen werden dann meist in wässrigen, alkoholischen oder wässrigorganischen Lösungsmittel durchgeführt, wobei das Diastereomer mit der geringeren Löslichkeit gegebenenfalls nach Animpfen zunächst ausfällt. Das eine Enantiomer der Verbindung der Formel (I) wird danach aus dem ausgefällten Salz oder das andere aus dem Kristallisat durch Ansäuern bzw. mit Base freigesetzt.

Gegenstand der Erfindung ist daher auch das Verfahren zur Herstellung der optisch aktiven Verbindungen (I) [Variant (d)], vorzugsweise (Ia) dadurch gekennzeichet, dass man
(d) mit Verbindungen (I), vorzugsweise den Erythro-Verbindungen oder insbesondere den Threo-Verbindungen der Formel (I), eine Racemattrennung durchführt und das gewünschte Enantiomer, vorzugsweise die Verbindung (Ia), in einer stereochemischen Reinheit von 60 bis 100 %, vorzugsweise 70 bis 100 %, mehr bevorzugt 80 bis 100 % , insbesondere 90 bis 100 %, bezogen auf das vorliegende Gemisch der Erythro- bzw. Threo-Enantiomeren isoliert.

Alternativ zu den genannten Racemattrennungsverfahren eignen sich zur Herstellung der optisch aktiven Enantiomeren, vorzugsweise der Threo-2-Enantiomeren (la) prinzipiell auch enantioselektive Verfahren ausgehend von stereochemisch reinen Ausgangsstoffen.

Zur Herstellung der Säureadditionssalze der Verbindungen der Formel (I) kommen allgemein folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzol und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat oder Kalium-tert.-butylat, oder Ammoniak, Ethanolamin oder quartäres Ammoniumhydroxid der Formel [NRR'R"R"']⁺ OH-.

Eine Kollektion aus Verbindungen der Formel (I), die nach den obengenannten Verfahren synthetisiert werden können, können zusätzlich in parallelisierter Weise hergestellt werden, wobei dies in manueller, teilweise automatisierter oder vollständig automatisierter Weise geschehen kann. Dabei ist es möglich, sowohl die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S. H. DeWitt in "Annual Reports in Combinatorial Chemistry and Molecular Diversity: Automated Synthesis", Band 1, Verlag Escom, 1997, Seite 69 bis 77 beschrieben wird.

Zur parallelisierten Reaktionsdurchführung und Aufarbeitung können eine Reihe von im Handel erhältlichen Geräten verwendet werden wie sie beispielsweise von den Firmen Stem Corporation, Woodrolfe Road, Tollesbury, Essex, CM9 8SE, England oder H + P Labortechnik GmbH, Bruckmannring 28, 85764 Oberschleißheim, Deutschland angeboten werden. Für die parallelisierte Aufreinigung von Verbindungen (I) oder von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA. Die aufgeführten Apparaturen ermöglichen eine modulare Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise von Roboter bedient werden. Derartige Automationssysteme können zum Beispiel von der Firma Zymark Corporation, Zymark Center, Hopkinton, MA 01748, USA bezogen werden.

Neben den beschriebenen Methoden kann die Herstellung von Verbindungen der Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen unterstützte Synthesemethoden sind in der Fachliteratur hinreichend beschrieben, z. B.: Barry A. Bunin in "The Combinatorial Index", Verlag Academic Press, 1998.
Die Verwendung von Festphasen unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci., 1985, 82, 5131 - 5135) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß den hier beschriebenen Verfahren liefert Verbindungen der Formel (I) in Form von Substanzkollektionen oder -bibliotheken. Gegenstand der vorliegenden Erfindung sind daher auch Bibliotheken der Verbindungen der Formel (I), die mindestens zwei Verbindungen der Formel (I) enthalten, und deren Vorprodukte.

Die erfindungsgemäßen Verbindungen der Formel (I) (und/oder deren Salze), oben und im Folgenden zusammen als "erfindungsgemäße Verbindungen", "erfindungsgemäße Verbindungen (I)" oder kurz als "Verbindungen (I)" bezeichnet, weisen eine ausgezeichnete herbizide Wirksamkeit gegen ein breites Spektrum wirtschaftlich wichtiger mono und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt. Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen), den Boden, in dem oder auf dem die Pflanzen wachsen, (z. B. den Boden von Kulturland oder Nicht-Kulturland) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.
Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.
Obgleich die erfindungsgemäßen Verbindungen eine ausgezeichnete herbizide Aktivität gegenüber mono und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen. Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono und dikotylen Kulturen eine große Rolle, da beispielsweise die Lagerbildung hierdurch verringert oder völlig verhindert werden kann.
Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.
Bevorzugt bezüglich transgener Kulturen ist die Anwendung der erfindungsgemäßen Verbindungen und/oder deren Salze in wirtschaftlich bedeutenden transgenen Kulturen von Nutz und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.
Vorzugsweise können die erfindungsgemäßen Verbindungen auch als Herbizide in Nutzpflanzenkulturen eingesetzt werden, welche gegenüber den phytotoxischen Wirkungen der Herbizide resistent sind bzw. gentechnisch resistent gemacht worden sind.
Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.
Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.
Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).
Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996
Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.
Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.
Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.
Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.
So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.
Vorzugsweise können die erfindungsgemäßen Verbindungen (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) und/oder deren Salze als Herbizide zur Bekämpfung von Schadpflanzen in Kulturen von Nutz- oder Zierpflanzen, gegebenenfalls in transgenen Kulturpflanzen.
Bevorzugt ist die Verwendung in Getreide wie Weizen, Gerste, Roggen, Hafer, Hirse und Reis im Vor- oder Nachauflauf, insbesondere im Weizen im Nachauflauf. Bevorzugt ist auch die Verwendung in Mais im Vor- oder Nachauflauf, insbesondere im Mais im Vorauflauf.
Bevorzugt ist auch die Verwendung in Soja im Vor- oder Nachauflauf, insbesondere Soja im Nachauflauf.
Die erfindungsgemäße Verwendung zur Bekämpfung von Schadpflanzen oder zur Wachstumsregulierung von Pflanzen schließt auch den Fall ein, bei dem der Wirkstoff der Formel (I) oder dessen Salz erst nach der Ausbringung auf der Pflanze, in der Pflanze oder im Boden aus einer Vorläufersubstanz ("Prodrug") gebildet wird.

Gegenstand der Erfindung ist auch das Verfahren (Anwendungsverfahren) zur Bekämpfung von Schadpflanzen oder zur Wachstumsregulierung von Pflanzen, dadurch gekennzeichnet, dass man eine wirksame Menge von einer oder mehreren Verbindungen der Formel (I) oder deren Salzen auf die Pflanzen (Schadpflanzen, ggf. zusammen mit den Nutzpflanzen) Pflanzensamen, den Boden, in dem oder auf dem die Pflanzen wachsen, oder die Anbaufläche appliziert.

Die erfindungsgemäßen Verbindungen (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) und/oder deren Salze enthalten.

Die Verbindungen der Formel (I) und/oder deren Salze können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.
Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.
Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.
Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.
Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57. Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I) und/oder dessen Salze.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2006 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminocyclopyrachlor-potassium, Aminocyclopyrachlor-methyl, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bicyclopyrone, Bifenox, Bilanafos, Bilanafossodium, Bispyribac, Bispyribac-sodium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthal-dimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clofencet-potassium, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1 H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofenethyl, Flupoxam, Flupropacil, Flupropanate, Flupyrsulfuron, Flupyrsulfuron-methylsodium, Flurenol, Flurenol-butyl, Fluridone, Flurochloridone, Fluroxypyr, Fluroxypyrmeptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-sodium, Glyphosate, Glyphosate-isopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HW-02, d. h. 1-(Dimethoxyphosphoryl)-ethyl-(2,4-dichlorphenoxy)acetat, Imazamethabenz, Imazamethabenz-methyl, Imazamox, Imazamox-ammonium, Imazapic, Imazapyr, Imazapyr-isopropylammonium, Imazaquin, Imazaquin-ammonium, Imazethapyr, Imazethapyr-ammonium, Imazosulfuron, Inabenfide, Indanofan, Indaziflam, Indolessigsäure (IAA), 4-Indol-3-ylbuttersäure (IBA), lodosulfuron, Iodosulfuron-methylsodium, lofensulfuron, lofensulfuron-sodium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -sodium, Mecoprop, Mecoprop-sodium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-sodium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufenethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobacmethyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosate-trimesium), Sulfosulfuron, SW-065, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triafamone, Triallate, Triasulfuron, Triaziflam, Triazofenamide, Tribenuron, Tribenuronmethyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen: 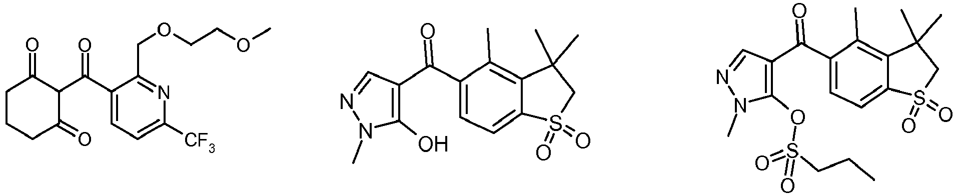 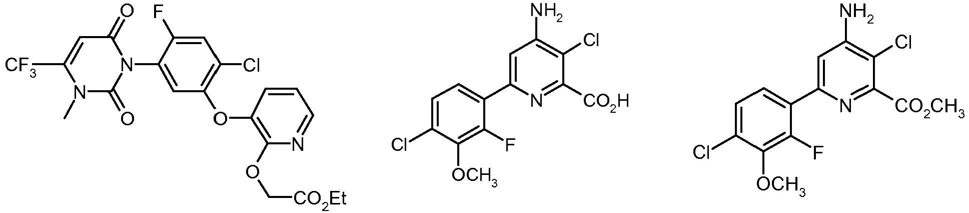
Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide. Folgende Gruppen von Verbindungen kommen beispielsweise als Safener für die Verbindungen (I) und deren Kombinationen mit weiteren Pestiziden in Frage:
A) Verbindungen der Formel (S-I), 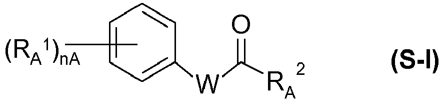 wobei die Symbole und Indizes folgende Bedeutungen haben:
   n_{A} ist eine natürliche Zahl von 0 bis 5, vorzugsweise 0 bis 3;
   R_{A}¹ ist Halogen, (C₁-C₄)Alkyl, (C₁-C₄)Alkoxy, Nitro oder (C₁-C₄)Haloalkyl,
   W_{A} ist ein unsubstituierter oder substituierter divalenter heterocyclischer Rest aus der Gruppe der teilungesättigten oder aromatischen Fünfring-Heterocyclen mit 1 bis 3 Heteroringatomen des Typs N oder O, wobei mindestens ein N-Atom und höchstens ein O-Atom im Ring enthalten ist, vorzugsweise ein Rest aus der Gruppe (W_{A}¹) bis (W_{A}⁴),

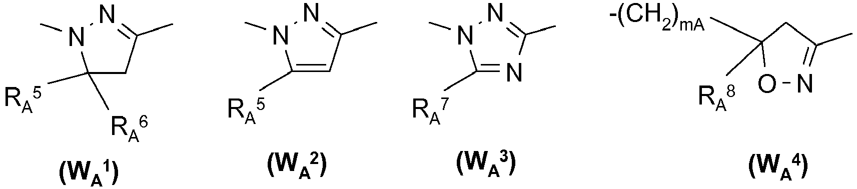

   m_{A} ist 0 oder 1;
   R_{A}² ist OR_{A}³, SR_{A}³ oder NR_{A}³R_{A}⁴ oder ein gesättigter
      oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-I) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   R_{A}³ ist Wasserstoff oder ein unsubstituierter oder substituierter aliphatischer Kohlenwasserstoffrest, vorzugsweise mit insgesamt 1 bis 18 C-Atomen;
   R_{A}⁴ ist Wasserstoff, (C₁-C₆)Alkyl, (C₁-C₆)Alkoxy oder substituiertes oder unsubstituiertes Phenyl;
   R_{A}⁵ ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   R_{A}⁶ R_{A}⁷, R_{A}⁸ sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure, vorzugsweise Verbindungen wie 1-(2,4-Dichlorphenyl)-5-(ethoxycarbonyl)-5-methyl-2-pyrazolin-3-carbonsäure-ethylester (S1-1) ("Mefenpyr-diethyl", siehe Pestic. Man.), und verwandte Verbindungen, wie sie in der WO 91/07874 beschrieben sind;
   b) Derivate der Dichlorphenylpyrazolcarbonsäure, vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4), 1-(2,4-Dichlorphenyl)-5-phenyl-pyrazol-3-carbonsäureethylester (S1-5) und verwandte Verbindungen, wie sie in EP-A-333 131 und EP-A-269 806 beschrieben sind;
   c) Verbindungen vom Typ der Triazolcarbonsäuren, vorzugsweise Verbindungen wie Fenchlorazol(-ethylester), d.h.
      1-(2,4-Dichlorphenyl)-5-trichlormethyl-(1H)-1,2,4-triazol-3-carbonsäureethylester (S1-6), und verwandte Verbindungen wie sie in EP-A-174 562 und EP-A-346 620 beschrieben sind;
   d) Verbindungen vom Typ der 5-Benzyl- oder 5-Phenyl-2-isoxazolin-3- carbonsäure, oder der 5,5-Diphenyl-2-isoxazolin-3-carbonsäure vorzugsweise Verbindungen wie 5-(2,4-Dichlorbenzyl)-2-isoxazolin-3-carbonsäureethylester (S1-7) oder 5-Phenyl-2-isoxazolin-3-carbonsäureethylester (S1-8) und verwandte Verbindungen, wie sie in WO 91/08202 beschrieben sind, bzw. der 5,5-Diphenyl-2-isoxazolin-carbonsäureethylester (S1-9) ("Isoxadifen-ethyl") oder -n-propylester (S1-10) oder der 5-(4-Fluorphenyl)-5-phenyl-2-isoxazolin-3-carbonsäureethylester (S1-11), wie sie in der Patentanmeldung WO-A-95/07897 beschrieben sind.
B) Chinolinderivate der Formel (S-II), 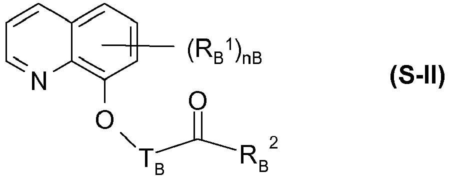 wobei die Symbole und Indizes folgende Bedeutungen haben:
   R_{B}¹ Halogen, (C₁-C₄)Alkyl, (C₁-C₄)Alkoxy, Nitro oder (C₁-C₄)Haloalkyl,
   n_{B} ist eine natürliche Zahl von 0 bis 5, vorzugsweise 0 bis 3;
   R_{B}² OR_{B}³, SR_{B}³ oder NR_{B}³R_{B}⁴ oder ein gesättigter
      oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-II) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   R_{B}³ ist Wasserstoff oder ein unsubstituierter oder substituierter aliphatischer Kohlenwasserstoffrest, vorzugsweise mit insgesamt 1 bis 18 C-Atomen;
   R_{B}⁴ ist Wasserstoff, (C₁-C₆)Alkyl, (C₁-C₆)Alkoxy oder substituiertes oder unsubstituiertes Phenyl;
   T_{B} ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2), vorzugsweise (5-Chlor-8-chinolinoxy)essigsäure-(1-methylhexyl)-ester (Common name "Cloquintocet-mexyl" (S2-1) (siehe Pestic. Man.), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie deren Hydrate und Salze wie sie in der WO-A-2002/034048 beschrieben sind.
   b) Verbindungen vom Typ der (5-Chlor-8-chinolinoxy)malonsäure, vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
C) Verbindungen der Formel (S-III) 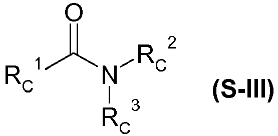 wobei die Symbole und Indizes folgende Bedeutungen haben:
   R_{c}¹ ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   R_{c}², R_{c}³ ist gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{c}² und R_{c}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B.
   "Dichlormid" (siehe Pestic.Man.) (= N,N-Diallyl-2,2-dichloracetamid),
   "R-29148" (= 3-Dichloracetyl-2,2,5-trimethyl-1,3-oxazolidin von der Firma Stauffer),
   "R-28725" (= 3-Dichloracetyl-2,2,-dimethyl-1,3-oxazolidin von der Firma Stauffer),
   "Benoxacor" (siehe Pestic. Man.) (= 4-Dichloracetyl-3,4-dihydro-3-methyl-2H-1,4-benzoxazin),
   "PPG-1292" (= N-Allyl-N-[(1,3-dioxolan-2-yl)methyl]-dichloracetamid von der Firma PPG Industries),
   "DKA-24" (= N-Allyl-N-[(allylaminocarbonyl)methyl]-dichloracetamid von der Firma Sagro-Chem),
   "AD-67" oder "MON 4660" (= 3-Dichloracetyl-1-oxa-3-aza-spiro[4,5]decan von der Firma Nitrokemia bzw. Monsanto),
   "TI-35" (= 1-Dichloracetyl-azepan von der Firma TRI-Chemical RT) "Diclonon" (Dicyclonon) oder "BAS145138" oder "LAB145138" (= 3-Dichloracetyl-2,5,5-trimethyl-1,3-diazabicyclo[4.3.0]nonan von der Firma BASF) und
   "Furilazol" oder "MON 13900" (siehe Pestic. Man.) (= (RS)-3-Dichloracetyl-5-(2-furyl)-2,2-dimethyloxazolidin)
D) N-Acylsulfonamide der Formel (S-IV) und ihre Salze, 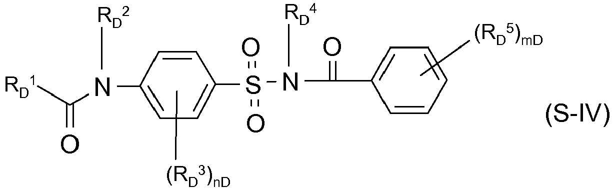 worin
   R_{D}¹ Wasserstoff, einen Kohlenwasserstoffrest, einen Kohlenwasserstoffoxyrest, einen Kohlenwasserstoffthiorest oder einen Heterocyclylrest, der vorzugsweise über ein C-Atom gebunden ist, wobei jeder der letztgenannten 4 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a}-R^{a} substituiert ist, wobei jeder Kohlenwasserstoffteil vorzugsweise 1 bis 20 C-Atome aufweist und ein C-haltiger Rest R_{D}¹ inklusive Substituenten vorzugsweise 1 bis 30 C-Atome aufweist;
   R_{D}² Wasserstoff oder (C₁-C₄)Alkyl, vorzugsweise Wasserstoff, oder
   R_{D}1 und R_{D}² zusammen mit der Gruppe der Formel -CO-N- den Rest eines 3- bis 8-gliedrigen gesättigten oder ungesättigten Rings;
   R_{D}³ gleich oder verschieden Halogen, Cyano, Nitro, Amino, Hydroxy, Carboxy, Formyl, CONH₂, SO₂NH₂ oder einen Rest der Formel -Z^{b}-R^{b};
   R_{D}⁴ Wasserstoff oder (C₁-C₄)Alkyl, vorzugsweise H;
   R_{D}⁵ gleich oder verschieden Halogen, Cyano, Nitro, Amino, Hydroxy, Carboxy, CHO, CONH₂, SO₂NH₂ oder einen Rest der Formel -Z^{c}-R^{c};
   R^{a} einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   R^{b},R^{c} gleich oder verschieden einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)-alkoxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   Z^{a} eine divalente Gruppe der
      Formel -O-, -S-, -CO-, -CS-, -CO-O-, -CO-S-, -O-CO-, -S-CO-, -SO-, -SO₂-, -NR *-, -CO-NR*-, -NR*-CO-, -SO₂-NR*- oder -NR*-SO₂-, wobei die rechts angegebene Bindung der jeweiligen divalenten Gruppe die Bindung zum Rest R^{a} ist und wobei die R* in den letztgenannten 5 Resten unabhängig voneinander jeweils H, (C₁-C₄)-Alkyl oder Halo-(C₁-C₄)-alkyl bedeuten;
   Z^{b},Z^{c} unabhängig voneinander eine direkte Bindung oder eine divalente Gruppe der Formel -O-, -S-, -CO-, -CS-, -CO-O-, -CO-S-, -O-CO-,
      -S-CO-, -SO-, -SO₂-, -NR*-, -SO₂-NR*-, -NR*-SO₂-, -CO-NR*-
      oder -NR*-CO-, wobei die rechts angegebene Bindung der jeweiligen divalenten Gruppe die Bindung zum Rest R^{b} bzw. R^{c} ist und wobei die R* in den letztgenannten 5 Resten unabhängig voneinander jeweils H, (C₁-C₄)-Alkyl oder Halo-(C₁-C₄)-alkyl bedeuten;
   n_{D} eine ganze Zahl von 0 bis 4, vorzugsweise 0, 1 oder 2, insbesondere 0 oder 1, und
   m_{D} eine ganze Zahl von 0 bis 5, vorzugsweise 0, 1, 2 oder 3, insbesondere 0, 1 oder 2;
   bedeuten;
E) Acylsulfamoylbenzoesäureamide der allgemeinen Formel (S-V), gegebenenfalls auch in Salzform, 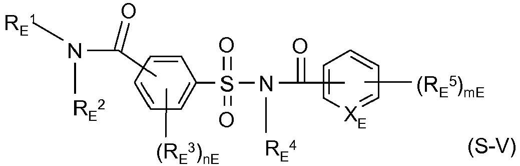 worin
   X_{E} CH oder N;
   R_{E}¹ Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a}-R^{a} substituiert sind;
   R_{E}² Wasserstoff, Hydroxy, (C₁-C₆)Alkyl, (C₂-C₆)Alkenyl, (C₂-C₆)Alkinyl, (C₁-C₆)Alkoxy, (C₂-C₆)Alkenyloxy, wobei die fünf letztgenannten Reste gegebenenfalls durch einen oder mehrere, gleiche oder verschiedene Reste aus der Gruppe Halogen, Hydroxy, (C₁-C₄)Alkyl, (C₁-C₄)Alkoxy und (C₁-C₄)Alkylthio substituiert sind, oder
   R_{E}1 und R_{E}² zusammen mit dem sie tragenden Stickstoffatom einen 3- bis 8-gliedrigen gesättigten oder ungesättigten Ring;
   R_{E}³ Halogen, Cyano, Nitro, Amino, Hydroxy, Carboxy, CHO, CONH₂, SO₂NH₂ oder Z^{b}-R^{b},
   R_{E}⁴ Wasserstoff, (C₁-C₄)Alkyl, (C₂-C₄)Alkenyl oder (C₂-C₄)Alkinyl;
   R_{E}⁵ Halogen, Cyano, Nitro, Amino, Hydroxy, Carboxy, Phosphoryl, CHO, CONH₂, SO₂NH₂ oder Z^{c}-R^{c},
   R^{a} einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[(C₁-C₄)-alkyl]-amino substituiert sind;
   R^{b}, R^{c} gleich oder verschieden einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)-alkyl]-amino substituiert sind;
   Z^{a} eine divalente Einheit aus der Gruppe O, S, CO, CS, C(O)O, C(O)S, SO, SO₂, NR^{d}, C(O)NR^{d} oder SO₂NR^{d};
   Z^{b}, Z^{c} gleich oder verschieden eine direkte Bindung oder eine divalente Einheit aus der Gruppe O, S, CO, CS, C(O)O, C(O)S, SO, SO₂, NR^{d}, SO₂NR^{d} oder C(O)NR^{d}_{;}
   R^{d} Wasserstoff, (C₁-C₄)-Alkyl oder (C₁-C₄)-Haloalkyl,
   n_{E} eine ganze Zahl von 0 bis 4, und
   m_{E} für den Fall, daß X für CH steht, eine ganze Zahl von 0 bis 5, und für den Fall, daß X für N steht, eine ganze Zahl von 0 bis 4
   bedeuten;
   davon bevorzugt sind Verbindungen (auch in Form ihrer Salze) vom Typ der Acylsulfamoylbenzoesäureamide, z.B. der nachfolgenden Formel (S-VI), die z.B. bekannt sind aus WO 99/16744,

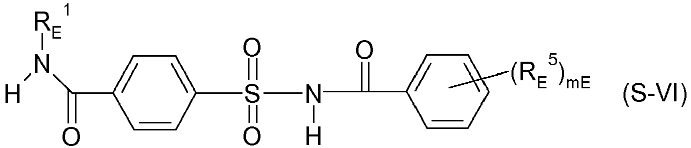

   z.B. solche worin
   R_{E}¹ = Cyclopropyl und R_{E}⁵ = 2-OMe ist ("Cyprosulfamide", S3-1),
   R_{E}¹ = Cyclopropyl und R_{E}⁵ = 5-Cl-2-OMe ist (S3-2),
   R_{E}¹ = Ethyl und R_{E}⁵ = 2-OMe ist (S3-3),
   R_{E}¹ = Isopropyl und R_{E}⁵ = 5-Cl-2-OMe ist (S3-4) und
   R_{E}¹ = Isopropyl und R_{E}⁵ = 2-OMe ist (S3-5);
F) Verbindungen vom Typ der N-Acylsulfamoylphenylharnstoffe der Formel (S-VII), die z.B. bekannt sind aus der EP-A-365484, 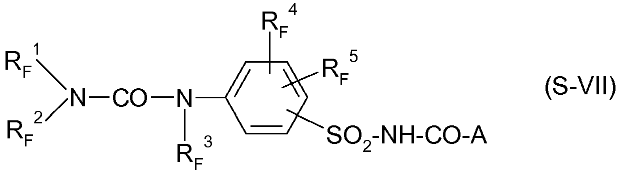 worin
   A für einen Rest aus der Gruppe 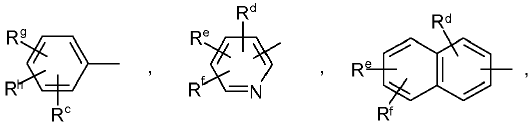 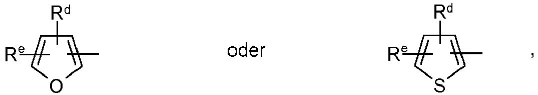
   R_{F}¹ und R_{F}² unabhängig voneinander für Wasserstoff, (C₁-C₈)Alkyl, (C₃-C₈)Cycloalkyl, (C₃-C₆)Alkenyl, (C₃-C₆)Alkinyl, 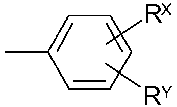 oder durch (C₁-C₄)Alkoxy oder 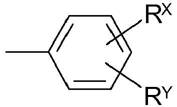 substituiertes (C₁-C₄)Alkoxy, oder
   R_{F}¹ und R_{F}² gemeinsam für eine (C₄-C₆)Alkylenbrücke oder eine durch Sauerstoff, Schwefel, SO, SO₂, NH oder -N(C₁-C₄-Alkyl)- unterbrochene (C₄-C₆)Alkylenbrücke,
   R_{F}³ für Wasserstoff oder (C₁-C₄)Alkyl,
   R_{F}⁴ und R_{F}⁵ unabhängig voneinander für Wasserstoff, Halogen, Cyan, Nitro, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl, -COOR^{j}, -CONR^{k}R^{m}, -CORⁿ, -SO₂NR^{k}R^{m} oder -OSO₂-C₁-C₄-Alkyl, oder R^{a} und R^{b} gemeinsam für eine (C₃-C₄)Alkylenbrücke, die durch Halogen oder C₁-C₄-Alkyl substituiert sein kann, oder eine (C₃-C₄)Alkenylenbrücke, die durch Halogen oder (C₁-C₄)Alkyl substituiert sein kann, oder eine C₄-Alkadienylenbrücke, die durch Halogen oder (C₁-C₄)Alkyl substituiert sein kann, und
   R^{g} und R^{h} unabhängig voneinander für Wasserstoff, Halogen, C₁-C₄-Alkyl, Trifluormethyl, Methoxy, Methylthio oder -COOR^{j} stehen, wobei
   R^{c} Wasserstoff, Halogen, (C₁-C₄)Alkyl oder Methoxy,
   R^{d} Wasserstoff, Halogen, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, -COOR^{j} oder -CONR^{k}R^{m},
   R^{e} Wasserstoff, Halogen, C₁-C₄-Alkyl, -COOR^{j}, Trifluormethyl oder Methoxy, oder R^{d} und R^{e} gemeinsam für eine (C₃-C₄)Alkylenbrücke,
   R^{f} Wasserstoff, Halogen oder (C₁-C₄)Alkyl,
   R^{X} und R^{Y} unabhängig voneinander Wasserstoff, Halogen, (C₁-C₄)Alkyl, (C₁-C₄)Alkoxy, (C₁-C₄)Alkylthio, -COOR⁴, Trifluormethyl, Nitro oder Cyan,
   R^{j}, R^{k} und R^{m} unabhängig voneinander Wasserstoff oder (C₁-C₄)Alkyl,
   R^{k} und R^{m} gemeinsam eine (C₄-C₆)Alkylenbrücke oder eine durch Sauerstoff, NH oder -N(C₁-C₄-Alkyl)- unterbrochene C₄-C₆-Alkylenbrücke, und
   Rⁿ (C₁-C₄)Alkyl, Phenyl oder durch Halogen, (C₁-C₄)Alkyl, Methoxy, Nitro oder Trifluormethyl substituiertes Phenyl bedeuten,
   davon bevorzugt sind:
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff,
   1-[4-(N-Naphthoylsulfamoyl)phenyl]-3,3-dimethylharnstoff, einschließlich der
   Stereoisomeren und der in der Landwirtschaft gebräuchlichen Salze,
G) Wirkstoffe aus der Klasse der Hydroxyaromaten und der aromatischaliphatischen Carbonsäurederivate, z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 1,2-Dihydro-2-oxo-6-trifluoromethylpyridin-3-carboxamid, 2-Hydroxyzimtsäure, 2,4-Dichlorzimtsäure, wie sie in der WO 2004084631, WO 2005015994, WO 2006007981, WO 2005016001 beschrieben sind;
H) Wirkstoffe aus der Klasse der 1,2-Dihydrochinoxalin-2-one, z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO 2005112630 beschrieben sind,
I) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (siehe Pestic. Man.) (= Piperidin-1-thiocarbonsäure-S-1-methyl-1-phenylethylester), das als Safener für Reis gegen Schäden des Herbizids Molinate bekannt ist,
   "Daimuron" oder "SK 23" (siehe Pestic. Man.) (= 1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (= 3-(2-Chlorphenylmethyl)-1-(1-methyl-1-phenyl-ethyl)-harnstoff, siehe JP-A-60087254), das als Safener für Reis gegen Schäden einiger Herbizide bekannt ist,
   "Methoxyphenon" oder "NK 049" (= 3,3'-Dimethyl-4-methoxy-benzophenon), das als Safener für Reis gegen Schäden einiger Herbizide bekannt ist, "CSB" (= 1-Brom-4-(chlormethylsulfonyl)-benzol) (CAS-Reg. Nr. 54091-06-4 von Kumiai), das als Safener gegen Schäden einiger Herbizide in Reis bekannt ist,
K) Verbindungen der Formel (S-IX), wie sie in der WO-A-1998/38856 beschrieben sind 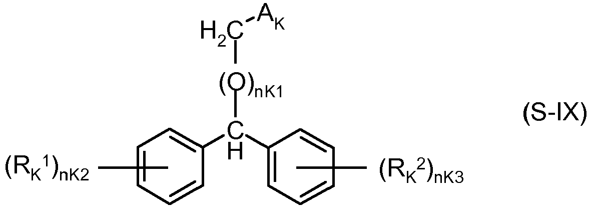 worin die Symbole und Indizes folgende Bedeutungen haben:
   R_{K}¹, R_{K}² unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   A_{K} COOR_{K}³ oder COOR_{K}⁴
   R_{K}³, R_{K}⁴ unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   n_{K}¹ 0 oder 1 und
   n_{K}², n_{K}³ unabhängig voneinander 0, 1 oder 2;
   vorzugsweise:
   Methyl-(diphenylmethoxy)acetat (CAS-Regno: 41858-19-9),
L) Verbindungen der Formel (S-X),
   wie sie in der WO A-98/27049 beschrieben sind 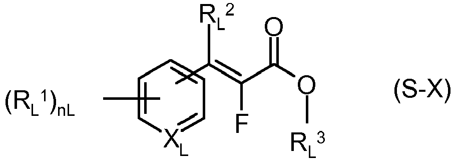 worin die Symbole und Indizes folgende Bedeutungen haben:
   X_{L} CH oder N,
   n_{L} für den Fall, dass X=N ist, eine ganze Zahl von 0 bis 4 und für den Fall, dass X=CH ist, eine ganze Zahl von 0 bis 5 ,
   R_{L}¹ Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)Alkylsulfonyl, (C₁-C₄)Alkoxycarbonyl, ggf. substituiertes. Phenyl, ggf. substituiertes Phenoxy,
   R_{L}² Wasserstoff oder (C₁-C₄)Alkyl
   R_{L}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze.
M) Wirkstoffe aus der Klasse der 3-(5-Tetrazolylcarbonyl)-2-chinolone, z.B. 1,2-Dihydro-4-hydroxy-1-ethyl-3-(5-tetrazolylcarbonyl)-2-chinolon (CAS-Regno: 219479-18-2), 1,2-Dihydro-4-hydroxy-1-methyl-3-(5-tetrazolyl-carbonyl)-2-chinolon (CAS-Regno: 95855-00-8), wie sie in der WO-A-1999000020 beschrieben sind,
N) Verbindungen der Formeln (S-XI) oder (S-XII)
   wie sie in der WO-A-2007023719 und WO-A-2007023764 beschrieben sind 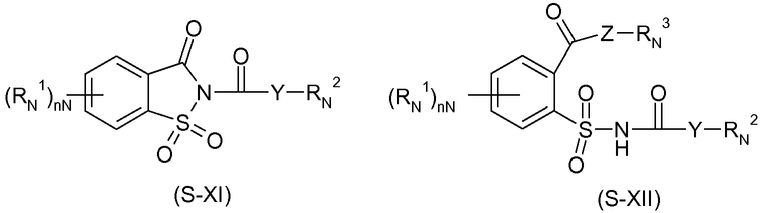 worin
   R_{N}¹ Halogen, (C₁-C₄)Alkyl, Methoxy, Nitro, Cyano, CF₃, OCF₃
   Y, Z unabhängig voneinander O oder S,
   n_{N} eine ganze Zahl von 0 bis 4,
   R_{N}² (C₁-C₁₆)Alkyl, (C₂-C₆)Alkenyl, (C₃-C₆)Cycloalkyl, Aryl; Benzyl, Halobenzyl,
   R_{N}³ Wasserstoff, (C₁-C₆)Alkyl bedeuten;
O) eine oder mehreren Verbindungen aus Gruppe:
   1,8-Naphthalsäureanhydrid,
   O,O-Diethyl S-2-ethylthioethyl phosphordithioat (Disulfoton),
   4-Chlorphenyl-methylcarbamat (Mephenate),
   O,O-Diethyl-O-phenylphosphorotioat (Dietholate),
   4-Carboxy-3,4-dihydro-2H-1-benzopyran-4-essigsäure (CL-304415, CAS-Regno: 31541-57-8),
   2-propenyl 1-oxa-4-azaspiro[4.5]decane-4-carbodithioate (MG-838, CAS-Regno: 133993-74-5),
   Methyl-[(3-oxo-1H-2-benzothiopyran-4(3H)-yliden)methoxy]acetate (aus WO-A-98/13361; CAS-Regno: 205121-04-6),
   Cyanomethoxyimino(phenyl)acetonitril (Cyometrinil),
   1,3-Dioxolan-2-ylmethoxyimino(phenyl)acetonitril (Oxabetrinil),
   4'-Chlor-2,2,2-trifluoracetophenon-O-1,3-dioxolan-2-ylmethyloxim (Fluxofenim), 4,6-Dichlor-2-phenylpyrimidin (Fenclorim),
   Benzyl-2-chlor-4-trifluormethyl-1,3-thiazol-5-carboxylat (Flurazole), 2-Dichlormethyl-2-methyl-1,3-dioxolan (MG-191),
   einschließlich der jeweils möglichen Stereoisomeren und der in der Landwirtschaft gebräuchlichen Salze.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I) und/oder deren Salze. Sie kann innerhalb weiter Grenzen schwanken. Für die Anwendung als Herbizid zur Bekämpfung von Schadpflanzen liegt sie beispielsweise im Bereich von 0,001 bis 10,0 kg/ha oder mehr Aktivsubstanz, vorzugsweise im Bereich von 0,005 bis 5 kg/ha, insbesondere im Bereich von 0,01 bis 1 kg/ha Aktivsubstanz. Dies gilt sowohl für die Anwendung im Vorauflauf oder im Nachauflauf.

Bei der Anwendung als Pflanzenwachstumsregulator, beispielsweise als Halmverkürzer bei Kulturpflanzen, wie sie oben genannt worden sind, vorzugsweise Getreidepflanzen wie Weizen, Gerste, Roggen, Triticale, Hirse, Reis oder Mais, liegt die Aufwandmenge beispielsweise im Bereich von 0,001 bis 2 kg/ha oder mehr Aktivsubstanz, vorzugsweise im Bereich von 0,005 bis 1 kg/ha, insbesondere im Bereich von 10 bis 500 g/ha Aktivsubstanz, ganz besonders von 20 bis 250 g/ha Aktivsubstanz. (bitte prüfen, ob dies genannt werden soll) Dies gilt sowohl für die Anwendung im Vorauflauf oder im Nachauflauf, wobei die Nachauflaufbehandlung in der Regel bevorzugt ist.
Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.
Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Nachfolgend sind beispielhaft einige Synthesebeispiele von Verbindungen der allgemeinen Formel (I) beschrieben. In den Beispielen beziehen sich Mengenangaben (auch Prozentangaben) auf das Gewicht, sofern nichts anderes speziell angegeben ist. Die Symbole ">" und "<" bedeuten "größer als" beziehungsweise "kleiner als". Das Symbol "≥" bedeutet "größer als oder gleich", das Symbol "≤" bedeutet "kleiner als oder gleich".
Wenn im Rahmen der Beschreibung und der Beispiele Bezeichnungen "R" und "S" für die absolute Konfiguration an einem Chiralitätszentrum der Stereoisomeren der Formel (I) angegeben ist, folgt diese der RS-Nomenklatur nach der Cahn-Ingold- Prelog-Regel, wenn nichts anderes näher definiert ist.

### (A) Synthesebeispiele

### Beispiel A1: erythro- und threo-4-Cyan-4-(5-brompyridin-3-yl)-3-(4-chlorphenyl)-butansäuremethylester (Tabelle 2, Beispiele erythro-Ibb1082 und threo-Ibb1082)

Zu 1,048 g (5,329 mmol) Methyl-3-(4-chlorphenyl)acrylat und 1,000 g (5,075 mmol) (5-Brompyridin-3-yl)acetonitril in 8,0 ml Toluol gab man unter Schutzgas (Ar) 0,055 g (1,015 mmol) Kalium-tert.-butylat. Man fügte 3 ml Dimethylformamid zu und rührte 8 h bei 80°C. Man entfernte das Lösemittel im Vakuum, nahm den Rückstand in Dichlormethan auf und wusch zweimal mit je 25 ml Wasser. Die vereinigten organischen Phasen wurden nacheinander mit 1 N wässriger Salzsäure und mit gesättigter wässriger Natriumchlorid-Lösung gewaschen und über Natriumsulfat getrocknet. Das Lösemittel wurde unter reduziertem Druck entfernt. Nach Chromatographie des Rückstands an Kieselgel (Gradient: Start bei Ethylacetat / Heptan = 5:95 innerhalb von 20 Minuten auf Ethylacetat / Heptan = 30:70) erhielt man nacheinander 0,578 g (29% d. Th.) *threo*-4-Cyan-4-(5-brompyridin-3-yl)-3-(4-chlorphenyl)-butansäuremethylester und 0,800 g (40% d. Th.) *erythro*-4-Cyan-4-(5-brompyridin-3-yl)-3-(4-chlorphenyl)-butansäuremethylester. Die Zuordnung der Konfiguration erfolgte durch Vergleich der chemischen Verschiebungen der jeweiligen CHCN Dubletts bei 4,12 ppm bzw. 4,49 ppm im ¹H-NMR (CDCl₃). Das Signal bei tieferem Feld wurde in Analogie zur Literatur dem *erythro*-Diastereomer zugeordnet. ¹H-NMR in CDCl₃ siehe Tabelle 2.

### Beispiel A2: (3S,4S)-4-Cyan-4-(5-brompyridin-3-yl)-3-(4-chlorphenyl)-butansäuremethylester (Tabelle 2, Beispiel threo-1-Ibb1082)

Durch präparative Chromatographie [(80 ml/min n-Heptan/2-Propanol (70:30)] des unter Beispiel A1 erhaltenen Gemisches der racemischen *threo*-4-Cyan-4-(5-brompyridin-3-yl)-3-(4-chlorphenyl)-butansäuremethylester an einer chiralen Festphase [Chiralpak IC, 20µm, (250 x 50)-mm Säule] erhielt man 232,0 mg (3S,4S)-4-Cyan-4-(5-brompyridin-3-yl)-3-(4-chlorphenyl)-butansäuremethylester [chemische Reinheit > 95% (NMR), Isomerenreinheit > 99% (chirale HPLC)], der als erster der beiden Stereoisomere (Retentionszeit = 12,17 min) eluiert wurde. Spezifischer Drehwert [α]: + 61°. ¹H-NMR in CDCl₃ und Retentionszeit bei analytischer HPLC siehe Tabelle 2.

### Beispiel A3: (3R,4R)-4-Cyan-4-(5-brompyridin-3-yl)-3-(4-chlorphenyl)-butansäuremethylester (Tabelle 2, Beispiel threo-2-Ibb1082)

Durch präparative Chromatographie [(80 ml/min n-Heptan/2-Propanol (70:30)] des unter Beispiel A1 erhaltenen Gemisches der racemischen *threo*-4-Cyan-4-(5-brompyridin-3-yl)-3-(4-chlorphenyl)-butansäuremethylester an einer chiralen Festphase [Chiralpak IC, 20µm, (250 x 50)-mm Säule] erhielt man 73,0 mg (3R,4R)-4-Cyan-4-(5-brompyridin-3-yl)-3-(4-chlorphenyl)-butansäuremethylester [chemische Reinheit > 95% (NMR), Isomerenreinheit > 99% (chirale HPLC)], der als letzter der beiden Stereoisomere (Retentionszeit = 20,01 min) eluiert wurde. Spezifischer Drehwert [α]: - 61°. ¹H-NMR in CDCl₃ und Retentionszeit bei analytischer HPLC siehe Tabelle 2.

Die in den nachfolgenden Tabellen beschriebenen Verbindungen mit der absoluten Konfiguration (3R, 4R) erhält man gemäß oder analog zu den oben beschriebenen Beispielen.

### Beispiel A4: 4-Cyan-4-(5-brompyridin-3-yl)-3-(4-chlorphenyl)-butansäure (Tabelle 2, Beispiele Iba1082)

Zu 0,630 g (1,600 mmol) 4-Cyan-3-(2,6-difluorphenyl)-4-(3,4-difluorphenyl)-butansäuremethylester (Beispiel A1) in 160 ml Methanol gab man unter Schutzgas (Ar) 3,0 ml 2 molare wässrige Natronlauge und rührte 2 h bei 25°C. Man entfernte das Methanol unter reduziertem Druck. Der Rückstand wurde mit 2N wäßriger Salzsäure angesäuert (pH = 3). Nach Filtration und Trocknen des Rückstands im Hochvakuum bei 40°C erhielt man 0,593 g (99,8 % d. Th.) der Titelverbindung als farblosen Feststoff [(erythro:threo = 35:65, Vergleich der Dubletts im ¹H-NMR in CDCl₃ bei 4,14 (threo) und 4,50 ppm (erythro)]. ¹H-NMR in CDCl₃ siehe Tabelle 2.

### Beispiel A5: 4-Cyan-4-(5-brompyridin-3-yl)-3-(4-chlorphenyl)-butansäureethylester (Tabelle 2, Beispiele, Ibc1082)

Zu 0,200 g (0,527 mmol) 4-Cyan-4-(5-brompyridin-3-yl)-3-(4-chlorphenyl)-butansäure (Beispiel A4) in 4 ml Dichlormethan gab man unter Schutzgas (Ar) 0,121 g (0,632 mmol) N'-(3-dimethylaminopropyl)-N-ethyl-carbodiimid (EDC), eine Spatelspitze 4-Dimethylaminopyridin (DMAP) und 0,061 g (1,317 mmol) Ethanol und rührte 8 h bei 25°C. Man entfernte das Lösemittel unter reduziertem Druck, nahm den Rückstand in Dichlormethan auf und wusch zweimal mit je 15 ml Wasser. Die vereinigten organischen Phasen wurden über Natriumsulfat getrocknet und das Lösemittel unter reduziertem Druck entfernt. Nach Chromatographie des Rückstands an Kieselgel (Gradient: Start bei Ethylacetat / Heptan = 5:95 innerhalb von 20 Minuten auf Ethylacetat / Heptan = 20:80) erhielt man 0,143 g (67% d. Th.) der Titelverbindung (erythro:threo = 58:42). ¹H-NMR in CDCl₃ siehe Tabelle 2.

Die in den nachfolgenden Tabellen beschriebenen Verbindungen erhält man gemäß oder analog zu den oben beschriebenen Beispielen.

In den Tabellen bedeuten:
- Bsp.: = Beispiel Nummer
- H: = Wasserstoff(-atom)
- Me: = Methyl
- Et: = Ethyl
- n-Pr: = n-Propyl
- i-Pr: = Isopropyl
- Rtz: = Retentionszeit
- F, Cl, Br, I: = Fluor, Chlor, Brom bzw. Jod entsprechend den üblichen chemischen Atomsymbolen
- CN: = Cyano
- NO₂: = Nitro
- MeO oder OMe: = Methoxy
- CF₃: = Trifluormethyl
- OCF₃: = Trifluormethoxy
- OCF₂H: = Diflluormethoxy
- CO₂Me: = Methoxycarbonyl ("Methylester-Gruppe")

Die Position eines Substituenten am Phenylring, beispielsweise in Position 2, ist dem Symbol oder der Abkürzung des Restes vorangestellt, z.B.
- 2-Cl: = 2-Chlor
- 2-F: = 2-Fluor

Nummerierungen der Substituentenpositionen bei di- oder trisubstituiertem Substitutionsmuster sind analog vorangestellt, z. B.
- 3,4-Me₂: = 3,4-Dimethyl (z. B. als Substitution am Phenylring)
- 3,5-F₂: = 3,5-Difluor (z. B. als Substitution am Phenylring)
- 3,4-F₂: = 3,4-Difluor (z. B. als Substitution am Phenylring)

Andere Abkürzungen sind analog der oben angegebenen Beispiele zu verstehen.
- "(R¹)ₘ = "H": = neben R³ kein weiterer Substituent vorhanden (m = 0)
- "(R²)ₙ = "H": = unsubstituierter Cyclus (n = 0)

Im Übrigen gelten die üblichen chemischen Symbole und Formeln, wie z. B. CH₂ für Methylen oder CF₃ für Trifluormethyl oder OH für Hydroxyl. Zusammengesetzte Bedeutungen sind entsprechend aus den genannten Abkürzungen zusammengesetzt definiert.

Die für Verbindungen aus den Tabellen 2a - 2f angegebenen Retentionszeiten ("Rtz") wurden durch analytische HPLC der Verbindungen (I) an einer chiralen Festphase erhalten. Die Verbindungen der Formel (I) wurden in einer Konzentration von 1 mg/mL in analysenreinem Dichlormethan gelöst und direkt der HPLC zugeführt. Die chromatographisch gereinigten Verbindungen (I) haben eine stereochemische Reinheit ≥ 90%.

**Tabelle 1: Definitionen von Strukturkombinationen der Gruppen (R¹)ₘ, (R²)ₙ und R³ für die nachfolgenden Tabellen von erfindungsgemäßen Verbindungen der allgemeinen Formel (I)**

| Nr. | (R¹)ₘ | (R²)ₙ | R³ |
|---|---|---|---|
| 1 | H | 4-F | F |
| 2 | H | 4-Cl | F |
| 3 | H | 4-Br | F |
| 4 | H | H | F |
| 5 | H | 4-Me | F |
| 6 | H | 4-CN | F |
| 7 | H | 4-NO₂ | F |
| 8 | H | 4-OMe | F |
| 9 | H | 3-F | F |
| 10 | H | 3-Cl | F |
| 11 | H | 3-Br | F |
| 12 | H | 3-Me | F |
| 13 | H | 3-CN | F |
| 14 | H | 3-NO₂ | F |
| 15 | H | 3-OMe | F |
| 16 | H | 2-F | F |
| 17 | H | 2-Cl | F |
| 18 | H | 2-Br | F |
| 19 | H | 2-Me | F |
| 20 | H | 2-CN | F |
| 21 | H | 2-NO₂ | F |
| 22 | H | 2-OMe | F |
| 23 | H | 2,3-F₂ | F |
| 24 | H | 2,4-F₂ | F |
| 25 | H | 2,5-F₂ | F |
| 26 | H | 2,6-F₂ | F |
| 27 | H | 3,4-F₂ | F |
| 28 | H | 3,5-F₂ | F |
| 29 | H | 2,3-Cl₂ | F |
| 30 | H | 2,4-Cl₂ | F |
| 31 | H | 2,5-Cl₂ | F |
| 32 | H | 2,6-Cl₂ | F |
| 33 | H | 3,4-Cl₂ | F |
| 34 | H | 3,5-Cl₂ | F |
| 35 | H | 2-F, 3-Cl | F |
| 36 | H | 2-F, 4-Cl | F |
| 37 | H | 2-F, 5-Cl | F |
| 38 | H | 2-F, 6-Cl | F |
| 39 | H | 2,6-F₂, 4-Cl | F |
| 40 | H | 3-F, 4-Cl | F |
| 41 | H | 3-Cl, 5-F | F |
| 42 | H | 2-Cl, 5-F | F |
| 43 | H | 3-CN, 4-Cl | F |
| 44 | H | 3-NO₂, 4-Cl | F |
| 45 | H | 2-F, 4-Br | F |
| 46 | 6-F | 4-F | F |
| 47 | 6-F | 4-Cl | F |
| 48 | 6-F | 4-Br | F |
| 49 | 6-F | H | F |
| 50 | 6-F | 4-Me | F |
| 51 | 6-F | 4-CN | F |
| 52 | 6-F | 4-NO₂ | F |
| 53 | 6-F | 4-OMe | F |
| 54 | 6-F | 3-F | F |
| 55 | 6-F | 3-Cl | F |
| 56 | 6-F | 3-Br | F |
| 57 | 6-F | 3-Me | F |
| 58 | 6-F | 3-CN | F |
| 59 | 6-F | 3-NO₂ | F |
| 60 | 6-F | 3-OMe | F |
| 61 | 6-F | 2-F | F |
| 62 | 6-F | 2-Cl | F |
| 63 | 6-F | 2-Br | F |
| 64 | 6-F | 2-Me | F |
| 65 | 6-F | 2-CN | F |
| 66 | 6-F | 2-NO₂ | F |
| 67 | 6-F | 2-OMe | F |
| 68 | 6-F | 2,3-F₂ | F |
| 69 | 6-F | 2,4-F₂ | F |
| 70 | 6-F | 2,5-F₂ | F |
| 71 | 6-F | 2,6-F₂ | F |
| 72 | 6-F | 3,4-F₂ | F |
| 73 | 6-F | 3,5-F₂ | F |
| 74 | 6-F | 2,3-Cl₂ | F |
| 75 | 6-F | 2,4-Cl₂ | F |
| 76 | 6-F | 2,5-Cl₂ | F |
| 77 | 6-F | 2,6-Cl₂ | F |
| 78 | 6-F | 3,4-Cl₂ | F |
| 79 | 6-F | 3,5-Cl₂ | F |
| 80 | 6-F | 2-F, 3-Cl | F |
| 81 | 6-F | 2-F, 4-Cl | F |
| 82 | 6-F | 2-F, 5-Cl | F |
| 83 | 6-F | 2-F, 6-Cl | F |
| 84 | 6-F | 2,6-F₂, 4-Cl | F |
| 85 | 6-F | 3-F, 4-Cl | F |
| 86 | 6-F | 3-Cl, 5-F | F |
| 87 | 6-F | 2-Cl, 5-F | F |
| 88 | 6-F | 3-CN, 4-Cl | F |
| 89 | 6-F | 3-NO₂, 4-Cl | F |
| 90 | 6-F | 2-F, 4-Br | F |
| 91 | 6-Cl | 4-F | F |
| 92 | 6-Cl | 4-Cl | F |
| 93 | 6-Cl | 4-Br | F |
| 94 | 6-Cl | H | F |
| 95 | 6-Cl | 4-Me | F |
| 96 | 6-Cl | 4-CN | F |
| 97 | 6-Cl | 4-NO₂ | F |
| 98 | 6-Cl | 4-OMe | F |
| 99 | 6-Cl | 3-F | F |
| 100 | 6-Cl | 3-Cl | F |
| 101 | 6-Cl | 3-Br | F |
| 102 | 6-Cl | 3-Me | F |
| 103 | 6-Cl | 3-CN | F |
| 104 | 6-Cl | 3-NO₂ | F |
| 105 | 6-Cl | 3-OMe | F |
| 106 | 6-Cl | 2-F | F |
| 107 | 6-Cl | 2-Cl | F |
| 108 | 6-Cl | 2-Br | F |
| 109 | 6-Cl | 2-Me | F |
| 110 | 6-Cl | 2-CN | F |
| 111 | 6-Cl | 2-NO₂ | F |
| 112 | 6-Cl | 2-OMe | F |
| 113 | 6-Cl | 2,3-F₂ | F |
| 114 | 6-Cl | 2,4-F₂ | F |
| 115 | 6-Cl | 2,5-F₂ | F |
| 116 | 6-Cl | 2,6-F₂ | F |
| 117 | 6-Cl | 3,4-F₂ | F |
| 118 | 6-Cl | 3,5-F₂ | F |
| 119 | 6-Cl | 2,3-Cl₂ | F |
| 120 | 6-Cl | 2,4-Cl₂ | F |
| 121 | 6-Cl | 2,5-Cl₂ | F |
| 122 | 6-Cl | 2,6-Cl₂ | F |
| 123 | 6-Cl | 3,4-Cl₂ | F |
| 124 | 6-Cl | 3,5-Cl₂ | F |
| 125 | 6-Cl | 2-F, 3-Cl | F |
| 126 | 6-Cl | 2-F, 4-Cl | F |
| 127 | 6-Cl | 2-F, 5-Cl | F |
| 128 | 6-Cl | 2-F, 6-Cl | F |
| 129 | 6-Cl | 2,6-F₂, 4-Cl | F |
| 130 | 6-Cl | 3-F, 4-Cl | F |
| 131 | 6-Cl | 3-Cl, 5-F | F |
| 132 | 6-Cl | 2-Cl, 5-F | F |
| 133 | 6-Cl | 3-CN, 4-Cl | F |
| 134 | 6-Cl | 3-NO₂, 4-Cl | F |
| 135 | 6-Cl | 2-F, 4-Br | F |
| 136 | 6-Br | 4-F | F |
| 137 | 6-Br | 4-Cl | F |
| 138 | 6-Br | 4-Br | F |
| 139 | 6-Br | H | F |
| 140 | 6-Br | 4-Me | F |
| 141 | 6-Br | 4-CN | F |
| 142 | 6-Br | 4-NO₂ | F |
| 143 | 6-Br | 4-OMe | F |
| 144 | 6-Br | 3-F | F |
| 145 | 6-Br | 3-Cl | F |
| 146 | 6-Br | 3-Br | F |
| 147 | 6-Br | 3-Me | F |
| 148 | 6-Br | 3-CN | F |
| 149 | 6-Br | 3-NO₂ | F |
| 150 | 6-Br | 3-OMe | F |
| 151 | 6-Br | 2-F | F |
| 152 | 6-Br | 2-Cl | F |
| 153 | 6-Br | 2-Br | F |
| 154 | 6-Br | 2-Me | F |
| 155 | 6-Br | 2-CN | F |
| 156 | 6-Br | 2-NO₂ | F |
| 157 | 6-Br | 2-OMe | F |
| 158 | 6-Br | 2,3-F₂ | F |
| 159 | 6-Br | 2,4-F₂ | F |
| 160 | 6-Br | 2,5-F₂ | F |
| 161 | 6-Br | 2,6-F₂ | F |
| 162 | 6-Br | 3,4-F₂ | F |
| 163 | 6-Br | 3,5-F₂ | F |
| 164 | 6-Br | 2,3-Cl₂ | F |
| 165 | 6-Br | 2,4-Cl₂ | F |
| 166 | 6-Br | 2,5-Cl₂ | F |
| 167 | 6-Br | 2,6-Cl₂ | F |
| 168 | 6-Br | 3,4-Cl₂ | F |
| 169 | 6-Br | 3,5-Cl₂ | F |
| 170 | 6-Br | 2-F, 3-Cl | F |
| 171 | 6-Br | 2-F, 4-Cl | F |
| 172 | 6-Br | 2-F, 5-Cl | F |
| 173 | 6-Br | 2-F, 6-Cl | F |
| 174 | 6-Br | 2,6-F₂, 4-Cl | F |
| 175 | 6-Br | 3-F, 4-Cl | F |
| 176 | 6-Br | 3-Cl, 5-F | F |
| 177 | 6-Br | 2-Cl, 5-F | F |
| 178 | 6-Br | 3-CN, 4-Cl | F |
| 179 | 6-Br | 3-NO₂, 4-Cl | F |
| 180 | 6-Br | 2-F, 4-Br | F |
| 181 | 6-CN | 4-F | F |
| 182 | 6-CN | 4-Cl | F |
| 183 | 6-CN | 4-Br | F |
| 184 | 6-CN | H | F |
| 185 | 6-CN | 4-Me | F |
| 186 | 6-CN | 4-CN | F |
| 187 | 6-CN | 4-NO₂ | F |
| 188 | 6-CN | 4-OMe | F |
| 189 | 6-CN | 3-F | F |
| 190 | 6-CN | 3-Cl | F |
| 191 | 6-CN | 3-Br | F |
| 192 | 6-CN | 3-Me | F |
| 193 | 6-CN | 3-CN | F |
| 194 | 6-CN | 3-NO₂ | F |
| 195 | 6-CN | 3-OMe | F |
| 196 | 6-CN | 2-F | F |
| 197 | 6-CN | 2-Cl | F |
| 198 | 6-CN | 2-Br | F |
| 199 | 6-CN | 2-Me | F |
| 200 | 6-CN | 2-CN | F |
| 201 | 6-CN | 2-NO₂ | F |
| 202 | 6-CN | 2-OMe | F |
| 203 | 6-CN | 2,3-F₂ | F |
| 204 | 6-CN | 2,4-F₂ | F |
| 205 | 6-CN | 2,5-F₂ | F |
| 206 | 6-CN | 2,6-F₂ | F |
| 207 | 6-CN | 3,4-F₂ | F |
| 208 | 6-CN | 3,5-F₂ | F |
| 209 | 6-CN | 2,3-Cl₂ | F |
| 210 | 6-CN | 2,4-Cl₂ | F |
| 211 | 6-CN | 2,5-Cl₂ | F |
| 212 | 6-CN | 2,6-Cl₂ | F |
| 213 | 6-CN | 3,4-Cl₂ | F |
| 214 | 6-CN | 3,5-Cl₂ | F |
| 215 | 6-CN | 2-F, 3-Cl | F |
| 216 | 6-CN | 2-F, 4-Cl | F |
| 217 | 6-CN | 2-F, 5-Cl | F |
| 218 | 6-CN | 2-F, 6-Cl | F |
| 219 | 6-CN | 2,6-F₂, 4-Cl | F |
| 220 | 6-CN | 3-F, 4-Cl | F |
| 221 | 6-CN | 3-Cl, 5-F | F |
| 222 | 6-CN | 2-Cl, 5-F | F |
| 223 | 6-CN | 3-CN, 4-Cl | F |
| 224 | 6-CN | 3-NO₂, 4-Cl | F |
| 225 | 6-CN | 2-F, 4-Br | F |
| 226 | 6-Me | 4-F | F |
| 227 | 6-Me | 4-Cl | F |
| 228 | 6-Me | 4-Br | F |
| 229 | 6-Me | H | F |
| 230 | 6-Me | 4-Me | F |
| 231 | 6-Me | 4-CN | F |
| 232 | 6-Me | 4-NO₂ | F |
| 233 | 6-Me | 4-OMe | F |
| 234 | 6-Me | 3-F | F |
| 235 | 6-Me | 3-Cl | F |
| 236 | 6-Me | 3-Br | F |
| 237 | 6-Me | 3-Me | F |
| 238 | 6-Me | 3-CN | F |
| 239 | 6-Me | 3-NO₂ | F |
| 240 | 6-Me | 3-OMe | F |
| 241 | 6-Me | 2-F | F |
| 242 | 6-Me | 2-Cl | F |
| 243 | 6-Me | 2-Br | F |
| 244 | 6-Me | 2-Me | F |
| 245 | 6-Me | 2-CN | F |
| 246 | 6-Me | 2-NO₂ | F |
| 247 | 6-Me | 2-OMe | F |
| 248 | 6-Me | 2,3-F₂ | F |
| 249 | 6-Me | 2,4-F₂ | F |
| 250 | 6-Me | 2,5-F₂ | F |
| 251 | 6-Me | 2,6-F₂ | F |
| 252 | 6-Me | 3,4-F₂ | F |
| 253 | 6-Me | 3,5-F₂ | F |
| 254 | 6-Me | 2,3-Cl₂ | F |
| 255 | 6-Me | 2,4-Cl₂ | F |
| 256 | 6-Me | 2,5-Cl₂ | F |
| 257 | 6-Me | 2,6-Cl₂ | F |
| 258 | 6-Me | 3,4-Cl₂ | F |
| 259 | 6-Me | 3,5-Cl₂ | F |
| 260 | 6-Me | 2-F, 3-Cl | F |
| 261 | 6-Me | 2-F, 4-Cl | F |
| 262 | 6-Me | 2-F, 5-Cl | F |
| 263 | 6-Me | 2-F, 6-Cl | F |
| 264 | 6-Me | 2,6-F₂, 4-Cl | F |
| 265 | 6-Me | 3-F, 4-Cl | F |
| 266 | 6-Me | 3-Cl, 5-F | F |
| 267 | 6-Me | 2-Cl, 5-F | F |
| 268 | 6-Me | 3-CN, 4-Cl | F |
| 269 | 6-Me | 3-NO₂, 4-Cl | F |
| 270 | 6-Me | 2-F, 4-Br | F |
| 271 | 6-OMe | 4-F | F |
| 272 | 6-OMe | 4-Cl | F |
| 273 | 6-OMe | 4-Br | F |
| 274 | 6-OMe | H | F |
| 275 | 6-OMe | 4-Me | F |
| 276 | 6-OMe | 4-CN | F |
| 277 | 6-OMe | 4-NO₂ | F |
| 278 | 6-OMe | 4-OMe | F |
| 279 | 6-OMe | 3-F | F |
| 280 | 6-OMe | 3-Cl | F |
| 281 | 6-OMe | 3-Br | F |
| 282 | 6-OMe | 3-Me | F |
| 283 | 6-OMe | 3-CN | F |
| 284 | 6-OMe | 3-NO₂ | F |
| 285 | 6-OMe | 3-OMe | F |
| 286 | 6-OMe | 2-F | F |
| 287 | 6-OMe | 2-Cl | F |
| 288 | 6-OMe | 2-Br | F |
| 289 | 6-OMe | 2-Me | F |
| 290 | 6-OMe | 2-CN | F |
| 291 | 6-OMe | 2-NO₂ | F |
| 292 | 6-OMe | 2-OMe | F |
| 293 | 6-OMe | 2,3-F₂ | F |
| 294 | 6-OMe | 2,4-F₂ | F |
| 295 | 6-OMe | 2,5-F₂ | F |
| 296 | 6-OMe | 2,6-F₂ | F |
| 297 | 6-OMe | 3,4-F₂ | F |
| 298 | 6-OMe | 3,5-F₂ | F |
| 299 | 6-OMe | 2,3-Cl₂ | F |
| 300 | 6-OMe | 2,4-Cl₂ | F |
| 301 | 6-OMe | 2,5-Cl₂ | F |
| 302 | 6-OMe | 2,6-Cl₂ | F |
| 303 | 6-OMe | 3,4-Cl₂ | F |
| 304 | 6-OMe | 3,5-Cl₂ | F |
| 305 | 6-OMe | 2-F, 3-Cl | F |
| 306 | 6-OMe | 2-F, 4-Cl | F |
| 307 | 6-OMe | 2-F, 5-Cl | F |
| 308 | 6-OMe | 2-F, 6-Cl | F |
| 309 | 6-OMe | 2,6-F₂, 4-Cl | F |
| 310 | 6-OMe | 3-F, 4-Cl | F |
| 311 | 6-OMe | 3-Cl, 5-F | F |
| 312 | 6-OMe | 2-Cl, 5-F | F |
| 313 | 6-OMe | 3-CN, 4-Cl | F |
| 314 | 6-OMe | 3-NO₂, 4-Cl | F |
| 315 | 6-OMe | 2-F, 4-Br | F |
| 316 | 2-F | 4-F | F |
| 317 | 2-F | 4-Cl | F |
| 318 | 2-F | 4-Br | F |
| 319 | 2-F | H | F |
| 320 | 2-F | 4-Me | F |
| 321 | 2-F | 4-CN | F |
| 322 | 2-F | 4-NO₂ | F |
| 323 | 2-F | 4-OMe | F |
| 324 | 2-F | 3-F | F |
| 325 | 2-F | 3-Cl | F |
| 326 | 2-F | 3-Br | F |
| 327 | 2-F | 3-Me | F |
| 328 | 2-F | 3-CN | F |
| 329 | 2-F | 3-NO₂ | F |
| 330 | 2-F | 3-OMe | F |
| 331 | 2-F | 2-F | F |
| 332 | 2-F | 2-Cl | F |
| 333 | 2-F | 2-Br | F |
| 334 | 2-F | 2-Me | F |
| 335 | 2-F | 2-CN | F |
| 336 | 2-F | 2-NO₂ | F |
| 337 | 2-F | 2-OMe | F |
| 338 | 2-F | 2,3-F₂ | F |
| 339 | 2-F | 2,4-F₂ | F |
| 340 | 2-F | 2,5-F₂ | F |
| 341 | 2-F | 2,6-F₂ | F |
| 342 | 2-F | 3,4-F₂ | F |
| 343 | 2-F | 3,5-F₂ | F |
| 344 | 2-F | 2,3-Cl₂ | F |
| 345 | 2-F | 2,4-Cl₂ | F |
| 346 | 2-F | 2,5-Cl₂ | F |
| 347 | 2-F | 2,6-Cl₂ | F |
| 348 | 2-F | 3,4-Cl₂ | F |
| 349 | 2-F | 3,5-Cl₂ | F |
| 350 | 2-F | 2-F, 3-Cl | F |
| 351 | 2-F | 2-F, 4-Cl | F |
| 352 | 2-F | 2-F, 5-Cl | F |
| 353 | 2-F | 2-F, 6-Cl | F |
| 354 | 2-F | 2,6-F₂, 4-Cl | F |
| 355 | 2-F | 3-F, 4-Cl | F |
| 356 | 2-F | 3-Cl, 5-F | F |
| 357 | 2-F | 2-Cl, 5-F | F |
| 358 | 2-F | 3-CN, 4-Cl | F |
| 359 | 2-F | 3-NO₂, 4-Cl | F |
| 360 | 2-F | 2-F, 4-Br | F |
| 361 | 4-F | 4-F | F |
| 362 | 4-F | 4-Cl | F |
| 363 | 4-F | 4-Br | F |
| 364 | 4-F | H | F |
| 365 | 4-F | 4-Me | F |
| 366 | 4-F | 4-CN | F |
| 367 | 4-F | 4-NO₂ | F |
| 368 | 4-F | 4-OMe | F |
| 369 | 4-F | 3-F | F |
| 370 | 4-F | 3-Cl | F |
| 371 | 4-F | 3-Br | F |
| 372 | 4-F | 3-Me | F |
| 373 | 4-F | 3-CN | F |
| 374 | 4-F | 3-NO₂ | F |
| 375 | 4-F | 3-OMe | F |
| 376 | 4-F | 2-F | F |
| 377 | 4-F | 2-Cl | F |
| 378 | 4-F | 2-Br | F |
| 379 | 4-F | 2-Me | F |
| 380 | 4-F | 2-CN | F |
| 381 | 4-F | 2-NO₂ | F |
| 382 | 4-F | 2-OMe | F |
| 383 | 4-F | 2,3-F₂ | F |
| 384 | 4-F | 2,4-F₂ | F |
| 385 | 4-F | 2,5-F₂ | F |
| 386 | 4-F | 2,6-F₂ | F |
| 387 | 4-F | 3,4-F₂ | F |
| 388 | 4-F | 3,5-F₂ | F |
| 389 | 4-F | 2,3-Cl₂ | F |
| 390 | 4-F | 2,4-Cl₂ | F |
| 391 | 4-F | 2,5-Cl₂ | F |
| 392 | 4-F | 2,6-Cl₂ | F |
| 393 | 4-F | 3,4-Cl₂ | F |
| 394 | 4-F | 3,5-Cl₂ | F |
| 395 | 4-F | 2-F, 3-Cl | F |
| 396 | 4-F | 2-F, 4-Cl | F |
| 397 | 4-F | 2-F, 5-Cl | F |
| 398 | 4-F | 2-F, 6-Cl | F |
| 399 | 4-F | 2,6-F₂, 4-Cl | F |
| 400 | 4-F | 3-F, 4-Cl | F |
| 401 | 4-F | 3-Cl, 5-F | F |
| 402 | 4-F | 2-Cl, 5-F | F |
| 403 | 4-F | 3-CN, 4-Cl | F |
| 404 | 4-F | 3-NO₂, 4-Cl | F |
| 405 | 4-F | 2-F, 4-Br | F |
| 406 | 2-Cl | 4-F | F |
| 407 | 2-Cl | 4-Cl | F |
| 408 | 2-Cl | 4-Br | F |
| 409 | 2-Cl | H | F |
| 410 | 2-Cl | 4-Me | F |
| 411 | 2-Cl | 4-CN | F |
| 412 | 2-Cl | 4-NO₂ | F |
| 413 | 2-Cl | 4-OMe | F |
| 414 | 2-Cl | 3-F | F |
| 415 | 2-Cl | 3-Cl | F |
| 416 | 2-Cl | 3-Br | F |
| 417 | 2-Cl | 3-Me | F |
| 418 | 2-Cl | 3-CN | F |
| 419 | 2-Cl | 3-NO₂ | F |
| 420 | 2-Cl | 3-OMe | F |
| 421 | 2-Cl | 2-F | F |
| 422 | 2-Cl | 2-Cl | F |
| 423 | 2-Cl | 2-Br | F |
| 424 | 2-Cl | 2-Me | F |
| 425 | 2-Cl | 2-CN | F |
| 426 | 2-Cl | 2-NO₂ | F |
| 427 | 2-Cl | 2-OMe | F |
| 428 | 2-Cl | 2,3-F₂ | F |
| 429 | 2-Cl | 2,4-F₂ | F |
| 430 | 2-Cl | 2,5-F₂ | F |
| 431 | 2-Cl | 2,6-F₂ | F |
| 432 | 2-Cl | 3,4-F₂ | F |
| 433 | 2-Cl | 3,5-F₂ | F |
| 434 | 2-Cl | 2,3-Cl₂ | F |
| 435 | 2-Cl | 2,4-Cl₂ | F |
| 436 | 2-Cl | 2,5-Cl₂ | F |
| 437 | 2-Cl | 2,6-Cl₂ | F |
| 438 | 2-Cl | 3,4-Cl₂ | F |
| 439 | 2-Cl | 3,5-Cl₂ | F |
| 440 | 2-Cl | 2-F, 3-Cl | F |
| 441 | 2-Cl | 2-F, 4-Cl | F |
| 442 | 2-Cl | 2-F, 5-Cl | F |
| 443 | 2-Cl | 2-F, 6-Cl | F |
| 444 | 2-Cl | 2,6-F₂, 4-Cl | F |
| 445 | 2-Cl | 3-F, 4-Cl | F |
| 446 | 2-Cl | 3-Cl, 5-F | F |
| 447 | 2-Cl | 2-Cl, 5-F | F |
| 448 | 2-Cl | 3-CN, 4-Cl | F |
| 449 | 2-Cl | 3-NO₂, 4-Cl | F |
| 450 | 2-Cl | 2-F, 4-Br | F |
| 451 | 4-Cl | 4-F | F |
| 452 | 4-Cl | 4-Cl | F |
| 453 | 4-Cl | 4-Br | F |
| 454 | 4-Cl | H | F |
| 455 | 4-Cl | 4-Me | F |
| 456 | 4-Cl | 4-CN | F |
| 457 | 4-Cl | 4-NO₂ | F |
| 458 | 4-Cl | 4-OMe | F |
| 459 | 4-Cl | 3-F | F |
| 460 | 4-Cl | 3-Cl | F |
| 461 | 4-Cl | 3-Br | F |
| 462 | 4-Cl | 3-Me | F |
| 463 | 4-Cl | 3-CN | F |
| 464 | 4-Cl | 3-NO₂ | F |
| 465 | 4-Cl | 3-OMe | F |
| 466 | 4-Cl | 2-F | F |
| 467 | 4-Cl | 2-Cl | F |
| 468 | 4-Cl | 2-Br | F |
| 469 | 4-Cl | 2-Me | F |
| 470 | 4-Cl | 2-CN | F |
| 471 | 4-Cl | 2-NO₂ | F |
| 472 | 4-Cl | 2-OMe | F |
| 473 | 4-Cl | 2,3-F₂ | F |
| 474 | 4-Cl | 2,4-F₂ | F |
| 475 | 4-Cl | 2,5-F₂ | F |
| 476 | 4-Cl | 2,6-F₂ | F |
| 477 | 4-Cl | 3,4-F₂ | F |
| 478 | 4-Cl | 3,5-F₂ | F |
| 479 | 4-Cl | 2,3-Cl₂ | F |
| 480 | 4-Cl | 2,4-Cl₂ | F |
| 481 | 4-Cl | 2,5-Cl₂ | F |
| 482 | 4-Cl | 2,6-Cl₂ | F |
| 483 | 4-Cl | 3,4-Cl₂ | F |
| 484 | 4-Cl | 3,5-Cl₂ | F |
| 485 | 4-Cl | 2-F, 3-Cl | F |
| 486 | 4-Cl | 2-F, 4-Cl | F |
| 487 | 4-Cl | 2-F, 5-Cl | F |
| 488 | 4-Cl | 2-F, 6-Cl | F |
| 489 | 4-Cl | 2,6-F₂, 4-Cl | F |
| 490 | 4-Cl | 3-F, 4-CI | F |
| 491 | 4-Cl | 3-Cl, 5-F | F |
| 492 | 4-Cl | 2-Cl, 5-F | F |
| 493 | 4-Cl | 3-CN, 4-Cl | F |
| 494 | 4-Cl | 3-NO₂, 4-Cl | F |
| 495 | 4-Cl | 2-F, 4-Br | F |
| 496 | 6-OCF₂H | 4-F | F |
| 497 | 6-OCF₂H | 4-CI | F |
| 498 | 6-OCF₂H | 4-Br | F |
| 499 | 6-OCF₂H | H | F |
| 500 | 6-OCF₂H | 4-Me | F |
| 501 | 6-OCF₂H | 4-CN | F |
| 502 | 6-OCF₂H | 4-NO₂ | F |
| 503 | 6-OCF₂H | 4-OMe | F |
| 504 | 6-OCF₂H | 3-F | F |
| 505 | 6-OCF₂H | 3-Cl | F |
| 506 | 6-OCF₂H | 3-Br | F |
| 507 | 6-OCF₂H | 3-Me | F |
| 508 | 6-OCF₂H | 3-CN | F |
| 509 | 6-OCF₂H | 3-NO₂ | F |
| 510 | 6-OCF₂H | 3-OMe | F |
| 511 | 6-OCF₂H | 2-F | F |
| 512 | 6-OCF₂H | 2-Cl | F |
| 513 | 6-OCF₂H | 2-Br | F |
| 514 | 6-OCF₂H | 2-Me | F |
| 515 | 6-OCF₂H | 2-CN | F |
| 516 | 6-OCF₂H | 2-NO₂ | F |
| 517 | 6-OCF₂H | 2-OMe | F |
| 518 | 6-OCF₂H | 2,3-F₂ | F |
| 519 | 6-OCF₂H | 2,4-F₂ | F |
| 520 | 6-OCF₂H | 2,5-F₂ | F |
| 521 | 6-OCF₂H | 2,6-F₂ | F |
| 522 | 6-OCF₂H | 3,4-F₂ | F |
| 523 | 6-OCF₂H | 3,5-F₂ | F |
| 524 | 6-OCF₂H | 2,3-Cl₂ | F |
| 525 | 6-OCF₂H | 2,4-Cl₂ | F |
| 526 | 6-OCF₂H | 2,5-Cl₂ | F |
| 527 | 6-OCF₂H | 2,6-Cl₂ | F |
| 528 | 6-OCF₂H | 3,4-Cl₂ | F |
| 529 | 6-OCF₂H | 3,5-Cl₂ | F |
| 530 | 6-OCF₂H | 2-F, 3-Cl | F |
| 531 | 6-OCF₂H | 2-F, 4-Cl | F |
| 532 | 6-OCF₂H | 2-F, 5-Cl | F |
| 533 | 6-OCF₂H | 2-F, 6-Cl | F |
| 534 | 6-OCF₂H | 2,6-F₂, 4-Cl | F |
| 535 | 6-OCF₂H | 3-F, 4-Cl | F |
| 536 | 6-OCF₂H | 3-Cl, 5-F | F |
| 537 | 6-OCF₂H | 2-Cl, 5-F | F |
| 538 | 6-OCF₂H | 3-CN, 4-Cl | F |
| 539 | 6-OCF₂H | 3-NO₂, 4-Cl | F |
| 540 | 6-OCF₂H | 2-F, 4-Br | F |
| 541 | H | 4-F | Cl |
| 542 | H | 4-Cl | Cl |
| 543 | H | 4-Br | Cl |
| 544 | H | H | Cl |
| 545 | H | 4-Me | Cl |
| 546 | H | 4-CN | Cl |
| 547 | H | 4-NO₂ | Cl |
| 548 | H | 4-OMe | Cl |
| 549 | H | 3-F | Cl |
| 550 | H | 3-Cl | Cl |
| 551 | H | 3-Br | Cl |
| 552 | H | 3-Me | Cl |
| 553 | H | 3-CN | Cl |
| 554 | H | 3-NO₂ | Cl |
| 555 | H | 3-OMe | Cl |
| 556 | H | 2-F | Cl |
| 557 | H | 2-Cl | Cl |
| 558 | H | 2-Br | Cl |
| 559 | H | 2-Me | Cl |
| 560 | H | 2-CN | Cl |
| 561 | H | 2-NO₂ | Cl |
| 562 | H | 2-OMe | Cl |
| 563 | H | 2,3-F₂ | Cl |
| 564 | H | 2,4-F₂ | Cl |
| 565 | H | 2,5-F₂ | Cl |
| 566 | H | 2,6-F₂ | Cl |
| 567 | H | 3,4-F₂ | Cl |
| 568 | H | 3,5-F₂ | Cl |
| 569 | H | 2,3-Cl₂ | Cl |
| 570 | H | 2,4-Cl₂ | Cl |
| 571 | H | 2,5-Cl₂ | Cl |
| 572 | H | 2,6-Cl₂ | Cl |
| 573 | H | 3,4-Cl₂ | Cl |
| 574 | H | 3,5-Cl₂ | Cl |
| 575 | H | 2-F, 3-Cl | Cl |
| 576 | H | 2-F, 4-Cl | Cl |
| 577 | H | 2-F, 5-Cl | Cl |
| 578 | H | 2-F, 6-Cl | Cl |
| 579 | H | 2,6-F₂, 4-Cl | Cl |
| 580 | H | 3-F, 4-Cl | Cl |
| 581 | H | 3-Cl, 5-F | Cl |
| 582 | H | 2-Cl, 5-F | Cl |
| 583 | H | 3-CN, 4-Cl | Cl |
| 584 | H | 3-NO₂, 4-Cl | Cl |
| 585 | H | 2-F, 4-Br | Cl |
| 586 | 6-F | 4-F | Cl |
| 587 | 6-F | 4-Cl | Cl |
| 588 | 6-F | 4-Br | Cl |
| 589 | 6-F | H | Cl |
| 590 | 6-F | 4-Me | Cl |
| 591 | 6-F | 4-CN | Cl |
| 592 | 6-F | 4-NO₂ | Cl |
| 593 | 6-F | 4-OMe | Cl |
| 594 | 6-F | 3-F | Cl |
| 595 | 6-F | 3-Cl | Cl |
| 596 | 6-F | 3-Br | Cl |
| 597 | 6-F | 3-Me | Cl |
| 598 | 6-F | 3-CN | Cl |
| 599 | 6-F | 3-NO₂ | Cl |
| 600 | 6-F | 3-OMe | Cl |
| 601 | 6-F | 2-F | Cl |
| 602 | 6-F | 2-Cl | Cl |
| 603 | 6-F | 2-Br | Cl |
| 604 | 6-F | 2-Me | Cl |
| 605 | 6-F | 2-CN | Cl |
| 606 | 6-F | 2-NO₂ | Cl |
| 607 | 6-F | 2-OMe | Cl |
| 608 | 6-F | 2,3-F₂ | Cl |
| 609 | 6-F | 2,4-F₂ | Cl |
| 610 | 6-F | 2,5-F₂ | Cl |
| 611 | 6-F | 2,6-F₂ | Cl |
| 612 | 6-F | 3,4-F₂ | Cl |
| 613 | 6-F | 3,5-F₂ | Cl |
| 614 | 6-F | 2,3-Cl₂ | Cl |
| 615 | 6-F | 2,4-Cl₂ | Cl |
| 616 | 6-F | 2,5-Cl₂ | Cl |
| 617 | 6-F | 2,6-Cl₂ | Cl |
| 618 | 6-F | 3,4-Cl₂ | Cl |
| 619 | 6-F | 3,5-Cl₂ | Cl |
| 620 | 6-F | 2-F, 3-Cl | Cl |
| 621 | 6-F | 2-F, 4-Cl | Cl |
| 622 | 6-F | 2-F, 5-Cl | Cl |
| 623 | 6-F | 2-F, 6-Cl | Cl |
| 624 | 6-F | 2,6-F₂, 4-Cl | Cl |
| 625 | 6-F | 3-F, 4-Cl | Cl |
| 626 | 6-F | 3-Cl, 5-F | Cl |
| 627 | 6-F | 2-Cl, 5-F | Cl |
| 628 | 6-F | 3-CN, 4-Cl | Cl |
| 629 | 6-F | 3-NO₂, 4-Cl | Cl |
| 630 | 6-F | 2-F, 4-Br | Cl |
| 631 | 6-Cl | 4-F | Cl |
| 632 | 6-Cl | 4-Cl | Cl |
| 633 | 6-Cl | 4-Br | Cl |
| 634 | 6-Cl | H | Cl |
| 635 | 6-Cl | 4-Me | Cl |
| 636 | 6-Cl | 4-CN | Cl |
| 637 | 6-Cl | 4-NO₂ | Cl |
| 638 | 6-Cl | 4-OMe | Cl |
| 639 | 6-Cl | 3-F | Cl |
| 640 | 6-Cl | 3-Cl | Cl |
| 641 | 6-Cl | 3-Br | Cl |
| 642 | 6-Cl | 3-Me | Cl |
| 643 | 6-Cl | 3-CN | Cl |
| 644 | 6-Cl | 3-NO₂ | Cl |
| 645 | 6-Cl | 3-OMe | Cl |
| 646 | 6-Cl | 2-F | Cl |
| 647 | 6-Cl | 2-Cl | Cl |
| 648 | 6-Cl | 2-Br | Cl |
| 649 | 6-Cl | 2-Me | Cl |
| 650 | 6-Cl | 2-CN | Cl |
| 651 | 6-Cl | 2-NO₂ | Cl |
| 652 | 6-Cl | 2-OMe | Cl |
| 653 | 6-Cl | 2,3-F₂ | Cl |
| 654 | 6-Cl | 2,4-F₂ | Cl |
| 655 | 6-Cl | 2,5-F₂ | Cl |
| 656 | 6-Cl | 2,6-F₂ | Cl |
| 657 | 6-Cl | 3,4-F₂ | Cl |
| 658 | 6-Cl | 3,5-F₂ | Cl |
| 659 | 6-Cl | 2,3-Cl₂ | Cl |
| 660 | 6-Cl | 2,4-Cl₂ | Cl |
| 661 | 6-Cl | 2,5-Cl₂ | Cl |
| 662 | 6-Cl | 2,6-Cl₂ | Cl |
| 663 | 6-Cl | 3,4-Cl₂ | Cl |
| 664 | 6-Cl | 3,5-Cl₂ | Cl |
| 665 | 6-Cl | 2-F, 3-Cl | Cl |
| 666 | 6-Cl | 2-F, 4-Cl | Cl |
| 667 | 6-Cl | 2-F, 5-Cl | Cl |
| 668 | 6-Cl | 2-F, 6-Cl | Cl |
| 669 | 6-Cl | 2,6-F₂, 4-Cl | Cl |
| 670 | 6-Cl | 3-F, 4-Cl | Cl |
| 671 | 6-Cl | 3-Cl, 5-F | Cl |
| 672 | 6-Cl | 2-Cl, 5-F | Cl |
| 673 | 6-Cl | 3-CN, 4-Cl | Cl |
| 674 | 6-Cl | 3-NO₂, 4-Cl | Cl |
| 675 | 6-Cl | 2-F, 4-Br | Cl |
| 676 | 6-Br | 4-F | Cl |
| 677 | 6-Br | 4-Cl | Cl |
| 678 | 6-Br | 4-Br | Cl |
| 679 | 6-Br | H | Cl |
| 680 | 6-Br | 4-Me | Cl |
| 681 | 6-Br | 4-CN | Cl |
| 682 | 6-Br | 4-NO₂ | Cl |
| 683 | 6-Br | 4-OMe | Cl |
| 684 | 6-Br | 3-F | Cl |
| 685 | 6-Br | 3-Cl | Cl |
| 686 | 6-Br | 3-Br | Cl |
| 687 | 6-Br | 3-Me | Cl |
| 688 | 6-Br | 3-CN | Cl |
| 689 | 6-Br | 3-NO₂ | Cl |
| 690 | 6-Br | 3-OMe | Cl |
| 691 | 6-Br | 2-F | Cl |
| 692 | 6-Br | 2-Cl | Cl |
| 693 | 6-Br | 2-Br | Cl |
| 694 | 6-Br | 2-Me | Cl |
| 695 | 6-Br | 2-CN | Cl |
| 696 | 6-Br | 2-NO₂ | Cl |
| 697 | 6-Br | 2-OMe | Cl |
| 698 | 6-Br | 2,3-F₂ | Cl |
| 699 | 6-Br | 2,4-F₂ | Cl |
| 700 | 6-Br | 2,5-F₂ | Cl |
| 701 | 6-Br | 2,6-F₂ | Cl |
| 702 | 6-Br | 3,4-F₂ | Cl |
| 703 | 6-Br | 3,5-F₂ | Cl |
| 704 | 6-Br | 2,3-Cl₂ | Cl |
| 705 | 6-Br | 2,4-Cl₂ | Cl |
| 706 | 6-Br | 2,5-Cl₂ | Cl |
| 707 | 6-Br | 2,6-Cl₂ | Cl |
| 708 | 6-Br | 3,4-Cl₂ | Cl |
| 709 | 6-Br | 3,5-Cl₂ | Cl |
| 710 | 6-Br | 2-F, 3-Cl | Cl |
| 711 | 6-Br | 2-F, 4-Cl | Cl |
| 712 | 6-Br | 2-F, 5-Cl | Cl |
| 713 | 6-Br | 2-F, 6-Cl | Cl |
| 714 | 6-Br | 2,6-F₂, 4-Cl | Cl |
| 715 | 6-Br | 3-F, 4-Cl | Cl |
| 716 | 6-Br | 3-Cl, 5-F | Cl |
| 717 | 6-Br | 2-Cl, 5-F | Cl |
| 718 | 6-Br | 3-CN, 4-Cl | Cl |
| 719 | 6-Br | 3-NO₂, 4-Cl | Cl |
| 720 | 6-Br | 2-F, 4-Br | Cl |
| 721 | 6-CN | 4-F | Cl |
| 722 | 6-CN | 4-Cl | Cl |
| 723 | 6-CN | 4-Br | Cl |
| 724 | 6-CN | H | Cl |
| 725 | 6-CN | 4-Me | Cl |
| 726 | 6-CN | 4-CN | Cl |
| 727 | 6-CN | 4-NO₂ | Cl |
| 728 | 6-CN | 4-OMe | Cl |
| 729 | 6-CN | 3-F | Cl |
| 730 | 6-CN | 3-Cl | Cl |
| 731 | 6-CN | 3-Br | Cl |
| 732 | 6-CN | 3-Me | Cl |
| 733 | 6-CN | 3-CN | Cl |
| 734 | 6-CN | 3-NO₂ | Cl |
| 735 | 6-CN | 3-OMe | Cl |
| 736 | 6-CN | 2-F | Cl |
| 737 | 6-CN | 2-Cl | Cl |
| 738 | 6-CN | 2-Br | Cl |
| 739 | 6-CN | 2-Me | Cl |
| 740 | 6-CN | 2-CN | Cl |
| 741 | 6-CN | 2-NO₂ | Cl |
| 742 | 6-CN | 2-OMe | Cl |
| 743 | 6-CN | 2,3-F₂ | Cl |
| 744 | 6-CN | 2,4-F₂ | Cl |
| 745 | 6-CN | 2,5-F₂ | Cl |
| 746 | 6-CN | 2,6-F₂ | Cl |
| 747 | 6-CN | 3,4-F₂ | Cl |
| 748 | 6-CN | 3,5-F₂ | Cl |
| 749 | 6-CN | 2,3-Cl₂ | Cl |
| 750 | 6-CN | 2,4-Cl₂ | Cl |
| 751 | 6-CN | 2,5-Cl₂ | Cl |
| 752 | 6-CN | 2,6-Cl₂ | Cl |
| 753 | 6-CN | 3,4-Cl₂ | Cl |
| 754 | 6-CN | 3,5-Cl₂ | Cl |
| 755 | 6-CN | 2-F, 3-Cl | Cl |
| 756 | 6-CN | 2-F, 4-Cl | Cl |
| 757 | 6-CN | 2-F, 5-Cl | Cl |
| 758 | 6-CN | 2-F, 6-Cl | Cl |
| 759 | 6-CN | 2,6-F₂, 4-Cl | Cl |
| 760 | 6-CN | 3-F, 4-Cl | Cl |
| 761 | 6-CN | 3-Cl, 5-F | Cl |
| 762 | 6-CN | 2-Cl, 5-F | Cl |
| 763 | 6-CN | 3-CN, 4-Cl | Cl |
| 764 | 6-CN | 3-NO₂, 4-Cl | Cl |
| 765 | 6-CN | 2-F, 4-Br | Cl |
| 766 | 6-Me | 4-F | Cl |
| 767 | 6-Me | 4-Cl | Cl |
| 768 | 6-Me | 4-Br | Cl |
| 769 | 6-Me | H | Cl |
| 770 | 6-Me | 4-Me | Cl |
| 771 | 6-Me | 4-CN | Cl |
| 772 | 6-Me | 4-N0₂ | Cl |
| 773 | 6-Me | 4-OMe | Cl |
| 774 | 6-Me | 3-F | Cl |
| 775 | 6-Me | 3-Cl | Cl |
| 776 | 6-Me | 3-Br | Cl |
| 777 | 6-Me | 3-Me | Cl |
| 778 | 6-Me | 3-CN | Cl |
| 779 | 6-Me | 3-NO₂ | Cl |
| 780 | 6-Me | 3-OMe | Cl |
| 781 | 6-Me | 2-F | Cl |
| 782 | 6-Me | 2-Cl | Cl |
| 783 | 6-Me | 2-Br | Cl |
| 784 | 6-Me | 2-Me | Cl |
| 785 | 6-Me | 2-CN | Cl |
| 786 | 6-Me | 2-N0₂ | Cl |
| 787 | 6-Me | 2-OMe | Cl |
| 788 | 6-Me | 2,3-F₂ | Cl |
| 789 | 6-Me | 2,4-F₂ | Cl |
| 790 | 6-Me | 2,5-F₂ | Cl |
| 791 | 6-Me | 2,6-F₂ | Cl |
| 792 | 6-Me | 3,4-F₂ | Cl |
| 793 | 6-Me | 3,5-F₂ | Cl |
| 794 | 6-Me | 2,3-Cl₂ | Cl |
| 795 | 6-Me | 2,4-Cl₂ | Cl |
| 796 | 6-Me | 2,5-Cl₂ | Cl |
| 797 | 6-Me | 2,6-Cl₂ | Cl |
| 798 | 6-Me | 3,4-Cl₂ | Cl |
| 799 | 6-Me | 3,5-Cl₂ | Cl |
| 800 | 6-Me | 2-F, 3-Cl | Cl |
| 801 | 6-Me | 2-F, 4-Cl | Cl |
| 802 | 6-Me | 2-F, 5-Cl | Cl |
| 803 | 6-Me | 2-F, 6-Cl | Cl |
| 804 | 6-Me | 2,6-F₂, 4-Cl | Cl |
| 805 | 6-Me | 3-F, 4-Cl | Cl |
| 806 | 6-Me | 3-Cl, 5-F | Cl |
| 807 | 6-Me | 2-Cl, 5-F | Cl |
| 808 | 6-Me | 3-CN, 4-Cl | Cl |
| 809 | 6-Me | 3-NO₂, 4-Cl | Cl |
| 810 | 6-Me | 2-F, 4-Br | Cl |
| 811 | 6-OMe | 4-F | Cl |
| 812 | 6-OMe | 4-Cl | Cl |
| 813 | 6-OMe | 4-Br | Cl |
| 814 | 6-OMe | H | Cl |
| 815 | 6-OMe | 4-Me | Cl |
| 816 | 6-OMe | 4-CN | Cl |
| 817 | 6-OMe | 4-NO₂ | Cl |
| 818 | 6-OMe | 4-OMe | Cl |
| 819 | 6-OMe | 3-F | Cl |
| 820 | 6-OMe | 3-Cl | Cl |
| 821 | 6-OMe | 3-Br | Cl |
| 822 | 6-OMe | 3-Me | Cl |
| 823 | 6-OMe | 3-CN | Cl |
| 824 | 6-OMe | 3-NO₂ | Cl |
| 825 | 6-OMe | 3-OMe | Cl |
| 826 | 6-OMe | 2-F | Cl |
| 827 | 6-OMe | 2-Cl | Cl |
| 828 | 6-OMe | 2-Br | Cl |
| 829 | 6-OMe | 2-Me | Cl |
| 830 | 6-OMe | 2-CN | Cl |
| 831 | 6-OMe | 2-NO₂ | Cl |
| 832 | 6-OMe | 2-OMe | Cl |
| 833 | 6-OMe | 2,3-F₂ | Cl |
| 834 | 6-OMe | 2,4-F₂ | Cl |
| 835 | 6-OMe | 2,5-F₂ | Cl |
| 836 | 6-OMe | 2,6-F₂ | Cl |
| 837 | 6-OMe | 3,4-F₂ | Cl |
| 838 | 6-OMe | 3,5-F₂ | Cl |
| 839 | 6-OMe | 2,3-Cl₂ | Cl |
| 840 | 6-OMe | 2,4-Cl₂ | Cl |
| 841 | 6-OMe | 2,5-Cl₂ | Cl |
| 842 | 6-OMe | 2,6-Cl₂ | Cl |
| 843 | 6-OMe | 3,4-Cl₂ | Cl |
| 844 | 6-OMe | 3,5-Cl₂ | Cl |
| 845 | 6-OMe | 2-F, 3-Cl | Cl |
| 846 | 6-OMe | 2-F, 4-Cl | Cl |
| 847 | 6-OMe | 2-F, 5-Cl | Cl |
| 848 | 6-OMe | 2-F, 6-Cl | Cl |
| 849 | 6-OMe | 2,6-F₂, 4-Cl | Cl |
| 850 | 6-OMe | 3-F, 4-Cl | Cl |
| 851 | 6-OMe | 3-Cl, 5-F | Cl |
| 852 | 6-OMe | 2-Cl, 5-F | Cl |
| 853 | 6-OMe | 3-CN, 4-Cl | Cl |
| 854 | 6-OMe | 3-NO₂, 4-Cl | Cl |
| 855 | 6-OMe | 2-F, 4-Br | Cl |
| 856 | 2-F | 4-F | Cl |
| 857 | 2-F | 4-Cl | Cl |
| 858 | 2-F | 4-Br | Cl |
| 859 | 2-F | H | Cl |
| 860 | 2-F | 4-Me | Cl |
| 861 | 2-F | 4-CN | Cl |
| 862 | 2-F | 4-NO₂ | Cl |
| 863 | 2-F | 4-OMe | Cl |
| 864 | 2-F | 3-F | Cl |
| 865 | 2-F | 3-Cl | Cl |
| 866 | 2-F | 3-Br | Cl |
| 867 | 2-F | 3-Me | Cl |
| 868 | 2-F | 3-CN | Cl |
| 869 | 2-F | 3-NO₂ | Cl |
| 870 | 2-F | 3-OMe | Cl |
| 871 | 2-F | 2-F | Cl |
| 872 | 2-F | 2-Cl | Cl |
| 873 | 2-F | 2-Br | Cl |
| 874 | 2-F | 2-Me | Cl |
| 875 | 2-F | 2-CN | Cl |
| 876 | 2-F | 2-NO₂ | Cl |
| 877 | 2-F | 2-OMe | Cl |
| 878 | 2-F | 2,3-F₂ | Cl |
| 879 | 2-F | 2,4-F₂ | Cl |
| 880 | 2-F | 2,5-F₂ | Cl |
| 881 | 2-F | 2,6-F₂ | Cl |
| 882 | 2-F | 3,4-F₂ | Cl |
| 883 | 2-F | 3,5-F₂ | Cl |
| 884 | 2-F | 2,3-Cl₂ | Cl |
| 885 | 2-F | 2,4-Cl₂ | Cl |
| 886 | 2-F | 2,5-Cl₂ | Cl |
| 887 | 2-F | 2,6-Cl₂ | Cl |
| 888 | 2-F | 3,4-Cl₂ | Cl |
| 889 | 2-F | 3,5-Cl₂ | Cl |
| 890 | 2-F | 2-F, 3-Cl | Cl |
| 891 | 2-F | 2-F, 4-Cl | Cl |
| 892 | 2-F | 2-F, 5-Cl | Cl |
| 893 | 2-F | 2-F, 6-Cl | Cl |
| 894 | 2-F | 2,6-F₂, 4-Cl | Cl |
| 895 | 2-F | 3-F, 4-Cl | Cl |
| 896 | 2-F | 3-Cl, 5-F | Cl |
| 897 | 2-F | 2-Cl, 5-F | Cl |
| 898 | 2-F | 3-CN, 4-Cl | Cl |
| 899 | 2-F | 3-NO₂, 4-Cl | Cl |
| 900 | 2-F | 2-F, 4-Br | Cl |
| 901 | 4-F | 4-F | Cl |
| 902 | 4-F | 4-Cl | Cl |
| 903 | 4-F | 4-Br | Cl |
| 904 | 4-F | H | Cl |
| 905 | 4-F | 4-Me | Cl |
| 906 | 4-F | 4-CN | Cl |
| 907 | 4-F | 4-NO₂ | Cl |
| 908 | 4-F | 4-OMe | Cl |
| 909 | 4-F | 3-F | Cl |
| 910 | 4-F | 3-Cl | Cl |
| 911 | 4-F | 3-Br | Cl |
| 912 | 4-F | 3-Me | Cl |
| 913 | 4-F | 3-CN | Cl |
| 914 | 4-F | 3-NO₂ | Cl |
| 915 | 4-F | 3-OMe | Cl |
| 916 | 4-F | 2-F | Cl |
| 917 | 4-F | 2-Cl | Cl |
| 918 | 4-F | 2-Br | Cl |
| 919 | 4-F | 2-Me | Cl |
| 920 | 4-F | 2-CN | Cl |
| 921 | 4-F | 2-NO₂ | Cl |
| 922 | 4-F | 2-OMe | Cl |
| 923 | 4-F | 2,3-F₂ | Cl |
| 924 | 4-F | 2,4-F₂ | Cl |
| 925 | 4-F | 2,5-F₂ | Cl |
| 926 | 4-F | 2,6-F₂ | Cl |
| 927 | 4-F | 3,4-F₂ | Cl |
| 928 | 4-F | 3,5-F₂ | Cl |
| 929 | 4-F | 2,3-Cl₂ | Cl |
| 930 | 4-F | 2,4-Cl₂ | Cl |
| 931 | 4-F | 2,5-Cl₂ | Cl |
| 932 | 4-F | 2,6-Cl₂ | Cl |
| 933 | 4-F | 3,4-Cl₂ | Cl |
| 934 | 4-F | 3,5-Cl₂ | Cl |
| 935 | 4-F | 2-F, 3-Cl | Cl |
| 936 | 4-F | 2-F, 4-Cl | Cl |
| 937 | 4-F | 2-F, 5-Cl | Cl |
| 938 | 4-F | 2-F, 6-Cl | Cl |
| 939 | 4-F | 2,6-F₂, 4-Cl | Cl |
| 940 | 4-F | 3-F, 4-Cl | Cl |
| 941 | 4-F | 3-Cl, 5-F | Cl |
| 942 | 4-F | 2-Cl, 5-F | Cl |
| 943 | 4-F | 3-CN, 4-Cl | Cl |
| 944 | 4-F | 3-NO₂, 4-Cl | Cl |
| 945 | 4-F | 2-F, 4-Br | Cl |
| 946 | 2-Cl | 4-F | Cl |
| 947 | 2-Cl | 4-Cl | Cl |
| 948 | 2-Cl | 4-Br | Cl |
| 949 | 2-Cl | H | Cl |
| 950 | 2-Cl | 4-Me | Cl |
| 951 | 2-Cl | 4-CN | Cl |
| 952 | 2-Cl | 4-NO₂ | Cl |
| 953 | 2-Cl | 4-OMe | Cl |
| 954 | 2-Cl | 3-F | Cl |
| 955 | 2-Cl | 3-Cl | Cl |
| 956 | 2-Cl | 3-Br | Cl |
| 957 | 2-Cl | 3-Me | Cl |
| 958 | 2-Cl | 3-CN | Cl |
| 959 | 2-Cl | 3-NO₂ | Cl |
| 960 | 2-Cl | 3-OMe | Cl |
| 961 | 2-Cl | 2-F | Cl |
| 962 | 2-Cl | 2-Cl | Cl |
| 963 | 2-Cl | 2-Br | Cl |
| 964 | 2-Cl | 2-Me | Cl |
| 965 | 2-Cl | 2-CN | Cl |
| 966 | 2-Cl | 2-NO₂ | Cl |
| 967 | 2-Cl | 2-OMe | Cl |
| 968 | 2-Cl | 2,3-F₂ | Cl |
| 969 | 2-Cl | 2,4-F₂ | Cl |
| 970 | 2-Cl | 2,5-F₂ | Cl |
| 971 | 2-Cl | 2,6-F₂ | Cl |
| 972 | 2-Cl | 3,4-F₂ | Cl |
| 973 | 2-Cl | 3,5-F₂ | Cl |
| 974 | 2-Cl | 2,3-Cl₂ | Cl |
| 975 | 2-Cl | 2,4-Cl₂ | Cl |
| 976 | 2-Cl | 2,5-Cl₂ | Cl |
| 977 | 2-Cl | 2,6-Cl₂ | Cl |
| 978 | 2-Cl | 3,4-Cl₂ | Cl |
| 979 | 2-Cl | 3,5-Cl₂ | Cl |
| 980 | 2-Cl | 2-F, 3-Cl | Cl |
| 981 | 2-Cl | 2-F, 4-Cl | Cl |
| 982 | 2-Cl | 2-F, 5-Cl | Cl |
| 983 | 2-Cl | 2-F, 6-Cl | Cl |
| 984 | 2-Cl | 2,6-F₂, 4-Cl | Cl |
| 985 | 2-Cl | 3-F, 4-Cl | Cl |
| 986 | 2-Cl | 3-Cl, 5-F | Cl |
| 987 | 2-Cl | 2-Cl, 5-F | Cl |
| 988 | 2-Cl | 3-CN, 4-Cl | Cl |
| 989 | 2-Cl | 3-NO₂, 4-Cl | Cl |
| 990 | 2-Cl | 2-F, 4-Br | Cl |
| 991 | 4-Cl | 4-F | Cl |
| 992 | 4-Cl | 4-Cl | Cl |
| 993 | 4-Cl | 4-Br | Cl |
| 994 | 4-Cl | H | Cl |
| 995 | 4-Cl | 4-Me | Cl |
| 996 | 4-Cl | 4-CN | Cl |
| 997 | 4-Cl | 4-NO₂ | Cl |
| 998 | 4-Cl | 4-OMe | Cl |
| 999 | 4-Cl | 3-F | Cl |
| 1000 | 4-Cl | 3-Cl | Cl |
| 1001 | 4-Cl | 3-Br | Cl |
| 1002 | 4-Cl | 3-Me | Cl |
| 1003 | 4-Cl | 3-CN | Cl |
| 1004 | 4-Cl | 3-NO₂ | Cl |
| 1005 | 4-Cl | 3-OMe | Cl |
| 1006 | 4-Cl | 2-F | Cl |
| 1007 | 4-Cl | 2-Cl | Cl |
| 1008 | 4-Cl | 2-Br | Cl |
| 1009 | 4-Cl | 2-Me | Cl |
| 1010 | 4-Cl | 2-CN | Cl |
| 1011 | 4-Cl | 2-NO₂ | Cl |
| 1012 | 4-Cl | 2-OMe | Cl |
| 1013 | 4-Cl | 2,3-F₂ | Cl |
| 1014 | 4-Cl | 2,4-F₂ | Cl |
| 1015 | 4-Cl | 2,5-F₂ | Cl |
| 1016 | 4-Cl | 2,6-F₂ | Cl |
| 1017 | 4-Cl | 3,4-F₂ | Cl |
| 1018 | 4-Cl | 3,5-F₂ | Cl |
| 1019 | 4-Cl | 2,3-Cl₂ | Cl |
| 1020 | 4-Cl | 2,4-Cl₂ | Cl |
| 1021 | 4-Cl | 2,5-Cl₂ | Cl |
| 1022 | 4-Cl | 2,6-Cl₂ | Cl |
| 1023 | 4-Cl | 3,4-Cl₂ | Cl |
| 1024 | 4-Cl | 3,5-Cl₂ | Cl |
| 1025 | 4-Cl | 2-F, 3-Cl | Cl |
| 1026 | 4-Cl | 2-F, 4-Cl | Cl |
| 1027 | 4-Cl | 2-F, 5-Cl | Cl |
| 1028 | 4-Cl | 2-F, 6-Cl | Cl |
| 1029 | 4-Cl | 2,6-F₂, 4-Cl | Cl |
| 1030 | 4-Cl | 3-F, 4-Cl | Cl |
| 1031 | 4-Cl | 3-Cl, 5-F | Cl |
| 1032 | 4-Cl | 2-Cl, 5-F | Cl |
| 1033 | 4-Cl | 3-CN, 4-Cl | Cl |
| 1034 | 4-Cl | 3-NO₂, 4-Cl | Cl |
| 1035 | 4-Cl | 2-F, 4-Br | Cl |
| 1036 | 6-OCF₂H | 4-F | Cl |
| 1037 | 6-OCF₂H | 4-Cl | Cl |
| 1038 | 6-OCF₂H | 4-Br | Cl |
| 1039 | 6-OCF₂H | H | Cl |
| 1040 | 6-OCF₂H | 4-Me | Cl |
| 1041 | 6-OCF₂H | 4-CN | Cl |
| 1042 | 6-OCF₂H | 4-NO₂ | Cl |
| 1043 | 6-OCF₂H | 4-OMe | Cl |
| 1044 | 6-OCF₂H | 3-F | Cl |
| 1045 | 6-OCF₂H | 3-CI | Cl |
| 1046 | 6-OCF₂H | 3-Br | Cl |
| 1047 | 6-OCF₂H | 3-Me | Cl |
| 1048 | 6-OCF₂H | 3-CN | Cl |
| 1049 | 6-OCF₂H | 3-NO₂ | Cl |
| 1050 | 6-OCF₂H | 3-OMe | Cl |
| 1051 | 6-OCF₂H | 2-F | Cl |
| 1052 | 6-OCF₂H | 2-Cl | Cl |
| 1053 | 6-OCF₂H | 2-Br | Cl |
| 1054 | 6-OCF₂H | 2-Me | Cl |
| 1055 | 6-OCF₂H | 2-CN | Cl |
| 1056 | 6-OCF₂H | 2-NO₂ | Cl |
| 1057 | 6-OCF₂H | 2-OMe | Cl |
| 1058 | 6-OCF₂H | 2,3-F₂ | Cl |
| 1059 | 6-OCF₂H | 2,4-F₂ | Cl |
| 1060 | 6-OCF₂H | 2,5-F₂ | Cl |
| 1061 | 6-OCF₂H | 2,6-F₂ | Cl |
| 1062 | 6-OCF₂H | 3,4-F₂ | Cl |
| 1063 | 6-OCF₂H | 3,5-F₂ | Cl |
| 1064 | 6-OCF₂H | 2,3-Cl₂ | Cl |
| 1065 | 6-OCF₂H | 2,4-Cl₂ | Cl |
| 1066 | 6-OCF₂H | 2,5-Cl₂ | Cl |
| 1067 | 6-OCF₂H | 2,6-Cl₂ | Cl |
| 1068 | 6-OCF₂H | 3,4-Cl₂ | Cl |
| 1069 | 6-OCF₂H | 3,5-Cl₂ | Cl |
| 1070 | 6-OCF₂H | 2-F, 3-Cl | Cl |
| 1071 | 6-OCF₂H | 2-F, 4-Cl | Cl |
| 1072 | 6-OCF₂H | 2-F, 5-Cl | Cl |
| 1073 | 6-OCF₂H | 2-F, 6-Cl | Cl |
| 1074 | 6-OCF₂H | 2,6-F₂, 4-Cl | Cl |
| 1075 | 6-OCF₂H | 3-F, 4-Cl | Cl |
| 1076 | 6-OCF₂H | 3-Cl, 5-F | Cl |
| 1077 | 6-OCF₂H | 2-Cl, 5-F | Cl |
| 1078 | 6-OCF₂H | 3-CN, 4-Cl | Cl |
| 1079 | 6-OCF₂H | 3-NO₂, 4-Cl | Cl |
| 1080 | 6-OCF₂H | 2-F, 4-Br | Cl |
| 1081 | H | 4-F | Br |
| 1082 | H | 4-Cl | Br |
| 1083 | H | 4-Br | Br |
| 1084 | H | H | Br |
| 1085 | H | 4-Me | Br |
| 1086 | H | 4-CN | Br |
| 1087 | H | 4-NO₂ | Br |
| 1088 | H | 4-OMe | Br |
| 1089 | H | 3-F | Br |
| 1090 | H | 3-Cl | Br |
| 1091 | H | 3-Br | Br |
| 1092 | H | 3-Me | Br |
| 1093 | H | 3-CN | Br |
| 1094 | H | 3-NO₂ | Br |
| 1095 | H | 3-OMe | Br |
| 1096 | H | 2-F | Br |
| 1097 | H | 2-Cl | Br |
| 1098 | H | 2-Br | Br |
| 1099 | H | 2-Me | Br |
| 1100 | H | 2-CN | Br |
| 1101 | H | 2-NO₂ | Br |
| 1102 | H | 2-OMe | Br |
| 1103 | H | 2,3-F₂ | Br |
| 1104 | H | 2,4-F₂ | Br |
| 1105 | H | 2,5-F₂ | Br |
| 1106 | H | 2,6-F₂ | Br |
| 1107 | H | 3,4-F₂ | Br |
| 1108 | H | 3,5-F₂ | Br |
| 1109 | H | 2,3-Cl₂ | Br |
| 1110 | H | 2,4-Cl₂ | Br |
| 1111 | H | 2,5-Cl₂ | Br |
| 1112 | H | 2,6-Cl₂ | Br |
| 1113 | H | 3,4-Cl₂ | Br |
| 1114 | H | 3,5-Cl₂ | Br |
| 1115 | H | 2-F, 3-Cl | Br |
| 1116 | H | 2-F, 4-Cl | Br |
| 1117 | H | 2-F, 5-Cl | Br |
| 1118 | H | 2-F, 6-Cl | Br |
| 1119 | H | 2,6-F₂, 4-Cl | Br |
| 1120 | H | 3-F, 4-Cl | Br |
| 1121 | H | 3-Cl, 5-F | Br |
| 1122 | H | 2-Cl, 5-F | Br |
| 1123 | H | 3-CN, 4-Cl | Br |
| 1124 | H | 3-NO₂, 4-Cl | Br |
| 1125 | H | 2-F, 4-Br | Br |
| 1126 | 6-F | 4-F | Br |
| 1127 | 6-F | 4-Cl | Br |
| 1128 | 6-F | 4-Br | Br |
| 1129 | 6-F | H | Br |
| 1130 | 6-F | 4-Me | Br |
| 1131 | 6-F | 4-CN | Br |
| 1132 | 6-F | 4-NO₂ | Br |
| 1133 | 6-F | 4-OMe | Br |
| 1134 | 6-F | 3-F | Br |
| 1135 | 6-F | 3-Cl | Br |
| 1136 | 6-F | 3-Br | Br |
| 1137 | 6-F | 3-Me | Br |
| 1138 | 6-F | 3-CN | Br |
| 1139 | 6-F | 3-NO₂ | Br |
| 1140 | 6-F | 3-OMe | Br |
| 1141 | 6-F | 2-F | Br |
| 1142 | 6-F | 2-Cl | Br |
| 1143 | 6-F | 2-Br | Br |
| 1144 | 6-F | 2-Me | Br |
| 1145 | 6-F | 2-CN | Br |
| 1146 | 6-F | 2-NO₂ | Br |
| 1147 | 6-F | 2-OMe | Br |
| 1148 | 6-F | 2,3-F₂ | Br |
| 1149 | 6-F | 2,4-F₂ | Br |
| 1150 | 6-F | 2,5-F₂ | Br |
| 1151 | 6-F | 2,6-F₂ | Br |
| 1152 | 6-F | 3,4-F₂ | Br |
| 1153 | 6-F | 3,5-F₂ | Br |
| 1154 | 6-F | 2,3-Cl₂ | Br |
| 1155 | 6-F | 2,4-Cl₂ | Br |
| 1156 | 6-F | 2,5-Cl₂ | Br |
| 1157 | 6-F | 2,6-Cl₂ | Br |
| 1158 | 6-F | 3,4-Cl₂ | Br |
| 1159 | 6-F | 3,5-Cl₂ | Br |
| 1160 | 6-F | 2-F, 3-Cl | Br |
| 1161 | 6-F | 2-F, 4-Cl | Br |
| 1162 | 6-F | 2-F, 5-Cl | Br |
| 1163 | 6-F | 2-F, 6-Cl | Br |
| 1164 | 6-F | 2,6-F₂, 4-Cl | Br |
| 1165 | 6-F | 3-F, 4-Cl | Br |
| 1166 | 6-F | 3-Cl, 5-F | Br |
| 1167 | 6-F | 2-Cl, 5-F | Br |
| 1168 | 6-F | 3-CN, 4-Cl | Br |
| 1169 | 6-F | 3-NO₂, 4-Cl | Br |
| 1170 | 6-F | 2-F, 4-Br | Br |
| 1171 | 6-Cl | 4-F | Br |
| 1172 | 6-Cl | 4-CI | Br |
| 1173 | 6-Cl | 4-Br | Br |
| 1174 | 6-Cl | H | Br |
| 1175 | 6-Cl | 4-Me | Br |
| 1176 | 6-Cl | 4-CN | Br |
| 1177 | 6-Cl | 4-NO₂ | Br |
| 1178 | 6-Cl | 4-OMe | Br |
| 1179 | 6-Cl | 3-F | Br |
| 1180 | 6-Cl | 3-Cl | Br |
| 1181 | 6-Cl | 3-Br | Br |
| 1182 | 6-Cl | 3-Me | Br |
| 1183 | 6-Cl | 3-CN | Br |
| 1184 | 6-Cl | 3-NO₂ | Br |
| 1185 | 6-Cl | 3-OMe | Br |
| 1186 | 6-Cl | 2-F | Br |
| 1187 | 6-Cl | 2-Cl | Br |
| 1188 | 6-Cl | 2-Br | Br |
| 1189 | 6-Cl | 2-Me | Br |
| 1190 | 6-Cl | 2-CN | Br |
| 1191 | 6-Cl | 2-NO₂ | Br |
| 1192 | 6-Cl | 2-OMe | Br |
| 1193 | 6-Cl | 2,3-F₂ | Br |
| 1194 | 6-Cl | 2,4-F₂ | Br |
| 1195 | 6-Cl | 2,5-F₂ | Br |
| 1196 | 6-Cl | 2,6-F₂ | Br |
| 1197 | 6-Cl | 3,4-F₂ | Br |
| 1198 | 6-Cl | 3,5-F₂ | Br |
| 1199 | 6-Cl | 2,3-Cl₂ | Br |
| 1200 | 6-Cl | 2,4-Cl₂ | Br |
| 1201 | 6-Cl | 2,5-Cl₂ | Br |
| 1202 | 6-Cl | 2,6-Cl₂ | Br |
| 1203 | 6-Cl | 3,4-Cl₂ | Br |
| 1204 | 6-Cl | 3,5-Cl₂ | Br |
| 1205 | 6-Cl | 2-F, 3-Cl | Br |
| 1206 | 6-Cl | 2-F, 4-Cl | Br |
| 1207 | 6-Cl | 2-F, 5-Cl | Br |
| 1208 | 6-Cl | 2-F, 6-Cl | Br |
| 1209 | 6-Cl | 2,6-F₂, 4-Cl | Br |
| 1210 | 6-Cl | 3-F, 4-Cl | Br |
| 1211 | 6-Cl | 3-Cl, 5-F | Br |
| 1212 | 6-Cl | 2-Cl, 5-F | Br |
| 1213 | 6-Cl | 3-CN, 4-Cl | Br |
| 1214 | 6-Cl | 3-NO₂, 4-Cl | Br |
| 1215 | 6-Cl | 2-F, 4-Br | Br |
| 1216 | 6-Br | 4-F | Br |
| 1217 | 6-Br | 4-Cl | Br |
| 1218 | 6-Br | 4-Br | Br |
| 1219 | 6-Br | H | Br |
| 1220 | 6-Br | 4-Me | Br |
| 1221 | 6-Br | 4-CN | Br |
| 1222 | 6-Br | 4-NO₂ | Br |
| 1223 | 6-Br | 4-OMe | Br |
| 1224 | 6-Br | 3-F | Br |
| 1225 | 6-Br | 3-Cl | Br |
| 1226 | 6-Br | 3-Br | Br |
| 1227 | 6-Br | 3-Me | Br |
| 1228 | 6-Br | 3-CN | Br |
| 1229 | 6-Br | 3-NO₂ | Br |
| 1230 | 6-Br | 3-OMe | Br |
| 1231 | 6-Br | 2-F | Br |
| 1232 | 6-Br | 2-Cl | Br |
| 1233 | 6-Br | 2-Br | Br |
| 1234 | 6-Br | 2-Me | Br |
| 1235 | 6-Br | 2-CN | Br |
| 1236 | 6-Br | 2-NO₂ | Br |
| 1237 | 6-Br | 2-OMe | Br |
| 1238 | 6-Br | 2,3-F₂ | Br |
| 1239 | 6-Br | 2,4-F₂ | Br |
| 1240 | 6-Br | 2,5-F₂ | Br |
| 1241 | 6-Br | 2,6-F₂ | Br |
| 1242 | 6-Br | 3,4-F₂ | Br |
| 1243 | 6-Br | 3,5-F₂ | Br |
| 1244 | 6-Br | 2,3-Cl₂ | Br |
| 1245 | 6-Br | 2,4-Cl₂ | Br |
| 1246 | 6-Br | 2,5-Cl₂ | Br |
| 1247 | 6-Br | 2,6-Cl₂ | Br |
| 1248 | 6-Br | 3,4-Cl₂ | Br |
| 1249 | 6-Br | 3,5-Cl₂ | Br |
| 1250 | 6-Br | 2-F, 3-Cl | Br |
| 1251 | 6-Br | 2-F, 4-Cl | Br |
| 1252 | 6-Br | 2-F, 5-Cl | Br |
| 1253 | 6-Br | 2-F, 6-Cl | Br |
| 1254 | 6-Br | 2,6-F₂, 4-Cl | Br |
| 1255 | 6-Br | 3-F, 4-Cl | Br |
| 1256 | 6-Br | 3-Cl, 5-F | Br |
| 1257 | 6-Br | 2-Cl, 5-F | Br |
| 1258 | 6-Br | 3-CN, 4-Cl | Br |
| 1259 | 6-Br | 3-NO₂, 4-Cl | Br |
| 1260 | 6-Br | 2-F, 4-Br | Br |
| 1261 | 6-CN | 4-F | Br |
| 1262 | 6-CN | 4-Cl | Br |
| 1263 | 6-CN | 4-Br | Br |
| 1264 | 6-CN | H | Br |
| 1265 | 6-CN | 4-Me | Br |
| 1266 | 6-CN | 4-CN | Br |
| 1267 | 6-CN | 4-NO₂ | Br |
| 1268 | 6-CN | 4-OMe | Br |
| 1269 | 6-CN | 3-F | Br |
| 1270 | 6-CN | 3-Cl | Br |
| 1271 | 6-CN | 3-Br | Br |
| 1272 | 6-CN | 3-Me | Br |
| 1273 | 6-CN | 3-CN | Br |
| 1274 | 6-CN | 3-NO₂ | Br |
| 1275 | 6-CN | 3-OMe | Br |
| 1276 | 6-CN | 2-F | Br |
| 1277 | 6-CN | 2-Cl | Br |
| 1278 | 6-CN | 2-Br | Br |
| 1279 | 6-CN | 2-Me | Br |
| 1280 | 6-CN | 2-CN | Br |
| 1281 | 6-CN | 2-NO₂ | Br |
| 1282 | 6-CN | 2-OMe | Br |
| 1283 | 6-CN | 2,3-F₂ | Br |
| 1284 | 6-CN | 2,4-F₂ | Br |
| 1285 | 6-CN | 2,5-F₂ | Br |
| 1286 | 6-CN | 2,6-F₂ | Br |
| 1287 | 6-CN | 3,4-F₂ | Br |
| 1288 | 6-CN | 3,5-F₂ | Br |
| 1289 | 6-CN | 2,3-Cl₂ | Br |
| 1290 | 6-CN | 2,4-Cl₂ | Br |
| 1291 | 6-CN | 2,5-Cl₂ | Br |
| 1292 | 6-CN | 2,6-Cl₂ | Br |
| 1293 | 6-CN | 3,4-Cl₂ | Br |
| 1294 | 6-CN | 3,5-Cl₂ | Br |
| 1295 | 6-CN | 2-F, 3-Cl | Br |
| 1296 | 6-CN | 2-F, 4-Cl | Br |
| 1297 | 6-CN | 2-F, 5-Cl | Br |
| 1298 | 6-CN | 2-F, 6-Cl | Br |
| 1299 | 6-CN | 2,6-F₂, 4-Cl | Br |
| 1300 | 6-CN | 3-F, 4-Cl | Br |
| 1301 | 6-CN | 3-Cl, 5-F | Br |
| 1302 | 6-CN | 2-Cl, 5-F | Br |
| 1303 | 6-CN | 3-CN, 4-Cl | Br |
| 1304 | 6-CN | 3-NO₂, 4-Cl | Br |
| 1305 | 6-CN | 2-F, 4-Br | Br |
| 1306 | 6-Me | 4-F | Br |
| 1307 | 6-Me | 4-Cl | Br |
| 1308 | 6-Me | 4-Br | Br |
| 1309 | 6-Me | H | Br |
| 1310 | 6-Me | 4-Me | Br |
| 1311 | 6-Me | 4-CN | Br |
| 1312 | 6-Me | 4-NO₂ | Br |
| 1313 | 6-Me | 4-OMe | Br |
| 1314 | 6-Me | 3-F | Br |
| 1315 | 6-Me | 3-Cl | Br |
| 1316 | 6-Me | 3-Br | Br |
| 1317 | 6-Me | 3-Me | Br |
| 1318 | 6-Me | 3-CN | Br |
| 1319 | 6-Me | 3-NO₂ | Br |
| 1320 | 6-Me | 3-OMe | Br |
| 1321 | 6-Me | 2-F | Br |
| 1322 | 6-Me | 2-Cl | Br |
| 1323 | 6-Me | 2-Br | Br |
| 1324 | 6-Me | 2-Me | Br |
| 1325 | 6-Me | 2-CN | Br |
| 1326 | 6-Me | 2-NO₂ | Br |
| 1327 | 6-Me | 2-OMe | Br |
| 1328 | 6-Me | 2,3-F₂ | Br |
| 1329 | 6-Me | 2,4-F₂ | Br |
| 1330 | 6-Me | 2,5-F₂ | Br |
| 1331 | 6-Me | 2,6-F₂ | Br |
| 1332 | 6-Me | 3,4-F₂ | Br |
| 1333 | 6-Me | 3,5-F₂ | Br |
| 1334 | 6-Me | 2,3-Cl₂ | Br |
| 1335 | 6-Me | 2,4-Cl₂ | Br |
| 1336 | 6-Me | 2,5-Cl₂ | Br |
| 1337 | 6-Me | 2,6-Cl₂ | Br |
| 1338 | 6-Me | 3,4-Cl₂ | Br |
| 1339 | 6-Me | 3,5-Cl₂ | Br |
| 1340 | 6-Me | 2-F, 3-Cl | Br |
| 1341 | 6-Me | 2-F, 4-Cl | Br |
| 1342 | 6-Me | 2-F, 5-Cl | Br |
| 1343 | 6-Me | 2-F, 6-Cl | Br |
| 1344 | 6-Me | 2,6-F₂, 4-Cl | Br |
| 1345 | 6-Me | 3-F, 4-Cl | Br |
| 1346 | 6-Me | 3-Cl, 5-F | Br |
| 1347 | 6-Me | 2-Cl, 5-F | Br |
| 1348 | 6-Me | 3-CN, 4-Cl | Br |
| 1349 | 6-Me | 3-NO₂, 4-Cl | Br |
| 1350 | 6-Me | 2-F, 4-Br | Br |
| 1351 | 6-OMe | 4-F | Br |
| 1352 | 6-OMe | 4-Cl | Br |
| 1353 | 6-OMe | 4-Br | Br |
| 1354 | 6-OMe | H | Br |
| 1355 | 6-OMe | 4-Me | Br |
| 1356 | 6-OMe | 4-CN | Br |
| 1357 | 6-OMe | 4-NO₂ | Br |
| 1358 | 6-OMe | 4-OMe | Br |
| 1359 | 6-OMe | 3-F | Br |
| 1360 | 6-OMe | 3-Cl | Br |
| 1361 | 6-OMe | 3-Br | Br |
| 1362 | 6-OMe | 3-Me | Br |
| 1363 | 6-OMe | 3-CN | Br |
| 1364 | 6-OMe | 3-NO₂ | Br |
| 1365 | 6-OMe | 3-OMe | Br |
| 1366 | 6-OMe | 2-F | Br |
| 1367 | 6-OMe | 2-Cl | Br |
| 1368 | 6-OMe | 2-Br | Br |
| 1369 | 6-OMe | 2-Me | Br |
| 1370 | 6-OMe | 2-CN | Br |
| 1371 | 6-OMe | 2-NO₂ | Br |
| 1372 | 6-OMe | 2-OMe | Br |
| 1373 | 6-OMe | 2,3-F₂ | Br |
| 1374 | 6-OMe | 2,4-F₂ | Br |
| 1375 | 6-OMe | 2,5-F₂ | Br |
| 1376 | 6-OMe | 2,6-F₂ | Br |
| 1377 | 6-OMe | 3,4-F₂ | Br |
| 1378 | 6-OMe | 3,5-F₂ | Br |
| 1379 | 6-OMe | 2,3-Cl₂ | Br |
| 1380 | 6-OMe | 2,4-Cl₂ | Br |
| 1381 | 6-OMe | 2,5-Cl₂ | Br |
| 1382 | 6-OMe | 2,6-Cl₂ | Br |
| 1383 | 6-OMe | 3,4-Cl₂ | Br |
| 1384 | 6-OMe | 3,5-Cl₂ | Br |
| 1385 | 6-OMe | 2-F, 3-Cl | Br |
| 1386 | 6-OMe | 2-F, 4-Cl | Br |
| 1387 | 6-OMe | 2-F, 5-Cl | Br |
| 1388 | 6-OMe | 2-F, 6-Cl | Br |
| 1389 | 6-OMe | 2,6-F₂, 4-Cl | Br |
| 1390 | 6-OMe | 3-F, 4-Cl | Br |
| 1391 | 6-OMe | 3-Cl, 5-F | Br |
| 1392 | 6-OMe | 2-Cl, 5-F | Br |
| 1393 | 6-OMe | 3-CN, 4-Cl | Br |
| 1394 | 6-OMe | 3-NO₂, 4-Cl | Br |
| 1395 | 6-OMe | 2-F, 4-Br | Br |
| 1396 | 2-F | 4-F | Br |
| 1397 | 2-F | 4-Cl | Br |
| 1398 | 2-F | 4-Br | Br |
| 1399 | 2-F | H | Br |
| 1400 | 2-F | 4-Me | Br |
| 1401 | 2-F | 4-CN | Br |
| 1402 | 2-F | 4-NO₂ | Br |
| 1403 | 2-F | 4-OMe | Br |
| 1404 | 2-F | 3-F | Br |
| 1405 | 2-F | 3-Cl | Br |
| 1406 | 2-F | 3-Br | Br |
| 1407 | 2-F | 3-Me | Br |
| 1408 | 2-F | 3-CN | Br |
| 1409 | 2-F | 3-NO₂ | Br |
| 1410 | 2-F | 3-OMe | Br |
| 1411 | 2-F | 2-F | Br |
| 1412 | 2-F | 2-Cl | Br |
| 1413 | 2-F | 2-Br | Br |
| 1414 | 2-F | 2-Me | Br |
| 1415 | 2-F | 2-CN | Br |
| 1416 | 2-F | 2-NO₂ | Br |
| 1417 | 2-F | 2-OMe | Br |
| 1418 | 2-F | 2,3-F₂ | Br |
| 1419 | 2-F | 2,4-F₂ | Br |
| 1420 | 2-F | 2,5-F₂ | Br |
| 1421 | 2-F | 2,6-F₂ | Br |
| 1422 | 2-F | 3,4-F₂ | Br |
| 1423 | 2-F | 3,5-F₂ | Br |
| 1424 | 2-F | 2,3-Cl₂ | Br |
| 1425 | 2-F | 2,4-Cl₂ | Br |
| 1426 | 2-F | 2,5-Cl₂ | Br |
| 1427 | 2-F | 2,6-Cl₂ | Br |
| 1428 | 2-F | 3,4-Cl₂ | Br |
| 1429 | 2-F | 3,5-Cl₂ | Br |
| 1430 | 2-F | 2-F, 3-Cl | Br |
| 1431 | 2-F | 2-F, 4-Cl | Br |
| 1432 | 2-F | 2-F, 5-Cl | Br |
| 1433 | 2-F | 2-F, 6-Cl | Br |
| 1434 | 2-F | 2,6-F₂, 4-Cl | Br |
| 1435 | 2-F | 3-F, 4-Cl | Br |
| 1436 | 2-F | 3-Cl, 5-F | Br |
| 1437 | 2-F | 2-Cl, 5-F | Br |
| 1438 | 2-F | 3-CN, 4-Cl | Br |
| 1439 | 2-F | 3-NO₂, 4-Cl | Br |
| 1440 | 2-F | 2-F, 4-Br | Br |
| 1441 | 4-F | 4-F | Br |
| 1442 | 4-F | 4-Cl | Br |
| 1443 | 4-F | 4-Br | Br |
| 1444 | 4-F | H | Br |
| 1445 | 4-F | 4-Me | Br |
| 1446 | 4-F | 4-CN | Br |
| 1447 | 4-F | 4-NO₂ | Br |
| 1448 | 4-F | 4-OMe | Br |
| 1449 | 4-F | 3-F | Br |
| 1450 | 4-F | 3-Cl | Br |
| 1451 | 4-F | 3-Br | Br |
| 1452 | 4-F | 3-Me | Br |
| 1453 | 4-F | 3-CN | Br |
| 1454 | 4-F | 3-NO₂ | Br |
| 1455 | 4-F | 3-OMe | Br |
| 1456 | 4-F | 2-F | Br |
| 1457 | 4-F | 2-Cl | Br |
| 1458 | 4-F | 2-Br | Br |
| 1459 | 4-F | 2-Me | Br |
| 1460 | 4-F | 2-CN | Br |
| 1461 | 4-F | 2-NO₂ | Br |
| 1462 | 4-F | 2-OMe | Br |
| 1463 | 4-F | 2,3-F₂ | Br |
| 1464 | 4-F | 2,4-F₂ | Br |
| 1465 | 4-F | 2,5-F₂ | Br |
| 1466 | 4-F | 2,6-F₂ | Br |
| 1467 | 4-F | 3,4-F₂ | Br |
| 1468 | 4-F | 3,5-F₂ | Br |
| 1469 | 4-F | 2,3-Cl₂ | Br |
| 1470 | 4-F | 2,4-Cl₂ | Br |
| 1471 | 4-F | 2,5-Cl₂ | Br |
| 1472 | 4-F | 2,6-Cl₂ | Br |
| 1473 | 4-F | 3,4-Cl₂ | Br |
| 1474 | 4-F | 3,5-Cl₂ | Br |
| 1475 | 4-F | 2-F, 3-Cl | Br |
| 1476 | 4-F | 2-F, 4-Cl | Br |
| 1477 | 4-F | 2-F, 5-Cl | Br |
| 1478 | 4-F | 2-F, 6-Cl | Br |
| 1479 | 4-F | 2,6-F₂, 4-Cl | Br |
| 1480 | 4-F | 3-F, 4-Cl | Br |
| 1481 | 4-F | 3-Cl, 5-F | Br |
| 1482 | 4-F | 2-Cl, 5-F | Br |
| 1483 | 4-F | 3-CN, 4-Cl | Br |
| 1484 | 4-F | 3-NO₂, 4-Cl | Br |
| 1485 | 4-F | 2-F, 4-Br | Br |
| 1486 | 2-Cl | 4-F | Br |
| 1487 | 2-Cl | 4-Cl | Br |
| 1488 | 2-Cl | 4-Br | Br |
| 1489 | 2-Cl | H | Br |
| 1490 | 2-Cl | 4-Me | Br |
| 1491 | 2-Cl | 4-CN | Br |
| 1492 | 2-Cl | 4-NO₂ | Br |
| 1493 | 2-Cl | 4-OMe | Br |
| 1494 | 2-Cl | 3-F | Br |
| 1495 | 2-Cl | 3-Cl | Br |
| 1496 | 2-Cl | 3-Br | Br |
| 1497 | 2-Cl | 3-Me | Br |
| 1498 | 2-Cl | 3-CN | Br |
| 1499 | 2-Cl | 3-NO₂ | Br |
| 1500 | 2-Cl | 3-OMe | Br |
| 1501 | 2-Cl | 2-F | Br |
| 1502 | 2-Cl | 2-Cl | Br |
| 1503 | 2-Cl | 2-Br | Br |
| 1504 | 2-Cl | 2-Me | Br |
| 1505 | 2-Cl | 2-CN | Br |
| 1506 | 2-Cl | 2-NO₂ | Br |
| 1507 | 2-Cl | 2-OMe | Br |
| 1508 | 2-Cl | 2,3-F₂ | Br |
| 1509 | 2-Cl | 2,4-F₂ | Br |
| 1510 | 2-Cl | 2,5-F₂ | Br |
| 1511 | 2-Cl | 2,6-F₂ | Br |
| 1512 | 2-Cl | 3,4-F₂ | Br |
| 1513 | 2-Cl | 3,5-F₂ | Br |
| 1514 | 2-Cl | 2,3-Cl₂ | Br |
| 1515 | 2-Cl | 2,4-Cl₂ | Br |
| 1516 | 2-Cl | 2,5-Cl₂ | Br |
| 1517 | 2-Cl | 2,6-Cl₂ | Br |
| 1518 | 2-Cl | 3,4-Cl₂ | Br |
| 1519 | 2-Cl | 3,5-Cl₂ | Br |
| 1520 | 2-Cl | 2-F, 3-Cl | Br |
| 1521 | 2-Cl | 2-F, 4-Cl | Br |
| 1522 | 2-Cl | 2-F, 5-Cl | Br |
| 1523 | 2-Cl | 2-F, 6-Cl | Br |
| 1524 | 2-Cl | 2,6-F₂, 4-Cl | Br |
| 1525 | 2-Cl | 3-F, 4-Cl | Br |
| 1526 | 2-Cl | 3-Cl, 5-F | Br |
| 1527 | 2-Cl | 2-Cl, 5-F | Br |
| 1528 | 2-Cl | 3-CN, 4-Cl | Br |
| 1529 | 2-Cl | 3-NO₂, 4-Cl | Br |
| 1530 | 2-Cl | 2-F, 4-Br | Br |
| 1531 | 4-Cl | 4-F | Br |
| 1532 | 4-Cl | 4-Cl | Br |
| 1533 | 4-Cl | 4-Br | Br |
| 1534 | 4-Cl | H | Br |
| 1535 | 4-Cl | 4-Me | Br |
| 1536 | 4-Cl | 4-CN | Br |
| 1537 | 4-Cl | 4-NO₂ | Br |
| 1538 | 4-Cl | 4-OMe | Br |
| 1539 | 4-Cl | 3-F | Br |
| 1540 | 4-Cl | 3-CI | Br |
| 1541 | 4-Cl | 3-Br | Br |
| 1542 | 4-Cl | 3-Me | Br |
| 1543 | 4-Cl | 3-CN | Br |
| 1544 | 4-Cl | 3-NO₂ | Br |
| 1545 | 4-Cl | 3-OMe | Br |
| 1546 | 4-Cl | 2-F | Br |
| 1547 | 4-Cl | 2-Cl | Br |
| 1548 | 4-Cl | 2-Br | Br |
| 1549 | 4-Cl | 2-Me | Br |
| 1550 | 4-Cl | 2-CN | Br |
| 1551 | 4-Cl | 2-NO₂ | Br |
| 1552 | 4-Cl | 2-OMe | Br |
| 1553 | 4-Cl | 2,3-F₂ | Br |
| 1554 | 4-Cl | 2,4-F₂ | Br |
| 1555 | 4-Cl | 2,5-F₂ | Br |
| 1556 | 4-Cl | 2,6-F₂ | Br |
| 1557 | 4-Cl | 3,4-F₂ | Br |
| 1558 | 4-Cl | 3,5-F₂ | Br |
| 1559 | 4-Cl | 2,3-Cl₂ | Br |
| 1560 | 4-Cl | 2,4-Cl₂ | Br |
| 1561 | 4-Cl | 2,5-Cl₂ | Br |
| 1562 | 4-Cl | 2,6-Cl₂ | Br |
| 1563 | 4-Cl | 3,4-Cl₂ | Br |
| 1564 | 4-Cl | 3,5-Cl₂ | Br |
| 1565 | 4-Cl | 2-F, 3-Cl | Br |
| 1566 | 4-Cl | 2-F, 4-Cl | Br |
| 1567 | 4-Cl | 2-F, 5-Cl | Br |
| 1568 | 4-Cl | 2-F, 6-Cl | Br |
| 1569 | 4-Cl | 2,6-F₂, 4-Cl | Br |
| 1570 | 4-Cl | 3-F, 4-Cl | Br |
| 1571 | 4-Cl | 3-Cl, 5-F | Br |
| 1572 | 4-Cl | 2-Cl, 5-F | Br |
| 1573 | 4-Cl | 3-CN, 4-Cl | Br |
| 1574 | 4-Cl | 3-NO₂, 4-Cl | Br |
| 1575 | 4-Cl | 2-F, 4-Br | Br |
| 1576 | 6-OCF₂H | 4-F | Br |
| 1577 | 6-OCF₂H | 4-Cl | Br |
| 1578 | 6-OCF₂H | 4-Br | Br |
| 1579 | 6-OCF₂H | H | Br |
| 1580 | 6-OCF₂H | 4-Me | Br |
| 1581 | 6-OCF₂H | 4-CN | Br |
| 1582 | 6-OCF₂H | 4-NO₂ | Br |
| 1583 | 6-OCF₂H | 4-OMe | Br |
| 1584 | 6-OCF₂H | 3-F | Br |
| 1585 | 6-OCF₂H | 3-Cl | Br |
| 1586 | 6-OCF₂H | 3-Br | Br |
| 1587 | 6-OCF₂H | 3-Me | Br |
| 1588 | 6-OCF₂H | 3-CN | Br |
| 1589 | 6-OCF₂H | 3-NO₂ | Br |
| 1590 | 6-OCF₂H | 3-OMe | Br |
| 1591 | 6-OCF₂H | 2-F | Br |
| 1592 | 6-OCF₂H | 2-Cl | Br |
| 1593 | 6-OCF₂H | 2-Br | Br |
| 1594 | 6-OCF₂H | 2-Me | Br |
| 1595 | 6-OCF₂H | 2-CN | Br |
| 1596 | 6-OCF₂H | 2-NO₂ | Br |
| 1597 | 6-OCF₂H | 2-OMe | Br |
| 1598 | 6-OCF₂H | 2,3-F₂ | Br |
| 1599 | 6-OCF₂H | 2,4-F₂ | Br |
| 1600 | 6-OCF₂H | 2,5-F₂ | Br |
| 1601 | 6-OCF₂H | 2,6-F₂ | Br |
| 1602 | 6-OCF₂H | 3,4-F₂ | Br |
| 1603 | 6-OCF₂H | 3,5-F₂ | Br |
| 1604 | 6-OCF₂H | 2,3-Cl₂ | Br |
| 1605 | 6-OCF₂H | 2,4-Cl₂ | Br |
| 1606 | 6-OCF₂H | 2,5-Cl₂ | Br |
| 1607 | 6-OCF₂H | 2,6-Cl₂ | Br |
| 1608 | 6-OCF₂H | 3,4-Cl₂ | Br |
| 1609 | 6-OCF₂H | 3,5-Cl₂ | Br |
| 1610 | 6-OCF₂H | 2-F, 3-Cl | Br |
| 1611 | 6-OCF₂H | 2-F, 4-Cl | Br |
| 1612 | 6-OCF₂H | 2-F, 5-Cl | Br |
| 1613 | 6-OCF₂H | 2-F, 6-Cl | Br |
| 1614 | 6-OCF₂H | 2,6-F₂, 4-Cl | Br |
| 1615 | 6-OCF₂H | 3-F, 4-Cl | Br |
| 1616 | 6-OCF₂H | 3-Cl, 5-F | Br |
| 1617 | 6-OCF₂H | 2-Cl, 5-F | Br |
| 1618 | 6-OCF₂H | 3-CN, 4-Cl | Br |
| 1619 | 6-OCF₂H | 3-NO₂, 4-Cl | Br |
| 1620 | 6-OCF₂H | 2-F, 4-Br | Br |
| 1621 | H | 4-F | Me |
| 1622 | H | 4-Cl | Me |
| 1623 | H | 4-Br | Me |
| 1624 | H | H | Me |
| 1625 | H | 4-Me | Me |
| 1626 | H | 4-CN | Me |
| 1627 | H | 4-NO₂ | Me |
| 1628 | H | 4-OMe | Me |
| 1629 | H | 3-F | Me |
| 1630 | H | 3-Cl | Me |
| 1631 | H | 3-Br | Me |
| 1632 | H | 3-Me | Me |
| 1633 | H | 3-CN | Me |
| 1634 | H | 3-NO₂ | Me |
| 1635 | H | 3-OMe | Me |
| 1636 | H | 2-F | Me |
| 1637 | H | 2-Cl | Me |
| 1638 | H | 2-Br | Me |
| 1639 | H | 2-Me | Me |
| 1640 | H | 2-CN | Me |
| 1641 | H | 2-NO₂ | Me |
| 1642 | H | 2-OMe | Me |
| 1643 | H | 2,3-F₂ | Me |
| 1644 | H | 2,4-F₂ | Me |
| 1645 | H | 2,5-F₂ | Me |
| 1646 | H | 2,6-F₂ | Me |
| 1647 | H | 3,4-F₂ | Me |
| 1648 | H | 3,5-F₂ | Me |
| 1649 | H | 2,3-Cl₂ | Me |
| 1650 | H | 2,4-Cl₂ | Me |
| 1651 | H | 2,5-Cl₂ | Me |
| 1652 | H | 2,6-Cl₂ | Me |
| 1653 | H | 3,4-Cl₂ | Me |
| 1654 | H | 3,5-Cl₂ | Me |
| 1655 | H | 2-F, 3-Cl | Me |
| 1656 | H | 2-F, 4-Cl | Me |
| 1657 | H | 2-F, 5-Cl | Me |
| 1658 | H | 2-F, 6-Cl | Me |
| 1659 | H | 2,6-F₂, 4-Cl | Me |
| 1660 | H | 3-F, 4-Cl | Me |
| 1661 | H | 3-Cl, 5-F | Me |
| 1662 | H | 2-Cl, 5-F | Me |
| 1663 | H | 3-CN, 4-Cl | Me |
| 1664 | H | 3-NO₂, 4-Cl | Me |
| 1665 | H | 2-F, 4-Br | Me |
| 1666 | 6-F | 4-F | Me |
| 1667 | 6-F | 4-Cl | Me |
| 1668 | 6-F | 4-Br | Me |
| 1669 | 6-F | H | Me |
| 1670 | 6-F | 4-Me | Me |
| 1671 | 6-F | 4-CN | Me |
| 1672 | 6-F | 4-NO₂ | Me |
| 1673 | 6-F | 4-OMe | Me |
| 1674 | 6-F | 3-F | Me |
| 1675 | 6-F | 3-Cl | Me |
| 1676 | 6-F | 3-Br | Me |
| 1677 | 6-F | 3-Me | Me |
| 1678 | 6-F | 3-CN | Me |
| 1679 | 6-F | 3-NO₂ | Me |
| 1680 | 6-F | 3-OMe | Me |
| 1681 | 6-F | 2-F | Me |
| 1682 | 6-F | 2-Cl | Me |
| 1683 | 6-F | 2-Br | Me |
| 1684 | 6-F | 2-Me | Me |
| 1685 | 6-F | 2-CN | Me |
| 1686 | 6-F | 2-NO₂ | Me |
| 1687 | 6-F | 2-OMe | Me |
| 1688 | 6-F | 2,3-F₂ | Me |
| 1689 | 6-F | 2,4-F₂ | Me |
| 1690 | 6-F | 2,5-F₂ | Me |
| 1691 | 6-F | 2,6-F₂ | Me |
| 1692 | 6-F | 3,4-F₂ | Me |
| 1693 | 6-F | 3,5-F₂ | Me |
| 1694 | 6-F | 2,3-Cl₂ | Me |
| 1695 | 6-F | 2,4-Cl₂ | Me |
| 1696 | 6-F | 2,5-Cl₂ | Me |
| 1697 | 6-F | 2,6-Cl₂ | Me |
| 1698 | 6-F | 3,4-Cl₂ | Me |
| 1699 | 6-F | 3,5-Cl₂ | Me |
| 1700 | 6-F | 2-F, 3-Cl | Me |
| 1701 | 6-F | 2-F, 4-Cl | Me |
| 1702 | 6-F | 2-F, 5-Cl | Me |
| 1703 | 6-F | 2-F, 6-Cl | Me |
| 1704 | 6-F | 2,6-F₂, 4-Cl | Me |
| 1705 | 6-F | 3-F, 4-Cl | Me |
| 1706 | 6-F | 3-Cl, 5-F | Me |
| 1707 | 6-F | 2-Cl, 5-F | Me |
| 1708 | 6-F | 3-CN, 4-Cl | Me |
| 1709 | 6-F | 3-NO₂, 4-Cl | Me |
| 1710 | 6-F | 2-F, 4-Br | Me |
| 1711 | 6-Cl | 4-F | Me |
| 1712 | 6-Cl | 4-Cl | Me |
| 1713 | 6-Cl | 4-Br | Me |
| 1714 | 6-Cl | H | Me |
| 1715 | 6-Cl | 4-Me | Me |
| 1716 | 6-Cl | 4-CN | Me |
| 1717 | 6-Cl | 4-NO₂ | Me |
| 1718 | 6-Cl | 4-OMe | Me |
| 1719 | 6-Cl | 3-F | Me |
| 1720 | 6-Cl | 3-Cl | Me |
| 1721 | 6-Cl | 3-Br | Me |
| 1722 | 6-Cl | 3-Me | Me |
| 1723 | 6-Cl | 3-CN | Me |
| 1724 | 6-Cl | 3-NO₂ | Me |
| 1725 | 6-Cl | 3-OMe | Me |
| 1726 | 6-Cl | 2-F | Me |
| 1727 | 6-Cl | 2-CI | Me |
| 1728 | 6-Cl | 2-Br | Me |
| 1729 | 6-Cl | 2-Me | Me |
| 1730 | 6-Cl | 2-CN | Me |
| 1731 | 6-Cl | 2-NO₂ | Me |
| 1732 | 6-Cl | 2-OMe | Me |
| 1733 | 6-Cl | 2,3-F₂ | Me |
| 1734 | 6-Cl | 2,4-F₂ | Me |
| 1735 | 6-Cl | 2,5-F₂ | Me |
| 1736 | 6-Cl | 2,6-F₂ | Me |
| 1737 | 6-Cl | 3,4-F₂ | Me |
| 1738 | 6-Cl | 3,5-F₂ | Me |
| 1739 | 6-Cl | 2,3-Cl₂ | Me |
| 1740 | 6-Cl | 2,4-Cl₂ | Me |
| 1741 | 6-Cl | 2,5-Cl₂ | Me |
| 1742 | 6-Cl | 2,6-Cl₂ | Me |
| 1743 | 6-Cl | 3,4-Cl₂ | Me |
| 1744 | 6-Cl | 3,5-Cl₂ | Me |
| 1745 | 6-Cl | 2-F, 3-Cl | Me |
| 1746 | 6-Cl | 2-F, 4-Cl | Me |
| 1747 | 6-Cl | 2-F, 5-Cl | Me |
| 1748 | 6-Cl | 2-F, 6-Cl | Me |
| 1749 | 6-Cl | 2,6-F₂, 4-Cl | Me |
| 1750 | 6-Cl | 3-F, 4-Cl | Me |
| 1751 | 6-Cl | 3-Cl, 5-F | Me |
| 1752 | 6-Cl | 2-Cl, 5-F | Me |
| 1753 | 6-Cl | 3-CN, 4-Cl | Me |
| 1754 | 6-Cl | 3-NO₂, 4-Cl | Me |
| 1755 | 6-Cl | 2-F, 4-Br | Me |
| 1756 | 6-Br | 4-F | Me |
| 1757 | 6-Br | 4-Cl | Me |
| 1758 | 6-Br | 4-Br | Me |
| 1759 | 6-Br | H | Me |
| 1760 | 6-Br | 4-Me | Me |
| 1761 | 6-Br | 4-CN | Me |
| 1762 | 6-Br | 4-NO₂ | Me |
| 1763 | 6-Br | 4-OMe | Me |
| 1764 | 6-Br | 3-F | Me |
| 1765 | 6-Br | 3-Cl | Me |
| 1766 | 6-Br | 3-Br | Me |
| 1767 | 6-Br | 3-Me | Me |
| 1768 | 6-Br | 3-CN | Me |
| 1769 | 6-Br | 3-NO₂ | Me |
| 1770 | 6-Br | 3-OMe | Me |
| 1771 | 6-Br | 2-F | Me |
| 1772 | 6-Br | 2-Cl | Me |
| 1773 | 6-Br | 2-Br | Me |
| 1774 | 6-Br | 2-Me | Me |
| 1775 | 6-Br | 2-CN | Me |
| 1776 | 6-Br | 2-NO₂ | Me |
| 1777 | 6-Br | 2-OMe | Me |
| 1778 | 6-Br | 2,3-F₂ | Me |
| 1779 | 6-Br | 2,4-F₂ | Me |
| 1780 | 6-Br | 2,5-F₂ | Me |
| 1781 | 6-Br | 2,6-F₂ | Me |
| 1782 | 6-Br | 3,4-F₂ | Me |
| 1783 | 6-Br | 3,5-F₂ | Me |
| 1784 | 6-Br | 2,3-Cl₂ | Me |
| 1785 | 6-Br | 2,4-Cl₂ | Me |
| 1786 | 6-Br | 2,5-Cl₂ | Me |
| 1787 | 6-Br | 2,6-Cl₂ | Me |
| 1788 | 6-Br | 3,4-Cl₂ | Me |
| 1789 | 6-Br | 3,5-Cl₂ | Me |
| 1790 | 6-Br | 2-F, 3-Cl | Me |
| 1791 | 6-Br | 2-F, 4-Cl | Me |
| 1792 | 6-Br | 2-F, 5-Cl | Me |
| 1793 | 6-Br | 2-F, 6-Cl | Me |
| 1794 | 6-Br | 2,6-F₂, 4-Cl | Me |
| 1795 | 6-Br | 3-F, 4-Cl | Me |
| 1796 | 6-Br | 3-Cl, 5-F | Me |
| 1797 | 6-Br | 2-Cl, 5-F | Me |
| 1798 | 6-Br | 3-CN, 4-Cl | Me |
| 1799 | 6-Br | 3-NO₂, 4-Cl | Me |
| 1800 | 6-Br | 2-F, 4-Br | Me |
| 1801 | 6-CN | 4-F | Me |
| 1802 | 6-CN | 4-CI | Me |
| 1803 | 6-CN | 4-Br | Me |
| 1804 | 6-CN | H | Me |
| 1805 | 6-CN | 4-Me | Me |
| 1806 | 6-CN | 4-CN | Me |
| 1807 | 6-CN | 4-NO₂ | Me |
| 1808 | 6-CN | 4-OMe | Me |
| 1809 | 6-CN | 3-F | Me |
| 1810 | 6-CN | 3-Cl | Me |
| 1811 | 6-CN | 3-Br | Me |
| 1812 | 6-CN | 3-Me | Me |
| 1813 | 6-CN | 3-CN | Me |
| 1814 | 6-CN | 3-NO₂ | Me |
| 1815 | 6-CN | 3-OMe | Me |
| 1816 | 6-CN | 2-F | Me |
| 1817 | 6-CN | 2-Cl | Me |
| 1818 | 6-CN | 2-Br | Me |
| 1819 | 6-CN | 2-Me | Me |
| 1820 | 6-CN | 2-CN | Me |
| 1821 | 6-CN | 2-NO₂ | Me |
| 1822 | 6-CN | 2-OMe | Me |
| 1823 | 6-CN | 2,3-F₂ | Me |
| 1824 | 6-CN | 2,4-F₂ | Me |
| 1825 | 6-CN | 2,5-F₂ | Me |
| 1826 | 6-CN | 2,6-F₂ | Me |
| 1827 | 6-CN | 3,4-F₂ | Me |
| 1828 | 6-CN | 3,5-F₂ | Me |
| 1829 | 6-CN | 2,3-Cl₂ | Me |
| 1830 | 6-CN | 2,4-Cl₂ | Me |
| 1831 | 6-CN | 2,5-Cl₂ | Me |
| 1832 | 6-CN | 2,6-Cl₂ | Me |
| 1833 | 6-CN | 3,4-Cl₂ | Me |
| 1834 | 6-CN | 3,5-Cl₂ | Me |
| 1835 | 6-CN | 2-F, 3-Cl | Me |
| 1836 | 6-CN | 2-F, 4-Cl | Me |
| 1837 | 6-CN | 2-F, 5-Cl | Me |
| 1838 | 6-CN | 2-F, 6-Cl | Me |
| 1839 | 6-CN | 2,6-F₂, 4-Cl | Me |
| 1840 | 6-CN | 3-F, 4-Cl | Me |
| 1841 | 6-CN | 3-Cl, 5-F | Me |
| 1842 | 6-CN | 2-Cl, 5-F | Me |
| 1843 | 6-CN | 3-CN, 4-Cl | Me |
| 1844 | 6-CN | 3-NO₂, 4-Cl | Me |
| 1845 | 6-CN | 2-F, 4-Br | Me |
| 1846 | 6-Me | 4-F | Me |
| 1847 | 6-Me | 4-Cl | Me |
| 1848 | 6-Me | 4-Br | Me |
| 1849 | 6-Me | H | Me |
| 1850 | 6-Me | 4-Me | Me |
| 1851 | 6-Me | 4-CN | Me |
| 1852 | 6-Me | 4-NO₂ | Me |
| 1853 | 6-Me | 4-OMe | Me |
| 1854 | 6-Me | 3-F | Me |
| 1855 | 6-Me | 3-Cl | Me |
| 1856 | 6-Me | 3-Br | Me |
| 1857 | 6-Me | 3-Me | Me |
| 1858 | 6-Me | 3-CN | Me |
| 1859 | 6-Me | 3-NO₂ | Me |
| 1860 | 6-Me | 3-OMe | Me |
| 1861 | 6-Me | 2-F | Me |
| 1862 | 6-Me | 2-Cl | Me |
| 1863 | 6-Me | 2-Br | Me |
| 1864 | 6-Me | 2-Me | Me |
| 1865 | 6-Me | 2-CN | Me |
| 1866 | 6-Me | 2-NO₂ | Me |
| 1867 | 6-Me | 2-OMe | Me |
| 1868 | 6-Me | 2,3-F₂ | Me |
| 1869 | 6-Me | 2,4-F₂ | Me |
| 1870 | 6-Me | 2,5-F₂ | Me |
| 1871 | 6-Me | 2,6-F₂ | Me |
| 1872 | 6-Me | 3,4-F₂ | Me |
| 1873 | 6-Me | 3,5-F₂ | Me |
| 1874 | 6-Me | 2,3-Cl₂ | Me |
| 1875 | 6-Me | 2,4-Cl₂ | Me |
| 1876 | 6-Me | 2,5-Cl₂ | Me |
| 1877 | 6-Me | 2,6-Cl₂ | Me |
| 1878 | 6-Me | 3,4-Cl₂ | Me |
| 1879 | 6-Me | 3,5-Cl₂ | Me |
| 1880 | 6-Me | 2-F, 3-Cl | Me |
| 1881 | 6-Me | 2-F, 4-Cl | Me |
| 1882 | 6-Me | 2-F, 5-Cl | Me |
| 1883 | 6-Me | 2-F, 6-Cl | Me |
| 1884 | 6-Me | 2,6-F₂, 4-Cl | Me |
| 1885 | 6-Me | 3-F, 4-Cl | Me |
| 1886 | 6-Me | 3-Cl, 5-F | Me |
| 1887 | 6-Me | 2-Cl, 5-F | Me |
| 1888 | 6-Me | 3-CN, 4-Cl | Me |
| 1889 | 6-Me | 3-NO₂, 4-Cl | Me |
| 1890 | 6-Me | 2-F, 4-Br | Me |
| 1891 | 6-OMe | 4-F | Me |
| 1892 | 6-OMe | 4-Cl | Me |
| 1893 | 6-OMe | 4-Br | Me |
| 1894 | 6-OMe | H | Me |
| 1895 | 6-OMe | 4-Me | Me |
| 1896 | 6-OMe | 4-CN | Me |
| 1897 | 6-OMe | 4-NO₂ | Me |
| 1898 | 6-OMe | 4-OMe | Me |
| 1899 | 6-OMe | 3-F | Me |
| 1900 | 6-OMe | 3-Cl | Me |
| 1901 | 6-OMe | 3-Br | Me |
| 1902 | 6-OMe | 3-Me | Me |
| 1903 | 6-OMe | 3-CN | Me |
| 1904 | 6-OMe | 3-NO₂ | Me |
| 1905 | 6-OMe | 3-OMe | Me |
| 1906 | 6-OMe | 2-F | Me |
| 1907 | 6-OMe | 2-Cl | Me |
| 1908 | 6-OMe | 2-Br | Me |
| 1909 | 6-OMe | 2-Me | Me |
| 1910 | 6-OMe | 2-CN | Me |
| 1911 | 6-OMe | 2-NO₂ | Me |
| 1912 | 6-OMe | 2-OMe | Me |
| 1913 | 6-OMe | 2,3-F₂ | Me |
| 1914 | 6-OMe | 2,4-F₂ | Me |
| 1915 | 6-OMe | 2,5-F₂ | Me |
| 1916 | 6-OMe | 2,6-F₂ | Me |
| 1917 | 6-OMe | 3,4-F₂ | Me |
| 1918 | 6-OMe | 3,5-F₂ | Me |
| 1919 | 6-OMe | 2,3-Cl₂ | Me |
| 1920 | 6-OMe | 2,4-Cl₂ | Me |
| 1921 | 6-OMe | 2,5-Cl₂ | Me |
| 1922 | 6-OMe | 2,6-Cl₂ | Me |
| 1923 | 6-OMe | 3,4-Cl₂ | Me |
| 1924 | 6-OMe | 3,5-Cl₂ | Me |
| 1925 | 6-OMe | 2-F, 3-Cl | Me |
| 1926 | 6-OMe | 2-F, 4-Cl | Me |
| 1927 | 6-OMe | 2-F, 5-Cl | Me |
| 1928 | 6-OMe | 2-F, 6-Cl. | Me |
| 1929 | 6-OMe | 2,6-F₂, 4-Cl | Me |
| 1930 | 6-OMe | 3-F, 4-Cl | Me |
| 1931 | 6-OMe | 3-Cl, 5-F | Me |
| 1932 | 6-OMe | 2-Cl, 5-F | Me |
| 1933 | 6-OMe | 3-CN, 4-Cl | Me |
| 1934 | 6-OMe | 3-NO₂, 4-Cl | Me |
| 1935 | 6-OMe | 2-F, 4-Br | Me |
| 1936 | 2-F | 4-F | Me |
| 1937 | 2-F | 4-Cl | Me |
| 1938 | 2-F | 4-Br | Me |
| 1939 | 2-F | H | Me |
| 1940 | 2-F | 4-Me | Me |
| 1941 | 2-F | 4-CN | Me |
| 1942 | 2-F | 4-NO₂ | Me |
| 1943 | 2-F | 4-OMe | Me |
| 1944 | 2-F | 3-F | Me |
| 1945 | 2-F | 3-Cl | Me |
| 1946 | 2-F | 3-Br | Me |
| 1947 | 2-F | 3-Me | Me |
| 1948 | 2-F | 3-CN | Me |
| 1949 | 2-F | 3-NO₂ | Me |
| 1950 | 2-F | 3-OMe | Me |
| 1951 | 2-F | 2-F | Me |
| 1952 | 2-F | 2-Cl | Me |
| 1953 | 2-F | 2-Br | Me |
| 1954 | 2-F | 2-Me | Me |
| 1955 | 2-F | 2-CN | Me |
| 1956 | 2-F | 2-NO₂ | Me |
| 1957 | 2-F | 2-OMe | Me |
| 1958 | 2-F | 2,3-F₂ | Me |
| 1959 | 2-F | 2,4-F₂ | Me |
| 1960 | 2-F | 2,5-F₂ | Me |
| 1961 | 2-F | 2,6-F₂ | Me |
| 1962 | 2-F | 3,4-F₂ | Me |
| 1963 | 2-F | 3,5-F₂ | Me |
| 1964 | 2-F | 2,3-Cl₂ | Me |
| 1965 | 2-F | 2,4-Cl₂ | Me |
| 1966 | 2-F | 2,5-Cl₂ | Me |
| 1967 | 2-F | 2,6-Cl₂ | Me |
| 1968 | 2-F | 3,4-Cl₂ | Me |
| 1969 | 2-F | 3,5-Cl₂ | Me |
| 1970 | 2-F | 2-F, 3-Cl | Me |
| 1971 | 2-F | 2-F, 4-Cl | Me |
| 1972 | 2-F | 2-F, 5-Cl | Me |
| 1973 | 2-F | 2-F, 6-Cl | Me |
| 1974 | 2-F | 2,6-F₂, 4-Cl | Me |
| 1975 | 2-F | 3-F, 4-Cl | Me |
| 1976 | 2-F | 3-Cl, 5-F | Me |
| 1977 | 2-F | 2-Cl, 5-F | Me |
| 1978 | 2-F | 3-CN, 4-Cl | Me |
| 1979 | 2-F | 3-NO₂, 4-Cl | Me |
| 1980 | 2-F | 2-F, 4-Br | Me |
| 1981 | 4-F | 4-F | Me |
| 1982 | 4-F | 4-Cl | Me |
| 1983 | 4-F | 4-Br | Me |
| 1984 | 4-F | H | Me |
| 1985 | 4-F | 4-Me | Me |
| 1986 | 4-F | 4-CN | Me |
| 1987 | 4-F | 4-NO₂ | Me |
| 1988 | 4-F | 4-OMe | Me |
| 1989 | 4-F | 3-F | Me |
| 1990 | 4-F | 3-Cl | Me |
| 1991 | 4-F | 3-Br | Me |
| 1992 | 4-F | 3-Me | Me |
| 1993 | 4-F | 3-CN | Me |
| 1994 | 4-F | 3-NO₂ | Me |
| 1995 | 4-F | 3-OMe | Me |
| 1996 | 4-F | 2-F | Me |
| 1997 | 4-F | 2-Cl | Me |
| 1998 | 4-F | 2-Br | Me |
| 1999 | 4-F | 2-Me | Me |
| 2000 | 4-F | 2-CN | Me |
| 2001 | 4-F | 2-NO₂ | Me |
| 2002 | 4-F | 2-OMe | Me |
| 2003 | 4-F | 2,3-F₂ | Me |
| 2004 | 4-F | 2,4-F₂ | Me |
| 2005 | 4-F | 2,5-F₂ | Me |
| 2006 | 4-F | 2,6-F₂ | Me |
| 2007 | 4-F | 3,4-F₂ | Me |
| 2008 | 4-F | 3,5-F₂ | Me |
| 2009 | 4-F | 2,3-Cl₂ | Me |
| 2010 | 4-F | 2,4-Cl₂ | Me |
| 2011 | 4-F | 2,5-Cl₂ | Me |
| 2012 | 4-F | 2,6-Cl₂ | Me |
| 2013 | 4-F | 3,4-Cl₂ | Me |
| 2014 | 4-F | 3,5-Cl₂ | Me |
| 2015 | 4-F | 2-F, 3-Cl | Me |
| 2016 | 4-F | 2-F, 4-Cl | Me |
| 2017 | 4-F | 2-F, 5-Cl | Me |
| 2018 | 4-F | 2-F, 6-Cl | Me |
| 2019 | 4-F | 2,6-F₂, 4-Cl | Me |
| 2020 | 4-F | 3-F, 4-Cl | Me |
| 2021 | 4-F | 3-Cl, 5-F | Me |
| 2022 | 4-F | 2-Cl, 5-F | Me |
| 2023 | 4-F | 3-CN, 4-Cl | Me |
| 2024 | 4-F | 3-NO₂, 4-Cl | Me |
| 2025 | 4-F | 2-F, 4-Br | Me |
| 2026 | 2-Cl | 4-F | Me |
| 2027 | 2-Cl | 4-Cl | Me |
| 2028 | 2-Cl | 4-Br | Me |
| 2029 | 2-Cl | H | Me |
| 2030 | 2-Cl | 4-Me | Me |
| 2031 | 2-Cl | 4-CN | Me |
| 2032 | 2-Cl | 4-NO₂ | Me |
| 2033 | 2-Cl | 4-OMe | Me |
| 2034 | 2-Cl | 3-F | Me |
| 2035 | 2-Cl | 3-Cl | Me |
| 2036 | 2-Cl | 3-Br | Me |
| 2037 | 2-Cl | 3-Me | Me |
| 2038 | 2-Cl | 3-CN | Me |
| 2039 | 2-Cl | 3-NO₂ | Me |
| 2040 | 2-Cl | 3-OMe | Me |
| 2041 | 2-Cl | 2-F | Me |
| 2042 | 2-Cl | 2-Cl | Me |
| 2043 | 2-Cl | 2-Br | Me |
| 2044 | 2-Cl | 2-Me | Me |
| 2045 | 2-Cl | 2-CN | Me |
| 2046 | 2-Cl | 2-NO₂ | Me |
| 2047 | 2-Cl | 2-OMe | Me |
| 2048 | 2-Cl | 2,3-F₂ | Me |
| 2049 | 2-Cl | 2,4-F₂ | Me |
| 2050 | 2-Cl | 2,5-F₂ | Me |
| 2051 | 2-Cl | 2,6-F₂ | Me |
| 2052 | 2-Cl | 3,4-F₂ | Me |
| 2053 | 2-Cl | 3,5-F₂ | Me |
| 2054 | 2-Cl | 2,3-Cl₂ | Me |
| 2055 | 2-Cl | 2,4-Cl₂ | Me |
| 2056 | 2-Cl | 2,5-Cl₂ | Me |
| 2057 | 2-Cl | 2,6-Cl₂ | Me |
| 2058 | 2-Cl | 3,4-Cl₂ | Me |
| 2059 | 2-Cl | 3,5-Cl₂ | Me |
| 2060 | 2-Cl | 2-F, 3-Cl | Me |
| 2061 | 2-Cl | 2-F, 4-Cl | Me |
| 2062 | 2-Cl | 2-F, 5-Cl | Me |
| 2063 | 2-Cl | 2-F, 6-Cl | Me |
| 2064 | 2-Cl | 2,6-F₂, 4-Cl | Me |
| 2065 | 2-Cl | 3-F, 4-Cl | Me |
| 2066 | 2-Cl | 3-Cl, 5-F | Me |
| 2067 | 2-Cl | 2-Cl, 5-F | Me |
| 2068 | 2-Cl | 3-CN, 4-Cl | Me |
| 2069 | 2-Cl | 3-NO₂, 4-Cl | Me |
| 2070 | 2-Cl | 2-F, 4-Br | Me |
| 2071 | 4-Cl | 4-F | Me |
| 2072 | 4-Cl | 4-CI | Me |
| 2073 | 4-Cl | 4-Br | Me |
| 2074 | 4-Cl | H | Me |
| 2075 | 4-Cl | 4-Me | Me |
| 2076 | 4-Cl | 4-CN | Me |
| 2077 | 4-Cl | 4-NO₂ | Me |
| 2078 | 4-Cl | 4-OMe | Me |
| 2079 | 4-Cl | 3-F | Me |
| 2080 | 4-Cl | 3-Cl | Me |
| 2081 | 4-Cl | 3-Br | Me |
| 2082 | 4-Cl | 3-Me | Me |
| 2083 | 4-Cl | 3-CN | Me |
| 2084 | 4-Cl | 3-NO₂ | Me |
| 2085 | 4-Cl | 3-OMe | Me |
| 2086 | 4-Cl | 2-F | Me |
| 2087 | 4-Cl | 2-Cl | Me |
| 2088 | 4-Cl | 2-Br | Me |
| 2089 | 4-Cl | 2-Me | Me |
| 2090 | 4-Cl | 2-CN | Me |
| 2091 | 4-Cl | 2-NO₂ | Me |
| 2092 | 4-Cl | 2-OMe | Me |
| 2093 | 4-Cl | 2,3-F₂ | Me |
| 2094 | 4-Cl | 2,4-F₂ | Me |
| 2095 | 4-Cl | 2,5-F₂ | Me |
| 2096 | 4-Cl | 2,6-F₂ | Me |
| 2097 | 4-Cl | 3,4-F₂ | Me |
| 2098 | 4-Cl | 3,5-F₂ | Me |
| 2099 | 4-Cl | 2,3-Cl₂ | Me |
| 2100 | 4-Cl | 2,4-Cl₂ | Me |
| 2101 | 4-Cl | 2,5-Cl₂ | Me |
| 2102 | 4-Cl | 2,6-Cl₂ | Me |
| 2103 | 4-Cl | 3,4-Cl₂ | Me |
| 2104 | 4-Cl | 3,5-Cl₂ | Me |
| 2105 | 4-Cl | 2-F, 3-Cl | Me |
| 2106 | 4-Cl | 2-F, 4-Cl | Me |
| 2107 | 4-Cl | 2-F, 5-Cl | Me |
| 2108 | 4-Cl | 2-F, 6-Cl | Me |
| 2109 | 4-Cl | 2,6-F₂, 4-Cl | Me |
| 2110 | 4-Cl | 3-F, 4-Cl | Me |
| 2111 | 4-Cl | 3-Cl, 5-F | Me |
| 2112 | 4-Cl | 2-Cl, 5-F | Me |
| 2113 | 4-Cl | 3-CN, 4-Cl | Me |
| 2114 | 4-Cl | 3-NO₂, 4-Cl | Me |
| 2115 | 4-Cl | 2-F, 4-Br | Me |
| 2116 | 6-OCF₂H | 4-F | Me |
| 2117 | 6-OCF₂H | 4-Cl | Me |
| 2118 | 6-OCF₂H | 4-Br | Me |
| 2119 | 6-OCF₂H | H | Me |
| 2120 | 6-OCF₂H | 4-Me | Me |
| 2121 | 6-OCF₂H | 4-CN | Me |
| 2122 | 6-OCF₂H | 4-NO₂ | Me |
| 2123 | 6-OCF₂H | 4-OMe | Me |
| 2124 | 6-OCF₂H | 3-F | Me |
| 2125 | 6-OCF₂H | 3-Cl | Me |
| 2126 | 6-OCF₂H | 3-Br | Me |
| 2127 | 6-OCF₂H | 3-Me | Me |
| 2128 | 6-OCF₂H | 3-CN | Me |
| 2129 | 6-OCF₂H | 3-NO₂ | Me |
| 2130 | 6-OCF₂H | 3-OMe | Me |
| 2131 | 6-OCF₂H | 2-F | Me |
| 2132 | 6-OCF₂H | 2-Cl | Me |
| 2133 | 6-OCF₂H | 2-Br | Me |
| 2134 | 6-OCF₂H | 2-Me | Me |
| 2135 | 6-OCF₂H | 2-CN | Me |
| 2136 | 6-OCF₂H | 2-NO₂ | Me |
| 2137 | 6-OCF₂H | 2-OMe | Me |
| 2138 | 6-OCF₂H | 2,3-F₂ | Me |
| 2139 | 6-OCF₂H | 2,4-F₂ | Me |
| 2140 | 6-OCF₂H | 2,5-F₂ | Me |
| 2141 | 6-OCF₂H | 2,6-F₂ | Me |
| 2142 | 6-OCF₂H | 3,4-F₂ | Me |
| 2143 | 6-OCF₂H | 3,5-F₂ | Me |
| 2144 | 6-OCF₂H | 2,3-Cl₂ | Me |
| 2145 | 6-OCF₂H | 2,4-Cl₂ | Me |
| 2146 | 6-OCF₂H | 2,5-Cl₂ | Me |
| 2147 | 6-OCF₂H | 2,6-Cl₂ | Me |
| 2148 | 6-OCF₂H | 3,4-Cl₂ | Me |
| 2149 | 6-OCF₂H | 3,5-Cl₂ | Me |
| 2150 | 6-OCF₂H | 2-F, 3-Cl | Me |
| 2151 | 6-OCF₂H | 2-F, 4-Cl | Me |
| 2152 | 6-OCF₂H | 2-F, 5-Cl | Me |
| 2153 | 6-OCF₂H | 2-F, 6-Cl | Me |
| 2154 | 6-OCF₂H | 2,6-F₂, 4-Cl | Me |
| 2155 | 6-OCF₂H | 3-F, 4-Cl | Me |
| 2156 | 6-OCF₂H | 3-Cl, 5-F | Me |
| 2157 | 6-OCF₂H | 2-Cl, 5-F | Me |
| 2158 | 6-OCF₂H | 3-CN, 4-Cl | Me |
| 2159 | 6-OCF₂H | 3-NO₂, 4-Cl | Me |
| 2160 | 6-OCF₂H | 2-F, 4-Br | Me |
| 2161 | H | 4-F | CN |
| 2162 | H | 4-Cl | CN |
| 2163 | H | 4-Br | CN |
| 2164 | H | H | CN |
| 2165 | H | 4-Me | CN |
| 2166 | H | 4-CN | CN |
| 2167 | H | 4-NO₂ | CN |
| 2168 | H | 4-OMe | CN |
| 2169 | H | 3-F | CN |
| 2170 | H | 3-Cl | CN |
| 2171 | H | 3-Br | CN |
| 2172 | H | 3-Me | CN |
| 2173 | H | 3-CN | CN |
| 2174 | H | 3-NO₂ | CN |
| 2175 | H | 3-OMe | CN |
| 2176 | H | 2-F | CN |
| 2177 | H | 2-Cl | CN |
| 2178 | H | 2-Br | CN |
| 2179 | H | 2-Me | CN |
| 2180 | H | 2-CN | CN |
| 2181 | H | 2-NO₂ | CN |
| 2182 | H | 2-OMe | CN |
| 2183 | H | 2,3-F₂ | CN |
| 2184 | H | 2,4-F₂ | CN |
| 2185 | H | 2,5-F₂ | CN |
| 2186 | H | 2,6-F₂ | CN |
| 2187 | H | 3,4-F₂ | CN |
| 2188 | H | 3,5-F₂ | CN |
| 2189 | H | 2,3-Cl₂ | CN |
| 2190 | H | 2,4-Cl₂ | CN |
| 2191 | H | 2,5-Cl₂ | CN |
| 2192 | H | 2,6-Cl₂ | CN |
| 2193 | H | 3,4-Cl₂ | CN |
| 2194 | H | 3,5-Cl₂ | CN |
| 2195 | H | 2-F, 3-Cl | CN |
| 2196 | H | 2-F, 4-Cl | CN |
| 2197 | H | 2-F, 5-Cl | CN |
| 2198 | H | 2-F, 6-Cl | CN |
| 2199 | H | 2,6-F₂, 4-Cl | CN |
| 2200 | H | 3-F, 4-Cl | CN |
| 2201 | H | 3-Cl, 5-F | CN |
| 2202 | H | 2-Cl, 5-F | CN |
| 2203 | H | 3-CN, 4-Cl | CN |
| 2204 | H | 3-NO₂, 4-Cl | CN |
| 2205 | H | 2-F, 4-Br | CN |
| 2206 | 6-F | 4-F | CN |
| 2207 | 6-F | 4-Cl | CN |
| 2208 | 6-F | 4-Br | CN |
| 2209 | 6-F | H | CN |
| 2210 | 6-F | 4-Me | CN |
| 2211 | 6-F | 4-CN | CN |
| 2212 | 6-F | 4-NO₂ | CN |
| 2213 | 6-F | 4-OMe | CN |
| 2214 | 6-F | 3-F | CN |
| 2215 | 6-F | 3-Cl | CN |
| 2216 | 6-F | 3-Br | CN |
| 2217 | 6-F | 3-Me | CN |
| 2218 | 6-F | 3-CN | CN |
| 2219 | 6-F | 3-NO₂ | CN |
| 2220 | 6-F | 3-OMe | CN |
| 2221 | 6-F | 2-F | CN |
| 2222 | 6-F | 2-Cl | CN |
| 2223 | 6-F | 2-Br | CN |
| 2224 | 6-F | 2-Me | CN |
| 2225 | 6-F | 2-CN | CN |
| 2226 | 6-F | 2-NO₂ | CN |
| 2227 | 6-F | 2-OMe | CN |
| 2228 | 6-F | 2,3-F₂ | CN |
| 2229 | 6-F | 2,4-F₂ | CN |
| 2230 | 6-F | 2,5-F₂ | CN |
| 2231 | 6-F | 2,6-F₂ | CN |
| 2232 | 6-F | 3,4-F₂ | CN |
| 2233 | 6-F | 3,5-F₂ | CN |
| 2234 | 6-F | 2,3-Cl₂ | CN |
| 2235 | 6-F | 2,4-Cl₂ | CN |
| 2236 | 6-F | 2,5-Cl₂ | CN |
| 2237 | 6-F | 2,6-Cl₂ | CN |
| 2238 | 6-F | 3,4-Cl₂ | CN |
| 2239 | 6-F | 3,5-Cl₂ | CN |
| 2240 | 6-F | 2-F, 3-Cl | CN |
| 2241 | 6-F | 2-F, 4-Cl | CN |
| 2242 | 6-F | 2-F, 5-Cl | CN |
| 2243 | 6-F | 2-F, 6-Cl | CN |
| 2244 | 6-F | 2,6-F₂, 4-Cl | CN |
| 2245 | 6-F | 3-F, 4-Cl | CN |
| 2246 | 6-F | 3-Cl, 5-F | CN |
| 2247 | 6-F | 2-Cl, 5-F | CN |
| 2248 | 6-F | 3-CN, 4-Cl | CN |
| 2249 | 6-F | 3-NO₂, 4-Cl | CN |
| 2250 | 6-F | 2-F, 4-Br | CN |
| 2251 | 6-Cl | 4-F | CN |
| 2252 | 6-Cl | 4-Cl | CN |
| 2253 | 6-Cl | 4-Br | CN |
| 2254 | 6-Cl | H | CN |
| 2255 | 6-Cl | 4-Me | CN |
| 2256 | 6-Cl | 4-CN | CN |
| 2257 | 6-Cl | 4-NO₂ | CN |
| 2258 | 6-Cl | 4-OMe | CN |
| 2259 | 6-Cl | 3-F | CN |
| 2260 | 6-Cl | 3-Cl | CN |
| 2261 | 6-Cl | 3-Br | CN |
| 2262 | 6-Cl | 3-Me | CN |
| 2263 | 6-Cl | 3-CN | CN |
| 2264 | 6-Cl | 3-NO₂ | CN |
| 2265 | 6-Cl | 3-OMe | CN |
| 2266 | 6-Cl | 2-F | CN |
| 2267 | 6-Cl | 2-Cl | CN |
| 2268 | 6-Cl | 2-Br | CN |
| 2269 | 6-Cl | 2-Me | CN |
| 2270 | 6-Cl | 2-CN | CN |
| 2271 | 6-Cl | 2-NO₂ | CN |
| 2272 | 6-Cl | 2-OMe | CN |
| 2273 | 6-Cl | 2,3-F₂ | CN |
| 2274 | 6-Cl | 2,4-F₂ | CN |
| 2275 | 6-Cl | 2,5-F₂ | CN |
| 2276 | 6-Cl | 2,6-F₂ | CN |
| 2277 | 6-Cl | 3,4-F₂ | CN |
| 2278 | 6-Cl | 3,5-F₂ | CN |
| 2279 | 6-Cl | 2,3-Cl₂ | CN |
| 2280 | 6-Cl | 2,4-Cl₂ | CN |
| 2281 | 6-Cl | 2,5-Cl₂ | CN |
| 2282 | 6-Cl | 2,6-Cl₂ | CN |
| 2283 | 6-Cl | 3,4-Cl₂ | CN |
| 2284 | 6-Cl | 3,5-Cl₂ | CN |
| 2285 | 6-Cl | 2-F, 3-Cl | CN |
| 2286 | 6-Cl | 2-F, 4-Cl | CN |
| 2287 | 6-Cl | 2-F, 5-Cl | CN |
| 2288 | 6-Cl | 2-F, 6-Cl | CN |
| 2289 | 6-Cl | 2,6-F₂, 4-Cl | CN |
| 2290 | 6-Cl | 3-F, 4-Cl | CN |
| 2291 | 6-Cl | 3-Cl, 5-F | CN |
| 2292 | 6-Cl | 2-Cl, 5-F | CN |
| 2293 | 6-Cl | 3-CN, 4-Cl | CN |
| 2294 | 6-Cl | 3-NO₂, 4-Cl | CN |
| 2295 | 6-Cl | 2-F, 4-Br | CN |
| 2296 | 6-Br | 4-F | CN |
| 2297 | 6-Br | 4-Cl | CN |
| 2298 | 6-Br | 4-Br | CN |
| 2299 | 6-Br | H | CN |
| 2300 | 6-Br | 4-Me | CN |
| 2301 | 6-Br | 4-CN | CN |
| 2302 | 6-Br | 4-NO₂ | CN |
| 2303 | 6-Br | 4-OMe | CN |
| 2304 | 6-Br | 3-F | CN |
| 2305 | 6-Br | 3-Cl | CN |
| 2306 | 6-Br | 3-Br | CN |
| 2307 | 6-Br | 3-Me | CN |
| 2308 | 6-Br | 3-CN | CN |
| 2309 | 6-Br | 3-NO₂ | CN |
| 2310 | 6-Br | 3-OMe | CN |
| 2311 | 6-Br | 2-F | CN |
| 2312 | 6-Br | 2-Cl | CN |
| 2313 | 6-Br | 2-Br | CN |
| 2314 | 6-Br | 2-Me | CN |
| 2315 | 6-Br | 2-CN | CN |
| 2316 | 6-Br | 2-NO₂ | CN |
| 2317 | 6-Br | 2-OMe | CN |
| 2318 | 6-Br | 2,3-F₂ | CN |
| 2319 | 6-Br | 2,4-F₂ | CN |
| 2320 | 6-Br | 2,5-F₂ | CN |
| 2321 | 6-Br | 2,6-F₂ | CN |
| 2322 | 6-Br | 3,4-F₂ | CN |
| 2323 | 6-Br | 3,5-F₂ | CN |
| 2324 | 6-Br | 2,3-Cl₂ | CN |
| 2325 | 6-Br | 2,4-Cl₂ | CN |
| 2326 | 6-Br | 2,5-Cl₂ | CN |
| 2327 | 6-Br | 2,6-Cl₂ | CN |
| 2328 | 6-Br | 3,4-Cl₂ | CN |
| 2329 | 6-Br | 3,5-Cl₂ | CN |
| 2330 | 6-Br | 2-F, 3-Cl | CN |
| 2331 | 6-Br | 2-F, 4-Cl | CN |
| 2332 | 6-Br | 2-F, 5-Cl | CN |
| 2333 | 6-Br | 2-F, 6-Cl | CN |
| 2334 | 6-Br | 2,6-F₂, 4-Cl | CN |
| 2335 | 6-Br | 3-F, 4-Cl | CN |
| 2336 | 6-Br | 3-Cl, 5-F | CN |
| 2337 | 6-Br | 2-Cl, 5-F | CN |
| 2338 | 6-Br | 3-CN, 4-Cl | CN |
| 2339 | 6-Br | 3-NO₂, 4-Cl | CN |
| 2340 | 6-Br | 2-F, 4-Br | CN |
| 2341 | 6-CN | 4-F | CN |
| 2342 | 6-CN | 4-Cl | CN |
| 2343 | 6-CN | 4-Br | CN |
| 2344 | 6-CN | H | CN |
| 2345 | 6-CN | 4-Me | CN |
| 2346 | 6-CN | 4-CN | CN |
| 2347 | 6-CN | 4-NO₂ | CN |
| 2348 | 6-CN | 4-OMe | CN |
| 2349 | 6-CN | 3-F | CN |
| 2350 | 6-CN | 3-Cl | CN |
| 2351 | 6-CN | 3-Br | CN |
| 2352 | 6-CN | 3-Me | CN |
| 2353 | 6-CN | 3-CN | CN |
| 2354 | 6-CN | 3-NO₂ | CN |
| 2355 | 6-CN | 3-OMe | CN |
| 2356 | 6-CN | 2-F | CN |
| 2357 | 6-CN | 2-Cl | CN |
| 2358 | 6-CN | 2-Br | CN |
| 2359 | 6-CN | 2-Me | CN |
| 2360 | 6-CN | 2-CN | CN |
| 2361 | 6-CN | 2-NO₂ | CN |
| 2362 | 6-CN | 2-OMe | CN |
| 2363 | 6-CN | 2,3-F₂ | CN |
| 2364 | 6-CN | 2,4-F₂ | CN |
| 2365 | 6-CN | 2,5-F₂ | CN |
| 2366 | 6-CN | 2,6-F₂ | CN |
| 2367 | 6-CN | 3,4-F₂ | CN |
| 2368 | 6-CN | 3,5-F₂ | CN |
| 2369 | 6-CN | 2,3-Cl₂ | CN |
| 2370 | 6-CN | 2,4-Cl₂ | CN |
| 2371 | 6-CN | 2,5-Cl₂ | CN |
| 2372 | 6-CN | 2,6-Cl₂ | CN |
| 2373 | 6-CN | 3,4-Cl₂ | CN |
| 2374 | 6-CN | 3,5-Cl₂ | CN |
| 2375 | 6-CN | 2-F, 3-Cl | CN |
| 2376 | 6-CN | 2-F, 4-Cl | CN |
| 2377 | 6-CN | 2-F, 5-Cl | CN |
| 2378 | 6-CN | 2-F, 6-Cl | CN |
| 2379 | 6-CN | 2,6-F₂, 4-Cl | CN |
| 2380 | 6-CN | 3-F, 4-Cl | CN |
| 2381 | 6-CN | 3-Cl, 5-F | CN |
| 2382 | 6-CN | 2-Cl, 5-F | CN |
| 2383 | 6-CN | 3-CN, 4-Cl | CN |
| 2384 | 6-CN | 3-NO₂, 4-Cl | CN |
| 2385 | 6-CN | 2-F, 4-Br | CN |
| 2386 | 6-Me | 4-F | CN |
| 2387 | 6-Me | 4-Cl | CN |
| 2388 | 6-Me | 4-Br | CN |
| 2389 | 6-Me | H | CN |
| 2390 | 6-Me | 4-Me | CN |
| 2391 | 6-Me | 4-CN | CN |
| 2392 | 6-Me | 4-NO₂ | CN |
| 2393 | 6-Me | 4-OMe | CN |
| 2394 | 6-Me | 3-F | CN |
| 2395 | 6-Me | 3-Cl | CN |
| 2396 | 6-Me | 3-Br | CN |
| 2397 | 6-Me | 3-Me | CN |
| 2398 | 6-Me | 3-CN | CN |
| 2399 | 6-Me | 3-NO₂ | CN |
| 2400 | 6-Me | 3-OMe | CN |
| 2401 | 6-Me | 2-F | CN |
| 2402 | 6-Me | 2-Cl | CN |
| 2403 | 6-Me | 2-Br | CN |
| 2404 | 6-Me | 2-Me | CN |
| 2405 | 6-Me | 2-CN | CN |
| 2406 | 6-Me | 2-NO₂ | CN |
| 2407 | 6-Me | 2-OMe | CN |
| 2408 | 6-Me | 2,3-F₂ | CN |
| 2409 | 6-Me | 2,4-F₂ | CN |
| 2410 | 6-Me | 2,5-F₂ | CN |
| 2411 | 6-Me | 2,6-F₂ | CN |
| 2412 | 6-Me | 3,4-F₂ | CN |
| 2413 | 6-Me | 3,5-F₂ | CN |
| 2414 | 6-Me | 2,3-Cl₂ | CN |
| 2415 | 6-Me | 2,4-Cl₂ | CN |
| 2416 | 6-Me | 2,5-Cl₂ | CN |
| 2417 | 6-Me | 2,6-Cl₂ | CN |
| 2418 | 6-Me | 3,4-Cl₂ | CN |
| 2419 | 6-Me | 3,5-Cl₂ | CN |
| 2420 | 6-Me | 2-F, 3-Cl | CN |
| 2421 | 6-Me | 2-F, 4-Cl | CN |
| 2422 | 6-Me | 2-F, 5-Cl | CN |
| 2423 | 6-Me | 2-F, 6-Cl | CN |
| 2424 | 6-Me | 2,6-F₂, 4-Cl | CN |
| 2425 | 6-Me | 3-F, 4-Cl | CN |
| 2426 | 6-Me | 3-Cl, 5-F | CN |
| 2427 | 6-Me | 2-Cl, 5-F | CN |
| 2428 | 6-Me | 3-CN, 4-Cl | CN |
| 2429 | 6-Me | 3-NO₂, 4-Cl | CN |
| 2430 | 6-Me | 2-F, 4-Br | CN |
| 2431 | 6-OMe | 4-F | CN |
| 2432 | 6-OMe | 4-CI | CN |
| 2433 | 6-OMe | 4-Br | CN |
| 2434 | 6-OMe | H | CN |
| 2435 | 6-OMe | 4-Me | CN |
| 2436 | 6-OMe | 4-CN | CN |
| 2437 | 6-OMe | 4-NO₂ | CN |
| 2438 | 6-OMe | 4-OMe | CN |
| 2439 | 6-OMe | 3-F | CN |
| 2440 | 6-OMe | 3-Cl | CN |
| 2441 | 6-OMe | 3-Br | CN |
| 2442 | 6-OMe | 3-Me | CN |
| 2443 | 6-OMe | 3-CN | CN |
| 2444 | 6-OMe | 3-NO₂ | CN |
| 2445 | 6-OMe | 3-OMe | CN |
| 2446 | 6-OMe | 2-F | CN |
| 2447 | 6-OMe | 2-Cl | CN |
| 2448 | 6-OMe | 2-Br | CN |
| 2449 | 6-OMe | 2-Me | CN |
| 2450 | 6-OMe | 2-CN | CN |
| 2451 | 6-OMe | 2-NO₂ | CN |
| 2452 | 6-OMe | 2-OMe | CN |
| 2453 | 6-OMe | 2,3-F₂ | CN |
| 2454 | 6-OMe | 2,4-F₂ | CN |
| 2455 | 6-OMe | 2,5-F₂ | CN |
| 2456 | 6-OMe | 2,6-F₂ | CN |
| 2457 | 6-OMe | 3,4-F₂ | CN |
| 2458 | 6-OMe | 3,5-F₂ | CN |
| 2459 | 6-OMe | 2,3-Cl₂ | CN |
| 2460 | 6-OMe | 2,4-Cl₂ | CN |
| 2461 | 6-OMe | 2,5-Cl₂ | CN |
| 2462 | 6-OMe | 2,6-Cl₂ | CN |
| 2463 | 6-OMe | 3,4-Cl₂ | CN |
| 2464 | 6-OMe | 3,5-Cl₂ | CN |
| 2465 | 6-OMe | 2-F, 3-Cl | CN |
| 2466 | 6-OMe | 2-F, 4-Cl | CN |
| 2467 | 6-OMe | 2-F, 5-Cl | CN |
| 2468 | 6-OMe | 2-F, 6-Cl | CN |
| 2469 | 6-OMe | 2,6-F₂, 4-Cl | CN |
| 2470 | 6-OMe | 3-F, 4-Cl | CN |
| 2471 | 6-OMe | 3-Cl, 5-F | CN |
| 2472 | 6-OMe | 2-Cl, 5-F | CN |
| 2473 | 6-OMe | 3-CN, 4-Cl | CN |
| 2474 | 6-OMe | 3-NO₂, 4-Cl | CN |
| 2475 | 6-OMe | 2-F, 4-Br | CN |
| 2476 | 2-F | 4-F | CN |
| 2477 | 2-F | 4-Cl | CN |
| 2478 | 2-F | 4-Br | CN |
| 2479 | 2-F | H | CN |
| 2480 | 2-F | 4-Me | CN |
| 2481 | 2-F | 4-CN | CN |
| 2482 | 2-F | 4-NO₂ | CN |
| 2483 | 2-F | 4-OMe | CN |
| 2484 | 2-F | 3-F | CN |
| 2485 | 2-F | 3-Cl | CN |
| 2486 | 2-F | 3-Br | CN |
| 2487 | 2-F | 3-Me | CN |
| 2488 | 2-F | 3-CN | CN |
| 2489 | 2-F | 3-NO₂ | CN |
| 2490 | 2-F | 3-OMe | CN |
| 2491 | 2-F | 2-F | CN |
| 2492 | 2-F | 2-Cl | CN |
| 2493 | 2-F | 2-Br | CN |
| 2494 | 2-F | 2-Me | CN |
| 2495 | 2-F | 2-CN | CN |
| 2496 | 2-F | 2-NO₂ | CN |
| 2497 | 2-F | 2-OMe | CN |
| 2498 | 2-F | 2,3-F₂ | CN |
| 2499 | 2-F | 2,4-F₂ | CN |
| 2500 | 2-F | 2,5-F₂ | CN |
| 2501 | 2-F | 2,6-F₂ | CN |
| 2502 | 2-F | 3,4-F₂ | CN |
| 2503 | 2-F | 3,5-F₂ | CN |
| 2504 | 2-F | 2,3-Cl₂ | CN |
| 2505 | 2-F | 2,4-Cl₂ | CN |
| 2506 | 2-F | 2,5-Cl₂ | CN |
| 2507 | 2-F | 2,6-Cl₂ | CN |
| 2508 | 2-F | 3,4-Cl₂ | CN |
| 2509 | 2-F | 3,5-Cl₂ | CN |
| 2510 | 2-F | 2-F, 3-Cl | CN |
| 2511 | 2-F | 2-F, 4-Cl | CN |
| 2512 | 2-F | 2-F, 5-Cl | CN |
| 2513 | 2-F | 2-F, 6-Cl | CN |
| 2514 | 2-F | 2,6-F₂, 4-Cl | CN |
| 2515 | 2-F | 3-F, 4-Cl | CN |
| 2516 | 2-F | 3-Cl, 5-F | CN |
| 2517 | 2-F | 2-Cl, 5-F | CN |
| 2518 | 2-F | 3-CN, 4-Cl | CN |
| 2519 | 2-F | 3-NO₂, 4-Cl | CN |
| 2520 | 2-F | 2-F, 4-Br | CN |
| 2521 | 4-F | 4-F | CN |
| 2522 | 4-F | 4-Cl | CN |
| 2523 | 4-F | 4-Br | CN |
| 2524 | 4-F | H | CN |
| 2525 | 4-F | 4-Me | CN |
| 2526 | 4-F | 4-CN | CN |
| 2527 | 4-F | 4-NO₂ | CN |
| 2528 | 4-F | 4-OMe | CN |
| 2529 | 4-F | 3-F | CN |
| 2530 | 4-F | 3-Cl | CN |
| 2531 | 4-F | 3-Br | CN |
| 2532 | 4-F | 3-Me | CN |
| 2533 | 4-F | 3-CN | CN |
| 2534 | 4-F | 3-NO₂ | CN |
| 2535 | 4-F | 3-OMe | CN |
| 2536 | 4-F | 2-F | CN |
| 2537 | 4-F | 2-Cl | CN |
| 2538 | 4-F | 2-Br | CN |
| 2539 | 4-F | 2-Me | CN |
| 2540 | 4-F | 2-CN | CN |
| 2541 | 4-F | 2-NO₂ | CN |
| 2542 | 4-F | 2-OMe | CN |
| 2543 | 4-F | 2,3-F₂ | CN |
| 2544 | 4-F | 2,4-F₂ | CN |
| 2545 | 4-F | 2,5-F₂ | CN |
| 2546 | 4-F | 2,6-F₂ | CN |
| 2547 | 4-F | 3,4-F₂ | CN |
| 2548 | 4-F | 3,5-F₂ | CN |
| 2549 | 4-F | 2,3-Cl₂ | CN |
| 2550 | 4-F | 2,4-Cl₂ | CN |
| 2551 | 4-F | 2,5-Cl₂ | CN |
| 2552 | 4-F | 2,6-Cl₂ | CN |
| 2553 | 4-F | 3,4-Cl₂ | CN |
| 2554 | 4-F | 3,5-Cl₂ | CN |
| 2555 | 4-F | 2-F, 3-Cl | CN |
| 2556 | 4-F | 2-F, 4-Cl | CN |
| 2557 | 4-F | 2-F, 5-Cl | CN |
| 2558 | 4-F | 2-F, 6-Cl | CN |
| 2559 | 4-F | 2,6-F₂, 4-Cl | CN |
| 2560 | 4-F | 3-F, 4-Cl | CN |
| 2561 | 4-F | 3-Cl, 5-F | CN |
| 2562 | 4-F | 2-Cl, 5-F | CN |
| 2563 | 4-F | 3-CN, 4-Cl | CN |
| 2564 | 4-F | 3-NO₂, 4-Cl | CN |
| 2565 | 4-F | 2-F, 4-Br | CN |
| 2566 | 2-Cl | 4-F | CN |
| 2567 | 2-Cl | 4-Cl | CN |
| 2568 | 2-Cl | 4-Br | CN |
| 2569 | 2-Cl | H | CN |
| 2570 | 2-Cl | 4-Me | CN |
| 2571 | 2-Cl | 4-CN | CN |
| 2572 | 2-Cl | 4-NO₂ | CN |
| 2573 | 2-Cl | 4-OMe | CN |
| 2574 | 2-Cl | 3-F | CN |
| 2575 | 2-Cl | 3-Cl | CN |
| 2576 | 2-Cl | 3-Br | CN |
| 2577 | 2-Cl | 3-Me | CN |
| 2578 | 2-Cl | 3-CN | CN |
| 2579 | 2-Cl | 3-NO₂ | CN |
| 2580 | 2-Cl | 3-OMe | CN |
| 2581 | 2-Cl | 2-F | CN |
| 2582 | 2-Cl | 2-Cl | CN |
| 2583 | 2-Cl | 2-Br | CN |
| 2584 | 2-Cl | 2-Me | CN |
| 2585 | 2-Cl | 2-CN | CN |
| 2586 | 2-Cl | 2-NO₂ | CN |
| 2587 | 2-Cl | 2-OMe | CN |
| 2588 | 2-Cl | 2,3-F₂ | CN |
| 2589 | 2-Cl | 2,4-F₂ | CN |
| 2590 | 2-Cl | 2,5-F₂ | CN |
| 2591 | 2-Cl | 2,6-F₂ | CN |
| 2592 | 2-Cl | 3,4-F₂ | CN |
| 2593 | 2-Cl | 3,5-F₂ | CN |
| 2594 | 2-Cl | 2,3-Cl₂ | CN |
| 2595 | 2-Cl | 2,4-Cl₂ | CN |
| 2596 | 2-Cl | 2,5-Cl₂ | CN |
| 2597 | 2-Cl | 2,6-Cl₂ | CN |
| 2598 | 2-Cl | 3,4-Cl₂ | CN |
| 2599 | 2-Cl | 3,5-Cl₂ | CN |
| 2600 | 2-Cl | 2-F, 3-Cl | CN |
| 2601 | 2-Cl | 2-F, 4-Cl | CN |
| 2602 | 2-Cl | 2-F, 5-Cl | CN |
| 2603 | 2-Cl | 2-F, 6-Cl | CN |
| 2604 | 2-Cl | 2,6-F₂, 4-Cl | CN |
| 2605 | 2-Cl | 3-F, 4-Cl | CN |
| 2606 | 2-Cl | 3-Cl, 5-F | CN |
| 2607 | 2-Cl | 2-Cl, 5-F | CN |
| 2608 | 2-Cl | 3-CN, 4-Cl | CN |
| 2609 | 2-Cl | 3-NO₂, 4-Cl | CN |
| 2610 | 2-Cl | 2-F, 4-Br | CN |
| 2611 | 4-Cl | 4-F | CN |
| 2612 | 4-Cl | 4-Cl | CN |
| 2613 | 4-Cl | 4-Br | CN |
| 2614 | 4-Cl | H | CN |
| 2615 | 4-Cl | 4-Me | CN |
| 2616 | 4-Cl | 4-CN | CN |
| 2617 | 4-Cl | 4-NO₂ | CN |
| 2618 | 4-Cl | 4-OMe | CN |
| 2619 | 4-Cl | 3-F | CN |
| 2620 | 4-Cl | 3-Cl | CN |
| 2621 | 4-Cl | 3-Br | CN |
| 2622 | 4-Cl | 3-Me | CN |
| 2623 | 4-Cl | 3-CN | CN |
| 2624 | 4-Cl | 3-NO₂ | CN |
| 2625 | 4-Cl | 3-OMe | CN |
| 2626 | 4-Cl | 2-F | CN |
| 2627 | 4-Cl | 2-Cl | CN |
| 2628 | 4-Cl | 2-Br | CN |
| 2629 | 4-Cl | 2-Me | CN |
| 2630 | 4-Cl | 2-CN | CN |
| 2631 | 4-Cl | 2-NO₂ | CN |
| 2632 | 4-Cl | 2-OMe | CN |
| 2633 | 4-Cl | 2,3-F₂ | CN |
| 2634 | 4-Cl | 2,4-F₂ | CN |
| 2635 | 4-Cl | 2,5-F₂ | CN |
| 2636 | 4-Cl | 2,6-F₂ | CN |
| 2637 | 4-Cl | 3,4-F₂ | CN |
| 2638 | 4-Cl | 3,5-F₂ | CN |
| 2639 | 4-Cl | 2,3-Cl₂ | CN |
| 2640 | 4-Cl | 2,4-Cl₂ | CN |
| 2641 | 4-Cl | 2,5-Cl₂ | CN |
| 2642 | 4-Cl | 2,6-Cl₂ | CN |
| 2643 | 4-Cl | 3,4-Cl₂ | CN |
| 2644 | 4-Cl | 3,5-Cl₂ | CN |
| 2645 | 4-Cl | 2-F, 3-Cl | CN |
| 2646 | 4-Cl | 2-F, 4-Cl | CN |
| 2647 | 4-Cl | 2-F, 5-Cl | CN |
| 2648 | 4-Cl | 2-F, 6-Cl | CN |
| 2649 | 4-Cl | 2,6-F₂, 4-Cl | CN |
| 2650 | 4-Cl | 3-F, 4-Cl | CN |
| 2651 | 4-Cl | 3-Cl, 5-F | CN |
| 2652 | 4-Cl | 2-Cl, 5-F | CN |
| 2653 | 4-Cl | 3-CN, 4-Cl | CN |
| 2654 | 4-Cl | 3-NO₂, 4-Cl | CN |
| 2655 | 4-Cl | 2-F, 4-Br | CN |
| 2656 | 6-OCF₂H | 4-F | CN |
| 2657 | 6-OCF₂H | 4-Cl | CN |
| 2658 | 6-OCF₂H | 4-Br | CN |
| 2659 | 6-OCF₂H | H | CN |
| 2660 | 6-OCF₂H | 4-Me | CN |
| 2661 | 6-OCF₂H | 4-CN | CN |
| 2662 | 6-OCF₂H | 4-NO₂ | CN |
| 2663 | 6-OCF₂H | 4-OMe | CN |
| 2664 | 6-OCF₂H | 3-F | CN |
| 2665 | 6-OCF₂H | 3-Cl | CN |
| 2666 | 6-OCF₂H | 3-Br | CN |
| 2667 | 6-OCF₂H | 3-Me | CN |
| 2668 | 6-OCF₂H | 3-CN | CN |
| 2669 | 6-OCF₂H | 3-NO₂ | CN |
| 2670 | 6-OCF₂H | 3-OMe | CN |
| 2671 | 6-OCF₂H | 2-F | CN |
| 2672 | 6-OCF₂H | 2-Cl | CN |
| 2673 | 6-OCF₂H | 2-Br | CN |
| 2674 | 6-OCF₂H | 2-Me | CN |
| 2675 | 6-OCF₂H | 2-CN | CN |
| 2676 | 6-OCF₂H | 2-NO₂ | CN |
| 2677 | 6-OCF₂H | 2-OMe | CN |
| 2678 | 6-OCF₂H | 2,3-F₂ | CN |
| 2679 | 6-OCF₂H | 2,4-F₂ | CN |
| 2680 | 6-OCF₂H | 2,5-F₂ | CN |
| 2681 | 6-OCF₂H | 2,6-F₂ | CN |
| 2682 | 6-OCF₂H | 3,4-F₂ | CN |
| 2683 | 6-OCF₂H | 3,5-F₂ | CN |
| 2684 | 6-OCF₂H | 2,3-Cl₂ | CN |
| 2685 | 6-OCF₂H | 2,4-Cl₂ | CN |
| 2686 | 6-OCF₂H | 2,5-Cl₂ | CN |
| 2687 | 6-OCF₂H | 2,6-Cl₂ | CN |
| 2688 | 6-OCF₂H | 3,4-Cl₂ | CN |
| 2689 | 6-OCF₂H | 3,5-Cl₂ | CN |
| 2690 | 6-OCF₂H | 2-F, 3-Cl | CN |
| 2691 | 6-OCF₂H | 2-F, 4-Cl | CN |
| 2692 | 6-OCF₂H | 2-F, 5-Cl | CN |
| 2693 | 6-OCF₂H | 2-F, 6-Cl | CN |
| 2694 | 6-OCF₂H | 2,6-F₂, 4-Cl | CN |
| 2695 | 6-OCF₂H | 3-F, 4-Cl | CN |
| 2696 | 6-OCF₂H | 3-Cl, 5-F | CN |
| 2697 | 6-OCF₂H | 2-Cl, 5-F | CN |
| 2698 | 6-OCF₂H | 3-CN, 4-Cl | CN |
| 2699 | 6-OCF₂H | 3-NO₂, 4-Cl | CN |
| 2700 | 6-OCF₂H | 2-F, 4-Br | CN |
| 2701 | H | 4-F | NO₂ |
| 2702 | H | 4-Cl | NO₂ |
| 2703 | H | 4-Br | NO₂ |
| 2704 | H | H | NO₂ |
| 2705 | H | 4-Me | NO₂ |
| 2706 | H | 4-CN | NO₂ |
| 2707 | H | 4-NO₂ | NO₂ |
| 2708 | H | 4-OMe | NO₂ |
| 2709 | H | 3-F | NO₂ |
| 2710 | H | 3-Cl | NO₂ |
| 2711 | H | 3-Br | NO₂ |
| 2712 | H | 3-Me | NO₂ |
| 2713 | H | 3-CN | NO₂ |
| 2714 | H | 3-NO₂ | NO₂ |
| 2715 | H | 3-OMe | NO₂ |
| 2716 | H | 2-F | NO₂ |
| 2717 | H | 2-Cl | NO₂ |
| 2718 | H | 2-Br | NO₂ |
| 2719 | H | 2-Me | NO₂ |
| 2720 | H | 2-CN | NO₂ |
| 2721 | H | 2-NO₂ | NO₂ |
| 2722 | H | 2-OMe | NO₂ |
| 2723 | H | 2,3-F₂ | NO₂ |
| 2724 | H | 2,4-F₂ | NO₂ |
| 2725 | H | 2,5-F₂ | NO₂ |
| 2726 | H | 2,6-F₂ | NO₂ |
| 2727 | H | 3,4-F₂ | NO₂ |
| 2728 | H | 3,5-F₂ | NO₂ |
| 2729 | H | 2,3-Cl₂ | NO₂ |
| 2730 | H | 2,4-Cl₂ | NO₂ |
| 2731 | H | 2,5-Cl₂ | NO₂ |
| 2732 | H | 2,6-Cl₂ | NO₂ |
| 2733 | H | 3,4-Cl₂ | NO₂ |
| 2734 | H | 3,5-Cl₂ | NO₂ |
| 2735 | H | 2-F, 3-Cl | NO₂ |
| 2736 | H | 2-F, 4-Cl | NO₂ |
| 2737 | H | 2-F, 5-Cl | NO₂ |
| 2738 | H | 2-F, 6-Cl | NO₂ |
| 2739 | H | 2,6-F₂, 4-Cl | NO₂ |
| 2740 | H | 3-F, 4-Cl | NO₂ |
| 2741 | H | 3-Cl, 5-F | NO₂ |
| 2742 | H | 2-Cl, 5-F | NO₂ |
| 2743 | H | 3-CN, 4-Cl | NO₂ |
| 2744 | H | 3-NO₂, 4-Cl | NO₂ |
| 2745 | H | 2-F, 4-Br | NO₂ |
| 2746 | 6-F | 4-F | NO₂ |
| 2747 | 6-F | 4-Cl | NO₂ |
| 2748 | 6-F | 4-Br | NO₂ |
| 2749 | 6-F | H | NO₂ |
| 2750 | 6-F | 4-Me | NO₂ |
| 2751 | 6-F | 4-CN | NO₂ |
| 2752 | 6-F | 4-NO₂ | NO₂ |
| 2753 | 6-F | 4-OMe | NO₂ |
| 2754 | 6-F | 3-F | NO₂ |
| 2755 | 6-F | 3-Cl | NO₂ |
| 2756 | 6-F | 3-Br | NO₂ |
| 2757 | 6-F | 3-Me | NO₂ |
| 2758 | 6-F | 3-CN | NO₂ |
| 2759 | 6-F | 3-NO₂ | NO₂ |
| 2760 | 6-F | 3-OMe | NO₂ |
| 2761 | 6-F | 2-F | NO₂ |
| 2762 | 6-F | 2-Cl | NO₂ |
| 2763 | 6-F | 2-Br | NO₂ |
| 2764 | 6-F | 2-Me | NO₂ |
| 2765 | 6-F | 2-CN | NO₂ |
| 2766 | 6-F | 2-NO₂ | NO₂ |
| 2767 | 6-F | 2-OMe | NO₂ |
| 2768 | 6-F | 2,3-F₂ | NO₂ |
| 2769 | 6-F | 2,4-F₂ | NO₂ |
| 2770 | 6-F | 2,5-F₂ | NO₂ |
| 2771 | 6-F | 2,6-F₂ | NO₂ |
| 2772 | 6-F | 3,4-F₂ | NO₂ |
| 2773 | 6-F | 3,5-F₂ | NO₂ |
| 2774 | 6-F | 2,3-Cl₂ | NO₂ |
| 2775 | 6-F | 2,4-Cl₂ | NO₂ |
| 2776 | 6-F | 2,5-Cl₂ | NO₂ |
| 2777 | 6-F | 2,6-Cl₂ | NO₂ |
| 2778 | 6-F | 3,4-Cl₂ | NO₂ |
| 2779 | 6-F | 3,5-Cl₂ | NO₂ |
| 2780 | 6-F | 2-F, 3-Cl | NO₂ |
| 2781 | 6-F | 2-F, 4-Cl | NO₂ |
| 2782 | 6-F | 2-F, 5-Cl | NO₂ |
| 2783 | 6-F | 2-F, 6-Cl | NO₂ |
| 2784 | 6-F | 2,6-F₂, 4-Cl | NO₂ |
| 2785 | 6-F | 3-F, 4-Cl | NO₂ |
| 2786 | 6-F | 3-Cl, 5-F | NO₂ |
| 2787 | 6-F | 2-Cl, 5-F | NO₂ |
| 2788 | 6-F | 3-CN, 4-Cl | NO₂ |
| 2789 | 6-F | 3-NO₂, 4-Cl | NO₂ |
| 2790 | 6-F | 2-F, 4-Br | NO₂ |
| 2791 | 6-Cl | 4-F | NO₂ |
| 2792 | 6-Cl | 4-Cl | NO₂ |
| 2793 | 6-Cl | 4-Br | NO₂ |
| 2794 | 6-Cl | H | NO₂ |
| 2795 | 6-Cl | 4-Me | NO₂ |
| 2796 | 6-Cl | 4-CN | NO₂ |
| 2797 | 6-Cl | 4-NO₂ | NO₂ |
| 2798 | 6-Cl | 4-OMe | NO₂ |
| 2799 | 6-Cl | 3-F | NO₂ |
| 2800 | 6-Cl | 3-Cl | NO₂ |
| 2801 | 6-Cl | 3-Br | NO₂ |
| 2802 | 6-Cl | 3-Me | NO₂ |
| 2803 | 6-Cl | 3-CN | NO₂ |
| 2804 | 6-Cl | 3-NO₂ | NO₂ |
| 2805 | 6-Cl | 3-OMe | NO₂ |
| 2806 | 6-Cl | 2-F | NO₂ |
| 2807 | 6-Cl | 2-Cl | NO₂ |
| 2808 | 6-Cl | 2-Br | NO₂ |
| 2809 | 6-Cl | 2-Me | NO₂ |
| 2810 | 6-Cl | 2-CN | NO₂ |
| 2811 | 6-Cl | 2-NO₂ | NO₂ |
| 2812 | 6-Cl | 2-OMe | NO₂ |
| 2813 | 6-Cl | 2,3-F₂ | NO₂ |
| 2814 | 6-Cl | 2,4-F₂ | NO₂ |
| 2815 | 6-Cl | 2,5-F₂ | NO₂ |
| 2816 | 6-Cl | 2,6-F₂ | NO₂ |
| 2817 | 6-Cl | 3,4-F₂ | NO₂ |
| 2818 | 6-Cl | 3,5-F₂ | NO₂ |
| 2819 | 6-Cl | 2,3-Cl₂ | NO₂ |
| 2820 | 6-Cl | 2,4-Cl₂ | NO₂ |
| 2821 | 6-Cl | 2,5-Cl₂ | NO₂ |
| 2822 | 6-Cl | 2,6-Cl₂ | NO₂ |
| 2823 | 6-Cl | 3,4-Cl₂ | NO₂ |
| 2824 | 6-Cl | 3,5-Cl₂ | NO₂ |
| 2825 | 6-Cl | 2-F, 3-Cl | NO₂ |
| 2826 | 6-Cl | 2-F, 4-Cl | NO₂ |
| 2827 | 6-Cl | 2-F, 5-Cl | NO₂ |
| 2828 | 6-Cl | 2-F, 6-Cl | NO₂ |
| 2829 | 6-Cl | 2,6-F₂, 4-Cl | NO₂ |
| 2830 | 6-Cl | 3-F, 4-Cl | NO₂ |
| 2831 | 6-Cl | 3-Cl, 5-F | NO₂ |
| 2832 | 6-Cl | 2-Cl, 5-F | NO₂ |
| 2833 | 6-Cl | 3-CN, 4-Cl | NO₂ |
| 2834 | 6-Cl | 3-NO₂, 4-Cl | NO₂ |
| 2835 | 6-Cl | 2-F, 4-Br | NO₂ |
| 2836 | 6-Br | 4-F | NO₂ |
| 2837 | 6-Br | 4-Cl | NO₂ |
| 2838 | 6-Br | 4-Br | NO₂ |
| 2839 | 6-Br | H | NO₂ |
| 2840 | 6-Br | 4-Me | NO₂ |
| 2841 | 6-Br | 4-CN | NO₂ |
| 2842 | 6-Br | 4-NO₂ | NO₂ |
| 2843 | 6-Br | 4-OMe | NO₂ |
| 2844 | 6-Br | 3-F | NO₂ |
| 2845 | 6-Br | 3-Cl | NO₂ |
| 2846 | 6-Br | 3-Br | NO₂ |
| 2847 | 6-Br | 3-Me | NO₂ |
| 2848 | 6-Br | 3-CN | NO₂ |
| 2849 | 6-Br | 3-NO₂ | NO₂ |
| 2850 | 6-Br | 3-OMe | NO₂ |
| 2851 | 6-Br | 2-F | NO₂ |
| 2852 | 6-Br | 2-Cl | NO₂ |
| 2853 | 6-Br | 2-Br | NO₂ |
| 2854 | 6-Br | 2-Me | NO₂ |
| 2855 | 6-Br | 2-CN | NO₂ |
| 2856 | 6-Br | 2-NO₂ | NO₂ |
| 2857 | 6-Br | 2-OMe | NO₂ |
| 2858 | 6-Br | 2,3-F₂ | NO₂ |
| 2859 | 6-Br | 2,4-F₂ | NO₂ |
| 2860 | 6-Br | 2,5-F₂ | NO₂ |
| 2861 | 6-Br | 2,6-F₂ | NO₂ |
| 2862 | 6-Br | 3,4-F₂ | NO₂ |
| 2863 | 6-Br | 3,5-F₂ | NO₂ |
| 2864 | 6-Br | 2,3-Cl₂ | NO₂ |
| 2865 | 6-Br | 2,4-Cl₂ | NO₂ |
| 2866 | 6-Br | 2,5-Cl₂ | NO₂ |
| 2867 | 6-Br | 2,6-Cl₂ | NO₂ |
| 2868 | 6-Br | 3,4-Cl₂ | NO₂ |
| 2869 | 6-Br | 3,5-Cl₂ | NO₂ |
| 2870 | 6-Br | 2-F, 3-Cl | NO₂ |
| 2871 | 6-Br | 2-F, 4-Cl | NO₂ |
| 2872 | 6-Br | 2-F, 5-Cl | NO₂ |
| 2873 | 6-Br | 2-F, 6-Cl | NO₂ |
| 2874 | 6-Br | 2,6-F₂, 4-Cl | NO₂ |
| 2875 | 6-Br | 3-F, 4-Cl | NO₂ |
| 2876 | 6-Br | 3-Cl, 5-F | NO₂ |
| 2877 | 6-Br | 2-Cl, 5-F | NO₂ |
| 2878 | 6-Br | 3-CN, 4-Cl | NO₂ |
| 2879 | 6-Br | 3-NO₂, 4-Cl | NO₂ |
| 2880 | 6-Br | 2-F, 4-Br | NO₂ |
| 2881 | 6-CN | 4-F | NO₂ |
| 2882 | 6-CN | 4-Cl | NO₂ |
| 2883 | 6-CN | 4-Br | NO₂ |
| 2884 | 6-CN | H | NO₂ |
| 2885 | 6-CN | 4-Me | NO₂ |
| 2886 | 6-CN | 4-CN | NO₂ |
| 2887 | 6-CN | 4-NO₂ | NO₂ |
| 2888 | 6-CN | 4-OMe | NO₂ |
| 2889 | 6-CN | 3-F | NO₂ |
| 2890 | 6-CN | 3-Cl | NO₂ |
| 2891 | 6-CN | 3-Br | NO₂ |
| 2892 | 6-CN | 3-Me | NO₂ |
| 2893 | 6-CN | 3-CN | NO₂ |
| 2894 | 6-CN | 3-NO₂ | NO₂ |
| 2895 | 6-CN | 3-OMe | NO₂ |
| 2896 | 6-CN | 2-F | NO₂ |
| 2897 | 6-CN | 2-Cl | NO₂ |
| 2898 | 6-CN | 2-Br | NO₂ |
| 2899 | 6-CN | 2-Me | NO₂ |
| 2900 | 6-CN | 2-CN | NO₂ |
| 2901 | 6-CN | 2-NO₂ | NO₂ |
| 2902 | 6-CN | 2-OMe | NO₂ |
| 2903 | 6-CN | 2,3-F₂ | NO₂ |
| 2904 | 6-CN | 2,4-F₂ | NO₂ |
| 2905 | 6-CN | 2,5-F₂ | NO₂ |
| 2906 | 6-CN | 2,6-F₂ | NO₂ |
| 2907 | 6-CN | 3,4-F₂ | NO₂ |
| 2908 | 6-CN | 3,5-F₂ | NO₂ |
| 2909 | 6-CN | 2,3-Cl₂ | NO₂ |
| 2910 | 6-CN | 2,4-Cl₂ | NO₂ |
| 2911 | 6-CN | 2,5-Cl₂ | NO₂ |
| 2912 | 6-CN | 2,6-Cl₂ | NO₂ |
| 2913 | 6-CN | 3,4-Cl₂ | NO₂ |
| 2914 | 6-CN | 3,5-Cl₂ | NO₂ |
| 2915 | 6-CN | 2-F, 3-Cl | NO₂ |
| 2916 | 6-CN | 2-F, 4-Cl | NO₂ |
| 2917 | 6-CN | 2-F, 5-Cl | NO₂ |
| 2918 | 6-CN | 2-F, 6-Cl | NO₂ |
| 2919 | 6-CN | 2,6-F₂, 4-Cl | NO₂ |
| 2920 | 6-CN | 3-F, 4-Cl | NO₂ |
| 2921 | 6-CN | 3-Cl, 5-F | NO₂ |
| 2922 | 6-CN | 2-Cl, 5-F | NO₂ |
| 2923 | 6-CN | 3-CN, 4-Cl | NO₂ |
| 2924 | 6-CN | 3-NO₂, 4-Cl | NO₂ |
| 2925 | 6-CN | 2-F, 4-Br | NO₂ |
| 2926 | 6-Me | 4-F | NO₂ |
| 2927 | 6-Me | 4-Cl | NO₂ |
| 2928 | 6-Me | 4-Br | NO₂ |
| 2929 | 6-Me | H | NO₂ |
| 2930 | 6-Me | 4-Me | NO₂ |
| 2931 | 6-Me | 4-CN | NO₂ |
| 2932 | 6-Me | 4-NO₂ | NO₂ |
| 2933 | 6-Me | 4-OMe | NO₂ |
| 2934 | 6-Me | 3-F | NO₂ |
| 2935 | 6-Me | 3-Cl | NO₂ |
| 2936 | 6-Me | 3-Br | NO₂ |
| 2937 | 6-Me | 3-Me | NO₂ |
| 2938 | 6-Me | 3-CN | NO₂ |
| 2939 | 6-Me | 3-NO₂ | NO₂ |
| 2940 | 6-Me | 3-OMe | NO₂ |
| 2941 | 6-Me | 2-F | NO₂ |
| 2942 | 6-Me | 2-Cl | NO₂ |
| 2943 | 6-Me | 2-Br | NO₂ |
| 2944 | 6-Me | 2-Me | NO₂ |
| 2945 | 6-Me | 2-CN | NO₂ |
| 2946 | 6-Me | 2-NO₂ | NO₂ |
| 2947 | 6-Me | 2-OMe | NO₂ |
| 2948 | 6-Me | 2,3-F₂ | NO₂ |
| 2949 | 6-Me | 2,4-F₂ | NO₂ |
| 2950 | 6-Me | 2,5-F₂ | NO₂ |
| 2951 | 6-Me | 2,6-F₂ | NO₂ |
| 2952 | 6-Me | 3,4-F₂ | NO₂ |
| 2953 | 6-Me | 3,5-F₂ | NO₂ |
| 2954 | 6-Me | 2,3-Cl₂ | NO₂ |
| 2955 | 6-Me | 2,4-Cl₂ | NO₂ |
| 2956 | 6-Me | 2,5-Cl₂ | NO₂ |
| 2957 | 6-Me | 2,6-Cl₂ | NO₂ |
| 2958 | 6-Me | 3,4-Cl₂ | NO₂ |
| 2959 | 6-Me | 3,5-Cl₂ | NO₂ |
| 2960 | 6-Me | 2-F, 3-Cl | NO₂ |
| 2961 | 6-Me | 2-F, 4-Cl | NO₂ |
| 2962 | 6-Me | 2-F, 5-Cl | NO₂ |
| 2963 | 6-Me | 2-F, 6-Cl | NO₂ |
| 2964 | 6-Me | 2,6-F₂, 4-Cl | NO₂ |
| 2965 | 6-Me | 3-F, 4-Cl | NO₂ |
| 2966 | 6-Me | 3-Cl, 5-F | NO₂ |
| 2967 | 6-Me | 2-Cl, 5-F | NO₂ |
| 2968 | 6-Me | 3-CN, 4-Cl | NO₂ |
| 2969 | 6-Me | 3-NO₂, 4-Cl | NO₂ |
| 2970 | 6-Me | 2-F, 4-Br | NO₂ |
| 2971 | 6-OMe | 4-F | NO₂ |
| 2972 | 6-OMe | 4-Cl | NO₂ |
| 2973 | 6-OMe | 4-Br | NO₂ |
| 2974 | 6-OMe | H | NO₂ |
| 2975 | 6-OMe | 4-Me | NO₂ |
| 2976 | 6-OMe | 4-CN | NO₂ |
| 2977 | 6-OMe | 4-NO₂ | NO₂ |
| 2978 | 6-OMe | 4-OMe | NO₂ |
| 2979 | 6-OMe | 3-F | NO₂ |
| 2980 | 6-OMe | 3-Cl | NO₂ |
| 2981 | 6-OMe | 3-Br | NO₂ |
| 2982 | 6-OMe | 3-Me | NO₂ |
| 2983 | 6-OMe | 3-CN | NO₂ |
| 2984 | 6-OMe | 3-NO₂ | NO₂ |
| 2985 | 6-OMe | 3-OMe | NO₂ |
| 2986 | 6-OMe | 2-F | NO₂ |
| 2987 | 6-OMe | 2-Cl | NO₂ |
| 2988 | 6-OMe | 2-Br | NO₂ |
| 2989 | 6-OMe | 2-Me | NO₂ |
| 2990 | 6-OMe | 2-CN | NO₂ |
| 2991 | 6-OMe | 2-NO₂ | NO₂ |
| 2992 | 6-OMe | 2-OMe | NO₂ |
| 2993 | 6-OMe | 2,3-F₂ | NO₂ |
| 2994 | 6-OMe | 2,4-F₂ | NO₂ |
| 2995 | 6-OMe | 2,5-F₂ | NO₂ |
| 2996 | 6-OMe | 2,6-F₂ | NO₂ |
| 2997 | 6-OMe | 3,4-F₂ | NO₂ |
| 2998 | 6-OMe | 3,5-F₂ | NO₂ |
| 2999 | 6-OMe | 2,3-Cl₂ | NO₂ |
| 3000 | 6-OMe | 2,4-Cl₂ | NO₂ |
| 3001 | 6-OMe | 2,5-Cl₂ | NO₂ |
| 3002 | 6-OMe | 2,6-Cl₂ | NO₂ |
| 3003 | 6-OMe | 3,4-Cl₂ | NO₂ |
| 3004 | 6-OMe | 3,5-Cl₂ | NO₂ |
| 3005 | 6-OMe | 2-F, 3-Cl | NO₂ |
| 3006 | 6-OMe | 2-F, 4-Cl | NO₂ |
| 3007 | 6-OMe | 2-F, 5-Cl | NO₂ |
| 3008 | 6-OMe | 2-F, 6-Cl | NO₂ |
| 3009 | 6-OMe | 2,6-F₂, 4-Cl | NO₂ |
| 3010 | 6-OMe | 3-F, 4-Cl | NO₂ |
| 3011 | 6-OMe | 3-Cl, 5-F | NO₂ |
| 3012 | 6-OMe | 2-Cl, 5-F | NO₂ |
| 3013 | 6-OMe | 3-CN, 4-Cl | NO₂ |
| 3014 | 6-OMe | 3-NO₂, 4-Cl | NO₂ |
| 3015 | 6-OMe | 2-F, 4-Br | NO₂ |
| 3016 | 2-F | 4-F | NO₂ |
| 3017 | 2-F | 4-Cl | NO₂ |
| 3018 | 2-F | 4-Br | NO₂ |
| 3019 | 2-F | H | NO₂ |
| 3020 | 2-F | 4-Me | NO₂ |
| 3021 | 2-F | 4-CN | NO₂ |
| 3022 | 2-F | 4-NO₂ | NO₂ |
| 3023 | 2-F | 4-OMe | NO₂ |
| 3024 | 2-F | 3-F | NO₂ |
| 3025 | 2-F | 3-Cl | NO₂ |
| 3026 | 2-F | 3-Br | NO₂ |
| 3027 | 2-F | 3-Me | NO₂ |
| 3028 | 2-F | 3-CN | NO₂ |
| 3029 | 2-F | 3-NO₂ | NO₂ |
| 3030 | 2-F | 3-OMe | NO₂ |
| 3031 | 2-F | 2-F | NO₂ |
| 3032 | 2-F | 2-Cl | NO₂ |
| 3033 | 2-F | 2-Br | NO₂ |
| 3034 | 2-F | 2-Me | NO₂ |
| 3035 | 2-F | 2-CN | NO₂ |
| 3036 | 2-F | 2-NO₂ | NO₂ |
| 3037 | 2-F | 2-OMe | NO₂ |
| 3038 | 2-F | 2,3-F₂ | NO₂ |
| 3039 | 2-F | 2,4-F₂ | NO₂ |
| 3040 | 2-F | 2,5-F₂ | NO₂ |
| 3041 | 2-F | 2,6-F₂ | NO₂ |
| 3042 | 2-F | 3,4-F₂ | NO₂ |
| 3043 | 2-F | 3,5-F₂ | NO₂ |
| 3044 | 2-F | 2,3-Cl₂ | NO₂ |
| 3045 | 2-F | 2,4-Cl₂ | NO₂ |
| 3046 | 2-F | 2,5-Cl₂ | NO₂ |
| 3047 | 2-F | 2,6-Cl₂ | NO₂ |
| 3048 | 2-F | 3,4-Cl₂ | NO₂ |
| 3049 | 2-F | 3,5-Cl₂ | NO₂ |
| 3050 | 2-F | 2-F, 3-Cl | NO₂ |
| 3051 | 2-F | 2-F, 4-Cl | NO₂ |
| 3052 | 2-F | 2-F, 5-Cl | NO₂ |
| 3053 | 2-F | 2-F, 6-Cl | NO₂ |
| 3054 | 2-F | 2,6-F₂, 4-Cl | NO₂ |
| 3055 | 2-F | 3-F, 4-Cl | NO₂ |
| 3056 | 2-F | 3-Cl, 5-F | NO₂ |
| 3057 | 2-F | 2-Cl, 5-F | NO₂ |
| 3058 | 2-F | 3-CN, 4-Cl | NO₂ |
| 3059 | 2-F | 3-NO₂, 4-Cl | NO₂ |
| 3060 | 2-F | 2-F, 4-Br | NO₂ |
| 3061 | 4-F | 4-F | NO₂ |
| 3062 | 4-F | 4-Cl | NO₂ |
| 3063 | 4-F | 4-Br | NO₂ |
| 3064 | 4-F | H | NO₂ |
| 3065 | 4-F | 4-Me | NO₂ |
| 3066 | 4-F | 4-CN | NO₂ |
| 3067 | 4-F | 4-NO₂ | NO₂ |
| 3068 | 4-F | 4-OMe | NO₂ |
| 3069 | 4-F | 3-F | NO₂ |
| 3070 | 4-F | 3-Cl | NO₂ |
| 3071 | 4-F | 3-Br | NO₂ |
| 3072 | 4-F | 3-Me | NO₂ |
| 3073 | 4-F | 3-CN | NO₂ |
| 3074 | 4-F | 3-NO₂ | NO₂ |
| 3075 | 4-F | 3-OMe | NO₂ |
| 3076 | 4-F | 2-F | NO₂ |
| 3077 | 4-F | 2-Cl | NO₂ |
| 3078 | 4-F | 2-Br | NO₂ |
| 3079 | 4-F | 2-Me | NO₂ |
| 3080 | 4-F | 2-CN | NO₂ |
| 3081 | 4-F | 2-NO₂ | NO₂ |
| 3082 | 4-F | 2-OMe | NO₂ |
| 3083 | 4-F | 2,3-F₂ | NO₂ |
| 3084 | 4-F | 2,4-F₂ | NO₂ |
| 3085 | 4-F | 2,5-F₂ | NO₂ |
| 3086 | 4-F | 2,6-F₂ | NO₂ |
| 3087 | 4-F | 3,4-F₂ | NO₂ |
| 3088 | 4-F | 3,5-F₂ | NO₂ |
| 3089 | 4-F | 2,3-Cl₂ | NO₂ |
| 3090 | 4-F | 2,4-Cl₂ | NO₂ |
| 3091 | 4-F | 2,5-Cl₂ | NO₂ |
| 3092 | 4-F | 2,6-Cl₂ | NO₂ |
| 3093 | 4-F | 3,4-Cl₂ | NO₂ |
| 3094 | 4-F | 3,5-Cl₂ | NO₂ |
| 3095 | 4-F | 2-F, 3-Cl | NO₂ |
| 3096 | 4-F | 2-F, 4-Cl | NO₂ |
| 3097 | 4-F | 2-F, 5-Cl | NO₂ |
| 3098 | 4-F | 2-F, 6-Cl | NO₂ |
| 3099 | 4-F | 2,6-F₂, 4-Cl | NO₂ |
| 3100 | 4-F | 3-F, 4-Cl | NO₂ |
| 3101 | 4-F | 3-Cl, 5-F | NO₂ |
| 3102 | 4-F | 2-Cl, 5-F | NO₂ |
| 3103 | 4-F | 3-CN, 4-Cl | NO₂ |
| 3104 | 4-F | 3-NO₂, 4-Cl | NO₂ |
| 3105 | 4-F | 2-F, 4-Br | NO₂ |
| 3106 | 2-Cl | 4-F | NO₂ |
| 3107 | 2-Cl | 4-Cl | NO₂ |
| 3108 | 2-Cl | 4-Br | NO₂ |
| 3109 | 2-Cl | H | NO₂ |
| 3110 | 2-Cl | 4-Me | NO₂ |
| 3111 | 2-Cl | 4-CN | NO₂ |
| 3112 | 2-Cl | 4-NO₂ | NO₂ |
| 3113 | 2-Cl | 4-OMe | NO₂ |
| 3114 | 2-Cl | 3-F | NO₂ |
| 3115 | 2-Cl | 3-Cl | NO₂ |
| 3116 | 2-Cl | 3-Br | NO₂ |
| 3117 | 2-Cl | 3-Me | NO₂ |
| 3118 | 2-Cl | 3-CN | NO₂ |
| 3119 | 2-Cl | 3-NO₂ | NO₂ |
| 3120 | 2-Cl | 3-OMe | NO₂ |
| 3121 | 2-Cl | 2-F | NO₂ |
| 3122 | 2-Cl | 2-Cl | NO₂ |
| 3123 | 2-Cl | 2-Br | NO₂ |
| 3124 | 2-Cl | 2-Me | NO₂ |
| 3125 | 2-Cl | 2-CN | NO₂ |
| 3126 | 2-Cl | 2-NO₂ | NO₂ |
| 3127 | 2-Cl | 2-OMe | NO₂ |
| 3128 | 2-Cl | 2,3-F₂ | NO₂ |
| 3129 | 2-Cl | 2,4-F₂ | NO₂ |
| 3130 | 2-Cl | 2,5-F₂ | NO₂ |
| 3131 | 2-Cl | 2,6-F₂ | NO₂ |
| 3132 | 2-Cl | 3,4-F₂ | NO₂ |
| 3133 | 2-Cl | 3,5-F₂ | NO₂ |
| 3134 | 2-Cl | 2,3-Cl₂ | NO₂ |
| 3135 | 2-Cl | 2,4-Cl₂ | NO₂ |
| 3136 | 2-Cl | 2,5-Cl₂ | NO₂ |
| 3137 | 2-Cl | 2,6-Cl₂ | NO₂ |
| 3138 | 2-Cl | 3,4-Cl₂ | NO₂ |
| 3139 | 2-Cl | 3,5-Cl₂ | NO₂ |
| 3140 | 2-Cl | 2-F, 3-Cl | NO₂ |
| 3141 | 2-Cl | 2-F, 4-Cl | NO₂ |
| 3142 | 2-Cl | 2-F, 5-Cl | NO₂ |
| 3143 | 2-Cl | 2-F, 6-Cl | NO₂ |
| 3144 | 2-Cl | 2,6-F₂, 4-Cl | NO₂ |
| 3145 | 2-Cl | 3-F, 4-Cl | NO₂ |
| 3146 | 2-Cl | 3-Cl, 5-F | NO₂ |
| 3147 | 2-Cl | 2-Cl, 5-F | NO₂ |
| 3148 | 2-Cl | 3-CN, 4-Cl | NO₂ |
| 3149 | 2-Cl | 3-NO₂, 4-Cl | NO₂ |
| 3150 | 2-Cl | 2-F, 4-Br | NO₂ |
| 3151 | 4-Cl | 4-F | NO₂ |
| 3152 | 4-Cl | 4-Cl | NO₂ |
| 3153 | 4-Cl | 4-Br | NO₂ |
| 3154 | 4-Cl | H | NO₂ |
| 3155 | 4-Cl | 4-Me | NO₂ |
| 3156 | 4-Cl | 4-CN | NO₂ |
| 3157 | 4-Cl | 4-NO₂ | NO₂ |
| 3158 | 4-Cl | 4-OMe | NO₂ |
| 3159 | 4-Cl | 3-F | NO₂ |
| 3160 | 4-Cl | 3-Cl | NO₂ |
| 3161 | 4-Cl | 3-Br | NO₂ |
| 3162 | 4-Cl | 3-Me | NO₂ |
| 3163 | 4-Cl | 3-CN | NO₂ |
| 3164 | 4-Cl | 3-NO₂ | NO₂ |
| 3165 | 4-Cl | 3-OMe | NO₂ |
| 3166 | 4-Cl | 2-F | NO₂ |
| 3167 | 4-Cl | 2-Cl | NO₂ |
| 3168 | 4-Cl | 2-Br | NO₂ |
| 3169 | 4-Cl | 2-Me | NO₂ |
| 3170 | 4-Cl | 2-CN | NO₂ |
| 3171 | 4-Cl | 2-NO₂ | NO₂ |
| 3172 | 4-Cl | 2-OMe | NO₂ |
| 3173 | 4-Cl | 2,3-F₂ | NO₂ |
| 3174 | 4-Cl | 2,4-F₂ | NO₂ |
| 3175 | 4-Cl | 2,5-F₂ | NO₂ |
| 3176 | 4-Cl | 2,6-F₂ | NO₂ |
| 3177 | 4-Cl | 3,4-F₂ | NO₂ |
| 3178 | 4-Cl | 3,5-F₂ | NO₂ |
| 3179 | 4-Cl | 2,3-Cl₂ | NO₂ |
| 3180 | 4-Cl | 2,4-Cl₂ | NO₂ |
| 3181 | 4-Cl | 2,5-Cl₂ | NO₂ |
| 3182 | 4-Cl | 2,6-Cl₂ | NO₂ |
| 3183 | 4-Cl | 3,4-Cl₂ | NO₂ |
| 3184 | 4-Cl | 3,5-Cl₂ | NO₂ |
| 3185 | 4-Cl | 2-F, 3-Cl | NO₂ |
| 3186 | 4-Cl | 2-F, 4-Cl | NO₂ |
| 3187 | 4-Cl | 2-F, 5-Cl | NO₂ |
| 3188 | 4-Cl | 2-F, 6-Cl | NO₂ |
| 3189 | 4-Cl | 2,6-F₂, 4-Cl | NO₂ |
| 3190 | 4-Cl | 3-F, 4-Cl | NO₂ |
| 3191 | 4-Cl | 3-Cl, 5-F | NO₂ |
| 3192 | 4-Cl | 2-Cl, 5-F | NO₂ |
| 3193 | 4-Cl | 3-CN, 4-Cl | NO₂ |
| 3194 | 4-Cl | 3-NO₂, 4-Cl | NO₂ |
| 3195 | 4-Cl | 2-F, 4-Br | NO₂ |
| 3196 | 6-OCF₂H | 4-F | NO₂ |
| 3197 | 6-OCF₂H | 4-Cl | NO₂ |
| 3198 | 6-OCF₂H | 4-Br | NO₂ |
| 3199 | 6-OCF₂H | H | NO₂ |
| 3200 | 6-OCF₂H | 4-Me | NO₂ |
| 3201 | 6-OCF₂H | 4-CN | NO₂ |
| 3202 | 6-OCF₂H | 4-NO₂ | NO₂ |
| 3203 | 6-OCF₂H | 4-OMe | NO₂ |
| 3204 | 6-OCF₂H | 3-F | NO₂ |
| 3205 | 6-OCF₂H | 3-Cl | NO₂ |
| 3206 | 6-OCF₂H | 3-Br | NO₂ |
| 3207 | 6-OCF₂H | 3-Me | NO₂ |
| 3208 | 6-OCF₂H | 3-CN | NO₂ |
| 3209 | 6-OCF₂H | 3-NO₂ | NO₂ |
| 3210 | 6-OCF₂H | 3-OMe | NO₂ |
| 3211 | 6-OCF₂H | 2-F | NO₂ |
| 3212 | 6-OCF₂H | 2-Cl | NO₂ |
| 3213 | 6-OCF₂H | 2-Br | NO₂ |
| 3214 | 6-OCF₂H | 2-Me | NO₂ |
| 3215 | 6-OCF₂H | 2-CN | NO₂ |
| 3216 | 6-OCF₂H | 2-NO₂ | NO₂ |
| 3217 | 6-OCF₂H | 2-OMe | NO₂ |
| 3218 | 6-OCF₂H | 2,3-F₂ | NO₂ |
| 3219 | 6-OCF₂H | 2,4-F₂ | NO₂ |
| 3220 | 6-OCF₂H | 2,5-F₂ | NO₂ |
| 3221 | 6-OCF₂H | 2,6-F₂ | NO₂ |
| 3222 | 6-OCF₂H | 3,4-F₂ | NO₂ |
| 3223 | 6-OCF₂H | 3,5-F₂ | NO₂ |
| 3224 | 6-OCF₂H | 2,3-Cl₂ | NO₂ |
| 3225 | 6-OCF₂H | 2,4-Cl₂ | NO₂ |
| 3226 | 6-OCF₂H | 2,5-Cl₂ | NO₂ |
| 3227 | 6-OCF₂H | 2,6-Cl₂ | NO₂ |
| 3228 | 6-OCF₂H | 3,4-Cl₂ | NO₂ |
| 3229 | 6-OCF₂H | 3,5-Cl₂ | NO₂ |
| 3230 | 6-OCF₂H | 2-F, 3-Cl | NO₂ |
| 3231 | 6-OCF₂H | 2-F, 4-Cl | NO₂ |
| 3232 | 6-OCF₂H | 2-F, 5-Cl | NO₂ |
| 3233 | 6-OCF₂H | 2-F, 6-Cl | NO₂ |
| 3234 | 6-OCF₂H | 2,6-F₂, 4-Cl | NO₂ |
| 3235 | 6-OCF₂H | 3-F, 4-Cl | NO₂ |
| 3236 | 6-OCF₂H | 3-Cl, 5-F | NO₂ |
| 3237 | 6-OCF₂H | 2-Cl, 5-F | NO₂ |
| 3238 | 6-OCF₂H | 3-CN, 4-Cl | NO₂ |
| 3239 | 6-OCF₂H | 3-NO₂, 4-Cl | NO₂ |
| 3240 | 6-OCF₂H | 2-F, 4-Br | NO₂ |
| 3241 | H | 4-F | CO₂Me |
| 3242 | H | 4-Cl | CO₂Me |
| 3243 | H | 4-Br | CO₂Me |
| 3244 | H | H | CO₂Me |
| 3245 | H | 4-Me | CO₂Me |
| 3246 | H | 4-CN | CO₂Me |
| 3247 | H | 4-NO₂ | CO₂Me |
| 3248 | H | 4-OMe | CO₂Me |
| 3249 | H | 3-F | CO₂Me |
| 3250 | H | 3-Cl | CO₂Me |
| 3251 | H | 3-Br | CO₂Me |
| 3252 | H | 3-Me | CO₂Me |
| 3253 | H | 3-CN | CO₂Me |
| 3254 | H | 3-NO₂ | CO₂Me |
| 3255 | H | 3-OMe | CO₂Me |
| 3256 | H | 2-F | CO₂Me |
| 3257 | H | 2-Cl | CO₂Me |
| 3258 | H | 2-Br | CO₂Me |
| 3259 | H | 2-Me | CO₂Me |
| 3260 | H | 2-CN | CO₂Me |
| 3261 | H | 2-NO₂ | CO₂Me |
| 3262 | H | 2-OMe | CO₂Me |
| 3263 | H | 2,3-F₂ | CO₂Me |
| 3264 | H | 2,4-F₂ | CO₂Me |
| 3265 | H | 2,5-F₂ | CO₂Me |
| 3266 | H | 2,6-F₂ | CO₂Me |
| 3267 | H | 3,4-F₂ | CO₂Me |
| 3268 | H | 3,5-F₂ | CO₂Me |
| 3269 | H | 2,3-Cl₂ | CO₂Me |
| 3270 | H | 2,4-Cl₂ | CO₂Me |
| 3271 | H | 2,5-Cl₂ | CO₂Me |
| 3272 | H | 2,6-Cl₂ | CO₂Me |
| 3273 | H | 3,4-Cl₂ | CO₂Me |
| 3274 | H | 3,5-Cl₂ | CO₂Me |
| 3275 | H | 2-F, 3-Cl | CO₂Me |
| 3276 | H | 2-F, 4-Cl | CO₂Me |
| 3277 | H | 2-F, 5-Cl | CO₂Me |
| 3278 | H | 2-F, 6-Cl | CO₂Me |
| 3279 | H | 2,6-F₂, 4-Cl | CO₂Me |
| 3280 | H | 3-F, 4-Cl | CO₂Me |
| 3281 | H | 3-Cl, 5-F | CO₂Me |
| 3282 | H | 2-Cl, 5-F | CO₂Me |
| 3283 | H | 3-CN, 4-Cl | CO₂Me |
| 3284 | H | 3-NO₂, 4-Cl | CO₂Me |
| 3285 | H | 2-F, 4-Br | CO₂Me |
| 3286 | 6-F | 4-F | CO₂Me |
| 3287 | 6-F | 4-Cl | CO₂Me |
| 3288 | 6-F | 4-Br | CO₂Me |
| 3289 | 6-F | H | CO₂Me |
| 3290 | 6-F | 4-Me | CO₂Me |
| 3291 | 6-F | 4-CN | CO₂Me |
| 3292 | 6-F | 4-NO₂ | CO₂Me |
| 3293 | 6-F | 4-OMe | CO₂Me |
| 3294 | 6-F | 3-F | CO₂Me |
| 3295 | 6-F | 3-Cl | CO₂Me |
| 3296 | 6-F | 3-Br | CO₂Me |
| 3297 | 6-F | 3-Me | CO₂Me |
| 3298 | 6-F | 3-CN | CO₂Me |
| 3299 | 6-F | 3-NO₂ | CO₂Me |
| 3300 | 6-F | 3-OMe | CO₂Me |
| 3301 | 6-F | 2-F | CO₂Me |
| 3302 | 6-F | 2-Cl | CO₂Me |
| 3303 | 6-F | 2-Br | CO₂Me |
| 3304 | 6-F | 2-Me | CO₂Me |
| 3305 | 6-F | 2-CN | CO₂Me |
| 3306 | 6-F | 2-NO₂ | CO₂Me |
| 3307 | 6-F | 2-OMe | CO₂Me |
| 3308 | 6-F | 2,3-F₂ | CO₂Me |
| 3309 | 6-F | 2,4-F₂ | CO₂Me |
| 3310 | 6-F | 2,5-F₂ | CO₂Me |
| 3311 | 6-F | 2,6-F₂ | CO₂Me |
| 3312 | 6-F | 3,4-F₂ | CO₂Me |
| 3313 | 6-F | 3,5-F₂ | CO₂Me |
| 3314 | 6-F | 2,3-Cl₂ | CO₂Me |
| 3315 | 6-F | 2,4-Cl₂ | CO₂Me |
| 3316 | 6-F | 2,5-Cl₂ | CO₂Me |
| 3317 | 6-F | 2,6-Cl₂ | CO₂Me |
| 3318 | 6-F | 3,4-Cl₂ | CO₂Me |
| 3319 | 6-F | 3,5-Cl₂ | CO₂Me |
| 3320 | 6-F | 2-F, 3-Cl | CO₂Me |
| 3321 | 6-F | 2-F, 4-Cl | CO₂Me |
| 3322 | 6-F | 2-F, 5-Cl | CO₂Me |
| 3323 | 6-F | 2-F, 6-Cl | CO₂Me |
| 3324 | 6-F | 2,6-F₂, 4-Cl | CO₂Me |
| 3325 | 6-F | 3-F, 4-Cl | CO₂Me |
| 3326 | 6-F | 3-Cl, 5-F | CO₂Me |
| 3327 | 6-F | 2-Cl, 5-F | CO₂Me |
| 3328 | 6-F | 3-CN, 4-Cl | CO₂Me |
| 3329 | 6-F | 3-NO₂, 4-Cl | CO₂Me |
| 3330 | 6-F | 2-F, 4-Br | CO₂Me |
| 3331 | 6-Cl | 4-F | CO₂Me |
| 3332 | 6-Cl | 4-Cl | CO₂Me |
| 3333 | 6-Cl | 4-Br | CO₂Me |
| 3334 | 6-Cl | H | CO₂Me |
| 3335 | 6-Cl | 4-Me | CO₂Me |
| 3336 | 6-Cl | 4-CN | CO₂Me |
| 3337 | 6-Cl | 4-NO₂ | CO₂Me |
| 3338 | 6-Cl | 4-OMe | CO₂Me |
| 3339 | 6-Cl | 3-F | CO₂Me |
| 3340 | 6-Cl | 3-Cl | CO₂Me |
| 3341 | 6-Cl | 3-Br | CO₂Me |
| 3342 | 6-Cl | 3-Me | CO₂Me |
| 3343 | 6-Cl | 3-CN | CO₂Me |
| 3344 | 6-Cl | 3-NO₂ | CO₂Me |
| 3345 | 6-Cl | 3-OMe | CO₂Me |
| 3346 | 6-Cl | 2-F | CO₂Me |
| 3347 | 6-Cl | 2-Cl | CO₂Me |
| 3348 | 6-Cl | 2-Br | CO₂Me |
| 3349 | 6-Cl | 2-Me | CO₂Me |
| 3350 | 6-Cl | 2-CN | CO₂Me |
| 3351 | 6-Cl | 2-NO₂ | CO₂Me |
| 3352 | 6-Cl | 2-OMe | CO₂Me |
| 3353 | 6-Cl | 2,3-F₂ | CO₂Me |
| 3354 | 6-Cl | 2,4-F₂ | CO₂Me |
| 3355 | 6-Cl | 2,5-F₂ | CO₂Me |
| 3356 | 6-Cl | 2,6-F₂ | CO₂Me |
| 3357 | 6-Cl | 3,4-F₂ | CO₂Me |
| 3358 | 6-Cl | 3,5-F₂ | CO₂Me |
| 3359 | 6-Cl | 2,3-Cl₂ | CO₂Me |
| 3360 | 6-Cl | 2,4-Cl₂ | CO₂Me |
| 3361 | 6-Cl | 2,5-Cl₂ | CO₂Me |
| 3362 | 6-Cl | 2,6-Cl₂ | CO₂Me |
| 3363 | 6-Cl | 3,4-Cl₂ | CO₂Me |
| 3364 | 6-Cl | 3,5-Cl₂ | CO₂Me |
| 3365 | 6-Cl | 2-F, 3-Cl | CO₂Me |
| 3366 | 6-Cl | 2-F, 4-Cl | CO₂Me |
| 3367 | 6-Cl | 2-F, 5-Cl | CO₂Me |
| 3368 | 6-Cl | 2-F, 6-Cl | CO₂Me |
| 3369 | 6-Cl | 2,6-F₂, 4-Cl | CO₂Me |
| 3370 | 6-Cl | 3-F, 4-Cl | CO₂Me |
| 3371 | 6-Cl | 3-Cl, 5-F | CO₂Me |
| 3372 | 6-Cl | 2-Cl, 5-F | CO₂Me |
| 3373 | 6-Cl | 3-CN, 4-Cl | CO₂Me |
| 3374 | 6-Cl | 3-NO₂, 4-Cl | CO₂Me |
| 3375 | 6-Cl | 2-F, 4-Br | CO₂Me |
| 3376 | 6-Br | 4-F | CO₂Me |
| 3377 | 6-Br | 4-Cl | CO₂Me |
| 3378 | 6-Br | 4-Br | CO₂Me |
| 3379 | 6-Br | H | CO₂Me |
| 3380 | 6-Br | 4-Me | CO₂Me |
| 3381 | 6-Br | 4-CN | CO₂Me |
| 3382 | 6-Br | 4-NO₂ | CO₂Me |
| 3383 | 6-Br | 4-OMe | CO₂Me |
| 3384 | 6-Br | 3-F | CO₂Me |
| 3385 | 6-Br | 3-Cl | CO₂Me |
| 3386 | 6-Br | 3-Br | CO₂Me |
| 3387 | 6-Br | 3-Me | CO₂Me |
| 3388 | 6-Br | 3-CN | CO₂Me |
| 3389 | 6-Br | 3-NO₂ | CO₂Me |
| 3390 | 6-Br | 3-OMe | CO₂Me |
| 3391 | 6-Br | 2-F | CO₂Me |
| 3392 | 6-Br | 2-Cl | CO₂Me |
| 3393 | 6-Br | 2-Br | CO₂Me |
| 3394 | 6-Br | 2-Me | CO₂Me |
| 3395 | 6-Br | 2-CN | CO₂Me |
| 3396 | 6-Br | 2-NO₂ | CO₂Me |
| 3397 | 6-Br | 2-OMe | CO₂Me |
| 3398 | 6-Br | 2,3-F₂ | CO₂Me |
| 3399 | 6-Br | 2,4-F₂ | CO₂Me |
| 3400 | 6-Br | 2,5-F₂ | CO₂Me |
| 3401 | 6-Br | 2,6-F₂ | CO₂Me |
| 3402 | 6-Br | 3,4-F₂ | CO₂Me |
| 3403 | 6-Br | 3,5-F₂ | CO₂Me |
| 3404 | 6-Br | 2,3-Cl₂ | CO₂Me |
| 3405 | 6-Br | 2,4-Cl₂ | CO₂Me |
| 3406 | 6-Br | 2,5-Cl₂ | CO₂Me |
| 3407 | 6-Br | 2,6-Cl₂ | CO₂Me |
| 3408 | 6-Br | 3,4-Cl₂ | CO₂Me |
| 3409 | 6-Br | 3,5-Cl₂ | CO₂Me |
| 3410 | 6-Br | 2-F, 3-Cl | CO₂Me |
| 3411 | 6-Br | 2-F, 4-Cl | CO₂Me |
| 3412 | 6-Br | 2-F, 5-Cl | CO₂Me |
| 3413 | 6-Br | 2-F, 6-Cl | CO₂Me |
| 3414 | 6-Br | 2,6-F₂, 4-Cl | CO₂Me |
| 3415 | 6-Br | 3-F, 4-Cl | CO₂Me |
| 3416 | 6-Br | 3-Cl, 5-F | CO₂Me |
| 3417 | 6-Br | 2-Cl, 5-F | CO₂Me |
| 3418 | 6-Br | 3-CN, 4-Cl | CO₂Me |
| 3419 | 6-Br | 3-NO₂, 4-Cl | CO₂Me |
| 3420 | 6-Br | 2-F, 4-Br | CO₂Me |
| 3421 | 6-CN | 4-F | CO₂Me |
| 3422 | 6-CN | 4-Cl | CO₂Me |
| 3423 | 6-CN | 4-Br | CO₂Me |
| 3424 | 6-CN | H | CO₂Me |
| 3425 | 6-CN | 4-Me | CO₂Me |
| 3426 | 6-CN | 4-CN | CO₂Me |
| 3427 | 6-CN | 4-NO₂ | CO₂Me |
| 3428 | 6-CN | 4-OMe | CO₂Me |
| 3429 | 6-CN | 3-F | CO₂Me |
| 3430 | 6-CN | 3-Cl | CO₂Me |
| 3431 | 6-CN | 3-Br | CO₂Me |
| 3432 | 6-CN | 3-Me | CO₂Me |
| 3433 | 6-CN | 3-CN | CO₂Me |
| 3434 | 6-CN | 3-NO₂ | CO₂Me |
| 3435 | 6-CN | 3-OMe | CO₂Me |
| 3436 | 6-CN | 2-F | CO₂Me |
| 3437 | 6-CN | 2-Cl | CO₂Me |
| 3438 | 6-CN | 2-Br | CO₂Me |
| 3439 | 6-CN | 2-Me | CO₂Me |
| 3440 | 6-CN | 2-CN | CO₂Me |
| 3441 | 6-CN | 2-NO₂ | CO₂Me |
| 3442 | 6-CN | 2-OMe | CO₂Me |
| 3443 | 6-CN | 2,3-F₂ | CO₂Me |
| 3444 | 6-CN | 2,4-F₂ | CO₂Me |
| 3445 | 6-CN | 2,5-F₂ | CO₂Me |
| 3446 | 6-CN | 2,6-F₂ | CO₂Me |
| 3447 | 6-CN | 3,4-F₂ | CO₂Me |
| 3448 | 6-CN | 3,5-F₂ | CO₂Me |
| 3449 | 6-CN | 2,3-Cl₂ | CO₂Me |
| 3450 | 6-CN | 2,4-Cl₂ | CO₂Me |
| 3451 | 6-CN | 2,5-Cl₂ | CO₂Me |
| 3452 | 6-CN | 2,6-Cl₂ | CO₂Me |
| 3453 | 6-CN | 3,4-Cl₂ | CO₂Me |
| 3454 | 6-CN | 3,5-Cl₂ | CO₂Me |
| 3455 | 6-CN | 2-F, 3-Cl | CO₂Me |
| 3456 | 6-CN | 2-F, 4-Cl | CO₂Me |
| 3457 | 6-CN | 2-F, 5-Cl | CO₂Me |
| 3458 | 6-CN | 2-F, 6-Cl | CO₂Me |
| 3459 | 6-CN | 2,6-F₂, 4-Cl | CO₂Me |
| 3460 | 6-CN | 3-F, 4-Cl | CO₂Me |
| 3461 | 6-CN | 3-Cl, 5-F | CO₂Me |
| 3462 | 6-CN | 2-Cl, 5-F | CO₂Me |
| 3463 | 6-CN | 3-CN, 4-Cl | CO₂Me |
| 3464 | 6-CN | 3-NO₂, 4-Cl | CO₂Me |
| 3465 | 6-CN | 2-F, 4-Br | CO₂Me |
| 3466 | 6-Me | 4-F | CO₂Me |
| 3467 | 6-Me | 4-Cl | CO₂Me |
| 3468 | 6-Me | 4-Br | CO₂Me |
| 3469 | 6-Me | H | CO₂Me |
| 3470 | 6-Me | 4-Me | CO₂Me |
| 3471 | 6-Me | 4-CN | CO₂Me |
| 3472 | 6-Me | 4-NO₂ | CO₂Me |
| 3473 | 6-Me | 4-OMe | CO₂Me |
| 3474 | 6-Me | 3-F | CO₂Me |
| 3475 | 6-Me | 3-Cl | CO₂Me |
| 3476 | 6-Me | 3-Br | CO₂Me |
| 3477 | 6-Me | 3-Me | CO₂Me |
| 3478 | 6-Me | 3-CN | CO₂Me |
| 3479 | 6-Me | 3-NO₂ | CO₂Me |
| 3480 | 6-Me | 3-OMe | CO₂Me |
| 3481 | 6-Me | 2-F | CO₂Me |
| 3482 | 6-Me | 2-Cl | CO₂Me |
| 3483 | 6-Me | 2-Br | CO₂Me |
| 3484 | 6-Me | 2-Me | CO₂Me |
| 3485 | 6-Me | 2-CN | CO₂Me |
| 3486 | 6-Me | 2-NO₂ | CO₂Me |
| 3487 | 6-Me | 2-OMe | CO₂Me |
| 3488 | 6-Me | 2,3-F₂ | CO₂Me |
| 3489 | 6-Me | 2,4-F₂ | CO₂Me |
| 3490 | 6-Me | 2,5-F₂ | CO₂Me |
| 3491 | 6-Me | 2,6-F₂ | CO₂Me |
| 3492 | 6-Me | 3,4-F₂ | CO₂Me |
| 3493 | 6-Me | 3,5-F₂ | CO₂Me |
| 3494 | 6-Me | 2,3-Cl₂ | CO₂Me |
| 3495 | 6-Me | 2,4-Cl₂ | CO₂Me |
| 3496 | 6-Me | 2,5-Cl₂ | CO₂Me |
| 3497 | 6-Me | 2,6-Cl₂ | CO₂Me |
| 3498 | 6-Me | 3,4-Cl₂ | CO₂Me |
| 3499 | 6-Me | 3,5-Cl₂ | CO₂Me |
| 3500 | 6-Me | 2-F, 3-Cl | CO₂Me |
| 3501 | 6-Me | 2-F, 4-Cl | CO₂Me |
| 3502 | 6-Me | 2-F, 5-Cl | CO₂Me |
| 3503 | 6-Me | 2-F, 6-Cl | CO₂Me |
| 3504 | 6-Me | 2,6-F₂, 4-Cl | CO₂Me |
| 3505 | 6-Me | 3-F, 4-Cl | CO₂Me |
| 3506 | 6-Me | 3-Cl, 5-F | CO₂Me |
| 3507 | 6-Me | 2-Cl, 5-F | CO₂Me |
| 3508 | 6-Me | 3-CN, 4-Cl | CO₂Me |
| 3509 | 6-Me | 3-NO₂, 4-Cl | CO₂Me |
| 3510 | 6-Me | 2-F, 4-Br | CO₂Me |
| 3511 | 6-OMe | 4-F | CO₂Me |
| 3512 | 6-OMe | 4-Cl | CO₂Me |
| 3513 | 6-OMe | 4-Br | CO₂Me |
| 3514 | 6-OMe | H | CO₂Me |
| 3515 | 6-OMe | 4-Me | CO₂Me |
| 3516 | 6-OMe | 4-CN | CO₂Me |
| 3517 | 6-OMe | 4-NO₂ | CO₂Me |
| 3518 | 6-OMe | 4-OMe | CO₂Me |
| 3519 | 6-OMe | 3-F | CO₂Me |
| 3520 | 6-OMe | 3-Cl | CO₂Me |
| 3521 | 6-OMe | 3-Br | CO₂Me |
| 3522 | 6-OMe | 3-Me | CO₂Me |
| 3523 | 6-OMe | 3-CN | CO₂Me |
| 3524 | 6-OMe | 3-NO₂ | CO₂Me |
| 3525 | 6-OMe | 3-OMe | CO₂Me |
| 3526 | 6-OMe | 2-F | CO₂Me |
| 3527 | 6-OMe | 2-Cl | CO₂Me |
| 3528 | 6-OMe | 2-Br | CO₂Me |
| 3529 | 6-OMe | 2-Me | CO₂Me |
| 3530 | 6-OMe | 2-CN | CO₂Me |
| 3531 | 6-OMe | 2-NO₂ | CO₂Me |
| 3532 | 6-OMe | 2-OMe | CO₂Me |
| 3533 | 6-OMe | 2,3-F₂ | CO₂Me |
| 3534 | 6-OMe | 2,4-F₂ | CO₂Me |
| 3535 | 6-OMe | 2,5-F₂ | CO₂Me |
| 3536 | 6-OMe | 2,6-F₂ | CO₂Me |
| 3537 | 6-OMe | 3,4-F₂ | CO₂Me |
| 3538 | 6-OMe | 3,5-F₂ | CO₂Me |
| 3539 | 6-OMe | 2,3-Cl₂ | CO₂Me |
| 3540 | 6-OMe | 2,4-Cl₂ | CO₂Me |
| 3541 | 6-OMe | 2,5-Cl₂ | CO₂Me |
| 3542 | 6-OMe | 2,6-Cl₂ | CO₂Me |
| 3543 | 6-OMe | 3,4-Cl₂ | CO₂Me |
| 3544 | 6-OMe | 3,5-Cl₂ | CO₂Me |
| 3545 | 6-OMe | 2-F, 3-Cl | CO₂Me |
| 3546 | 6-OMe | 2-F, 4-Cl | CO₂Me |
| 3547 | 6-OMe | 2-F, 5-Cl | CO₂Me |
| 3548 | 6-OMe | 2-F, 6-Cl | CO₂Me |
| 3549 | 6-OMe | 2,6-F₂, 4-Cl | CO₂Me |
| 3550 | 6-OMe | 3-F, 4-Cl | CO₂Me |
| 3551 | 6-OMe | 3-Cl, 5-F | CO₂Me |
| 3552 | 6-OMe | 2-Cl, 5-F | CO₂Me |
| 3553 | 6-OMe | 3-CN, 4-Cl | CO₂Me |
| 3554 | 6-OMe | 3-NO₂, 4-Cl | CO₂Me |
| 3555 | 6-OMe | 2-F, 4-Br | CO₂Me |
| 3556 | 2-F | 4-F | CO₂Me |
| 3557 | 2-F | 4-Cl | CO₂Me |
| 3558 | 2-F | 4-Br | CO₂Me |
| 3559 | 2-F | H | CO₂Me |
| 3560 | 2-F | 4-Me | CO₂Me |
| 3561 | 2-F | 4-CN | CO₂Me |
| 3562 | 2-F | 4-NO₂ | CO₂Me |
| 3563 | 2-F | 4-OMe | CO₂Me |
| 3564 | 2-F | 3-F | CO₂Me |
| 3565 | 2-F | 3-Cl | CO₂Me |
| 3566 | 2-F | 3-Br | CO₂Me |
| 3567 | 2-F | 3-Me | CO₂Me |
| 3568 | 2-F | 3-CN | CO₂Me |
| 3569 | 2-F | 3-NO₂ | CO₂Me |
| 3570 | 2-F | 3-OMe | CO₂Me |
| 3571 | 2-F | 2-F | CO₂Me |
| 3572 | 2-F | 2-Cl | CO₂Me |
| 3573 | 2-F | 2-Br | CO₂Me |
| 3574 | 2-F | 2-Me | CO₂Me |
| 3575 | 2-F | 2-CN | CO₂Me |
| 3576 | 2-F | 2-NO₂ | CO₂Me |
| 3577 | 2-F | 2-OMe | CO₂Me |
| 3578 | 2-F | 2,3-F₂ | CO₂Me |
| 3579 | 2-F | 2,4-F₂ | CO₂Me |
| 3580 | 2-F | 2,5-F₂ | CO₂Me |
| 3581 | 2-F | 2,6-F₂ | CO₂Me |
| 3582 | 2-F | 3,4-F₂ | CO₂Me |
| 3583 | 2-F | 3,5-F₂ | CO₂Me |
| 3584 | 2-F | 2,3-Cl₂ | CO₂Me |
| 3585 | 2-F | 2,4-Cl₂ | CO₂Me |
| 3586 | 2-F | 2,5-Cl₂ | CO₂Me |
| 3587 | 2-F | 2,6-Cl₂ | CO₂Me |
| 3588 | 2-F | 3,4-Cl₂ | CO₂Me |
| 3589 | 2-F | 3,5-Cl₂ | CO₂Me |
| 3590 | 2-F | 2-F, 3-Cl | CO₂Me |
| 3591 | 2-F | 2-F, 4-Cl | CO₂Me |
| 3592 | 2-F | 2-F, 5-Cl | CO₂Me |
| 3593 | 2-F | 2-F, 6-Cl | CO₂Me |
| 3594 | 2-F | 2,6-F₂, 4-Cl | CO₂Me |
| 3595 | 2-F | 3-F, 4-Cl | CO₂Me |
| 3596 | 2-F | 3-Cl, 5-F | CO₂Me |
| 3597 | 2-F | 2-Cl, 5-F | CO₂Me |
| 3598 | 2-F | 3-CN, 4-Cl | CO₂Me |
| 3599 | 2-F | 3-NO₂, 4-Cl | CO₂Me |
| 3600 | 2-F | 2-F, 4-Br | CO₂Me |
| 3601 | 4-F | 4-F | CO₂Me |
| 3602 | 4-F | 4-Cl | CO₂Me |
| 3603 | 4-F | 4-Br | CO₂Me |
| 3604 | 4-F | H | CO₂Me |
| 3605 | 4-F | 4-Me | CO₂Me |
| 3606 | 4-F | 4-CN | CO₂Me |
| 3607 | 4-F | 4-NO₂ | CO₂Me |
| 3608 | 4-F | 4-OMe | CO₂Me |
| 3609 | 4-F | 3-F | CO₂Me |
| 3610 | 4-F | 3-Cl | CO₂Me |
| 3611 | 4-F | 3-Br | CO₂Me |
| 3612 | 4-F | 3-Me | CO₂Me |
| 3613 | 4-F | 3-CN | CO₂Me |
| 3614 | 4-F | 3-NO₂ | CO₂Me |
| 3615 | 4-F | 3-OMe | CO₂Me |
| 3616 | 4-F | 2-F | CO₂Me |
| 3617 | 4-F | 2-Cl | CO₂Me |
| 3618 | 4-F | 2-Br | CO₂Me |
| 3619 | 4-F | 2-Me | CO₂Me |
| 3620 | 4-F | 2-CN | CO₂Me |
| 3621 | 4-F | 2-NO₂ | CO₂Me |
| 3622 | 4-F | 2-OMe | CO₂Me |
| 3623 | 4-F | 2,3-F₂ | CO₂Me |
| 3624 | 4-F | 2,4-F₂ | CO₂Me |
| 3625 | 4-F | 2,5-F₂ | CO₂Me |
| 3626 | 4-F | 2,6-F₂ | CO₂Me |
| 3627 | 4-F | 3,4-F₂ | CO₂Me |
| 3628 | 4-F | 3,5-F₂ | CO₂Me |
| 3629 | 4-F | 2,3-Cl₂ | CO₂Me |
| 3630 | 4-F | 2,4-Cl₂ | CO₂Me |
| 3631 | 4-F | 2,5-Cl₂ | CO₂Me |
| 3632 | 4-F | 2,6-Cl₂ | CO₂Me |
| 3633 | 4-F | 3,4-Cl₂ | CO₂Me |
| 3634 | 4-F | 3,5-Cl₂ | CO₂Me |
| 3635 | 4-F | 2-F, 3-Cl | CO₂Me |
| 3636 | 4-F | 2-F, 4-Cl | CO₂Me |
| 3637 | 4-F | 2-F, 5-Cl | CO₂Me |
| 3638 | 4-F | 2-F, 6-Cl | CO₂Me |
| 3639 | 4-F | 2,6-F₂, 4-Cl | CO₂Me |
| 3640 | 4-F | 3-F, 4-Cl | CO₂Me |
| 3641 | 4-F | 3-Cl, 5-F | CO₂Me |
| 3642 | 4-F | 2-Cl, 5-F | CO₂Me |
| 3643 | 4-F | 3-CN, 4-Cl | CO₂Me |
| 3644 | 4-F | 3-NO₂, 4-Cl | CO₂Me |
| 3645 | 4-F | 2-F, 4-Br | CO₂Me |
| 3646 | 2-Cl | 4-F | CO₂Me |
| 3647 | 2-Cl | 4-Cl | CO₂Me |
| 3648 | 2-Cl | 4-Br | CO₂Me |
| 3649 | 2-Cl | H | CO₂Me |
| 3650 | 2-Cl | 4-Me | CO₂Me |
| 3651 | 2-Cl | 4-CN | CO₂Me |
| 3652 | 2-Cl | 4-NO₂ | CO₂Me |
| 3653 | 2-Cl | 4-OMe | CO₂Me |
| 3654 | 2-Cl | 3-F | CO₂Me |
| 3655 | 2-Cl | 3-Cl | CO₂Me |
| 3656 | 2-Cl | 3-Br | CO₂Me |
| 3657 | 2-Cl | 3-Me | CO₂Me |
| 3658 | 2-Cl | 3-CN | CO₂Me |
| 3659 | 2-Cl | 3-NO₂ | CO₂Me |
| 3660 | 2-Cl | 3-OMe | CO₂Me |
| 3661 | 2-Cl | 2-F | CO₂Me |
| 3662 | 2-Cl | 2-Cl | CO₂Me |
| 3663 | 2-Cl | 2-Br | CO₂Me |
| 3664 | 2-Cl | 2-Me | CO₂Me |
| 3665 | 2-Cl | 2-CN | CO₂Me |
| 3666 | 2-Cl | 2-NO₂ | CO₂Me |
| 3667 | 2-Cl | 2-OMe | CO₂Me |
| 3668 | 2-Cl | 2,3-F₂ | CO₂Me |
| 3669 | 2-Cl | 2,4-F₂ | CO₂Me |
| 3670 | 2-Cl | 2,5-F₂ | CO₂Me |
| 3671 | 2-Cl | 2,6-F₂ | CO₂Me |
| 3672 | 2-Cl | 3,4-F₂ | CO₂Me |
| 3673 | 2-Cl | 3,5-F₂ | CO₂Me |
| 3674 | 2-Cl | 2,3-Cl₂ | CO₂Me |
| 3675 | 2-Cl | 2,4-Cl₂ | CO₂Me |
| 3676 | 2-Cl | 2,5-Cl₂ | CO₂Me |
| 3677 | 2-Cl | 2,6-Cl₂ | CO₂Me |
| 3678 | 2-Cl | 3,4-Cl₂ | CO₂Me |
| 3679 | 2-Cl | 3,5-Cl₂ | CO₂Me |
| 3680 | 2-Cl | 2-F, 3-Cl | CO₂Me |
| 3681 | 2-Cl | 2-F, 4-Cl | CO₂Me |
| 3682 | 2-Cl | 2-F, 5-Cl | CO₂Me |
| 3683 | 2-Cl | 2-F, 6-Cl | CO₂Me |
| 3684 | 2-Cl | 2,6-F₂, 4-Cl | CO₂Me |
| 3685 | 2-Cl | 3-F, 4-Cl | CO₂Me |
| 3686 | 2-Cl | 3-Cl, 5-F | CO₂Me |
| 3687 | 2-Cl | 2-Cl, 5-F | CO₂Me |
| 3688 | 2-Cl | 3-CN, 4-Cl | CO₂Me |
| 3689 | 2-Cl | 3-NO₂, 4-Cl | CO₂Me |
| 3690 | 2-Cl | 2-F, 4-Br | CO₂Me |
| 3691 | 4-Cl | 4-F | CO₂Me |
| 3692 | 4-Cl | 4-Cl | CO₂Me |
| 3693 | 4-Cl | 4-Br | CO₂Me |
| 3694 | 4-Cl | H | CO₂Me |
| 3695 | 4-Cl | 4-Me | CO₂Me |
| 3696 | 4-Cl | 4-CN | CO₂Me |
| 3697 | 4-Cl | 4-NO₂ | CO₂Me |
| 3698 | 4-Cl | 4-OMe | CO₂Me |
| 3699 | 4-Cl | 3-F | CO₂Me |
| 3700 | 4-Cl | 3-Cl | CO₂Me |
| 3701 | 4-Cl | 3-Br | CO₂Me |
| 3702 | 4-Cl | 3-Me | CO₂Me |
| 3703 | 4-Cl | 3-CN | CO₂Me |
| 3704 | 4-Cl | 3-NO₂ | CO₂Me |
| 3705 | 4-Cl | 3-OMe | CO₂Me |
| 3706 | 4-Cl | 2-F | CO₂Me |
| 3707 | 4-Cl | 2-Cl | CO₂Me |
| 3708 | 4-Cl | 2-Br | CO₂Me |
| 3709 | 4-Cl | 2-Me | CO₂Me |
| 3710 | 4-Cl | 2-CN | CO₂Me |
| 3711 | 4-Cl | 2-NO₂ | CO₂Me |
| 3712 | 4-Cl | 2-OMe | CO₂Me |
| 3713 | 4-Cl | 2,3-F₂ | CO₂Me |
| 3714 | 4-Cl | 2,4-F₂ | CO₂Me |
| 3715 | 4-Cl | 2,5-F₂ | CO₂Me |
| 3716 | 4-Cl | 2,6-F₂ | CO₂Me |
| 3717 | 4-Cl | 3,4-F₂ | CO₂Me |
| 3718 | 4-Cl | 3,5-F₂ | CO₂Me |
| 3719 | 4-Cl | 2,3-Cl₂ | CO₂Me |
| 3720 | 4-Cl | 2,4-Cl₂ | CO₂Me |
| 3721 | 4-Cl | 2,5-Cl₂ | CO₂Me |
| 3722 | 4-Cl | 2,6-Cl₂ | CO₂Me |
| 3723 | 4-Cl | 3,4-Cl₂ | CO₂Me |
| 3724 | 4-Cl | 3,5-Cl₂ | CO₂Me |
| 3725 | 4-Cl | 2-F, 3-Cl | CO₂Me |
| 3726 | 4-Cl | 2-F, 4-Cl | CO₂Me |
| 3727 | 4-Cl | 2-F, 5-Cl | CO₂Me |
| 3728 | 4-Cl | 2-F, 6-Cl | CO₂Me |
| 3729 | 4-Cl | 2,6-F₂, 4-Cl | CO₂Me |
| 3730 | 4-Cl | 3-F, 4-Cl | CO₂Me |
| 3731 | 4-Cl | 3-Cl, 5-F | CO₂Me |
| 3732 | 4-Cl | 2-Cl, 5-F | CO₂Me |
| 3733 | 4-Cl | 3-CN, 4-Cl | CO₂Me |
| 3734 | 4-Cl | 3-NO₂, 4-Cl | CO₂Me |
| 3735 | 4-Cl | 2-F, 4-Br | CO₂Me |
| 3736 | 6-OCF₂H | 4-F | CO₂Me |
| 3737 | 6-OCF₂H | 4-Cl | CO₂Me |
| 3738 | 6-OCF₂H | 4-Br | CO₂Me |
| 3739 | 6-OCF₂H | H | CO₂Me |
| 3740 | 6-OCF₂H | 4-Me | CO₂Me |
| 3741 | 6-OCF₂H | 4-CN | CO₂Me |
| 3742 | 6-OCF₂H | 4-NO₂ | CO₂Me |
| 3743 | 6-OCF₂H | 4-OMe | CO₂Me |
| 3744 | 6-OCF₂H | 3-F | CO₂Me |
| 3745 | 6-OCF₂H | 3-Cl | CO₂Me |
| 3746 | 6-OCF₂H | 3-Br | CO₂Me |
| 3747 | 6-OCF₂H | 3-Me | CO₂Me |
| 3748 | 6-OCF₂H | 3-CN | CO₂Me |
| 3749 | 6-OCF₂H | 3-NO₂ | CO₂Me |
| 3750 | 6-OCF₂H | 3-OMe | CO₂Me |
| 3751 | 6-OCF₂H | 2-F | CO₂Me |
| 3752 | 6-OCF₂H | 2-Cl | CO₂Me |
| 3753 | 6-OCF₂H | 2-Br | CO₂Me |
| 3754 | 6-OCF₂H | 2-Me | CO₂Me |
| 3755 | 6-OCF₂H | 2-CN | CO₂Me |
| 3756 | 6-OCF₂H | 2-NO₂ | CO₂Me |
| 3757 | 6-OCF₂H | 2-OMe | CO₂Me |
| 3758 | 6-OCF₂H | 2,3-F₂ | CO₂Me |
| 3759 | 6-OCF₂H | 2,4-F₂ | CO₂Me |
| 3760 | 6-OCF₂H | 2,5-F₂ | CO₂Me |
| 3761 | 6-OCF₂H | 2,6-F₂ | CO₂Me |
| 3762 | 6-OCF₂H | 3,4-F₂ | CO₂Me |
| 3763 | 6-OCF₂H | 3,5-F₂ | CO₂Me |
| 3764 | 6-OCF₂H | 2,3-Cl₂ | CO₂Me |
| 3765 | 6-OCF₂H | 2,4-Cl₂ | CO₂Me |
| 3766 | 6-OCF₂H | 2,5-Cl₂ | CO₂Me |
| 3767 | 6-OCF₂H | 2,6-Cl₂ | CO₂Me |
| 3768 | 6-OCF₂H | 3,4-Cl₂ | CO₂Me |
| 3769 | 6-OCF₂H | 3,5-Cl₂ | CO₂Me |
| 3770 | 6-OCF₂H | 2-F, 3-Cl | CO₂Me |
| 3771 | 6-OCF₂H | 2-F, 4-Cl | CO₂Me |
| 3772 | 6-OCF₂H | 2-F, 5-Cl | CO₂Me |
| 3773 | 6-OCF₂H | 2-F, 6-Cl | CO₂Me |
| 3774 | 6-OCF₂H | 2,6-F₂, 4-Cl | CO₂Me |
| 3775 | 6-OCF₂H | 3-F, 4-Cl | CO₂Me |
| 3776 | 6-OCF₂H | 3-Cl, 5-F | CO₂Me |
| 3777 | 6-OCF₂H | 2-Cl, 5-F | CO₂Me |
| 3778 | 6-OCF₂H | 3-CN, 4-Cl | CO₂Me |
| 3779 | 6-OCF₂H | 3-NO₂, 4-Cl | CO₂Me |
| 3780 | 6-OCF₂H | 2-F, 4-Br | CO₂Me |
| 3781 | H | 3-F, 4-OMe | F |
| 3782 | H | 3-F, 4-OMe | Cl |
| 3783 | H | 3-F, 4-OMe | Br |
| 3784 | H | 3-F, 4-OMe | Me |
| 3785 | H | 3-F, 4-OMe | CN |
| 3786 | H | 3-F, 4-OMe | NO₂ |
| 3787 | H | 3-F, 4-OMe | CO₂Me |
| 3788 | 6-F | 3-F, 4-OMe | F |
| 3789 | 6-F | 3-F, 4-OMe | Cl |
| 3790 | 6-F | 3-F, 4-OMe | Br |
| 3791 | 6-F | 3-F, 4-OMe | Me |
| 3792 | 6-F | 3-F, 4-OMe | CN |
| 3793 | 6-F | 3-F, 4-OMe | NO₂ |
| 3794 | 6-F | 3-F, 4-OMe | CO₂Me |
| 3795 | H | 3-F, 4-CN | F |
| 3796 | H | 3-F, 4-CN | Cl |
| 3797 | H | 3-F, 4-CN | Br |
| 3798 | H | 3-F, 4-CN | Me |
| 3799 | H | 3-F, 4-CN | CN |
| 3800 | H | 3-F, 4-CN | NO₂ |
| 3801 | H | 3-F, 4-CN | CO₂Me |
| 3802 | 6-F | 3-F, 4-CN | F |
| 3803 | 6-F | 3-F, 4-CN | Cl |
| 3804 | 6-F | 3-F, 4-CN | Br |
| 3805 | 6-F | 3-F, 4-CN | Me |
| 3806 | 6-F | 3-F, 4-CN | CN |
| 3807 | 6-F | 3-F, 4-CN | NO₂ |
| 3808 | 6-F | 3-F, 4-CN | CO₂Me |
| 3809 | 6-CI | 3-F, 4-CN | F |
| 3810 | 6-CI | 3-F, 4-CN | Cl |
| 3811 | 6-CI | 3-F, 4-CN | Br |
| 3812 | 6-CI | 3-F, 4-CN | Me |
| 3813 | 6-CI | 3-F, 4-CN | CN |
| 3814 | 6-CI | 3-F, 4-CN | NO₂ |
| 3815 | 6-CI | 3-F, 4-CN | CO₂Me |
| 3816 | 6-CN | 3-F, 4-CN | F |
| 3817 | 6-CN | 3-F, 4-CN | Cl |
| 3818 | 6-CN | 3-F, 4-CN | Br |
| 3819 | 6-CN | 3-F, 4-CN | Me |
| 3820 | 6-CN | 3-F, 4-CN | CN |
| 3821 | 6-CN | 3-F, 4-CN | NO₂ |
| 3822 | 6-CN | 3-F, 4-CN | CO₂Me |
| 3823 | H | 3-F, 4-NO₂ | F |
| 3824 | H | 3-F, 4-NO₂ | Cl |
| 3825 | H | 3-F, 4-NO₂ | Br |
| 3826 | H | 3-F, 4-NO₂ | Me |
| 3827 | H | 3-F, 4-NO₂ | CN |
| 3828 | H | 3-F, 4-NO₂ | NO₂ |
| 3829 | H | 3-F, 4-NO₂ | CO₂Me |
| 3830 | 6-F | 3-F, 4-NO₂ | F |
| 3831 | 6-F | 3-F, 4-NO₂ | Cl |
| 3832 | 6-F | 3-F, 4-NO₂ | Br |
| 3833 | 6-F | 3-F, 4-NO₂ | Me |
| 3834 | 6-F | 3-F, 4-NO₂ | CN |
| 3835 | 6-F | 3-F, 4-NO₂ | NO₂ |
| 3836 | 6-F | 3-F, 4-NO₂ | CO₂Me |
| 3837 | 6-Cl | 3-F, 4-NO₂ | F |
| 3838 | 6-Cl | 3-F, 4-NO₂ | Cl |
| 3839 | 6-Cl | 3-F, 4-NO₂ | Br |
| 3840 | 6-Cl | 3-F, 4-NO₂ | Me |
| 3841 | 6-Cl | 3-F, 4-NO₂ | CN |
| 3842 | 6-Cl | 3-F, 4-NO₂ | NO₂ |
| 3843 | 6-Cl | 3-F, 4-NO₂ | CO₂Me |
| 3844 | 6-CN | 3-F, 4-NO₂ | F |
| 3845 | 6-CN | 3-F, 4-NO₂ | Cl |
| 3846 | 6-CN | 3-F, 4-NO₂ | Br |
| 3847 | 6-CN | 3-F, 4-NO₂ | Me |
| 3848 | 6-CN | 3-F, 4-NO₂ | CN |
| 3849 | 6-CN | 3-F, 4-NO₂ | NO₂ |
| 3850 | 6-CN | 3-F, 4-NO₂ | CO₂Me |
| 3851 | H | 3-Cl, 4-F | F |
| 3852 | H | 3-Cl, 4-F | Cl |
| 3853 | H | 3-Cl, 4-F | Br |
| 3854 | H | 3-Cl, 4-F | Me |
| 3855 | H | 3-Cl, 4-F | CN |
| 3856 | H | 3-Cl, 4-F | NO₂ |
| 3857 | H | 3-Cl, 4-F | CO₂Me |
| 3858 | 6-F | 3-Cl, 4-F | F |
| 3859 | 6-F | 3-Cl, 4-F | Cl |
| 3860 | 6-F | 3-Cl, 4-F | Br |
| 3861 | 6-F | 3-Cl, 4-F | Me |
| 3862 | 6-F | 3-Cl, 4-F | CN |
| 3863 | 6-F | 3-Cl, 4-F | NO₂ |
| 3864 | 6-F | 3-Cl, 4-F | CO₂Me |
| 3865 | 6-Cl | 3-Cl, 4-F | F |
| 3866 | 6-Cl | 3-Cl, 4-F | Cl |
| 3867 | 6-Cl | 3-Cl, 4-F | Br |
| 3868 | 6-Cl | 3-Cl, 4-F | Me |
| 3869 | 6-Cl | 3-Cl, 4-F | CN |
| 3870 | 6-Cl | 3-Cl, 4-F | NO₂ |
| 3871 | 6-Cl | 3-Cl, 4-F | CO₂Me |
| 3872 | 6-CN | 3-Cl, 4-F | F |
| 3873 | 6-CN | 3-Cl, 4-F | Cl |
| 3874 | 6-CN | 3-Cl, 4-F | Br |
| 3875 | 6-CN | 3-Cl, 4-F | Me |
| 3876 | 6-CN | 3-Cl, 4-F | CN |
| 3877 | 6-CN | 3-Cl, 4-F | NO₂ |
| 3878 | 6-CN | 3-Cl, 4-F | CO₂Me |
| 3879 | H | 3-F, 4-Br | F |
| 3880 | H | 3-F, 4-Br | Cl |
| 3881 | H | 3-F, 4-Br | Br |
| 3882 | H | 3-F, 4-Br | Me |
| 3883 | H | 3-F, 4-Br | CN |
| 3884 | H | 3-F, 4-Br | NO₂ |
| 3885 | H | 3-F, 4-Br | CO₂Me |
| 3886 | 6-F | 3-F, 4-Br | F |
| 3887 | 6-F | 3-F, 4-Br | Cl |
| 3888 | 6-F | 3-F, 4-Br | Br |
| 3889 | 6-F | 3-F, 4-Br | Me |
| 3890 | 6-F | 3-F, 4-Br | CN |
| 3891 | 6-F | 3-F, 4-Br | NO₂ |
| 3892 | 6-F | 3-F, 4-Br | CO₂Me |
| 3893 | 6-Cl | 3-F, 4-Br | F |
| 3894 | 6-Cl | 3-F, 4-Br | Cl |
| 3895 | 6-Cl | 3-F, 4-Br | Br |
| 3896 | 6-Cl | 3-F, 4-Br | Me |
| 3897 | 6-Cl | 3-F, 4-Br | CN |
| 3898 | 6-Cl | 3-F, 4-Br | NO₂ |
| 3899 | 6-Cl | 3-F, 4-Br | CO₂Me |
| 3900 | 6-CN | 3-F, 4-Br | F |
| 3901 | 6-CN | 3-F, 4-Br | Cl |
| 3902 | 6-CN | 3-F, 4-Br | Br |
| 3903 | 6-CN | 3-F, 4-Br | Me |
| 3904 | 6-CN | 3-F, 4-Br | CN |
| 3905 | 6-CN | 3-F, 4-Br | NO₂ |
| 3906 | 6-CN | 3-F, 4-Br | CO₂Me |
| 3907 | H | 3-CN, 4-F | F |
| 3908 | H | 3-CN, 4-F | Cl |
| 3909 | H | 3-CN, 4-F | Br |
| 3910 | H | 3-CN, 4-F | Me |
| 3911 | H | 3-CN, 4-F | CN |
| 3912 | H | 3-CN, 4-F | NO₂ |
| 3913 | H | 3-CN, 4-F | CO₂Me |
| 3914 | 6-F | 3-CN, 4-F | F |
| 3915 | 6-F | 3-CN, 4-F | Cl |
| 3916 | 6-F | 3-CN, 4-F | Br |
| 3917 | 6-F | 3-CN, 4-F | Me |
| 3918 | 6-F | 3-CN, 4-F | CN |
| 3919 | 6-F | 3-CN, 4-F | NO₂ |
| 3920 | 6-F | 3-CN, 4-F | CO₂Me |
| 3921 | 6-Cl | 3-CN, 4-F | F |
| 3922 | 6-Cl | 3-CN, 4-F | Cl |
| 3923 | 6-Cl | 3-CN, 4-F | Br |
| 3924 | 6-Cl | 3-CN, 4-F | Me |
| 3925 | 6-Cl | 3-CN, 4-F | CN |
| 3926 | 6-Cl | 3-CN, 4-F | NO₂ |
| 3927 | 6-Cl | 3-CN, 4-F | CO₂Me |
| 3928 | 6-CN | 3-CN, 4-F | F |
| 3929 | 6-CN | 3-CN, 4-F | Cl |
| 3930 | 6-CN | 3-CN, 4-F | Br |
| 3931 | 6-CN | 3-CN, 4-F | Me |
| 3932 | 6-CN | 3-CN, 4-F | CN |
| 3933 | 6-CN | 3-CN, 4-F | NO₂ |
| 3934 | 6-CN | 3-CN, 4-F | CO₂Me |
| 3935 | H | 4-CF₃ | F |
| 3936 | H | 4-CF₃ | Cl |
| 3937 | H | 4-CF₃ | Br |
| 3938 | H | 4-CF₃ | Me |
| 3939 | H | 4-CF₃ | CN |
| 3940 | H | 4-CF₃ | NO₂ |
| 3941 | H | 4-CF₃ | CO₂Me |
| 3942 | 6-F | 4-CF₃ | F |
| 3943 | 6-F | 4-CF₃ | Cl |
| 3944 | 6-F | 4-CF₃ | Br |
| 3945 | 6-F | 4-CF₃ | Me |
| 3946 | 6-F | 4-CF₃ | CN |
| 3947 | 6-F | 4-CF₃ | NO₂ |
| 3948 | 6-F | 4-CF₃ | CO₂Me |
| 3949 | 6-Cl | 4-CF₃ | F |
| 3950 | 6-Cl | 4-CF₃ | Cl |
| 3951 | 6-Cl | 4-CF₃ | Br |
| 3952 | 6-Cl | 4-CF₃ | Me |
| 3953 | 6-Cl | 4-CF₃ | CN |
| 3954 | 6-Cl | 4-CF₃ | NO₂ |
| 3955 | 6-Cl | 4-CF₃ | CO₂Me |
| 3956 | 6-CN | 4-CF₃ | F |
| 3957 | 6-CN | 4-CF₃ | Cl |
| 3958 | 6-CN | 4-CF₃ | Br |
| 3959 | 6-CN | 4-CF₃ | Me |
| 3960 | 6-CN | 4-CF₃ | CN |
| 3961 | 6-CN | 4-CF₃ | NO₂ |
| 3962 | 6-CN | 4-CF₃ | CO₂Me |
| 3963 | H | 4-OCF₃ | F |
| 3964 | H | 4-OCF₃ | Cl |
| 3965 | H | 4-OCF₃ | Br |
| 3966 | H | 4-OCF₃ | Me |
| 3967 | H | 4-OCF₃ | CN |
| 3968 | H | 4-OCF₃ | NO₂ |
| 3969 | H | 4-OCF₃ | CO₂Me |
| 3970 | 6-F | 4-OCF₃ | F |
| 3971 | 6-F | 4-OCF₃ | Cl |
| 3972 | 6-F | 4-OCF₃ | Br |
| 3973 | 6-F | 4-OCF₃ | Me |
| 3974 | 6-F | 4-OCF₃ | CN |
| 3975 | 6-F | 4-OCF₃ | NO₂ |
| 3976 | 6-F | 4-OCF₃ | CO₂Me |
| 3977 | 6-Cl | 4-OCF₃ | F |
| 3978 | 6-Cl | 4-OCF₃ | Cl |
| 3979 | 6-Cl | 4-OCF₃ | Br |
| 3980 | 6-Cl | 4-OCF₃ | Me |
| 3981 | 6-Cl | 4-OCF₃ | CN |
| 3982 | 6-Cl | 4-OCF₃ | NO₂ |
| 3983 | 6-Cl | 4-OCF₃ | CO₂Me |
| 3984 | 6-CN | 4-OCF₃ | F |
| 3985 | 6-CN | 4-OCF₃ | Cl |
| 3986 | 6-CN | 4-OCF₃ | Br |
| 3987 | 6-CN | 4-OCF₃ | Me |
| 3988 | 6-CN | 4-OCF₃ | CN |
| 3989 | 6-CN | 4-OCF₃ | NO₂ |
| 3990 | 6-CN | 4-OCF₃ | CO₂Me |

Definition der Beispiele in den nachfolgenden Tabellen 2 bis 2f:
Zu Referenzzwecken sind in den nachfolgenden Tabellen 2 bis 2f die einzelnen Verbindungen einzelnen Nummern (= Beispielnummern) zugeordnet, wobei die jeweilige Beispielnummer sich zusammensetzt aus der Nummer der chemischen Formel, die der jeweiligen Tabelle zugeordnet ist und einer "Zeilennummer" (Zeilennummer), die sich auf dieselbe Nummer in der Zeile der ersten Spalte der Tabelle 1 bezieht. Die chemische Struktur des Beispiels Nr. "(Formelnummer)(Zeilennummer)" ist damit durch die der jeweiligen Tabelle vorangestellten Formel gemäß Formelnummer und der Zeilennummer aus Tabelle 1 eindeutig definiert, zum Beispiel:
   Das Beispiel mit der Nr. "Iba1" aus Tabelle 2 ist die Verbindung der Formel (Ib), worin R⁴ = H (= Wasserstoff) bedeutet [= Formel (Iba)] und (R¹)ₘ = H, (R²)ₙ = 4-F und R³ = F gemäß der Zeile 1 aus Tabelle 1 definiert ist.
   Das Beispiel mit der Nr. "Ibd1801" aus Tabelle 2 ist die Verbindung der Formel (Ib), worin R⁴ = n-Propyl bedeutet [= Formel (Ibd)] und (R¹)ₘ = 6-CN, (R²)ₙ = 4-F und R³ = Me = CH₃ gemäß der Zeile 1801 aus Tabelle 1 definiert ist.

Entsprechendes gilt für die Zuordnung von racemischen oder optisch aktiven threo-Stereoisomeren oder erythro-Stereoisomeren. Beispielsweise sind die Verbindungen der Tabelle 2a zu Referenzzwecken einzelnen Nummern (= Beispielnummern) zugeordnet, wobei die Nummer "threo-Iba(Zeilennummer)" das racemische Gemisch der threo-Enantiomeren mit der chemischen Struktur der Formeln (threo-1-Iba) und (threo-2-Iba), welche jeweils die Strukturkombination der Gruppen (R¹)ₘ, (R²)ₙ und R³ gemäß der Zeilennummer aus der Tabelle 1 aufweist, bezeichnet.

Tabelle 2: Verbindungen der Formel (Ib), (Iba), (Ibb), (Ibc), (Ibd), (Ibe), (Ibf), (Ibg) (Ibh), (Ibi), (Ibj), (Ibk), (Ibl), (Ibm), (Ibn), (Ibo), (Ibp), (Ibq), (Ibr) (Ibs), (Ibt), (Ibu), (Ibv), (Ibw), (Ibx), (Iby) und (Ibz), worin jeweils (R¹)ₘ, (R²)ₙ und R³ wie in Tabelle 1 definiert sind

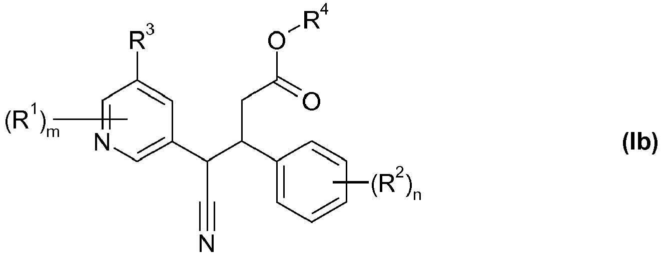

Definition von Unterformeln zu Formel (Ib), siehe nachfolgende Tabelle U1:

**Tabelle U1**

| Formel | Rest R⁴ in Formel (Ib) |
|---|---|
| (Iba) | H (Wasserstoff-atom) |
| (Ibb) | Methyl |
| (Ibc) | Ethyl |
| (Ibd) | n-Propyl |
| (Ibe) | i-Propyl (= Isopropyl) |
| (Ibf) | 2,2-Difluorethyl |
| (Ibg) | 2,2,2-Trifluorethyl |
| (Ibh) | 2-Methoxyethyl |
| (Ibi) | Cyclopropyl methyl |
| (Ibj) | (1-Methylcyclopropyl)methyl |
| (Ibk) | Allyl |
| (Ibl) | Prop-2-in-1-yl |
| (Ibm) | Ethinyl |
| (Ibn) | Prop-1-in-1-yl |
| (Ibo) | Benzyl |
| (Ibp) | 4-Chlorbenzyl |
| (Ibq) | Phenyl |
| (Ibr) | Methoxymethyl |
| (Ibs) | Difluormethyl |
| (Ibt) | Oxetan-3-yl |
| (Ibu) | Thietan-3-yl |
| (Ibv) | 2-(Phenylsulfanyl)ethyl |
| (Ibw) | 2-(Phenylsulfinyl)ethyl |
| (Ibx) | 2-(Ethylsulfanyl)ethyl |
| (Iby) | 2-(Ethylsulfinyl)ethyl |
| (Ibz) | Tetrahydrofuran-2-yl-methyl |

### Erythro-threo-Gemische der Formeln (Iba) bis (Ibz):

Beispiele für Verbindungen der Formeln (Iba) bis (Ibz) sind die Verbindungen der betreffenden Formel (Iba) bis (Ibz) in Form eines racemischen erythro-threo-Gemischs (Mengenverhältnis 70:30 bis 30:70), worin die Strukturkombination der Gruppen (R¹)ₘ, (R²)ₙ und R³ gemäß einer Zeilennummer aus der Tabelle 1 definiert ist.

Die Nummerierung erfolgt gemäß "(Formel)(Zeilennummer)" ohne Schreiben der Klammern, z. B. Iba200 = Verbindung der Formel (Iba) mit der Strukturkombination von Zeile 200 aus Tabelle 1.

### Tabellen 2a, 2b und 2c:

Threo-, threo-1- und threo-2-Verbindungen zu den Verbindungen der Formel (Ib), (Iba), (Ibb), (Ibc), (Ibd), (Ibe), (Ibf), (Ibg) (Ibh), (Ibi), (Ibj), (Ibk), (Ibl), (Ibm), (Ibn), (Ibo), (Ibp), (Ibq), (Ibr) (Ibs), (Ibt), (Ibu), (Ibv), (Ibw), (Ibx), (Iby) und (Ibz), worin jeweils (R¹)ₘ, (R²)ₙ und R³ wie in Tabelle 1 definiert sind

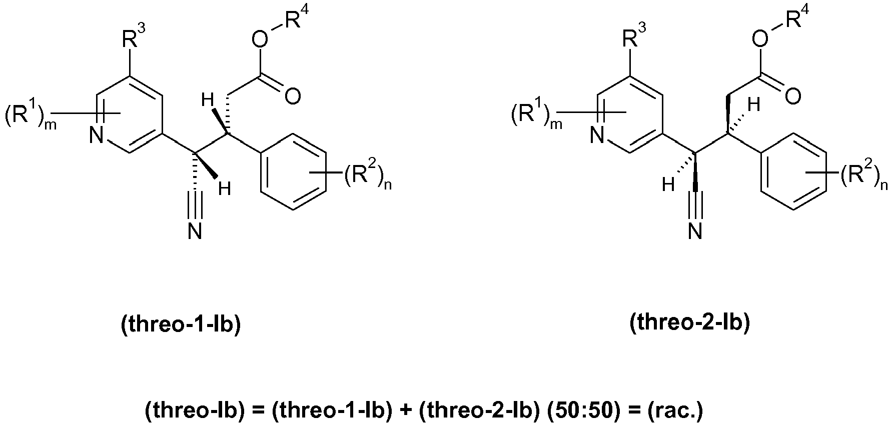

Definition von Unterformeln zu Formeln (threo-Ib), (threo-1-Ib) und (threo-2-Ib), siehe nachfolgende Tabelle U2:

**Tabelle U2**

| Formel | Rest R⁴ in Formel (threo-Ib) |
|---|---|
| (threo-Iba) | H (Wasserstoff-atom) |
| (threo-1-Iba) | H (Wasserstoff-atom) |
| (threo-2-Iba) | H (Wasserstoff-atom) |
| (threo-Ibb) | Methyl |
| (threo-1-Ibb) | Methyl |
| (threo-2-Ibb) | Methyl |
| (threo-Ibc) | Ethyl |
| (threo-1-Ibc) | Ethyl |
| (threo-2-Ibc) | Ethyl |
| (threo-Ibd) | n-Propyl |
| (threo-1-Ibd) | n-Propyl |
| (threo-2-Ibd) | n-Propyl |
| (threo-Ibe) | i-Propyl (= Isopropyl) |
| (threo-1-Ibe) | i-Propyl |
| (threo-2-Ibe) | i-Propyl |
| (threo-Ibf) | 2,2-Difluorethyl |
| (threo-1-Ibf) | 2,2-Difluorethyl |
| (threo-2-Ibf) | 2,2-Difluorethyl |
| (threo-Ibg) | 2,2,2-Trifluorethyl |
| (threo-1-Ibg) | 2,2,2-Trifluorethyl |
| (threo-2-Ibg) | 2,2,2-Trifluorethyl |
| (threo-Ibh) | 2-Methoxyethyl |
| (threo-1-Ibh) | 2-Methoxyethyl |
| (threo-2-Ibh) | 2-Methoxyethyl |
| (threo-Ibi) | Cyclopropyl methyl |
| (threo-1-Ibi) | Cyclopropyl methyl |
| (threo-2-Ibi) | Cyclopropyl methyl |
| (threo-Ibj) | (1-Methylcyclopropyl)methyl |
| (threo-1-Ibj) | (1-Methylcyclopropyl)methyl |
| (threo-2-Ibj) | (1-Methylcyclopropyl)methyl |
| (threo-Ibk) | Allyl |
| (threo-1-Ibk) | Allyl |
| (threo-2-Ibk) | Allyl |
| (threo-Ibl) | Prop-2-in-1-yl |
| (threo-1-Ibl) | Prop-2-in-1-yl |
| (threo-2-Ibl) | Prop-2-in-1-yl |
| (threo-Ibm) | Ethinyl |
| (threo-1-Ibm) | Ethinyl |
| (threo-2-Ibm) | Ethinyl |
| (threo-Ibn) | Prop-1-in-1-yl |
| (threo-1-Ibn) | Prop-1-in-1-yl |
| (threo-2-Ibn) | Prop-1-in-1-yl |
| (threo-Ibo) | Benzyl |
| (threo-1-Ibo) | Benzyl |
| (threo-2-Ibo) | Benzyl |
| (threo-Ibp) | 4-Chlorbenzyl |
| (threo-1-Ibp) | 4-Chlorbenzyl |
| (threo-2-Ibp) | 4-Chlorbenzyl |
| (threo-Ibq) | Phenyl |
| (threo-1-Ibq) | Phenyl |
| (threo-2-Ibq) | Phenyl |
| (threo-Ibr) | Methoxymethyl |
| (threo-1-Ibr) | Methoxymethyl |
| (threo-2-Ibr) | Methoxymethyl |
| (threo-Ibs) | Difluormethyl |
| (threo-1-Ibs) | Difluormethyl |
| (threo-2-Ibs) | Difluormethyl |
| (threo-Ibt) | Oxetan-3-yl |
| (threo-1-Ibt) | Oxetan-3-yl |
| (threo-2-Ibt) | Oxetan-3-yl |
| (threo-Ibu) | Thietan-3-yl |
| (threo-1-Ibu) | Thietan-3-yl |
| (threo-2-Ibu) | Thietan-3-yl |
| (threo-Ibv) | 2-(Phenylsulfanyl)ethyl |
| (threo-1-Ibv) | 2-(Phenylsulfanyl)ethyl |
| (threo-2-Ibv) | 2-(Phenylsulfanyl)ethyl |
| (threo-Ibw) | 2-(Phenylsulfinyl)ethyl |
| (threo-1-Ibw) | 2-(Phenylsulfinyl)ethyl |
| (threo-2-Ibw) | 2-(Phenylsulfinyl)ethyl |
| (threo-Ibx) | 2-(Ethylsulfanyl)ethyl |
| (threo-1-Ibx) | 2-(Ethylsulfanyl)ethyl |
| (threo-2-Ibx) | 2-(Ethylsulfanyl)ethyl |
| (threo-Iby) | 2-(Ethylsulfinyl)ethyl |
| (threo-1-Iby) | 2-(Ethylsulfinyl)ethyl |
| (threo-2-Iby) | 2-(Ethylsulfinyl)ethyl |
| (threo-Ibz) | Tetrahydrofuran-2-yl-methyl |
| (threo-1-Ibz) | Tetrahydrofuran-2-yl-methyl |
| (threo-2-Ibz) | Tetrahydrofuran-2-yl-methyl |

### Tabelle 2a (threo-Racemate), Beispiele:

Beispiele für Verbindungen der Formeln (threo-Iba) bis (threo-Ibz) (siehe Tabelle U2) sind die Verbindungen der betreffenden Formeln in Form des racemischen Gemischs der threo-Isomeren, worin die Strukturkombination der Gruppen (R¹)ₘ, (R²)ₙ und R³ gemäß einer Zeilennummer aus der Tabelle 1 definiert ist.

Die Nummerierung erfolgt gemäß "(Formel)(Zeilennummer)" ohne Schreiben der Klammern, z. B. threo-Iba200 = Verbindung der Formel (threo-Iba) mit der Strukturkombination von Zeile 200 aus Tabelle 1.

### Tabelle 2b (optisch aktive threo-2-Enantiomere): Beispiele:

Beispiele für Verbindungen der Formeln (threo-2-Iba) bis (threo-2-Ibz) (siehe Tabelle U2) sind die optisch aktiven threo-2-Verbindungen der betreffenden Formeln in angereicherter Form [= (3R,4R)-Form mit mehr als 90%ee], worin die Strukturkombination der Gruppen (R¹)ₘ, (R²)ₙ und R³ gemäß einer Zeilennummer aus der Tabelle 1 definiert ist.

Die Nummerierung erfolgt gemäß "(Formel)(Zeilennummer)" ohne Klammern. Beispielsweise bezeichnet die Nr. threo-2-Iba1789 die Verbindung der Formel (threo-2-Iba), worin (R¹)ₘ = 6-Br, (R²)ₙ = 3,5-Cl₂ und R³ = Me bedeuten.

### Tabelle 2c (optisch aktive threo-1-Enantiomere): Beispiele:

Beispiele für Verbindungen der Formeln (threo-1-Iba) bis (threo-1-Ibz) (siehe Tabelle U2) sind die optisch aktiven threo-1-Verbindungen der betreffenden Formeln in angereicherter Form [= (3S,4S)-Form mit mehr als 90%ee], worin die Strukturkombination der Gruppen (R¹)ₘ, (R²)ₙ und R³ gemäß einer Zeilennummer aus der Tabelle 1 definiert ist.

Die Nummerierung erfolgt gemäß "(Formel)(Zeilennummer)" ohne Klammern. Beispielsweise bezeichnet die Nr. threo-1-Ibb5 die Verbindung der Formel (threo-1-Ibb), worin, worin (R¹)ₘ = H, (R²)ₙ = 4-Me und R³ = F bedeuten.

### Tabellen 2d, 2e und 2f:

Erythro-, erythro-1- und erythro-2-Verbindungen zu den Verbindungen der Formel (Ib), (Iba), (Ibb), (Ibc), (Ibd), (Ibe), (Ibf), (Ibg) (Ibh), (Ibi), (Ibj), (Ibk), (Ibl), (Ibm), (Ibn), (Ibo), (Ibp), (Ibq), (Ibr) (Ibs), (Ibt), (Ibu), (Ibv), (Ibw), (Ibx), (Iby) und (Ibz), worin jeweils (R¹)ₘ, (R²)ₙ und R³ wie in Tabelle 1 definiert sind

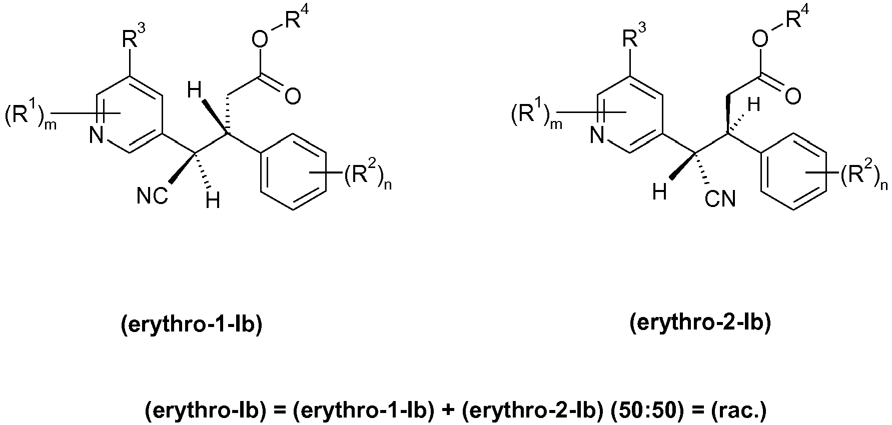

Definition von Unterformeln zu Formeln (erythro-Ib), (erythro-1-Ib) und (erythro-2-Ib), siehe nachfolgende Tabelle U3:

**Tabelle U3**

| Formel | Rest R⁴ in Formel (erythro-Ib) |
|---|---|
| (erythro-Iba) | H (Wasserstoff-atom) |
| (erythro-1-Iba) | H (Wasserstoff-atom) |
| (erythro-2-Iba) | H (Wasserstoff-atom) |
| (erythro-Ibb) | Methyl |
| (erythro-1-Ibb) | Methyl |
| (erythro-2-Ibb) | Methyl |
| (erythro-Ibc) | Ethyl |
| (erythro-1-Ibc) | Ethyl |
| (erythro-2-Ibc) | Ethyl |
| (erythro-Ibd) | n-Propyl |
| (erythro-1-Ibd) | n-Propyl |
| (erythro-2-Ibd) | n-Propyl |
| (erythro-Ibe) | i-Propyl (= Isopropyl) |
| (erythro-1-Ibe) | i-Propyl |
| (erythro-2-Ibe) | i-Propyl |
| (erythro-Ibf) | 2,2-Difluorethyl |
| (erythro-1-Ibf) | 2,2-Difluorethyl |
| (erythro-2-Ibf) | 2,2-Difluorethyl |
| (erythro-Ibg) | 2,2,2-Trifluorethyl |
| (erythro-1-Ibg) | 2,2,2-Trifluorethyl |
| (erythro-2-Ibg) | 2,2,2-Trifluorethyl |
| (erythro-Ibh) | 2-Methoxyethyl |
| (erythro-1-Ibh) | 2-Methoxyethyl |
| (erythro-2-Ibh) | 2-Methoxyethyl |
| (erythro-Ibi) | Cyclopropyl methyl |
| (erythro-1-Ibi) | Cyclopropyl methyl |
| (erythro-2-Ibi) | Cyclopropyl methyl |
| (erythro-Ibj) | (1-Methylcyclopropyl)methyl |
| (erythro-1-Ibj) | (1-Methylcyclopropyl)methyl |
| (erythro-2-Ibj) | (1-Methylcyclopropyl)methyl |
| (erythro-Ibk) | Allyl |
| (erythro-1-Ibk) | Allyl |
| (erythro-2-Ibk) | Allyl |
| (erythro-Ibl) | Prop-2-in-1-yl |
| (erythro-1-Ibl) | Prop-2-in-1-yl |
| (erythro-2-Ibl) | Prop-2-in-1-yl |
| (erythro-Ibm) | Ethinyl |
| (erythro-1-Ibm) | Ethinyl |
| (erythro-2-Ibm) | Ethinyl |
| (erythro-Ibn) | Prop-1-in-1-yl |
| (erythro-1-Ibn) | Prop-1-in-1-yl |
| (erythro-2-Ibn) | Prop-1-in-1-yl |
| (erythro-Ibo) | Benzyl |
| (erythro-1-Ibo) | Benzyl |
| (erythro-2-Ibo) | Benzyl |
| (erythro-Ibp) | 4-Chlorbenzyl |
| (erythro-1-Ibp) | 4-Chlorbenzyl |
| (erythro-2-Ibp) | 4-Chlorbenzyl |
| (erythro-Ibq) | Phenyl |
| (erythro-1-Ibq) | Phenyl |
| (erythro-2-Ibq) | Phenyl |
| (erythro-Ibr) | Methoxymethyl |
| (erythro-1-Ibr) | Methoxymethyl |
| (erythro-2-Ibr) | Methoxymethyl |
| (erythro-Ibs) | Difluormethyl |
| (erythro-1-Ibs) | Difluormethyl |
| (erythro-2-Ibs) | Difluormethyl |
| (erythro-Ibt) | Oxetan-3-yl |
| (erythro-1-Ibt) | Oxetan-3-yl |
| (erythro-2-Ibt) | Oxetan-3-yl |
| (erythro-Ibu) | Thietan-3-yl |
| (erythro-1-Ibu) | Thietan-3-yl |
| (erythro-2-Ibu) | Thietan-3-yl |
| (erythro-Ibv) | 2-(Phenylsulfanyl)ethyl |
| (erythro-1-Ibv) | 2-(Phenylsulfanyl)ethyl |
| (erythro-2-Ibv) | 2-(Phenylsulfanyl)ethyl |
| (erythro-Ibw) | 2-(Phenylsulfinyl)ethyl |
| (erythro-1-Ibw) | 2-(Phenylsulfinyl)ethyl |
| (erythro-2-Ibw) | 2-(Phenylsulfinyl)ethyl |
| (erythro-Ibx) | 2-(Ethylsulfanyl)ethyl |
| (erythro-1-Ibx) | 2-(Ethylsulfanyl)ethyl |
| (erythro-2-Ibx) | 2-(Ethylsulfanyl)ethyl |
| (erythro-Iby) | 2-(Ethylsulfinyl)ethyl |
| (erythro-1-Iby) | 2-(Ethylsulfinyl)ethyl |
| (erythro-2-Iby) | 2-(Ethylsulfinyl)ethyl |
| (erythro-Ibz) | Tetrahydrofuran-2-yl-methyl |
| (erythro-1-Ibz) | Tetrahydrofuran-2-yl-methyl |
| (erythro-2-Ibz) | Tetrahydrofuran-2-yl-methyl |

### Tabelle 2d (erythro-Racemate), Beispiele:

Beispiele für Verbindungen der Formeln (erythro-Iba) bis (erythro-Ibz) (siehe Tabelle U3) sind die Verbindungen der betreffenden Formeln in Form des racemischen Gemischs der erythro-Isomeren, worin die Strukturkombination der Gruppen (R¹)ₘ, (R²)ₙ und R³ gemäß einer Zeilennummer aus der Tabelle 1 definiert ist.

Die Nummerierung erfolgt gemäß "(Formel)(Zeilennummer)" ohne Schreiben der Klammern, z. B. erythro-Iba200 = Verbindung der Formel (erythro-Iba) mit der Strukturkombination von Zeile 200 aus Tabelle 1.

### Tabelle 2e (optisch aktive erythro-2-Enantiomere): Beispiele:

Beispiele für Verbindungen der Formeln (erythro-2-Iba) bis (erythro-2-Ibz) (siehe Tabelle U3) sind die optisch aktiven erythro-2-Verbindungen der betreffenden Formeln in angereicherter Form [= (3R,4S)-Form mit mehr als 90%ee], worin die Strukturkombination der Gruppen (R¹)ₘ, (R²)ₙ und R³ gemäß einer Zeilennummer aus der Tabelle 1 definiert ist.

Die Nummerierung erfolgt gemäß "(Formel)(Zeilennummer)" ohne Klammern. Beispielsweise bezeichnet die Nr. erythro-2-Iba1789 die Verbindung der Formel (erythro-2-Iba), worin (R¹)ₘ = 6-Br, (R²)ₙ = 3,5-Cl₂ und R³ = Me bedeuten.

### Tabelle 2f (optisch aktive erythro-1-Enantiomere): Beispiele:

Beispiele für Verbindungen der Formeln (erythro-1-Iba) bis (erythro-1-Ibz) (siehe Tabelle U3) sind die optisch aktiven erythro-1-Verbindungen der betreffenden Formeln in angereicherter Form [= (3S,4R)-Form mit mehr als 90%ee], worin die Strukturkombination der Gruppen (R¹)ₘ, (R²)ₙ und R³ gemäß einer Zeilennummer aus der Tabelle 1 definiert ist.

Die Nummerierung erfolgt gemäß "(Formel)(Zeilennummer)" ohne Klammern. Beispielsweise bezeichnet die Nr. erythro-1-Ibb5 die Verbindung der Formel (erythro-1-Ibb), worin, worin (R¹)ₘ = H, (R²)ₙ = 4-Me und R³ = F bedeuten.

### Physikalische Daten zu Tabellen 2a - 2f:

### Testmethoden:

1) NMR = ¹H-NMR-Daten (400 MHz, CDCl₃), charakteristische chemische Verschiebungen [in ppm] sind zum jeweiligen Beispiel angegeben,
2) MS = Massenspektrum, gemessen mit Quadrupolgerät; Elektrospray-Ionisierung (+-), Massenbereich 100-1000; Molpeak M bzw [M+H]+ oder [M-1]+ oder [M-2]+ oder [M+1]+ beim jeweiligen Beispiel angegeben,
3) HPLC = Hochdruckflüssigchromatographie (High Performance Liquid Chromatography), Säule: Zorbax Eclipse, 50x3,0, C18 1,8 ym, Laufmittel: Wasser + 0,06% Ameisensäure / Acrylnitril + 0,06% Ameisensäure, Gradient: 90:10, nach 2min 5:95; Detektor: DAD (210 - 400 nm); Retentionszeit (Rtz.) beim jeweiligen Beispiel angegeben,
4) HPLC-Chiral = HPLC an chiraler Säule, Säule: Chiralpak IC, 250 x 4,6mm, 5µm DAIC 83325, Detektorwellenlänge: 210 nm; Säulentemperatur 25°C, Eluent a: (n-Heptan : 2-Propanol) , (60 : 40), Chromasolv, Fluss: 1,0 mL/min Eluent b: (n-Heptan : 2-Propanol) , (70 : 30), Chromasolv, Fluss: 1,0 mL/min Eluent c: (n-Heptan : 2-Propanol) , (80 : 20), Chromasolv, Fluss: 1,0 mL/min Eluent d: (n-Heptan : 2-Propanol) , (90 : 10), Chromasolv, Fluss: 0,6 mL/min
   Bsp. erythro-Ibb2, NMR: 2,85 (dd, 1 H), 3,06 (dd, 1 H), 3,53 (m, 1 H), 3,71 (s, 3H), 4,56 (d, 1 H), 6,95 (d, 2H), 7,08 (dt, 1 H), 7,25 (d, 2H), 8,24 (bs, 1 H), 8,43 (d, 1 H)
   Bsp. erythro-1-Ibb2, NMR: 2,85 (dd, 1 H), 3,06 (dd, 1 H), 3,53 (m, 1 H), 3,71 (s, 3H), 4,56 (d, 1 H), 6,95 (d, 2H), 7,08 (dt, 1 H), 7,25 (d, 2H), 8,24 (bs, 1 H), 8,43 (d, 1H)); HPLC-Chiral: 29,9 min, Eluent d
   Bsp. erythro-2-Ibb2, NMR: 2,85 (dd, 1 H), 3,06 (dd, 1 H), 3,53 (m, 1 H), 3,71 (s, 3H), 4,56 (d, 1 H), 6,95 (d, 2H), 7,08 (dt, 1 H), 7,25 (d, 2H), 8,24 (bs, 1 H), 8,43 (d, 1H)); HPLC-Chiral: 32,9 min, Eluent d
   Bsp. threo-Ibb2, NMR: 2,92 (m, 2H), 3,60 (s, 3H), 3,66 (q, 1 H), 4,18 (d, 1 H), 7,04 (d, 2H), 7,25 (dt, 1 H), 7,28 (d, 2H), 8,15 (bs, 1 H), 8,44 (d, 1 H)
   Bsp. threo-1-Ibb2, NMR: 2,92 (m, 2H), 3,60 (s, 3H), 3,66 (q, 1 H), 4,18 (d, 1 H), 7,04 (d, 2H), 7,25 (dt, 1 H), 7,28 (d, 2H), 8,15 (bs, 1 H), 8,44 (d, 1H)); HPLC-Chiral: 11,7 min, Eluent c
   Bsp. threo-2-Ibb2, NMR: 2,92 (m, 2H), 3,60 (s, 3H), 3,66 (q, 1 H), 4,18 (d, 1 H), 7,04 (d, 2H), 7,25 (dt, 1 H), 7,28 (d, 2H), 8,15 (bs, 1 H), 8,44 (d, 1H)); HPLC-Chiral: 23,2 min, Eluent c
   Bsp. erythro-Ibb4, NMR: 2,89 (dd, 1 H), 3,10 (dd, 1 H), 3,53 (m, 1 H), 3,71 (s, 3H), 4,57 (d, 1 H), 7,01 (m, 3H), 8,22 (m, 1 H), 8,40 (d, 1 H)
   Bsp. threo-Ibb4, NMR: 2,94 (d, 2H), 3,59 (s, 3H), 3,66 (q, 1 H), 4,20 (d, 1 H), 7,07 (m, 2H), 7,20 (m, 1 H), 8,14 (m, 1 H), 8,40 (d, 1 H)
   Bsp. erythro-Ibb7, NMR: 2,90 (dd, 1 H), 3,11 (dd, 1 H), 3,69 (m, 1 H), 3,71 (s, 3H), 4,62 (d, 1 H), 7,12 (dt, 1 H), 7,23 (d, 2H), 8,15 (d, 2H), 8,27 (bs, 1 H), 8,46 (d, 1 H)
   Bsp. threo-Ibb7, NMR: 2,98 (d, 2H), 3,60 (s, 3H), 3,80 (q, 1 H), 4,25 (d, 1 H), 7,32 (m, 1 H), 7,33 (d, 2H), 8,19 (d, 2H), 8,19 (m, 1 H), 8,44 (bs, 1 H)
   Bsp. erythro-Ibb9, NMR: 2,87 (dd, 1 H), 3,06 (dd, 1 H), 3,54 (m, 1 H), 3,71 (s, 3H), 4,56 (d, 1 H), 6,78 (m, 2H), 7,00 (m, 1 H), 7,08 (t, 1 H), 8,25 (m, 1 H), 8,43 (d, 1 H)
   Bsp. threo-Ibb9, NMR: 2,93 (m, 2H), 3,60 (s, 3H), 3,66 (q, 1 H), 4,19 (d, 1 H), 6,83 (m, 1 H), 6,88 (d, 1 H), 6,98 (m,1 H), 8,17 (t, 1 H), 8,43 (d, 1 H)
   Bsp. threo-Ibb10, NMR: 2,93 (d, 2H), 3,61 (s, 3H), 3,64 (q, 1 H), 4,19 (d, 1 H), 6,99 (m, 1 H), 7,09 (m, 1 H), 8,17 (bs, 1 H), 8,44 (d, 1 H)
   Bsp. erythro-Ibb25, NMR: 2,81 (dd, 1 H), 3,00 (dd, 1 H), 3,70 (s, 3H), 3,96 (q, 1 H), 4,59 (d, 1 H), 6,91 (m, 1 H), 6,99 (m, 1 H), 7,11 (m, 1 H), 7,21 (dt, 1 H), 8,28 (bs, 1 H), 8,45 (d, 1 H)
   Bsp. threo-Ibb25, NMR: 3,00 (m, 2H), 3,60 (s, 3H), 3,91 (q, 1 H), 4,26 (d, 1 H), 6,85 (m, 1 H), 7,00 (m,2H), 7,40 (dt, 1 H), 8,25 (bs, 1 H), 8,44 (d, 1 H)
   Bsp. erythro-Ibb26, NMR: 2,88 (d, 2H), 3,59 (s, 3H), 4,11 (q, 1 H), 4,46 (d, 1 H), 6,92 (t, 2H), 7,44 (dt, 1 H), 8,39 (bs, 1 H), 8,49 (d, 1 H)
   Bsp. erythro-1-Ibb26, NMR: 2,88 (d, 2H), 3,59 (s, 3H), 4,11 (q, 1 H), 4,46 (d, 1 H), 6,92 (t, 2H), 7,44 (dt, 1 H), 8,39 (bs, 1 H), 8,49 (d, 1 H); HPLC-Chiral: 12,5 min, Eluent c
   Bsp. erythro-2-Ibb26, NMR: 2,88 (d, 2H), 3,59 (s, 3H), 4,11 (q, 1 H), 4,46 (d, 1 H), 6,92 (t, 2H), 7,44 (dt, 1 H), 8,39 (bs, 1 H), 8,49 (d, 1 H); HPLC-Chiral: 13,5 min, Eluent c
   Bsp. threo-Ibb26, NMR: 3,15 (dd, 1 H), 3,23 (dd, 1 H), 3,63 (s, 3H), 4,17 (m, 1 H), 4,26 (d, 1 H), 6,78 (t, 2H), 7,39 (dt, 1 H), 8,10 (bs, 1 H), 8,36 (bs, 1 H)
   Bsp. threo-1-Ibb26, NMR: 3,15 (dd, 1 H), 3,23 (dd, 1 H), 3,63 (s, 3H), 4,17 (m, 1 H), 4,26 (d, 1 H), 6,78 (t, 2H), 7,39 (dt, 1 H), 8,10 (bs, 1 H), 8,36 (bs, 1 H); HPLC-Chiral: 11,7 min, Eluent c
   Bsp. threo-2-Ibb26, NMR: 3,15 (dd, 1 H), 3,23 (dd, 1 H), 3,63 (s, 3H), 4,17 (m, 1 H), 4,26 (d, 1H), 6,78 (t, 2H), 7,39 (dt, 1H), 8,10 (bs, 1H), 8,36 (bs, 1H); HPLC-Chiral: 16,5 min, Eluent c
   Bsp. erythro-Ibb541, NMR: 2,86 (dd, 1 H), 3,06 (dd, 1 H), 3,52 (m, 1 H), 3,71 (s, 3H), 4,50 (d, 1 H), 6,98 (d, 2H), 7,32 (t, 1 H), 8,24 (d, 1 H), 8,52 (d, 1 H)
   Bsp. threo-Ibb541, NMR: 2,90 (m, 2H), 3,60 (s, 3H), 3,66 (q, 1 H), 4,14 (d, 1 H), 7,00 (m, 2H), 7,06 (m, 2H), 7,48 (t, 1 H), 8,18 (d, 1 H), 8,52 (d, 1 H)
   Bsp. erythro-Ibb542, NMR: 2,85 (dd, 1 H), 3,05 (dd, 1 H), 3,51 (m, 1 H), 3,70 (s, 3H), 4,50 (d, 1 H), 6,95 (d, 2H), 7,27 (d, 2H), 7,35 (t, 1 H), 8,25 (d, 1 H), 8,52 (d, 1 H)
   Bsp. threo-Ibb542, NMR: 2,89 (d, 2H), 3,60 (s, 3H), 3,64 (q, 1 H), 4,14 (d, 1 H), 7,04 (d, 2H), 7,29 (d, 2H), 7,50 (t, 1 H), 8,19 (d, 1 H), 8,53 (d, 1 H)
   Bsp. threo-1-Ibb542, NMR: 2,89 (d, 2H), 3,60 (s, 3H), 3,64 (q, 1 H), 4,14 (d, 1 H), 7,04 (d, 2H), 7,29 (d, 2H), 7,50 (t, 1H), 8,19 (d, 1H), 8,53 (d, 1H); HPLC-Chiral: 11,0 min, Eluent c
   Bsp. threo-2-Ibb542, NMR: 2,89 (d, 2H), 3,60 (s, 3H), 3,64 (q, 1 H), 4,14 (d, 1 H), 7,04 (d, 2H), 7,29 (d, 2H), 7,50 (t, 1H), 8,19 (d, 1H), 8,53 (d, 1H); HPLC-Chiral: 18,5 min, Eluent c
   Bsp. erythro-Ibb543, NMR: 2,85 (dd, 1 H), 3,05 (dd, 1 H), 3,50 (m, 1 H), 3,70 (s, 3H), 4,50 (d, 1 H), 6,89 (d, 2H), 7,36 (s, 1 H), 7,42 (d, 2H), 8,25 (s, 1 H), 8,53 (s, 1 H)
   Bsp. threo-Ibb543, NMR: 2,90 (d, 2H), 3,60 (s, 3H), 3,63 (q, 1 H), 4,14 (d, 1 H), 6,98 (d, 2H), 7,44 (d, 2H), 7,51 (t, 1 H), 8,19 (d, 1 H), 8,53 (d, 1 H)
   Bsp. erythro-Ibb544, NMR: 2,89 (dd, 1 H), 3,10 (dd, 1 H), 3,52 (m, 1 H), 3,70 (s, 3H), 4,52 (d, 1 H), 7,00 (m, 2H), 7,29 (m, 4H), 8,23 (d, 1 H), 8,50 (d, 1 H)
   Bsp. threo-Ibb544, NMR: 2,94 (d, 2H), 3,59 (s, 3H), 3,65 (q, 1 H), 4,16 (d, 1 H), 7,07 (m, 2H), 7,29 (m, 3H), 7,45 (t, 1 H), 8,16 (d, 1 H), 8,60 (d, 1 H)
   Bsp. erythro-Ibb546, NMR: 2,87 (dd, 1 H), 3,08 (dd, 1 H), 3,60 (m, 1 H), 3,71 (s, 3H), 4,55 (d, 1 H), 7,15 (d, 2H), 7,35 (t, 1 H), 7,60 (d, 2H), 8,27 (d, 1 H), 8,55 (d, 1 H)
   Bsp. threo-Ibb546, NMR: 2,94 (d, 2H), 3,60 (s, 3H), 3,73 (q, 1 H), 4,18 (d, 1 H), 7,26 (d, 2H), 7,51 (t, 1 H), 7,63 (d, 2H), 8,21 (bs, 1 H), 8,55 (bs, 1 H)
   Bsp. erythro-Ibb547, NMR: 2,90 (dd, 1 H), 3,10 (dd, 1 H), 3,68 (m, 1 H), 3,71 (s, 3H), 4,57 (d, 1 H), 7,22 (d, 2H), 7,40 (t, 1 H), 8,17 (d, 2H), 8,28 (d, 1 H), 8,55 (d, 1 H)
   Bsp. threo-Ibb547, NMR: 2,97 (d, 2H), 3,60 (s, 3H), 3,80 (q, 1 H), 4,21 (d, 1 H), 7,34 (d, 2H), 7,55 (t, 1 H), 8,20 (d, 2H), 8,23 (bs, 1 H), 8,56 (bs, 1 H)
   Bsp. erythro-Ibb548, NMR: 2,85 (dd, 1 H), 3,06 (dd, 1 H), 3,47 (m, 1 H), 3,70 (s, 3H), 3,78 (s, 3H), 4,47 (d, 1 H), 6,80 (d, 2H), 6,91 (d, 2H), 7,31 (m, 1 H), 8,22 (d, 1 H), 8,50 (d, 1 H)
   Bsp. threo-Ibb548, NMR: 2,89 (m, 2H), 3,59 (s, 3H), 3,60 (m, 1 H), 3,78 (s, 3H), 4,12 (d, 1 H), 6,81 (d, 2H), 6,98 (d, 2H), 7,46 (t, 1 H), 8,15 (d, 1 H), 8,50 (d, 1 H)
   Bsp. erythro-Ibb553, NMR: 2,87 (dd, 1 H), 3,07 (dd, 1 H), 3,58 (m, 1 H), 3,71 (s, 3H), 4,53 (d, 1 H), 7,31 (m, 2H), 7,36 (t, 1 H), 7,44 (t, 1 H), 7,62 (m, 1 H), 8,27 (d, 1 H), 8,55 (d, 1 H)
   Bsp. threo-Ibb553, NMR: 2,93 (d, 2H), 3,61 (s, 3H), 3,71 (q, 1 H), 4,17 (d, 1 H), 7,41 (m, 2H), 7,46 (t, 1 H), 7,51 (t, 1 H), 7,62 (m, 1 H), 8,21 (d, 1 H), 8,55 (d, 1 H)
   Bsp. erythro-Ibb565, NMR: 2,81 (dd, 1 H), 2,99 (dd, 1 H), 3,69 (s, 3H), 3,94 (q, 1 H), 4,54 (d, 1 H), 6,93 (m, 1 H), 6,99 (m, 1 H), 7,10 (m, 1 H), 7,45 (t, 1 H), 8,31 (bs, 1 H), 8,54 (d, 1 H)
   Bsp. threo-Ibb565, NMR: 2,99 (m, 2H), 3,60 (s, 3H), 3,91 (q, 1 H), 4,22 (d, 1 H), 6,86 (m, 1 H), 6,98 (m,1 H), 7,01 (m, 1 H), 7,65 (t, 1 H), 8,30 (d, 1 H), 8,53 (d, 1 H)
   Bsp. erythro-Ibb566, NMR: 2,88 (d, 2H), 3,59 (s, 3H), 4,10 (q, 1 H), 4,41 (d, 1 H), 6,92 (t, 2H), 7,32 (m, 1 H), 7,69 (t, 1 H), 8,44 (bs, 1 H), 8,59 (bs, 1 H)
   Bsp. threo-Ibb566, NMR: 3,12 (dd, 1 H), 3,23 (dd, 1 H), 3,63 (s, 3H), 4,18 (m, 1 H), 4,22 (d, 1 H), 6,79 (t, 2H), 7,18 (m, 1 H), 7,64 (t, 1 H), 8,15 (d, 1 H), 8,45 (d, 1 H)
   Bsp. threo-1-Ibb566, NMR: 3,12 (dd, 1 H), 3,23 (dd, 1 H), 3,63 (s, 3H), 4,18 (m, 1 H), 4,22 (d, 1H), 6,79 (t, 2H), 7,18 (m, 1 H), 7,64 (t, 1 H), 8,15 (d, 1 H), 8,45 (d, 1H)); HPLC-Chiral: 11,5 min, Eluent c
   Bsp. threo-2-Ibb566, NMR: 3,12 (dd, 1 H), 3,23 (dd, 1 H), 3,63 (s, 3H), 4,18 (m, 1 H), 4,22 (d, 1H), 6,79 (t, 2H), 7,18 (m, 1 H), 7,64 (t, 1 H), 8,15 (d, 1 H), 8,45 (d, 1H)); HPLC-Chiral: 15,4 min, Eluent c
   Bsp. Ibb587 (erythro-Ibb587 : threo-Ibb587 = 50:50), NMR:
      erythro-Ibb587: 2,85 (dd, 1 H), 3,06 (dd, 1 H), 3,48 (m, 1 H), 3,71 (s, 3H), 4,53 (d, 1 H), 6,96 (d, 2H), 7,28 (d, 2H), 7,46 (dd, 1 H), 7,83 (m, 1 H)
      threo-Ibb587: 2,89 (m, 2H), 3,61 (s, 3H), 3,63 (q, 1 H), 4,16 (d, 1 H), 7,03 (d, 2H), 7,30 (d, 2H), 7,61 (dd, 1 H), 7,77 (m, 1 H)
   Bsp. erythro-Ibb587: 2,85 (dd, 1 H), 3,06 (dd, 1 H), 3,48 (m, 1 H), 3,71 (s, 3H), 4,53 (d, 1 H), 6,96 (d, 2H), 7,28 (d, 2H), 7,46 (dd, 1 H), 7,83 (m, 1 H)
   Bsp. threo-Ibb587: 2,89 (m, 2H), 3,61 (s, 3H), 3,63 (q, 1 H), 4,16 (d, 1 H), 7,03 (d, 2H), 7,30 (d, 2H), 7,61 (dd, 1 H), 7,77 (m, 1 H)
   Bsp. Ibb589 (erythro-Ibb589 : threo-Ibb589 = 50:50), NMR:
      erythro-Ibb589: 2,90 (dd, 1 H), 3,11 (dd, 1 H), 3,48 (m, 1 H), 3,71 (s, 3H), 4,55 (d, 1 H), 7,00 (m, 2H), 7,30 (m, 3H), 7,37 (dd, 1 H), 7,81 (m, 1 H)
      threo-Ibb589: 2,94 (m, 2H), 3,61 (s, 3H), 3,63 (q, 1 H), 4,17 (d, 1 H), 7,05 (m, 2H), 7,54 (dd, 1 H), 7,74 (m, 1 H)
   Bsp. erythro-Ibb589: 2,90 (dd, 1 H), 3,11 (dd, 1 H), 3,48 (m, 1 H), 3,71 (s, 3H), 4,55 (d, 1 H), 7,00 (m, 2H), 7,30 (m, 3H), 7,37 (dd, 1 H), 7,81 (m, 1 H)
   Bsp. erythro-Ibb592: 2,90 (dd, 1 H), 3,10 (dd, 1 H), 3,65 (m, 1 H), 3,72 (s, 3H), 4,59 (d, 1 H), 7,25 (d, 2H), 7,53 (dd, 1 H), 7,87 (m, 1 H), 8,18 (d, 2H)
   Bsp. threo-Ibb592: 2,97 (d, 2H), 3,61 (s, 3H), 3,78 (q, 1 H), 4,23 (d, 1 H), 7,33 (d, 2H), 7,68 (dd, 1 H), 7,82 (m, 1 H), 8,20 (d, 2H)
   Bsp. Ibb594 (erythro-Ibb594 : threo-Ibb594 = 54:46), NMR:
      erythro-Ibb594: 2,87 (dd, 1 H), 3,06 (dd, 1 H), 3,50 (m, 1 H), 3,72 (s, 3H), 4,54 (d, 1 H), 6,79 (m, 2H), 7,00 (m, 1 H), 7,28 (m, 1 H), 7,45 (dd, 1 H), 7,85 (m, 1 H)
      threo-Ibb594: 2,92 (m, 2H), 3,62 (s, 3H), 3,63 (q, 1 H), 4,17 (d, 1 H), 6,83 (m, 2H), 7,01 (m, 1 H), 7,28 (m, 1 H), 7,61 (dd, 1 H), 7,78 (m, 1 H)
   Bsp. erythro-Ibb594: 2,87 (dd, 1 H), 3,06 (dd, 1 H), 3,50 (m, 1 H), 3,72 (s, 3H), 4,54 (d, 1 H), 6,79 (m, 2H), 7,00 (m, 1 H), 7,28 (m, 1 H), 7,45 (dd, 1 H), 7,85 (m, 1 H)
   Bsp. Ibb595 (erythro-Ibb595 : threo-Ibb595 = 48:52), NMR:
      erythro-Ibb595: 2,85 (dd, 1 H), 3,05 (dd, 1 H), 3,47 (m, 1 H), 3,71 (s, 3H), 4,53 (d, 1 H), 6,91 (dt, 1 H), 7,04 (t, 1 H), 7,45 (dd, 1 H), 7,86 (m, 1 H)
      threo-Ibb595: 2,91 (m, 2H), 3,61 (q, 1 H), 3,62 (s, 3H), 4,18 (d, 1 H), 6,98 (dt, 1 H), 7,10 (t, 1 H), 7,62 (dd, 1 H), 7,80 (m, 1 H)
   Bsp. erythro-Ibb595: 2,85 (dd, 1 H), 3,05 (dd, 1 H), 3,47 (m, 1 H), 3,71 (s, 3H), 4,53 (d, 1 H), 6,91 (dt, 1 H), 7,04 (t, 1 H), 7,24 (t, 1 H), 7,30 (m, 1 H), 7,45 (dd, 1 H), 7,86 (m, 1 H)
   Bsp. Ibb610 (erythro-Ibb610 : threo-Ibb610 = 55:45), NMR:
      erythro-Ibb610: 2,82 (dd, 1 H), 3,01 (dd, 1 H), 3,71 (s, 3H), 3,93 (m, 1 H), 4,58 (d, 1 H), 6,94 (m, 1 H), 6,99 (m, 1 H), 7,12 (m, 1 H), 7,59 (dd, 1 H), 7,90 (m, 1 H)
      threo-Ibb610: 2,99 (m, 2H), 3,61 (s, 3H), 3,90 (q, 1 H), 4,22 (d, 1 H), 6,86 (m, 1 H), 7,00 (m, 2H), 7,79 (m, 1 H), 7,88 (m, 2H)
   Bsp. erythro-Ibb610: 2,82 (dd, 1 H), 3,01 (dd, 1 H), 3,71 (s, 3H), 3,93 (m, 1 H), 4,58 (d, 1 H), 6,94 (m, 1 H), 6,99 (m, 1 H), 7,12 (m, 1 H), 7,59 (dd, 1 H), 7,90 (m, 1 H)
   Bsp. Ibb611 (erythro-Ibb611 : threo-Ibb611 = 50:50), NMR:
      erythro-Ibb611: 2,88 (dd, 1 H), 2,93 (dd, 1 H), 3,62 (s, 3H), 4,07 (q, 1 H), 4,45 (d, 1 H), 6,92 (t, 2H), 7,31 (m, 1 H), 7,81 (dd, 1 H), 8,02 (m, 1 H)
      threo-Ibb611: 3,11 (dd, 1 H), 3,24 (dd, 1 H), 3,63 (s, 3H), 4,16 (m, 1 H), 4,22 (d, 1 H), 6,81 (t, 2H), 7,23 (m, 1 H), 7,74 (m, 1 H), 7,78 (dd, 1 H)
   Bsp. erythro-Ibb624: 2,87 (m, 2H), 3,63 (s, 3H), 4,01 (q, 1 H), 4,40 (d, 1 H), 6,98 (d, 2H), 7,84 (dd, 1 H), 8,03 (m, 1 H)
   Bsp. threo-Ibb624: 3,07 (dd, 1 H), 3,22 (dd, 1 H), 3,64 (s, 3H), 4,10 (m, 1 H), 4,18 (d, 1 H), 6,87 (d, 2H), 7,76 (m, 1 H), 7,80 (dd, 1 H)
   Bsp. erythro-Ibb1081, NMR: 2,86 (dd, 1 H), 3,06 (dd, 1 H), 3,51 (m, 1 H), 3,70 (s, 3H), 4,49 (d, 1 H), 6,98 (d, 4H), 7,46 (t, 1 H), 8,27 (d, 1 H), 8,62 (d, 1 H)
   Bsp. threo-Ibb1081, NMR: 2,90 (m, 2H), 3,60 (s, 3H), 3,65 (q, 1 H), 4,12 (d, 1 H), 7,03 (m, 4H), 7,62 (t, 1 H), 8,22 (d, 1 H), 8,62 (d, 1 H)
   Bsp. Iba1082 (erythro-Iba1082: threo-Iba1082 = 35 : 65):
      erythro-Iba1082, NMR: 2,90 (dd, 1 H), 3,10 (dd, 1 H), 3,49 (m, 1 H), 4,50 (d, 1 H), 6,95 (d, 2H), 7,30 (d, 2H), 7,49 (m, 1 H)
      threo-Iba1082, NMR: 2,93 (m, 2H), 3,64 (q, 1 H), 4,14 (d, 1 H), 7,05 (d, 2H), 7,27 (d, 2H), 7,65(m, 1 H)
   Bsp. Ibb1082 (erythro-Ibb1082 : threo-lbb1082 = 58 : 42):
      erythro-Ibb1082, NMR: 2,85 (dd, 1 H), 3,06 (dd, 1 H), 3,51 (m, 1 H), 3,71 (s, 3H), 4,49 (d, 1 H), 6,95 (d, 2H), 7,27 (d, 2H), 7,49 (m, 1 H)
      threo-Ibb1082, NMR: 2,89 (d, 2H), 3,60 (s, 3H), 3,64 (q, 1 H), 4,12 (d, 1 H), 7,04 (d, 2H), 7,29 (d, 2H), 7,64 (m, 1 H)
   Bsp. erythro-Ibb1082, NMR: 2,85 (dd, 1 H), 3,06 (dd, 1 H), 3,51 (m, 1 H), 3,71 (s, 3H), 4,49 (d, 1 H), 6,95 (d, 2H), 7,27 (d, 2H), 7,49 (m, 1 H), 8,27 (d, 1 H), 8,62 (d, 1 H)
   Bsp. erythro-1-Ibb1082, NMR: 2,85 (dd, 1 H), 3,06 (dd, 1 H), 3,51 (m, 1 H), 3,71 (s, 3H), 4,49 (d, 1 H), 6,95 (d, 2H), 7,27 (d, 2H), 7,49 (m, 1 H), 8,27 (d, 1 H), 8,62 (d, 1 H); HPLC-Chiral: 28,2 min, Eluent d
   Bsp. erythro-2-Ibb1082, NMR: 2,85 (dd, 1 H), 3,06 (dd, 1 H), 3,51 (m, 1 H), 3,71 (s, 3H), 4,49 (d, 1 H), 6,95 (d, 2H), 7,27 (d, 2H), 7,49 (m, 1 H), 8,27 (d, 1 H), 8,62 (d, 1 H); HPLC-Chiral: Rtz = 30,4 min, Eluent d
   Bsp. threo-Ibb1082, NMR: 2,89 (d, 2H), 3,60 (s, 3H), 3,64 (q, 1 H), 4,12 (d, 1 H), 7,04 (d, 2H), 7,29 (d, 2H), 7,64 (m, 1 H), 8,22 (d, 1 H), 8,63 (d, 1 H)
   Bsp. threo-1-Ibb1082, NMR: 2,89 (d, 2H), 3,60 (s, 3H), 3,64 (q, 1 H), 4,12 (d, 1 H), 7,04 (d, 2H), 7,29 (d, 2H), 7,64 (t, 1 H), 8,22 (d, 1 H), 8,63 (d, 1 H); HPLC-Chiral: Rtz = 6,9 min, Eluent a
   Bsp. threo-2-Ibb1082, NMR: 2,89 (d, 2H), 3,60 (s, 3H), 3,64 (q, 1 H), 4,12 (d, 1 H), 7,04 (d, 2H), 7,29 (d, 2H), 7,64 (t, 1 H), 8,22 (d, 1 H), 8,63 (d, 1 H); HPLC-Chiral: Rtz = 9,9 min, Eluent a
   Bsp. Ibc1082 (erythro-Ibc1082 : threo-Ibc1082 = 58 : 42):
      erythro-Ibc1082, NMR: 1,24 (t, 3H), 2,83 (dd, 1 H), 3,03 (dd, 1 H), 3,51 (m, 1 H), 4,14 (q, 2H), 4,48 (d, 1 H), 6,95 (d, 2H), 7,28 (d, 2H), 7,49 (m, 1 H), 8,27 (m, 1 H), 8,63 (m, 1 H) threo-Ibc1082, NMR: 1,16 (t, 3H), 2,89 (d, 2H), 3,63 (q, 1 H), 4,03 (m, 2H), 4,12 (d, 1 H), 7,04 (d, 2H), 7,26 (d, 2H), 7,64 (m, 1 H), 8,22 (m, 1 H), 8,63 (m, 1 H)
      erythro-Ibc1082, NMR: 1,24 (t, 3H), 2,83 (dd, 1 H), 3,03 (dd, 1 H), 3,51 (m, 1 H), 4,14 (q, 2H), 4,48 (d, 1 H), 6,95 (d, 2H), 7,28 (d, 2H), 7,49 (m, 1 H), 8,27 (m, 1 H), 8,63 (m, 1 H) threo-Ibc1082, NMR: 1,16 (t, 3H), 2,89 (d, 2H), 3,63 (q, 1 H), 4,03 (m, 2H), 4,12 (d, 1 H), 7,04 (d, 2H), 7,26 (d, 2H), 7,64 (m, 1 H), 8,22 (m, 1 H), 8,63 (m, 1 H)
   Bsp. Ibv1082 (erythro-Ibv1082 : threo-Ibv1082 = 58 : 42):
      erythro-Ibv1082, NMR: 3,09 (t, 2H), 3,47 (m, 1 H), 4,28 (t, 2H), 4,47 (d, 1 H), 6,93 (d, 2H), 7,49 (t, 1 H), 8,28 (d, 1 H), 8,63 (d, 1 H)
      threo-Ibv1082, NMR: 3,02 (t, 2H), 3,59 (q, 1 H), 4,12 (d, 1 H), 4,16 (m, 2H), 7,01 (d, 2H), 7,67 (t, 1 H), 8,21 (d, 1 H), 8,63 (d, 1 H)
   Bsp. erythro-Ibb1083, NMR in [D₆-DMSO]: 2,63 (dd, 1 H), 2,76 (dd, 1 H), 3,39 (s, 3H), 3,75 (m, 1 H), 4,75 (d, 1 H), 7,28 (d, 2H), 7,57 (d, 2H), 8,11 (t, 1 H), 8,50 (d, 1 H), 8,72 (d, 1 H)
   Bsp. threo-Ibb1083, NMR in [D₆-DMSO]: 2,94 (m, 2H), 3,45 (s, 3H), 3,85 (m, 1 H), 4,74 (d, 1 H), 7,18 (d, 2H), 7,46 (d, 2H), 8,06 (t, 1 H), 8,33 (d, 1 H), 8,61 (d, 1 H)
   Bsp. erythro-Ibb1084, NMR: 2,89 (dd, 1 H), 3,10 (dd, 1 H), 3,51 (m, 1 H), 3,70 (s, 3H), 4,51 (d, 1 H), 7,00 (m, 2H), 7,29 (m, 3H), 7,42 (t, 1 H), 8,25 (d, 1 H), 8,60 (d, 1 H)
   Bsp. threo-Ibb1084, NMR: 2,94 (d, 2H), 3,64 (s, 3H), 3,65 (q, 1 H), 4,14 (d, 1 H), 7,08 (m, 2H), 7,30 (m, 3H), 7,60 (s, 1 H), 8,20 (s, 1 H), 8,60 (s, 1 H)
   Bsp. erythro-Ibb1085, NMR: 2,31 (s, 3H), 2,87 (dd, 1 H), 3,07 (dd, 1 H), 3,48 (m, 1 H), 3,70 (s, 3H), 4,47 (d, 1 H), 6,87 (d, 2H), 7,08 (d, 2H), 7,44 (m, 1 H), 8,23 (d, 1 H), 8,60 (d, 1 H)
   Bsp. threo-Ibb1085, NMR: 2,31 (s, 3H), 2,91 (d, 2H), 3,59 (s, 3H), 3,61 (q, 1 H), 4,12 (d, 1 H), 6,95 (d, 2H), 7,10 (d, 2H), 7,62 (t, 1 H), 8,18 (d, 1 H), 8,60 (d, 1 H)
   Bsp. erythro-Ibb1087, NMR: 2,90 (dd, 1 H), 3,10 (dd, 1 H), 3,67 (m, 1 H), 3,71 (s, 3H), 4,55 (d, 1 H), 7,22 (d, 2H), 7,53 (t, 1 H), 8,17 (d, 2H), 8,31 (d, 1 H), 8,65 (d, 1 H)
   Bsp. threo-Ibb1087, NMR: 2,97 (d, 2H), 3,60 (s, 3H), 3,79 (q, 1 H), 4,19 (d, 1 H), 7,33 (d, 2H), 7,69 (t, 1 H), 8,20 (d, 2H), 8,26 (bs, 1 H), 8,65 (bs, 1 H)
   Bsp. erythro-Ibb1088, NMR: 2,85 (dd, 1 H), 3,06 (dd, 1 H), 3,47 (m, 1 H), 3,70 (s, 3H), 3,78 (s, 3H), 4,46 (d, 1 H), 6,80 (d, 2H), 6,91 (d, 2H), 7,44 (s, 1 H), 8,25 (s, 1 H), 8,60 (s, 1 H)
   Bsp. threo-Ibb1088, NMR: 2,89 (m, 2H), 3,59 (s, 3H), 3,60 (m, 1 H), 3,78 (s, 3H), 4,11 (d, 1 H), 6,81 (d, 2H), 6,99 (d, 2H), 7,61 (t, 1 H), 8,19 (d, 1 H), 8,60 (d, 1 H)
   Bsp. erythro-Ibb1089, NMR: 2,87 (dd, 1 H), 3,05 (dd, 1 H), 3,52 (m, 1 H), 3,71 (s, 3H), 4,50 (d, 1 H), 6,77 (m, 2H), 7,02 (m, 1 H), 7,49 (m, 1 H), 8,29 (d, 1 H), 8,62 (d, 1 H)
   Bsp. threo-Ibb1089, NMR: 2,92 (d, 2H), 3,61 (s, 3H), 3,65 (q, 1 H), 4,13 (d, 1 H), 6,83 (m, 1 H), 6,88 (d, 1 H), 6,99 (m,1 H), 7,65 (m, 1 H), 8,23 (d, 1 H), 8,62 (d, 1 H)
   Bsp. threo-Ibb1090, NMR: 2,91 (d, 2H), 3,61 (s, 3H), 3,63 (q, 1 H), 4,14 (d, 1 H), 6,99 (m, 2H), 7,65 (m, 1 H), 8,25 (d, 1 H), 8,64 (d, 1 H)
   Bsp. erythro-Ibb1092, NMR: 2,28 (s, 3H), 2,87 (dd, 1 H), 3,07 (dd, 1 H), 3,47 (m, 1 H), 3,70 (s, 3H), 4,49 (d, 1 H), 6,77 (s, 1 H), 6,80 (d, 1 H), 7,11 (d, 1 H), 7,17 (t, 1 H), 7,40 (t, 1 H), 8,26 (d, 1 H), 8,60 (d, 1 H)
   Bsp. threo-Ibb1092, NMR: 2,28 (s, 3H), 2,91 (d, 2H), 3,60 (s, 3H), 3,61 (q, 1 H), 4,14 (d, 1 H), 6,87 (m, 2H), 7,09 (d, 1 H), 7,19 (t, 1 H), 7,59 (t, 1 H), 8,21 (d, 1 H), 8,61 (d, 1 H)
   Bsp. erythro-Ibb1093, NMR: 2,87 (dd, 1 H), 3,06 (dd, 1 H), 3,57 (m, 1 H), 3,71 (s, 3H), 4,51 (d, 1 H), 7,32 (m, 2H), 7,44 (t, 1 H), 7,50 (t, 1 H), 7,62 (m, 1 H), 8,30 (d, 1 H), 8,65 (d, 1 H)
   Bsp. threo-Ibb1093, NMR: 2,93 (d, 2H), 3,61 (s, 3H), 3,70 (q, 1 H), 4,16 (d, 1 H), 7,39 (m, 2H), 7,46 (t, 1 H), 7,62 (m, 1 H), 7,63 (m, 1 H), 8,25 (bs, 1 H), 8,66 (bs, 1 H)
   Bsp. erythro-Ibb1094, NMR: 2,90 (dd, 1 H), 3,09 (dd, 1 H), 3,68 (m, 1 H), 3,70 (s, 3H), 4,53 (d, 1 H), 7,42 (m, 1 H), 7,55 (m, 2H), 7,89 (t, 1 H), 8,19 (m, 1 H), 8,32 (d, 1 H), 8,66 (d, 1 H)
   Bsp. threo-Ibb1094, NMR: 2,98 (d, 2H), 3,61 (s, 3H), 3,80 (q, 1 H), 4,21 (d, 1 H), 7,51 (m, 2H), 7,68 (t, 1 H), 8,00 (t, 1 H), 8,17 (m, 1 H), 8,26 (d, 1 H), 8,65 (d, 1 H)
   Bsp. Ibb1096 (erythro-Ibb1096 : threo-Ibb1096 = 55:45), NMR:
      erythro-Ibb1096: 2,83 (dd, 1 H), 3,04 (dd, 1 H), 3,68 (s, 3H), 3,97 (q, 1 H), 4,53 (d, 1 H), 7,54 (t, 1 H), 8,30 (d, 1 H), 8,62 (d, 1 H)
      threo-Ibb1096: 3,01 (m, 2H), 3,58 (s, 3H), 3,93 (m, 1 H), 4,22 (d, 1 H), 7,77 (t, 1 H), 8,30 (d, 1 H), 8,60 (d, 1 H)
   Bsp. erythro-Ibb1103, NMR: 2,84 (dd, 1 H), 3.03 (dd, 1 H), 3,69 (s, 3H), 3,98 (m, 1 H), 4,54 (d, 1 H), 7,14 (m, 3H), 7,59 (t, 1 H), 8,33 (d, 1 H), 8,65 (d, 1 H)
   Bsp. threo-Ibb1103, NMR: 3,00 (m, 2H), 3,59 (s, 3H), 3,97 (m, 1 H), 4,20 (d, 1 H), 6,91 (m, 1 H), 7,13 (m, 2H), 7,80 (t, 1 H), 8,34 (d, 1 H), 8,64 (d, 1 H)
   Bsp. erythro-Ibb1104, NMR: 2,81 (dd, 1 H), 3,01 (dd, 1 H), 3,691 (s, 3H), 3,92 (q, 1 H), 4,51 (d, 1 H), 6,71 (m, 1 H), 6,94 (m, 1 H), 7,36 (m, 1 H), 7,58 (t, 1 H), 8,32 (d, 1 H), 8,64 (d, 1 H)
   Bsp. threo-Ibb1104, NMR: 2,98 (m, 2H), 3,58 (s, 3H), 3,90 (q, 1 H), 4,19 (d, 1 H), 6,82 (m, 2H), 7,10 (m, 1 H), 7,78 (t, 1 H), 8,32 (d, 1 H), 8,62 (d, 1 H)
   Bsp. erythro-Ibb1105, NMR: 2,81 (dd, 1 H), 2,99 (dd, 1 H), 3,71 (s, 3H), 3,95 (q, 1 H), 4,53 (d, 1 H), 6,92 (m, 1 H), 6,99 (m, 1 H), 7,10 (m, 1 H), 7,60 (m, 1 H), 8,36 (d, 1 H), 8,66 (d, 1 H)
   Bsp. erythro-1-Ibb1105, NMR: 2,81 (dd, 1 H), 2,99 (dd, 1 H), 3,69 (s, 3H), 3,94 (q, 1 H), 4,53 (d, 1 H), 6,92 (m, 1 H), 6,99 (m, 1 H), 7,10 (m, 1 H), 7,60 (t, 1 H), 8,35 (d, 1 H), 8,65 (d, 1 H), HPLC-Chiral: Rtz = 26,9 min, Eluent d
   Bsp. erythro-2-Ibb1105, NMR: 2,81 (dd, 1 H), 2,99 (dd, 1 H), 3,69 (s, 3H), 3,94 (q, 1 H), 4,53 (d, 1 H), 6,92 (m, 1 H), 6,99 (m, 1 H), 7,10 (m, 1 H), 7,60 (t, 1 H), 8,35 (d, 1 H), 8,65 (d, 1 H), HPLC-Chiral: Rtz = 29,2 min, Eluent d
   Bsp. threo-Ibb1105, NMR: 2,98 (m, 2H), 3,60 (s, 3H), 3,89 (q, 1 H), 4,20 (d, 1 H), 6,86 (m, 1 H), 7,00 (m, 2H), 7,80 (m, 1 H), 8,35 (d, 1 H), 8,63 (d, 1 H)
   Bsp. threo-1-Ibb1105, NMR: 2,98 (m, 2H), 3,60 (s, 3H), 3,90 (q, 1 H), 4,20 (d, 1 H), 6,86 (m, 1 H), 7,00 (m, 2H), 7,80 (t, 1 H), 8,34 (d, 1 H), 8,63 (d, 1 H), HPLC-Chiral: Rtz = 10,9 min, Eluent c
   Bsp. threo-2-Ibb1105, NMR: 2,98 (m, 2H), 3,60 (s, 3H), 3,90 (q, 1 H), 4,20 (d, 1 H), 6,85 (m, 1 H), 7,00 (m, 2H), 7,80 (t, 1 H), 8,34 (d, 1 H), 8,63 (d, 1 H), HPLC-Chiral: Rtz = 15,1 min, Eluent c
   Bsp. erythro-Ibb1106, NMR: 2,88 (d, 2H), 3,60 (s, 3H), 4,09 (q, 1 H), 4,39 (d, 1 H), 6,92 (t, 2H), 7,82 (m, 1 H), 8,47 (d, 1 H), 8,68 (d, 1 H)
   Bsp. erythro-1-Ibb1106, NMR: 2,88 (d, 2H), 3,60 (s, 3H), 4,09 (q, 1 H), 4,39 (d, 1 H), 6,92 (t, 2H), 7,83 (m, 1 H), 8,47 (d, 1 H), 8,69 (d, 1 H); HPLC-Chiral: Rtz 8,7 min, Eluent b
   Bsp. erythro-2-Ibb1106, NMR: 2,88 (d, 2H), 3,60 (s, 3H), 4,09 (q, 1 H), 4,39 (d, 1 H), 6,92 (t, 2H), 7,83 (m, 1 H), 8,47 (d, 1 H), 8,69 (d, 1 H); HPLC-Chiral: Rtz 9,8 min, Eluent b
   Bsp. threo-Ibb1106, NMR: 3,12 (dd, 1 H), 3,23 (dd, 1 H), 3,63 (s, 3H), 4,17 (m, 2H), 6,79 (t, 2H), 7,78 (m, 1 H), 8,18 (d, 1 H), 8,55 (d, 1 H)
   Bsp. threo-1-Ibb1106, NMR: 3,12 (dd, 1 H), 3,23 (dd, 1 H), 3,63 (s, 3H), 4,17 (m, 2H), 6,79 (t, 2H), 7,18 (m, 1 H), 7,78 (t, 1 H), 8,18 (d, 1 H), 8,55 (d, 1 H); HPLC-Chiral: Rtz 7,3 min, Eluent a
   Bsp. threo-2-Ibb1106, NMR: 3,12 (dd, 1 H), 3,23 (dd, 1 H), 3,63 (s, 3H), 4,17 (m, 2H), 6,79 (t, 2H), 7,18 (m, 1 H), 7,78 (t, 1 H), 8,18 (d, 1 H), 8,55 (d, 1 H); 7,3 min, HPLC-Chiral: Rtz 9,0 min, Eluent a
   Bsp. erythro-Ibb1107, NMR: 2,84 (dd, 1 H), 3,02 (dd, 1 H), 3,50 (m, 1 H), 3,71 (s, 3H), 4,48 (d, 1 H), 6,74 (m, 1 H), 6,91 (m, 1 H), 7,09 (m, 1 H), 7,53 (t, 1 H), 8,30 (d, 1 H), 8,64 (d, 1 H)
   Bsp. threo-Ibb1107, NMR: 2,88 (d, 2H), 3,61 (s, 3H), 3,62 (q, 1 H), 4,12 (d, 1 H), 6,86 (m, 1 H), 6,96 (m, 1 H), 7,11 (m, 1 H), 7,67 (t, 1 H), 8,24 (d, 1 H), 8,64 (d, 1 H)
   Bsp. threo-Ibb1108, NMR: 2,90 (d, 2H), 3,62 (s, 3H), 3,64 (q, 1 H), 4,13 (d, 1 H), 6,67 (m, 2H), 6,75 (m,1 H), 7,70 (m, 1 H), 8,27 (d, 1 H), 8,65 (d, 1 H)
   Bsp. erythro-Ibb1119, NMR: 2,84 (d, 2H), 3,60 (s, 3H), 4,03 (q, 1 H), 4,35 (d, 1 H), 6,98 (m, 2H), 7,85 (t, 1 H), 8,48 (d, 1 H), 8,70 (d, 1 H)
   Bsp. threo-Ibb1119, NMR: 3,11 (dd, 1 H), 3,21 (dd, 1 H), 3,64 (s, 3H), 4,14 (m, 2H), 6,85 (m, 2H), 7,81 (m, 1 H), 8,20 (d, 1 H), 8,59 (d, 1 H)
   Bsp. erythro-Ibb1123, NMR: 2,84 (dd, 1 H), 3,03 (dd, 1 H), 3,57 (q, 1 H), 3,71 (s, 3H), 4,50 (d, 1 H), 7,25 (m, 1 H), 7,32 (m, 1 H), 7,47 (m, 1 H), 7,57 (m, 1 H), 8,32 (m, 1 H), 8,68 (m, 1H)
   Bsp. threo-Ibb1123, NMR: 2,90 (d, 2H), 3,61 (s, 3H), 3,69 (q, 1 H), 4,15 (d, 1 H), 7,34 (d, 1 H), 7,45 (s, 1 H), 7,50 (dd, 1 H), 7,71 (s, 1 H), 8,28 (s, 1 H), 8,69 (s, 1 H)
   Bsp. Ibb1126 (erythro-lbb1126 : threo-Ibb26 = 56:44), NMR:
      erythro-Ibb1126: 2,87 (dd, 1 H), 3,07 (dd, 1 H), 3,47 (m, 1 H), 3,71 (s, 3H), 4,52 (d, 1 H), 6,99 (m, 4H), 7,56 (dd, 1 H), 7,86 (m, 1 H)
      threo-Ibb1126: 2,90 (m, 2H), 3,61 (s, 3H), 3,64 (q, 1 H), 4,15 (d, 1 H), 7,04 (m, 4H), 7,72 (dd, 1 H), 7,80 (m, 1 H)
   Bsp. threo-Ibb1126: 2,90 (m, 2H), 3,61 (s, 3H), 3,64 (q, 1 H), 4,15 (d, 1 H), 7,04 (m, 4H), 7,72 (dd, 1 H), 7,80 (m, 1 H)
   Bsp. Ibb1127 (erythro-Ibb1127 : threo-Ibb1127 = 34:66), NMR:
      erythro-Ibb1127: 2,86 (dd, 1 H), 3,06 (dd, 1 H), 3,48 (m, 1 H), 3,71 (s, 3H), 4,52 (d, 1 H), 6,96 (d, 2H), 7,29 (d, 2H), 7,61 (dd, 1 H), 7,86 (m, 1 H)
      threo-Ibb1127: 2,89 (m, 2H), 3,61 (s, 3H), 3,63 (q, 1 H), 4,15 (d, 1 H), 7,02 (d, 2H), 7,30 (d, 2H), 7,75 (dd, 1 H), 7,81 (m, 1 H)
   Bsp. erythro-Ibb1127: 2,86 (dd, 1 H), 3,06 (dd, 1 H), 3,48 (m, 1 H), 3,71 (s, 3H), 4,52 (d, 1 H), 6,96 (d, 2H), 7,29 (d, 2H), 7,61 (dd, 1 H), 7,86 (m, 1 H)
   Bsp. threo-Ibb1127: 2,89 (m, 2H), 3,61 (s, 3H), 3,63 (q, 1 H), 4,15 (d, 1 H), 7,02 (d, 2H), 7,30 (d, 2H), 7,75 (dd, 1 H), 7,81 (m, 1 H)
   Bsp. Ibb1128 (erythro-Ibb1128 : threo-Ibb1128 = 68:32), NMR:
      erythro-Ibb1128: 2,85 (dd, 1 H), 3,06 (dd, 1 H), 3,46 (m, 1 H), 3,71 (s, 3H), 4,52 (d, 1 H), 6,89 (d, 2H), 7,44 (d, 2H), 7,61 (dd, 1 H), 7,86 (m, 1 H)
      threo-Ibb1128: 2,88 (m, 2H), 3,61 (s, 3H), 3,62 (q, 1 H), 4,15 (d, 1 H), 6,96 (d, 2H), 7,46 (d, 2H), 7,75 (dd, 1 H), 7,81 (m, 1 H)
   Bsp. erythro-Ibb1128: 2,85 (dd, 1 H), 3,06 (dd, 1 H), 3,46 (m, 1 H), 3,71 (s, 3H), 4,52 (d, 1 H), 6,89 (d, 2H), 7,44 (d, 2H), 7,61 (dd, 1 H), 7,86 (m, 1 H)
   Bsp. threo-Ibb1128: 2,88 (m, 2H), 3,61 (s, 3H), 3,62 (q, 1 H), 4,15 (d, 1 H), 6,96 (d, 2H), 7,46 (d, 2H), 7,75 (dd, 1 H), 7,81 (m, 1 H)
   Bsp. Ibb1129 (erythro-Ibb1129 : threo-Ibb1129 = 59:41), NMR:
      erythro-Ibb1129: 2,90 (dd, 1 H), 3,11 (dd, 1 H), 3,48 (m, 1 H), 3,71 (s, 3H), 4,54 (d, 1 H), 6,99 (m, 2H), 7,51 (dd, 1 H), 7,84 (m, 1 H)
      threo-Ibb1129: 2,94 (m, 2H), 3,61 (s, 3H), 3,63 (q, 1 H), 4,16 (d, 1 H), 7,05 (m, 2H), 7,69 (dd, 1 H), 7,78 (m, 1 H)
   Bsp. Ibb1131 (erythro-Ibb1131 : threo-Ibb1131 = 40:60), NMR:
      erythro-Ibb1131: 2,89 (dd, 1 H), 3,08 (dd, 1 H), 3,57 (m, 1 H), 3,72 (s, 3H), 4,56 (d, 1 H), 7,17 (d, 2H), 7,63 (m, 3H), 7,89 (m, 1 H)
      threo-Ibb1131: 2,91 (m, 2H), 3,61 (s, 3H), 3,71 (q, 1 H), 4,20 (d, 1 H), 7,26 (d, 2H), 7,62 (d, 2H), 7,76 (dd, 1 H), 7,85 (m, 1 H)
   Bsp. Ibb1132 (erythro-Ibb1132 : threo-Ibb1132 = 34:66), NMR:
      erythro-Ibb1132: 2,90 (dd, 1 H), 3,10 (dd, 1 H), 3,65 (m, 1 H), 3,72 (s, 3H), 4,59 (d, 1 H), 7,25 (d, 2H), 7,68 (dd, 1 H), 7,91 (m, 1 H), 8,19 (d, 2H)
      threo-Ibb1132: 2,97 (m, 2H), 3,61 (s, 3H), 3,78 (q, 1 H), 4,23 (d, 1 H), 7,34 (d, 2H), 7,82 (dd, 1 H), 7,86 (m, 1 H), 8,21 (d, 2H)
   Bsp. Ibb1133 (erythro-Ibb1133 : threo-Ibb1133 = 57:43), NMR:
      erythro-Ibb1133: 2,86 (dd, 1 H), 3,07 (dd, 1 H), 3,43 (m, 1 H), 3,71 (s, 3H), 3,79 (s, 3H), 4,50 (d, 1 H), 6,82 (d, 2H), 6,91 (d, 2H), 7,53 (dd, 1 H), 7,83 (m, 1 H)
      threo-Ibb1133: 2,89 (m, 2H), 3,58 (q, 1 H), 3,61 (s, 3H), 3,78 (s, 3H), 4,12 (d, 1 H), 6,83 (d, 2H), 6,96 (d, 2H), 7,69 (dd, 1 H), 7,76 (m, 1 H)
   Bsp. erythro-Ibb1133: 2,86 (dd, 1 H), 3,07 (dd, 1 H), 3,43 (m, 1 H), 3,71 (s, 3H), 3,79 (s, 3H), 4,50 (d, 1 H), 6,82 (d, 2H), 6,91 (d, 2H), 7,53 (dd, 1 H), 7,83 (m, 1 H)
   Bsp. Ibb1134 (erythro-Ibb1134 : threo-Ibb1134 = 54:46), NMR:
      erythro-Ibb1134: 2,87 (dd, 1 H), 3,06 (dd, 1 H), 3,50 (m, 1 H), 3,72 (s, 3H), 4,53 (d, 1 H), 7,60 (dd, 1 H), 7,89 (d, 1 H)
      threo-Ibb1134: 2,92 (m, 2H), 3,62 (s, 3H), 3,63 (q, 1 H), 4,16 (d, 1 H), 7,76 (dd, 1 H), 7,82 (d, 1H)
   Bsp. erythro-Ibb1134: 2,87 (dd, 1 H), 3,06 (dd, 1 H), 3,50 (m, 1 H), 3,72 (s, 3H), 4,53 (d, 1 H), 7,60 (dd, 1 H), 7,89 (d, 1 H)
   Bsp. erythro-1-Ibb1134: 2,87 (dd, 1 H), 3,06 (dd, 1 H), 3,50 (m, 1 H), 3,72 (s, 3H), 4,53 (d, 1 H), 7,60 (dd, 1 H), 7,89 (d, 1 H)
   Bsp. erythro-2-Ibb1134: 2,87 (dd, 1 H), 3,06 (dd, 1 H), 3,50 (m, 1 H), 3,72 (s, 3H), 4,53 (d, 1 H), 7,60 (dd, 1 H), 7,89 (d, 1 H)
   Bsp. threo-Ibb1134: 2,92 (m, 2H), 3,62 (s, 3H), 3,63 (q, 1 H), 4,16 (d, 1 H), 7,76 (dd, 1 H), 7,82 (d, 1 H)
   Bsp. threo-1-Ibb1134: 2,92 (m, 2H), 3,62 (s, 3H), 3,63 (q, 1 H), 4,16 (d, 1 H), 7,76 (dd, 1 H), 7,82 (d, 1 H)
   Bsp. threo-2-Ibb1134: 2,92 (m, 2H), 3,62 (s, 3H), 3,63 (q, 1 H), 4,16 (d, 1 H), 7,76 (dd, 1 H), 7,82 (d, 1 H); HPLC-Chiral: 30,7 min, Eluent d
   Bsp. Ibb1139 (erythro-Ibb1139 : threo-Ibb1139 = 37:63), NMR:
      erythro-Ibb1139, NMR: 2,90 (dd, 1 H), 3,09 (dd, 1 H), 3,65 (m, 1 H), 3,71 (s, 3H), 4,57 (d, 1 H), 7,41 (dt, 1 H), 7,53 (t, 1 H), 7,68 (dd, 1 H), 7,93 (m, 2H), 8,21 (m, 1 H) threo-Ibb1139, NMR: 2,98 (d, 2H), 3,62 (s, 3H), 3,80 (q, 1 H), 4,26 (d, 1 H), 7,48 (dt, 1 H), 7,52 (t, 1 H), 7,82 (dd, 1 H), 7,88 (m, 1 H), 8,02 (m, 1 H), 8,20 (m, 1 H)
   Bsp. erythro-Ibb1148, NMR: 2,84 (dd, 1 H), 3.05 (dd, 1 H), 3,70 (s, 3H), 3,96 (m, 1 H), 4,58 (d, 1 H), 7,16 (m, 3H), 7,72 (dd, 1 H), 7,92 (m, 1 H)
   Bsp. threo-Ibb1148, NMR: 3,02 (m, 2H), 3,60 (s, 3H), 3,96 (q, 1 H), 4,22 (d, 1 H), 6,90 (m, 1 H), 7,06 (m, 1 H), 7,13 (m, 1 H), 7,93 (m, 2H)
   Bsp. erythro-Ibb1149, NMR: 2,82 (dd, 1 H), 3,04 (dd, 1 H), 3,70 (s, 3H), 3,90 (q, 1 H), 4,55 (d, 1 H), 6,71 (m, 1 H), 6,96 (m, 1 H), 7,38 (m, 1 H), 7,70 (dd, 1 H), 7,91 (m, 1 H)
   Bsp. threo-Ibb1149, NMR: 2,99 (m, 2H), 3,60 (s, 3H), 3,88 (q, 1 H), 4,20 (d, 1 H), 6,82 (m, 2H), 7,10 (m, 1 H), 7,91 (m, 2H)
   Bsp. Ibb1150 (erythro-Ibb1150 : threo-Ibb1150 = 53:47), NMR:
      erythro-Ibb1150: 2,81 (dd, 1 H), 2,99 (dd, 1 H), 3,71 (s, 3H), 3,91 (m, 1 H), 4,57 (d, 1 H), 6,94 (m, 1 H), 7,00 (m, 1 H), 7,12 (m, 1 H), 7,71 (dd, 1 H), 7,93 (m, 1 H)
      threo-Ibb1150: 2,99 (m, 2H), 3,61 (s, 3H), 3,89 (q, 1 H), 4,22 (d, 1 H), 6,86 (m, 1 H), 7,00 (m, 2H), 7,12 (m, 1 H), 7,93 (m, 2H)
   Bsp. erythro-Ibb1150: 2,81 (dd, 1 H), 2,99 (dd, 1 H), 3,71 (s, 3H), 3,91 (m, 1 H), 4,57 (d, 1 H), 6,94 (m, 1 H), 7,00 (m, 1 H), 7,12 (m, 1 H), 7,71 (dd, 1 H), 7,93 (m, 1 H)
   Bsp. Ibb1151 (erythro-Ibb1151 : threo-Ibb1151 = 52:48), NMR:
      erythro-Ibb1151: 2,88 (dd, 1 H), 2,95 (dd, 1 H), 3,62 (s, 3H), 4,16 (m, 1 H), 4,45 (d, 1 H), 6,93 (t, 2H), 7,31 (m, 1 H), 7,96 (dd, 1 H), 8,07 (m, 1 H)
      threo-Ibb1151: 3,11 (dd, 1 H), 3,23 (dd, 1 H), 3,63 (s, 3H), 4,07 (q, 1 H), 4,21 (d, 1 H), 6,81 (t, 2H), 7,21 (m, 1 H), 7,78 (m, 1 H), 7,94 (dd, 1 H)
   Bsp. Ibb1152 (erythro-Ibb1152 : threo-Ibb1152 = 63:37), NMR:
      erythro-Ibb1152, NMR: 2,83 (dd, 1 H), 3,02 (dd, 1 H), 3,47 (m, 1 H), 3,72 (s, 3H), 4,51 (d, 1 H), 6,73 (m, 1 H), 6,94 (m, 1 H), 7,10 (m, 1 H), 7,65 (dd, 1 H), 7,90 (m, 1 H) threo-Ibb1152, NMR: 2,88 (d, 2H), 3,61 (q, 1 H), 3,62 (s, 3H), 4,15 (d, 1 H), 6,82 (m, 1 H), 6,96 (m, 1 H), 7,11 (m, 1 H), 7,77 (dd, 1 H), 7,84 (m, 1 H)
   Bsp. erythro-Ibb1152, NMR: 2,83 (dd, 1 H), 3,02 (dd, 1 H), 3,47 (m, 1 H), 3,72 (s, 3H), 4,51 (d, 1 H), 6,73 (m, 1 H), 6,94 (m, 1 H), 7,10 (m, 1 H), 7,65 (dd, 1 H), 7,90 (m, 1 H)
   Bsp. erythro-Ibb1164: 2,87 (m, 2H), 3,62 (s, 3H), 4,00 (q, 1 H), 4,39 (d, 1 H), 6,99 (d, 2H), 7,79 (dd, 1 H), 8,07 (m, 1 H)
   Bsp. threo-Ibb1164: 3,08 (dd, 1 H), 3,22 (dd, 1 H), 3,64 (s, 3H), 4,10 (m, 1 H), 4,18 (d, 1 H), 6,87 (d, 2H), 7,80 (m, 1 H), 7,95 (dd, 1 H)
   Bsp. erythro-Ibb1622, NMR: 2,28 (s, 3H), 2,83 (dd, 1 H), 3,00 (dd, 1 H), 3,52 (m, 1 H), 3,67 (s, 3H), 4,40 (d, 1 H), 6,95 (d, 2H), 7,15 (t, 1 H), 7,24 (d, 2H), 8,19 (bs, 1 H), 8,39 (bs, 1 H)
   Bsp. threo-Ibb1622, NMR: 2,33 (s, 3H), 2,89 (d, 2H), 3,58 (s, 3H), 3,64 (q, 1 H), 4,10 (d, 1 H), 7,04 (d, 2H), 7,27 (d, 2H), 7,29 (t, 1 H), 8,14 (d, 1 H), 8,39 (d, 1 H)
   Bsp. erythro-Ibb2162, NMR: 2,87 (dd, 1 H), 3,11 (dd, 1 H), 3,50 (m, 1 H), 3,73 (s, 3H), 4,65 (d, 1 H), 6,92 (d, 2H), 7,27 (d, 2H), 7,62 (m, 1 H), 8,55 (d, 1 H), 8,82 (d, 1 H)
   Bsp. threo-Ibb2162, NMR: 2,91 (m, 2H), 3,61 (s, 3H), 3,64 (q, 1 H), 4,23 (d, 1 H), 7,01 (d, 2H), 7,30 (d, 2H), 7,76 (m, 1 H), 8,48 (d, 1 H), 8,82 (d, 1 H)
   Bsp. erythro-Ibb2186, NMR: 2,90 (dd, 1 H), 3,05 (dd, 1 H), 3,66 (s, 3H), 4,10 (m, 1 H), 4,58 (d, 1 H), 6,90 (t, 2H), 7,33 (m, 1 H), 7,90 (t, 1 H), 8,75 (d, 1 H), 8,87 (d, 1 H)
   Bsp. threo-Ibb2186, NMR: 3,15 (dd, 1 H), 3,24 (dd, 1 H), 3,64 (s, 3H), 4,17 (m, 1 H), 4,27 (d, 1 H), 6,80 (t, 2H), 7,21 (m, 1 H), 7,85 (t, 1 H), 8,50 (d, 1 H), 8,76 (d, 1 H)
   Bsp. erythro-Ibb3909, NMR: 2,87 (dd, 1 H), 3,02 (dd, 1 H), 3,57 (m, 1 H), 3,71 (s, 3H), 4,50 (d, 1 H), 7,21 (m, 3H), 7,55 (m, 1 H), 8,31 (m, 1 H), 8,68 (m, 1 H)
   Bsp. threo-Ibb3909, NMR: 2,89 (d, 2H), 3,61 (s, 3H), 3,70 (q, 1 H), 4,15 (d, 1 H), 7,21 (m, 1 H), 7,40 (d, 1 H), 8,27 (d, 1 H), 8,68 (d, 1 H)
   Bsp. erythro-Ibb3783, NMR: 2,84 (dd, 1 H), 3,02 (dd, 1 H), 3,46 (m, 1 H), 3,70 (s, 3H), 3,87 (s, 3H), 4,46 (d, 1 H), 6,69 (m, 1 H), 6,80 (dd, 1 H), 6,85 (t, 1 H), 7,50 (t, 1 H), 8,28 (d, 1 H), 8,63 (d, 1 H)
   Bsp. threo-Ibb3783, NMR: 2,88 (m, 2H), 3,58 (q, 1 H), 3,61 (s, 3H), 3,87 (s, 3H), 4,10 (d, 1 H), 6,85 (m, 3H), 7,66 (t, 1 H), 8,21 (d, 1 H), 8,62 (d, 1 H)
   Bsp. erythro-Ibb3937, NMR: 2,89 (dd, 1 H), 3,09 (dd, 1 H), 3,60 (m, 1 H), 3,71 (s, 3H), 4,53 (d, 1 H), 7,15 (d, 2H), 7,47 (m, 1 H), 7,56 (d, 2H), 8,30 (bs, 1 H), 8,64 (bs, 1 H)
   Bsp. threo-Ibb3937, NMR: 2,94 (m, 2H), 3,60 (s, 3H), 3,74 (q, 1 H), 4,17 (d, 1 H), 7,26 (d, 2H), 7,59 (d, 2H), 7,64 (t, 1 H), 8,26 (d, 1 H), 8,64 (d, 1 H)

### (B) Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| 75 Gewichtsteile | einer Verbindung der Formel (I), | |
|---|---|---|
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichtsteile einer Verbindung der Formel (I),

| | | |
|---|---|---|
| 5 | " | 2,2'-dinaphthylmethan-6,6'-disulfonsaures Natrium |
| 2 | " | oleoylmethyltaurinsaures Natrium, |
| 1 | Gewichtsteil | Polyvinylalkohol, |
| 17 | Gewichtsteile | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### (C) Biologische Beispiele

### 1. Herbizide Wirkung im Vorauflauf

Samen von mono- bzw. dikotylen Unkraut- bzw. Kulturpflanzen wurden in Holzfasertöpfen in sandiger Lehmerde ausgelegt und mit Erde abgedeckt. Die in Form von benetzbaren Pulvern (WP) formulierten erfindungsgemäßen Verbindungen (I) wurden dann als wäßrige Suspension bzw. Emulsion mit einer Wasseraufwandmenge von umgerechnet 600 I/ha unter Zusatz von 0,2% Netzmittel auf die Oberfläche der Abdeckerde appliziert.

Nach der Behandlung wurden die Töpfe im Gewächshaus aufgestellt und unter guten Wachstumsbedingungen für die Testpflanzen gehalten. Nach ca. 3 Wochen wurde die Wirkung der Präparate visuell im Vergleich zu unbehandelten Kontrollen in Prozentwerten bonitiert. Beispielsweise bedeutet 100% Wirkung = Pflanzen sind abgestorben, 50% herbizide Wirkung oder Schaden = Pflanzen zu 50% reduziert bzw. Pflanzenmasse um 50% reduziert, 0 % Wirkung = wie Kontrollpflanzen. Erfindungsgemäße Verbindungen (I), wie beispielsweise die Verbindungen Nr. erythro-Ibb2, threo-Ibb2, erythro-Ibb9, threo-Ibb9, threo-Ibb10, erythro-Ibb25, threo-Ibb25, erythro-Ibb26, threo-Ibb26, erythro-Ibb542, threo-Ibb542, erythro-Ibb565, threo-Ibb565, erythro-Ibb566, threo-Ibb566, erythro-Ibb1081, threo-Ibb1081, Ibb1082, erythro-Ibb1082, erythro-1-Ibb1082, erythro-2-Ibb1082, threo-Ibb1082, threo-1-Ibb1082, threo-2-Ibb1082, erythro-Ibb1084, threo-Ibb1084, erythro-Ibb1085, threo-Ibb1085, erythro-Ibb1088, threo-Ibb1088, erythro-Ibb1089, threo-Ibb1089, threo-Ibb1090, erythro-Ibb1092, erythro-Ibb1093, threo-Ibb1093, erythro-Ibb1094, threo-Ibb1094, Ibb1096, erythro-Ibb1096, threo-Ibb1096, erythro-Ibb1103, threo-Ibb1103, erythro-Ibb1104, threo-Ibb1104, erythro-Ibb1105, erythro-1-Ibb1105, erythro-2-Ibb1105, threo-Ibb1105, threo-1-Ibb1105, threo-2-Ibb1105, erythro-Ibb1106, erythro-1-Ibb1106, erythro-2-Ibb1106, threo-Ibb1106, threo-1-Ibb1106, threo-2-Ibb1106, erythro-Ibb1107, threo-Ibb1107, threo-Ibb1108, erythro-Ibb1622, threo-Ibb1622, erythro-Ibb3783, threo-Ibb3783 aus den obigen Tabellen 2 bis 2f weisen eine gute herbizide Wirksamkeit (70% bis 100% Wirkung) gegen mehrere Schadpflanzen bei einer Aufwandmenge von 320 g oder weniger Aktivsubstanz pro Hektar im Vorauflauf auf. Beispielsweise haben dabei die Verbindungen Nr. erythro-Ibb2, threo-Ibb2, threo-Ibb9, threo-Ibb10, erythro-Ibb25, threo-Ibb25, erythro-Ibb26, threo-Ibb26, Ibb1082, threo-Ibb1082, erythro-Ibb1084, threo-Ibb1084, erythro-Ibb1088, threo-Ibb1088, threo-Ibb1089, threo-Ibb1090, threo-Ibb1105, erythro-Ibb1106, threo-Ibb1106 und threo-Ibb1108 eine sehr gute Wirkung (90-100%) gegen Schadpflanzen wie Echinochloa crus-galli im Vorauflaufverfahren bei einer Aufwandmenge von 0,32 kg Aktivsubstanz pro Hektar.

Beispielsweise haben die Verbindungen Nr. erythro-Ibb2, threo-Ibb2, erythro-Ibb9, threo-Ibb10, erythro-Ibb26, Ibb1082, threo-Ibb1082, threo-Ibb1084, threo-Ibb1088, threo-Ibb1089, threo-Ibb1090, threo-Ibb1105, erythro-Ibb1106, threo-Ibb1106 und threo-Ibb1108 eine sehr gute Wirkung (90-100%) gegen Schadpflanzen wie Setaria virides im Vorauflaufverfahren bei einer Aufwandmenge von 0,32 kg Aktivsubstanz pro Hektar.

Beispielsweise haben die Verbindungen Nr. erythro-Ibb26, threo-Ibb26, threo-Ibb1089, erythro-Ibb1105, erythro-Ibb1106 und threo-Ibb1106 eine sehr gute Wirkung (80-100%) gegen Schadpflanzen wie Polygonum convolvulus im Vorauflaufverfahren bei einer Aufwandmenge von 0,32 kg Aktivsubstanz pro Hektar.

Beispielsweise haben die Verbindungen Nr. erythro-Ibb9, threo-Ibb9, Ibb1082, threo-Ibb1082, erythro-Ibb1088, threo-Ibb1088, erythro-Ibb1089, threo-Ibb1089, threo-Ibb1090, erythro-Ibb1105, threo-Ibb1105, erythro-Ibb1106, threo-Ibb1106 und threo-Ibb1108 eine sehr gute Wirkung (90-100%) gegen Schadpflanzen wie Veronica persica im Vorauflaufverfahren bei einer Aufwandmenge von 0,32 kg Aktivsubstanz pro Hektar.

Beispielsweise haben die Verbindungen Nr. erythro-Ibb9, threo-Ibb9, erythro-Ibb26, threo-Ibb26, Ibb1082, threo-Ibb1082, threo-Ibb1084, threo-Ibb1088, erythro-Ibb1105, threo-Ibb1105, erythro-Ibb1106 und threo-Ibb1106 eine sehr gute Wirkung (90-100%) gegen Schadpflanzen wie Viola tricolor im Vorauflaufverfahren bei einer Aufwandmenge von 0,32 kg Aktivsubstanz pro Hektar.

Beispielsweise haben die Verbindungen Nr. erythro-Ibb2, threo-Ibb2, erythro-Ibb9, threo-Ibb9, threo-Ibb10, erythro-Ibb26, threo-Ibb26, Ibb1082, threo-Ibb1082, erythro-Ibb1088, threo-Ibb1088, threo-Ibb1090, erythro-Ibb1106 und threo-Ibb1106 eine sehr gute Wirkung (80-100%) gegen Schadpflanzen wie Alopecurus myosuroides im Vorauflaufverfahren bei einer Aufwandmenge von 0,32 kg Aktivsubstanz pro Hektar. Beispielsweise haben die Verbindungen Nr. erythro-Ibb2, threo-Ibb10, erythro-Ibb26, threo-Ibb26, threo-Ibb1082, erythro-Ibb1105, threo-Ibb1105, erythro-Ibb1106 und threo-Ibb1106 eine sehr gute Wirkung (80-100%) gegen Schadpflanzen wie Amaranthus retroflexus im Vorauflaufverfahren bei einer Aufwandmenge von 0,32 kg Aktivsubstanz pro Hektar.

### 2. Herbizide Wirkung im Nachauflauf

Samen von mono- bzw. dikotylen Unkraut- bzw. Kulturpflanzen wurden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat wurden die Versuchspflanzen im Einblattstadium behandelt, wobei die in Form von benetzbaren Pulvern (WP) formulierten erfindungsgemäßen Verbindungen (I) als wäßrige Suspension bzw. Emulsion mit einer Wasseraufwandmenge von umgerechnet 600 I/ha unter Zusatz von 0,2% Netzmittel auf die grünen Pflanzenteile gesprüht wurden. Nach ca. 3 Wochen Standzeit der Versuchspflanzen im Gewächshaus unter optimalen Wachstumsbedingungen wurde die Wirkung der Präparate visuell im Vergleich zu unbehandelten Kontrollen in Prozentwerten bonitiert. Beispielsweise bedeutet 100% Wirkung = Pflanzen sind abgestorben, 50% herbizide Wirkung oder Schaden = Pflanzen zu 50% reduziert bzw. Pflanzenmasse um 50% reduziert, 0 % Wirkung = wie Kontrollpflanzen.

Wie die Ergebnisse zeigen, weisen erfindungsgemäße Verbindungen (I), wie beispielsweise die Verbindungen Nr. erythro-Ibb2, threo-Ibb2, erythro-Ibb9, threo-Ibb9, threo-Ibb10, erythro-Ibb25, threo-Ibb25, erythro-Ibb26, threo-Ibb26, erythro-Ibb542, threo-Ibb542, erythro-Ibb565, threo-Ibb565, erythro-Ibb566, threo-Ibb566, erythro-Ibb1081, threo-Ibb1081, Ibb1082, erythro-Ibb1082, erythro-1-Ibb1082, erythro-2-Ibb1082, threo-Ibb1082, threo-1-Ibb1082, threo-2-Ibb1082, erythro-Ibb1084, threo-Ibb1084, erythro-Ibb1085, threo-Ibb1085, erythro-Ibb1088, threo-Ibb1088, erythro-Ibb1089, threo-Ibb1089, threo-Ibb1090, erythro-Ibb1092, erythro-Ibb1093, threo-Ibb1093, erythro-Ibb1094, threo-Ibb1094, Ibb1096, erythro-Ibb1096, threo-Ibb1096, erythro-Ibb1103, threo-Ibb1103, erythro-Ibb1104, threo-Ibb1104, erythro-Ibb1105, erythro-1-Ibb1105, erythro-2-Ibb1105, threo-Ibb1105, threo-1-Ibb1105, threo-2-Ibb1105, erythro-Ibb1106, erythro-1-Ibb1106, erythro-2-Ibb1106, threo-Ibb1106, threo-1-Ibb1106, threo-2-Ibb1106, erythro-Ibb1107, threo-Ibb1107, threo-Ibb1108, erythro-Ibb1622, threo-Ibb1622, erythro-Ibb3783, threo-Ibb3783 aus den obigen Tabellen 2 bis 2f eine gute herbizide Wirksamkeit (70% bis 100% Wirkung) gegen mehrere Schadpflanzen bei einer Aufwandmenge von 320 g oder weniger Aktivsubstanz pro Hektar im Nachauflauf auf.

Beispielsweise haben dabei die Verbindungen Nr. erythro-Ibb2, threo-Ibb2, threo-Ibb9, threo-Ibb10, erythro-Ibb25, threo-Ibb25, erythro-Ibb26, threo-Ibb26, Ibb1082, threo-Ibb1082, erythro-Ibb1084, threo-Ibb1084, erythro-Ibb1088, threo-Ibb1088, erythro-Ibb1089, threo-Ibb1089, threo-Ibb1090, erythro-Ibb1105, threo-Ibb1105, erythro-Ibb1106, threo-Ibb1106 und threo-Ibb1108 eine sehr gute Wirkung (80-100%) gegen Schadpflanzen wie Echinochloa crus-galli im Nachauflaufverfahren bei einer Aufwandmenge von 0,32 kg Aktivsubstanz pro Hektar.

Beispielsweise haben dabei auch die Verbindungen Nr. erythro-Ibb2, threo-Ibb2, threo-Ibb9, threo-Ibb10, erythro-Ibb25, threo-Ibb25, erythro-Ibb26, threo-Ibb26, Ibb1082, threo-Ibb1082, erythro-Ibb1084, threo-Ibb1084, threo-Ibb1088, erythro-Ibb1089, threo-Ibb1089, threo-Ibb1090, erythro-Ibb1105, threo-Ibb1105, erythro-Ibb1106, threo-Ibb1106 und threo-Ibb1108 eine sehr gute Wirkung (80-100%) gegen Schadpflanzen wie Setaria virides im Nachauflaufverfahren bei einer Aufwandmenge von 0,32 kg Aktivsubstanz pro Hektar.

Beispielsweise haben dabei auch die Verbindungen Nr. erythro-Ibb2, threo-Ibb2, erythro-Ibb9, threo-Ibb9, threo-Ibb10, erythro-Ibb25, threo-Ibb25, erythro-Ibb26, threo-Ibb26, Ibb1082, threo-Ibb1082, erythro-Ibb1084, threo-Ibb1088, erythro-Ibb1089, threo-Ibb1089, threo-Ibb1090, erythro-Ibb1105, threo-Ibb1105, threo-Ibb1106 und threo-Ibb1108 eine sehr gute Wirkung (80-100%) gegen Schadpflanzen wie Veronica persica im Nachauflaufverfahren bei einer Aufwandmenge von 0,32 kg Aktivsubstanz pro Hektar.

Beispielsweise haben dabei auch die Verbindungen Nr. erythro-Ibb2, threo-Ibb2, erythro-Ibb9, threo-Ibb9, threo-Ibb10, erythro-Ibb25, threo-Ibb25, erythro-Ibb26, threo-Ibb26, Ibb1082, threo-Ibb1082, threo-Ibb1084, erythro-Ibb1088, threo-Ibb1088, threo-Ibb1090, erythro-Ibb1105, threo-Ibb1105, erythro-Ibb1106, threo-Ibb1106 und threo-Ibb1108 eine sehr gute Wirkung (80-100%) gegen Schadpflanzen wie Avena fatua im Nachauflaufverfahren bei einer Aufwandmenge von 0,32 kg Aktivsubstanz pro Hektar. Beispielsweise haben dabei auch die Verbindungen Nr. erythro-Ibb2, threo-Ibb2, threo-Ibb9, threo-Ibb10, erythro-Ibb25, threo-Ibb25, erythro-Ibb26, threo-Ibb26, Ibb1082, threo-Ibb1082, threo-Ibb1084, threo-Ibb1088, threo-Ibb1090 und threo-Ibb1105 eine sehr gute Wirkung (80-100%) gegen Schadpflanzen wie Alopecurus myosuroides im Nachauflaufverfahren bei einer Aufwandmenge von 0,32 kg Aktivsubstanz pro Hektar. Beispielsweise haben dabei auch die Verbindungen Nr. threo-Ibb10, erythro-Ibb25, threo-Ibb25, erythro-Ibb26, threo-Ibb26, threo-Ibb1082, threo-Ibb1084, erythro-Ibb1105, threo-Ibb1105 und threo-Ibb1108 eine sehr gute Wirkung (80-100%) gegen Schadpflanzen wie Cyprus esculentus im Nachauflaufverfahren bei einer Aufwandmenge von 0,32 kg Aktivsubstanz pro Hektar.

Beispielsweise haben dabei auch die Verbindungen Nr. erythro-Ibb2, threo-Ibb26 und threo-Ibb1082 eine sehr gute Wirkung (80-100%) gegen Schadpflanzen wie Lolium multiflorum im Nachauflaufverfahren bei einer Aufwandmenge von 0,32 kg Aktivsubstanz pro Hektar.

Beispielsweise haben dabei auch die Verbindungen Nr. erythro-Ibb9 und threo-Ibb26 eine sehr gute Wirkung (80-100%) gegen Schadpflanzen wie Stellaria media im Nachauflaufverfahren bei einer Aufwandmenge von 0,32 kg Aktivsubstanz pro Hektar. Beispielsweise haben dabei auch die Verbindungen Nr. erythro-Ibb2, threo-Ibb2, threo-Ibb9, threo-Ibb25, erythro-Ibb26, threo-Ibb26, threo-Ibb1082, threo-Ibb1084, erythro-Ibb1088, threo-Ibb1088, threo-Ibb1090, erythro-Ibb1105, threo-Ibb1105, erythro-Ibb1106, threo-Ibb1106 und threo-Ibb1108 eine sehr gute Wirkung (80-100%) gegen Schadpflanzen wie Abutilon theophrasti im Nachauflaufverfahren bei einer Aufwandmenge von 0,32 kg Aktivsubstanz pro Hektar.
einer Aufwandmenge von 0,32 kg Aktivsubstanz pro Hektar.

Beispielsweise haben dabei auch die Verbindungen Nr. erythro-Ibb2, threo-Ibb2, threo-Ibb9, erythro-Ibb25, threo-Ibb25, erythro-Ibb26, Ibb1082, threo-Ibb1082, threo-Ibb1084 und erythro-Ibb1105 eine sehr gute Wirkung (80-100%) gegen Schadpflanzen wie Amaranthus retroflexus im Nachauflaufverfahren bei einer Aufwandmenge von 0,32 kg Aktivsubstanz pro Hektar.

Beispielsweise haben dabei auch die Verbindungen Nr. threo-Ibb10, erythro-Ibb25, threo-Ibb25, erythro-Ibb26, threo-Ibb26, Ibb1082, threo-Ibb1082, erythro-Ibb1084, threo-Ibb1084, erythro-Ibb1088, threo-Ibb1088, threo-Ibb1090, erythro-Ibb1105, threo-Ibb1105 und threo-Ibb1108 eine sehr gute Wirkung (80-100%) gegen Schadpflanzen wie Polygonum convolvulus im Nachauflaufverfahren bei einer Aufwandmenge von 0,32 kg Aktivsubstanz pro Hektar.

Beispielsweise haben dabei auch die Verbindungen Nr. erythro-Ibb2, threo-Ibb2, threo-Ibb9, erythro-Ibb26, threo-Ibb26, Ibb1082, threo-Ibb1090 und threo-Ibb1105 eine sehr gute Wirkung (80-100%) gegen Schadpflanzen wie Viola tricolor virides im Nachauflaufverfahren bei einer Aufwandmenge von 0,32 kg Aktivsubstanz pro Hektar. Beispielsweise haben dabei auch die Verbindungen Nr. erythro-Ibb2, erythro-Ibb26, threo-Ibb26, Ibb1082, threo-Ibb1084, threo-Ibb1088, threo-Ibb1090, threo-Ibb1105 und threo-Ibb1106 eine sehr gute Wirkung (80-100%) gegen Schadpflanzen wie Pharbitis purpurea im Nachauflaufverfahren bei einer Aufwandmenge von 0,32 kg Aktivsubstanz pro Hektar.

## Patentansprüche

1. Verbindungen der Formel (I) oder deren Salze, 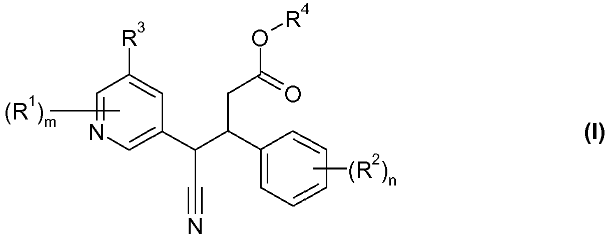 in welcher
(R¹)ₘ m Substituenten R¹ bedeutet,
wobei R¹, wenn m = 1, oder jeder der Substituenten R¹, wenn m größer als 1 ist, unabhängig voneinander Halogen, Cyano, Nitro, Hydroxy, (C₁-C₈)Alkyl, (C₂-C₆)Alkenyl, (C₂-C₆)Alkinyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Haloal kenyl, (C₂-C₆)Haloalkinyl, (C₁-C₆)Alkoxy-(C₁-C₄)alkyl, (C₁-C₆)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR⁵, C(O)NR⁶R⁷, C(O)-Het¹, NR⁸R⁹ oder Het² bedeutet oder wobei jeweils zwei am Ring ortho-ständige Gruppen R¹ oder R¹ und R³ gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht,
wobei die Gruppe -Z¹-A*-Z² zusammen mit den an die Gruppe gebundenen C-Atomen des Phenylrings einen ankondensierten 5 oder 6 Ring bilden,
(R²)ₙ n Substituenten R² bedeutet,
wobei R², wenn n = 1, oder jeder der Substituenten R², wenn n größer als 1 ist, unabhängig voneinander Halogen, Cyano, Nitro, Hydroxy, (C₁-C₈)Alkyl, (C₂-C₆)Alkenyl, (C₂-C₆)Alkinyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₈)Haloalkylthio, (C₁-C₆)Haloalkylsulfinyl, (C₁-C₆)Haloalkylsulfonyl, (C₂-C₆)Haloalkenyl, (C₂-C₆)Haloalkinyl, (C₁-C₆)Alkoxy-(C₁-C₄)alkyl, (C₁-C₆)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR¹⁰, C(O)NR¹¹R¹², C(O)-Het³, NR¹³R¹⁴ oder Het⁴ bedeutet oder wobei jeweils zwei am Ring ortho-ständige Gruppen R² gemeinsam eine Gruppe der Formel -Z³-A*-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³ -A**-Z⁴ zusammen mit den an die Gruppe gebundenen C-Atomen des Phenylrings einen ankondensierten 5 oder 6 Ring bilden,
R³ Halogen, Cyano, Nitro, Hydroxy, (C₁-C₈)Alkyl, (C₂-C₆)Alkenyl, (C₂-C₆)Alkinyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Haloalkenyl, (C₂-C₆)Haloalkinyl, (C₁-C₈)Alkoxy-(C₁-C₄)alkyl, (C₁-C₈)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR¹⁵, C(O)NR¹⁶R¹⁷, C(O)-Het⁵, NR¹⁸R¹⁹ oder Het⁶ bedeutet,
R⁴ Wasserstoff oder einen hydrolysierbaren Rest bedeutet, vorzugsweise R⁴ Wasserstoff oder einen gegebenenfalls substituierten Kohlenwasserstoffrest oder einen gegebenenfalls substituierten Heterocyclylrest, wobei jeder der beiden letztgenannten kohlenstoffhaltigen Reste inklusive Substituenten 1 bis 30 C-Atome, vorzugsweise 1 bis 24 C-Atome, insbesondere 1 bis 20 C-Atome aufweist, bedeutet
oder
R⁴ einen Rest der Formel SiR^{a}R^{b}R^{c}, -NR^{a}R^{b} oder -N=CR^{c}R^{d} bedeutet, wobei in den letztgenannten 3 Formeln jeder der Reste R^{a}, R^{b}, R^{c} und R^{d} unabhängig voneinander Wasserstoff oder einen gegebenenfalls substituierten Kohlenwasserstoffrest bedeutet, dabei aber SiH₃ für SiR^{a}R^{b}R^{c} ausgenommen ist, oder R^{a} und R^{b} zusammen mit dem N-Atom der Gruppe -NR^{a}R^{b} einen 3- bis 9-gliedrigen Heterocyclus, welcher zusätzlich zum N-Atom ein oder zwei weitere Heteroringatome aus der Gruppe N, O und S enthalten kann und welcher unsubstituiert oder substituiert ist, bedeuten oder R^{c} und R^{d} zusammen mit dem C-Atom der Gruppe -N=CR^{c}R^{d} einen 3- bis 9-gliedrigen carbocyclischen Rest oder einen heterocyclischen Rest, welcher 1 bis 3 Heteroringatome aus der Gruppe N, O und S enthalten kann, wobei der carbocyclische oder heterocyclischen Rest unsubstituiert oder substituiert ist, bedeuten, wobei jeder der Reste R^{a}, R^{b}, R^{c} und R^{d} inklusive Substituenten bis 30 C-Atome, vorzugsweise bis 24 C-Atome, insbesondere bis 20 C-Atome aufweist,
oder
R⁴ einen Rest der Formel -C(=O)-R^{e} oder -P(=O)(R^{f})₂ bedeutet, wobei R^{e} und die R^{f} unabhängig voneinander jeweils Wasserstoff, OH, (C₁-C₈)Alkyl, (C₁-C₄)Haloalkyl, (C₂-C₈)Alkenyl, (C₂-C₈)Alkinyl, (C₁-C₆)Alkoxy, (C₁-C₆)Alkoxy-(C₁-C₈)alkyl, (C₁-C₄)Haloalkoxy, (C₁-C₄)Haloalkoxy-(C₁-C₈)alkyl, (C₃-C₈)Alkenyloxy, (C₃-C₈)Alkenyloxy-(C₁-C₈)alkyl, (C₃-C₈)Alkinyloxy, (C₃-C₈)Alkinyloxy-(C₁-C₈)alkyl, -NR*R**, wobei R* und R** weiter unten definiert sind, Tri-[(C₁-C₄)alkyl]-silyl, Tri-[(C₁-C₄)alkyl]-silyl-(C₁-C₈)alkyl, (C₃-C₆)Cycloalkyl, (C₃-C₆)Cycloalkyl-(C₁-C₈)alkyl, (C₅-C₆)Cycloalkenyl, (C₅-C₆)Cycloalkenyl-(C₁-C₈)alkyl, (C₅-C₆)Cycloalkinyl, (C₅-C₆)Cycloalkinyl-(C₁-C₈)alkyl, Phenyl, Phenyl-(C₁-C₈)alkyl, Phenoxy, Phenoxy-(C₁-C₈)alkyl, Phenylamino, Phenylamino-(C₁-C₈)alkyl, einen Rest Het⁷, Het⁷-(C₁-C₆)alkyl oder Het⁷-O-(C₁-C₆)alkyl bedeuten,
wobei jeder der letztgenannten 15 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist,
R⁵, R¹⁰ und R¹⁵ jeweils unabhängig voneinander Wasserstoff, (C₁-C₆)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die unten genannte Gruppe M, vorzugsweise (C₁-C₄)Alkyl bedeuten,
R⁶, R⁷, R⁸, R⁹, R¹¹, R¹², R¹³, R¹⁴, R¹⁶, R¹⁷, R¹⁸ und R¹⁹ jeweils unabhängig voneinander Wasserstoff, (C₁-C₆)Alkyl, (C₂-C₆)Alkenyl oder (C₂-C₆)Alkinyl, wobei jeder der letztgenannten 3 Reste jeweils unabhängig voneinander unsubstituiert oder durch einen oder mehrere Reste aus der Gruppe Halogen, Nitro, Cyano und Phenyl, das gegebenenfalls substituiert ist, vorzugweise unsubstitutiert oder durch einen oder mehrere Reste aus der Gruppe Halogen, Nitro, Cyano, (C₁-C₄)Alkyl und (C₁-C₄)Haloalkyl substituiert ist, substituiert ist, oder (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, Nitro, Cyano, (C₁-C₄)Alkyl, (C₁-C₄)Haloalkyl, Phenyl und Benzyl, wobei jeder der letztgenannten 2 Reste gegebenenfalls substituiert ist, vorzugsweise unsubstituiert oder durch einen oder mehrere Reste aus der Gruppe Halogen, Nitro, Cyano, (C₁-C₄)Alkyl und (C₁-C₄)Haloalkyl substituiert ist, substituiert ist, bedeuten,
Het¹, Het², Het³, Het⁴, Het⁵ und Het⁶ jeweils unabhängig voneinander einen gesättigten oder teilungesättigten Rest eines Heterocyclus mit 3 bis 9 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, (C₁-C₄)Alkoxy, (C₁-C₄)Haloalkoxy, (C₁-C₄)Alkylthio und Oxo substituiert ist,
vorzugsweise den Rest eines gesättigten Heterocyclus der genannten Art, insbesondere eine Morpholino- Piperidino- oder Pyrrolidinogruppe bedeuten,
Het⁷ jeweils unabhängig voneinander einen gesättigten, teilweise ungesättigten oder heteroaromatischen monocyclischen Heterocyclylrest mit 3 bis 9 Ringatomen oder einen 9- oder 10-gliedrigen bicyclischen Heterocyclus, jeweils enthaltend 1, 2, 3 oder 4 Heteroatome, ausgewählt aus der Gruppe bestehend aus O, N und S, bedeutet,
R*, R** jeweils unabhängig voneinander (unabhängig auch von anderen Resten NR*R**) H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus gegebenenfalls durch einen oder mehrere gleiche oder verschiedene Reste aus der Gruppe Halogen, (C₁-C₄)Alkyl, (C₁-C₄)Haloalkyl, (C₁-C₄)Alkoxy oder (C₁-C₄)Haloalkoxy oder im Fall gesättigter oder teilungesättigter cyclischer Basisgruppen auch Oxo substituiert ist, bedeuten oder
R* und R** zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten,
R^{A} Halogen, Cyano, Hydroxy oder (C₁-C₆)Alkoxy bedeutet,
R^{B} Halogen, Cyano, Hydroxy, Oxo, Nitro, (C₁-C₈)Alkyl, (C₁-C₆)Haloalkyl, Cyano-(C₁-C₆)alkyl, Hydroxy-(C₁-C₆)alkyl, Nitro-(C₁-C₆)alkyl, (C₂-C₈)Alkenyl, (C₂-C₈)Haloalkenyl, (C₂-C₈)Alkinyl, (C₂-C₈)Haloalkinyl, (C₁-C₈)Alkoxy, (C₂-C₈)Alkenyloxy, (C₂-C₈)Alkinyloxy, (C₁-C₈)Haloalkoxy, (C₁-C₆)Alkoxy-(C₁-C₆)alkyl, (C₁-C₆)Alkoxy-(C₁-C₄)alkoxy, (C₁-C₆)Haloalkoxy-(C₁-C₄)alkyl, (C₁-C₆)Haioaikoxy-(C₁-C₄)alkoxy, (C₁-C₈)Alkylthio, (C₂-C₆)Alkenylthio, (C₂-C₆)Alkinylthio, (C₁-C₈)Alkylsulfinyl , (C₁-C₆)Haloalkylsulfinyl, (C₁-C₈)Alkylsulfonyl, (C₁-C₆)Haloalkylsulfonyl, einen Rest der Formel R^{aa}-C(=O)-, R^{aa}-C(=O)-(C₁-C₆)alkyₗ, wobei die R^{aa} weiter unten definiert sind, -NR*R**, wobei R* und R** weiter unten definiert sind, Tri-[(C₁-C₄)alkyl]-silyl, Tri-[(C₁-C₄)alkyl]-silyl-(C₁-C₆)alkyl, (C₃-C₈)Cycloalkyl, (C₃-C₈)Cycloalkoxy, (C₃-C₈)Cycloalkyl-(C₁-C₈)alkyl, (C₃-C₈)Cycloalkyl-(C₁-C₈)alkoxy, Phenyl, Phenyl-(C₁-C₈)alkyl, Phenoxy, Phenoxy-(C₁-C₈)alkyl, Phenylamino, Phenylamino-(C₁-C₈)alkyl oder einen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, wobei jeder der letztgenannten 11 Reste im cyclischen Teil gegebenenfalls durch einen oder mehrere gleiche oder verschiedene Reste R^{bb} substituiert ist, bedeutet,
R^{aa} jeweils unabhängig voneinander Wasserstoff, OH, (C₁-C₈)Alkyl, (C₁-C₆)Haloalkyl, (C₂-C₈)Alkenyl, (C₂-C₈)Alkinyl, (C₁-C₈)Alkoxy, (C₁-C₆)Alkoxy-(C₁-C₆)alkyl, (C₁-C₆)Alkoxy-(C₁-C₆)alkyloxy, (C₁-C₆)Haloalkoxy, (C₁-C₆)Haloalkoxy-(C₁-C₆)alkyl, (C₁-C₆)Haloalkoxy-(C₁-C₆)alkoxy, (C₃-C₈)Alkenyloxy, (C₃-C₈)Alkenyloxy-(C₁-C₆)alkyl, (C₃-C₈)Alkenyloxy-(C₁-C₆)alkoxy, (C₃-C₈)Alkinyloxy, (C₃-C₈)Alkinyloxy-(C₁-C₆)alkyl, (C₃-C₈)Alkinyloxy-(C₁-C₆)alkoxy, -NR*R*, wobei R* und R** wie oben definiert sind, Tri-[(C₁-C₄)alkyl]-silyl, Tri-[(C₁-C₄)alkyl]-silyl-(C₁-C₆)alkyl, Tri-[(C₁-C₄)alkyl]-silyl-(C₁-C₆)alkoxy, (C₃-C₈)Cycloalkyl, (C₃-C₈)Cycloalkoxy, (C₃-C₈)Cycloalkyl-(C₁-C₈)alkyl, (C₃-C₈)Cycloalkyl-(C₁-C₈)alkoxy, (C₅-C₈)Cycloalkenyl, (C₅-C₈)Cycloalkenyl-(C₁-C₆)alkyl, (C₅-C₈)Cycloalkenyloxy, (C₅-C₈)Cycloalkinyl, (C₅-C₈)Cycloalkinyl-(C₁-C₆)alkyl, (C₅-C₈)Cycloalkinyl-(C₁-C₆)alkoxy, Phenyl, Phenyl-(C₁-C₈)alkyl, Phenyl-(C₁-C₈)alkoxy, Phenoxy, Phenoxy-(C₁-C₈)alkyl, Phenoxy-(C₁-C₈)alkoxy, Phenylamino, Phenylamino-(C₁-C₈)alkyl, Phenylamino-(C₁-C₈)alkoxy oder einen gegebenenfalls über eine Alkylengruppe oder eine Alkoxygruppe gebundenen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, bedeutet, wobei jeder der letztgenannten 20 Reste im cyclischen Teil gegebenenfalls durch einen oder mehrere gleiche oder verschiedene Reste R^{cc} substituiert ist, bedeutet und
R^{bb} und R^{cc} jeweils unabhängig voneinander Halogen, (C₁-C₄)Alkyl, (C₁-C₄)Haloalkyl, (C₁-C₄)Alkoxy oder (C₁-C₄)Haloalkoxy oder im Fall gesättigter oder teilungesättigter cyclischer Basisgruppen auch Oxo bedeutet und
M ein Äquivalent eines Kations,
vorzugsweise ein Metallionenäquivalent, ein Ammoniumion, dass gegebenenfalls durch 1 bis 4 gleiche oder verschiedene Reste aus der Gruppe (C₁-C₆)Alkyl, (C₂-C₆)Alkenyl, (C₂-C₆)Alkinyl, (C₃-C₆)Cycloalkyl, (C₅-C₆)Cycloalkenyl, (C₅-C₆)Cycloalkinyl, Phenyl, (C₃-C₆)Cycloalkyl-(C₁-C₄)alkyl, (C₅-C₆)Cycloalkenyl-(C₁-C₄)alkyl, Phenyl(C₁-C₄)alkyl substituiert ist, oder ein tertiäres Sulfoniumion, das vorzugsweise durch 3 gleiche oder verschiedene Reste aus der Gruppe (C₁-C₆)Alkyl, (C₂-C₆)Alkenyl, (C₂-C₆)Alkinyl, (C₃-C₆)Cycloalkyl, (C₅-C₆)Cycloalkenyl, (C₅-C₆)Cycloalkinyl, Phenyl, (C₃-C₆)Cycloalkyl-(C₁-C₄)alkyl, (C₅-C₆)Cycloalkenyl-(C₁-C₄)alkyl, Phenyl(C₁-C₄)alkyl, insbesondere (C₁-C₄)Alkyl substituiert ist, bedeutet,
m 0, 1, 2 oder 3, vorzugsweise 0, 1 oder 2, insbesondere 0 oder 1, und
n 0, 1, 2, 3, 4 oder 5, vorzugsweise 0, 1, 2, oder 3 bedeuten.

2. Verbindungen oder deren Salze gemäß Anspruch 1, **dadurch gekennzeichnet, dass**
R⁴ Wasserstoff oder einen gegebenenfalls substituierten Kohlenwasserstoffrest oder einen gegebenenfalls substituierten Heterocyclylrest, wobei jeder der beiden letztgenannten kohlenstoffhaltigen Reste inklusive Substituenten 1 bis 30 C-Atome, bedeutet
oder
R⁴ einen Rest der Formel SiR^{a}R^{b}R^{c}, -NR^{a}R^{b} oder -N=CR^{c}R^{d} bedeutet,
wobei in den letztgenannten 3 Formeln jeder der Reste R^{a}, R^{b}, R^{c} und R^{d} unabhängig voneinander Wasserstoff oder einen gegebenenfalls substituierten Kohlenwasserstoffrest bedeutet, dabei aber SiH₃ für SiR^{a}R^{b}R^{c} ausgenommen ist, oder R^{a} und R^{b} zusammen mit dem N-Atom der Gruppe -NR^{a}R^{b} einen 3- bis 9-gliedrigen Heterocyclus, welcher zusätzlich zum N-Atom ein oder zwei weitere Heteroringatome aus der Gruppe N, O und S enthalten kann und welcher unsubstituiert oder substituiert ist, bedeuten oder R^{c} und R^{d} zusammen mit dem C-Atom der Gruppe -N=CR^{c}R^{d} einen 3- bis 9-gliedrigen carbocyclischen Rest oder einen heterocyclischen Rest, welcher 1 bis 3 Heteroringatome aus der Gruppe N, O und S enthalten kann, wobei der carbocyclische oder heterocyclischen Rest unsubstituiert oder substituiert ist, bedeuten,
wobei jeder der Reste R^{a}, R^{b}, R^{c} und R^{d} inklusive Substituenten bis 30 C-Atome aufweist,
oder
R⁴ einen Rest der Formel -C(=O)-R^{e} oder -P(=O)(R^{f})₂ bedeutet, wobei R^{e} und die
R^{f} unabhängig voneinander jeweils Wasserstoff, OH, (C₁-C₈)Alkyl, (C₁-C₄)Haloalkyl, (C₂-C₈)Alkenyl, (C₂-C₈)Alkinyl, (C₁-C₆)Alkoxy, (C₁-C₆)Alkoxy-(C₁-C₈)alkyl, (C₁-C₄)Haloalkoxy, (C₁-C₄)Haloalkoxy-(C₁-C₈)alkyl, (C₃-C₈)Alkenyloxy, (C₃-C₈)Alkenyloxy-(C₁-C₈)alkyl, (C₃-C₈)Alkinyloxy, (C₃-C₈)Alkinyloxy-(C₁-C₈)alkyl, -NR*R**, wobei R* und R** weiter unten definiert sind, Tri-[(C₁-C₄)alkyl]-silyl, Tri-[(C₁-C₄)alkyl]-silyl-(C₁-C₈)alkyl, (C₃-C₆)Cycloalkyl, (C₃-C₆)Cycloalkyl-(C₁-C₈)alkyl, (C₅-C₆)Cycloalkenyl, (C₅-C₆)Cycloalkenyl-(C₁-C₈)alkyl, (C₅-C₆)Cycloalkinyl, (C₅-C₆)Cycloalkinyl-(C₁-C₈)alkyl, Phenyl, Phenyl-(C₁-C₈)alkyl, Phenoxy, Phenoxy-(C₁-C₈)alkyl, Phenylamino, Phenylamino-(C₁-C₈)alkyl, ein Rest Het⁷, Het⁷-(C₁-C₆)alkyl oder Het⁷-O-(C₁-C₆)alkyl bedeuten,
wobei jeder der letztgenannten 15 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist,
Het⁷ jeweils unabhängig voneinander einen gesättigten, teilweise ungesättigten oder heteroaromatischen monocyclischen Heterocyclylrest mit 3 bis 9 Ringatomen oder einen 9- oder 10-gliedrigen bicyclischen Heterocyclus, jeweils enthaltend 1, 2, 3 oder 4 Heteroatome, ausgewählt aus der Gruppe bestehend aus O, N und S, bedeutet,
R*, R** jeweils unabhängig voneinander (unabhängig auch von anderen Resten NR*R**) H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus gegebenenfalls durch einen oder mehrere gleiche oder verschiedene Reste aus der Gruppe Halogen, (C₁-C₄)Alkyl, (C₁-C₄)Haloalkyl, (C₁-C₄)Alkoxy oder (C₁-C₄)Haloalkoxy oder im Fall gesättigter oder teilungesättigter cyclischer Basisgruppen auch Oxo substituiert ist, bedeuten oder
R* und R** zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten,
R^{A} Halogen, Cyano, Hydroxy oder (C₁-C₆)Alkoxy bedeutet,
R^{B} Halogen, Cyano, Hydroxy, Oxo, Nitro, (C₁-C₈)Alkyl, (C₁-C₆)Haloalkyl, Cyano-(C₁-C₆)alkyl, Hydroxy-(C₁-C₆)alkyl, Nitro-(C₁-C₆)alkyl, (C₂-C₈)Alkenyl, (C₂-C₈)Haloalkenyl, (C₂-C₈)Alkinyl, (C₂-C₈)Haloalkinyl, (C₁-C₈)Alkoxy, (C₂-C₈)Alkenyloxy, (C₂-C₈)Alkinyloxy, (C₁-C₈)Haloalkoxy, (C₁-C₆)Alkoxy-(C₁-C₆)alkyl, (C₁-C₆)Alkoxy-(C₁-C₄)alkoxy, (C₁-C₆)Haloalkoxy-(C₁-C₄)alkyl, (C₁-C₆)Haloalkoxy-(C₁-C₄)alkoxy, (C₁-C₈)Alkylthio, (C₂-C₆)Alkenylthio, (C₂-C₆)Alkinylthio, (C₁-C₈)Alkylsulfinyl , (C₁-C₆)Haloalkylsulfinyl, (C₁-C₈)Alkylsulfonyl, (C₁-C₆)Haloalkylsulfonyl, ein Rest der Formel R^{aa}-C(=O)-, R^{aa}-C(=O)-(C₁-C₆)alkyl, wobei die R^{aa} weiter unten definiert sind, -NR*R**,
wobei R* und R** weiter unten definiert sind, Tri-[(C₁-C₄)alkyl]-silyl, Tri-[(C₁-C₄)alkyl]-silyl-(C₁-C₆)alkyl, (C₃-C₈)Cycloalkyl, (C₃-C₈)Cycloalkoxy, (C₃-C₈)Cycloalkyl-(C₁-C₈)alkyl, (C₃-C₈)Cycloalkyl-(C₁-C₈)alkoxy, Phenyl, Phenyl-(C₁-C₈)alkyl, Phenoxy, Phenoxy-(C₁-C₈)alkyl, Phenylamino, Phenylamino-(C₁-C₈)alkyl oder einen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, wobei jeder der letztgenannten 11 Reste im cyclischen Teil gegebenenfalls durch einen oder mehrere gleiche oder verschiedene Reste R^{bb} substituiert ist, substituiert ist, bedeutet,
R^{aa} jeweils unabhängig voneinander Wasserstoff, OH, (C₁-C₈)Alkyl, (C₁-C₆)Haloalkyl, (C₂-C₈)Alkenyl, (C₂-C₈)Alkinyl, (C₁-C₈)Alkoxy, (C₁-C₆)Alkoxy-(C₁-C₆)alkyl, (C₁-C₆)Alkoxy-(C₁-C₆)alkyloxy, (C₁-C₆)Haloalkoxy, (C₁-C₆)Haloalkoxy-(C₁-C₆)alkyl, (C₁-C₆)Haloalkoxy-(C₁-C₆)alkoxy, (C₃-C₈)Alkenyloxy, (C₃-C₈)Alkenyloxy-(C₁-C₆)alkyl, (C₃-C₈)Alkenyloxy-(C₁-C₆)alkoxy, (C₃-C₈)Alkinyloxy, (C₃-C₈)Alkinyloxy-(C₁-C₆)alkyl, (C₃-C₈)Alkinyloxy-(C₁-C₆)alkoxy, -NR*R*, wobei R* und R** wie oben definiert sind, Tri-[(C₁-C₄)alkyl]-silyl, Tri-[(C₁-C₄)alkyl]-silyl-(C₁-C₆)alkyl, Tri-[(C₁-C₄)alkyl]-silyl-(C₁-C₆)alkoxy, (C₃-C₈)Cycloalkyl, (C₃-C₈)Cycloalkoxy, (C₃-C₈)Cycloalkyl-(C₁-C₈)alkyl, (C₃-C₈)Cycloalkyl-(C₁-C₈)alkoxy, (C₅-C₈)Cycloalkenyl, (C₅-C₈)Cycloalkenyl-(C₁-C₆)alkyl, (C₅-C₈)Cycloalkenyloxy, (C₅-C₈)Cycloalkinyl, (C₅-C₈)Cycloalkinyl-(C₁-C₆)alkyl, (C₅-C₈)Cycloalkinyl-(C₁-C₆)alkoxy, Phenyl, Phenyl-(C₁-C₈)alkyl, Phenyl-(C₁-C₈)alkoxy, Phenoxy, Phenoxy-(C₁-C₈)alkyl, Phenoxy-(C₁-C₈)alkoxy, Phenylamino, Phenylamino-(C₁-C₈)alkyl, Phenylamino-(C₁-C₈)alkoxy oder einen gegebenenfalls über eine Alkylengruppe oder eine Alkoxygruppe gebundenen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, bedeutet, wobei jeder der letztgenannten 20 Reste im cyclischen Teil gegebenenfalls durch einen oder mehrere gleiche oder verschiedene Reste R^{cc} substituiert ist, bedeutet und
R^{bb} und R^{cc} jeweils unabhängig voneinander Halogen, (C₁-C₄)Alkyl, (C₁-C₄)Haloalkyl, (C₁-C₄)Alkoxy oder (C₁-C₄)Haloalkoxy oder im Fall gesättigter oder teilungesättigter cyclischer Basisgruppen auch Oxo bedeutet,
bedeutet.

3. Verbindungen oder deren Salze gemäß Anspruch 1 oder 2, **dadurch gekennzeichnet, dass**
(R¹)ₘ m Substituenten R¹ bedeutet,
wobei R¹, wenn m = 1, oder jeder der Substituenten R¹, wenn m größer als 1, unabhängig voneinander Halogen, Cyano, Nitro, Hydroxy, (C₁-C₆)Alkyl, (C₂-C₄)Alkenyl, (C₂-C₄)Alkinyl, (C₁-C₆)Alkoxy, (C₁-C₆)Alkylthio, (C₁-C₆)Alkylsulfinyl, (C₁-C₆)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Haloalkenyl, (C₂-C₄)Haloalkinyl, (C₁-C₄)Alkoxy-(C₁-C₄)alkyl, (C₁-C₄)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR⁵, C(O)NR⁶R⁷, C(O)-Het¹, NR⁸R⁹ oder Het² bedeutet oder wobei jeweils zwei am Ring ortho-ständige Gruppen R¹ oder R¹ und R³ gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht,
wobei die Gruppe -Z¹-A*-Z² zusammen mit den an die Gruppe gebundenen C-Atomen des Phenylrings einen ankondensierten 5 oder 6 Ring bilden,
R⁵ Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die Gruppe M bedeutet,
R⁶, R⁷, R⁸ und R⁹ jeweils unabhängig voneinander Wasserstoff oder (C₁-C₄)Alkyl, das unsubstituiert oder durch einen oder mehrere Reste aus der Gruppe Halogen, Nitro, Cyano und Phenyl substituiert ist, oder
(C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, (C₁-C₄)Alkyl, (C₁-C₄)Haloalkyl, Phenyl und Benzyl substituiert ist,
Het¹ und Het² jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, und Oxo substituiert ist, bedeuten
M das Äquivalent eines Kations bedeutet und
m 0, 1, 2 oder 3 bedeutet.

4. Verbindungen oder deren Salze gemäß einem der Ansprüche 1 bis 3, **dadurch gekennzeichnet, dass**
(R²)ₙ n Substituenten R² bedeutet,
wobei R², wenn n = 1, oder jeder der Substituenten R², wenn n größer als 1 ist, unabhängig voneinander Halogen, Cyano, Nitro, Hydroxy, (C₁-C₆)Alkyl, (C₂-C₄)Alkenyl, (C₂-C₄)Alkinyl, (C₁-C₆)Alkoxy, (C₁-C₆)Alkylthio, (C₁-C₆)Alkylsulfinyl, (C₁-C₆)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Haloalkenyl, (C₂-C₄)Haloalkinyl, (C₁-C₄)Alkoxy-(C₁-C₄)alkyl, (C₁-C₄)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR¹⁰, C(O)NR¹¹R¹², C(O)-Het³, NR¹³R¹⁴ oder Het⁴ bedeutet oder wobei jeweils zwei am Ring ortho-ständige Gruppen R² gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴ zusammen mit den an die Gruppe gebundenen C-Atomen des Phenylrings einen ankondensierten 5 oder 6 Ring bilden,
R¹⁰ Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die Gruppe M bedeutet,
R¹¹, R¹², R¹³ und R¹⁴ jeweils unabhängig voneinander Wasserstoff oder (C₁-C₄)Alkyl, das unsubstituiert oder durch einen oder mehrere Reste aus der Gruppe Halogen, Nitro, Cyano und Phenyl substituiert ist, oder
(C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, (C₁-C₄)Alkyl, (C₁-C₄)Haloalkyl, Phenyl und Benzyl substituiert ist, bedeuten,
Het³ und Het⁴ jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl und Oxo substituiert ist, bedeuten,
M das Äquivalent eines Kations bedeutet und
n 0, 1, 2, 3, 4 oder 5 bedeutet.

5. Verbindungen oder deren Salze gemäß einem der Ansprüche 1 bis 4, **dadurch gekennzeichnet, dass**
R³ Halogen, Cyano, Nitro, Hydroxy, (C₁-C₆)Alkyl, (C₂-C₄)Alkenyl, (C₂-C₄)Alkinyl, (C₁-C₆)Alkoxy, (C₁-C₆)Alkylthio, (C₁-C₆)Alkylsulfinyl, (C₁-C₆)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Haloalkenyl, (C₂-C₄)Haloalkinyl, (C₁-C₄)Alkoxy-(C₁-C₄)alkyl, (C₁-C₄)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR¹⁵, C(O)NR¹⁶R¹⁷, C(O)-Het⁵, NR¹⁸R¹⁹ oder Het⁶ bedeutet,
R¹⁵ Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die genannte Gruppe M bedeutet, vorzugsweise Wasserstoff, (C₁-C₄)Alkyl oder die Gruppe M bedeutet,
R¹⁶, R¹⁷ , R¹⁸ und R¹⁹ jeweils unabhängig voneinander Wasserstoff oder (C₁-C₄)Alkyl, das unsubstituiert oder durch einen oder mehrere Reste aus der Gruppe Halogen, Nitro, Cyano und Phenyl substituiert ist, oder
(C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, (C₁-C₄)Alkyl, (C₁-C₄)Haloalkyl, Phenyl und Benzyl substituiert ist, bedeuten,
Het⁵ und Het⁶ jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, und Oxo substituiert ist, bedeuten und
M das Äquivalent eines Kations bedeutet.

6. Verbindungen oder deren Salze gemäß einem der Ansprüche 1 bis 5, **dadurch gekennzeichnet, dass**
(R¹)ₘ m Substituenten R¹ bedeutet,
wobei R¹, wenn m = 1, oder jeder der Substituenten R¹, wenn m größer als 1, unabhängig voneinander Halogen, Cyano, Nitro, Hydroxy, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₁-C₄)Alkoxy-(C₁-C₄)alkyl, (C₃-C₆)Cycloalkyl, oder einen Rest der Formel C(O)OR⁵, C(O)NR⁶R⁷, C(O)-Het¹, NR⁸R⁹ oder Het² bedeutet,
oder wobei jeweils zwei am Ring ortho-ständige Gruppen R¹ oder R¹ und R³ gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht,
wobei die Gruppe -Z¹-A*-Z² zusammen mit den an die Gruppe gebundenen C-Atomen des Phenylrings einen ankondensierten 5 oder 6 Ring bilden,
R⁵ Wasserstoff, (C₁-C₄)Alkyl oder die genannte Gruppe M bedeutet,
R⁶, R⁷, R⁸ und R⁹ jeweils unabhängig voneinander Wasserstoff, (C₁-C₄)Alkyl, (C₁-C₄)Haloalkyl, Benzyl, (C₃-C₆)Cycloalkyl oder Phenyl bedeuten,
Het¹ und Het² jeweils unabhängig voneinandereine Morpholino- Piperidino- oder Pyrrolidinogruppe bedeuten und
m 0, 1, 2 oder 3 bedeutet,
(R²)ₙ n Substituenten R² bedeutet,
wobei R², wenn n = 1, oder jeder der Substituenten R², wenn n größer als 1 ist, unabhängig voneinander Halogen, Cyano, Nitro, Hydroxy, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₁-C₄)Alkoxy-(C₁-C₄)alkyl, (C₃-C₆)Cycloalkyl oder einen Rest der Formel C(O)OR¹⁰, C(O)NR¹¹R¹², C(O)-Het³, NR¹³R¹⁴ oder Het⁴ bedeutet
oder wobei jeweils zwei am Ring ortho-ständige Gruppen R² gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴ zusammen mit den an die Gruppe gebundenen C-Atomen des Phenylrings einen ankondensierten 5 oder 6 Ring bilden,
R¹⁰ Wasserstoff, (C₁-C₄)Alkyl oder die genannte Gruppe M bedeutet,
R¹¹, R¹² , R¹³ und R¹⁴ jeweils unabhängig voneinander Wasserstoff oder (C₁-C₄)Alkyl, (C₁-C₄)Haloalkyl, Benzyl, (C₃-C₆)Cycloalkyl oder Phenyl bedeuten,
Het³ und Het⁴ jeweils unabhängig voneinander eine Morpholino- Piperidino- oder Pyrrolidinogruppe bedeuten und
n 0, 1, 2, 3, 4 oder 5, bedeutet,
R³ Halogen, Cyano, Nitro, Hydroxy, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₁-C₄)Alkoxy-(C₁-C₄)alkyl, (C₃-C₆)Cycloalkyl oder einen Rest der Formel C(O)OR¹⁵, C(O)NR¹⁶R¹⁷, C(O)-Het⁵, NR¹⁸R¹⁹ oder Het⁶ bedeutet,
R¹⁵ Wasserstoff, (C₁-C₄)Alkyl oder die genannte Gruppe M bedeutet,
R¹⁶, R¹⁷, R¹⁸ und R¹⁹ jeweils unabhängig voneinander Wasserstoff, (C₁-C₄)Alkyl, (C₁-C₄)Haloalkyl, Benzyl, (C₃-C₆)Cycloalkyl oder Phenyl bedeuten,
Het⁵ und Het⁶ jeweils unabhängig voneinandereine Morpholino- Piperidino- oder Pyrrolidinogruppe bedeuten und
M das Äquivalent eines Kations bedeutet.

7. Verfahren zur Herstellung einer Verbindung der Formel (I), wie sie gemäß einem der Ansprüche 1 bis 6 definiert ist, oder deren Salz, **dadurch gekennzeichnet, dass** man
Beispielsweise werden Diastereomerengemische der Verbindungen der Formel (I)] mit einem Gehalt an der herzustellenden Verbindung (I) erhalten, **dadurch gekennzeichnet, dass** man
(a) für den Fall der Herstellung von Diastereomerengemischen der Verbindungen der Formel (I) Verbindungen der Formel (II) 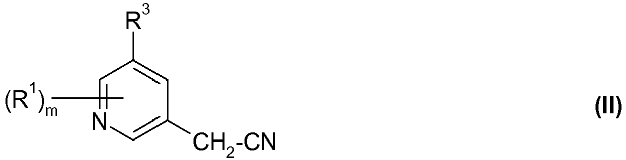 mit Verbindungen der Formel (III) oder deren Salzen, 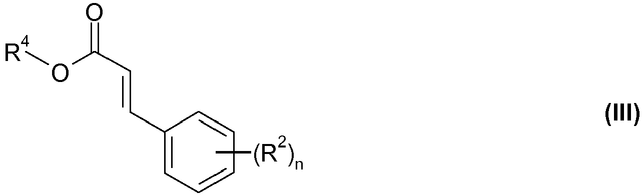 zu Verbindungen der Formel (I) 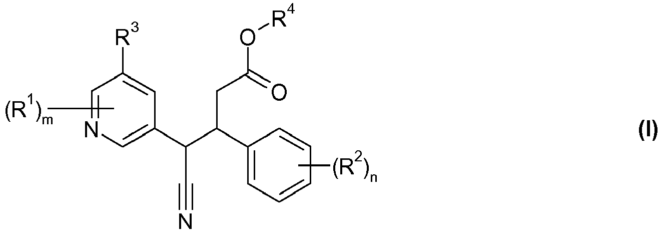 umsetzt,
wobei R¹, R², R³, R⁴, m und n in den Verbindungen (II) und (III) wie in der jeweils herzustellenden Verbindung der Formel (I) definiert sind,
(b) Verbindungen der Formel (I*), 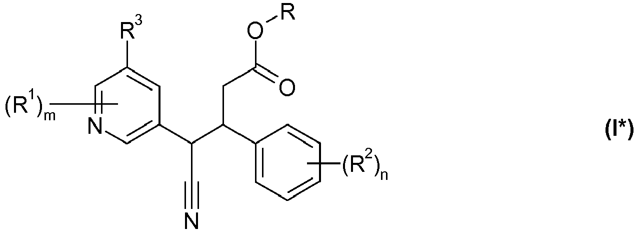 in denen R ein Rest aus der Gruppe der für R⁴ möglichen Reste bedeutet aber vom Rest R⁴ in der herzustellenden Verbindung (I) verschieden ist, mit einer Verbindung der Formel R⁴-OH, in der R⁴ wie in Formel (I) definiert ist, zur Verbindung (I) umsetzt, wobei R¹, R², R³, m und n in der Verbindung (I*) wie in der jeweils herzustellenden Verbindung der Formel (I) definiert sind.
(c) Verbindungen der Formel (I*) (siehe b), worin R ein Wasserstoffatom darstellt, mit einer Verbindung der Formel R⁴-OH, in der R⁴ wie in Formel (I) definiert ist, zur Verbindung (I) umsetzt, oder
(d) für den Fall, dass optisch aktive Verbindungen einer diastereomeren Form der Verbindung der Formel (I) hergestellt werden, mit den racemischen Gemisch der diastereomeren Form der Verbindung der Formel (I) eine Racemattrennung durchführt und das gewünschte Enantiomer in einer stereochemischen Reinheit von 60 bis 100 %, vorzugsweise 70 bis 100 %, mehr bevorzugt 80 bis 100 % , insbesondere 90 bis 100 %, bezogen auf das vorliegende Gemisch der Erythro- bzw. Threo-Enantiomeren isoliert.

8. Herbizides oder pflanzenwachstumsregulierendes Mittel, **dadurch gekennzeichnet, dass** es eine oder mehrere Verbindungen der Formel (I) oder deren Salze, wie sie nach einem der Ansprüche 1 bis 6 definiert sind, und im Pflanzenschutz übliche Formulierungshilfsmittel enthält.

9. Verfahren zur Bekämpfung von Schadpflanzen oder zur Wachstumsregulierung von Pflanzen, **dadurch gekennzeichnet, dass** man eine wirksame Menge von einer oder mehreren Verbindungen der Formel (I) oder deren Salzen, wie sie nach einem der Ansprüche 1 bis 6 definiert sind, auf die Pflanzen, Pflanzensamen, den Boden, in dem oder auf dem die Pflanzen wachsen, oder die Anbaufläche appliziert.

10. Verfahren nach Anspruch 9, **dadurch gekennzeichnet, dass** die Verbindungen der Formel (I) oder deren Salze zur selektiven Bekämpfung von Schadpflanzen oder zur Wachstumsregulierung in Kulturen von Nutz- oder Zierpflanzen eingesetzt werden.

11. Verwendung der Verbindungen der Formel (I) oder deren Salze gemäß einem Ansprüche 1 bis 6 als Herbizide oder Pflanzenwachstumsregulatoren.

## Claims

1. Compounds of the formula (I) or salts thereof 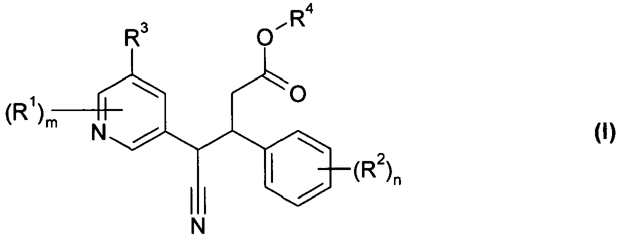 in which
(R¹)ₘ represents m substituents R¹,
where R¹, if m = 1, or each of the substituents R¹, if m is greater than 1, independently of the others represents halogen, cyano, nitro, hydroxy, (C₁-C₈)-alkyl, (C₂-C₆)-alkenyl, (C₂-C₆)-alkynyl, (C₁-C₈)-alkoxy, (C₁-C₈)-alkylthio, (C₁-C₈)-alkylsulphinyl, (C₁-C₈)-alkylsulphonyl, (C₁-C₆)-haloalkyl, (C₁-C₆)-haloalkoxy, (C₁-C₆)-haloalkylthio, (C₁-C₆)-haloalkylsulphinyl, (C₁-C₆)-haloalkylsulphonyl, (C₂-C₆)-haloalkenyl, (C₂-C₆)-haloalkynyl, (C₁-C₆)-alkoxy- (C₁-C₄)-alkyl, (C₁-C₆)-haloalkoxy-(C₁-C₄)-alkyl, (C₃-C₆) -cycloalkyl which is optionally substituted by one or more radicals from the group consisting of halogen and (C₁-C₄)-alkyl, (C₃-C₆)-cycloalkoxy which is optionally substituted by one or more radicals from the group consisting of halogen and (C₁-C₄)-alkyl, or a radical of the formula C(O)OR⁵, C(O)NR⁶R⁷, C(O)-Het¹, NR⁸R⁹ or Het²
or where in each case two groups R¹ located ortho at the ring or R¹ and R³ together represent a group of the formula -Z¹-A*-Z² in which
A* represents an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-**C₄**)-haloalkoxy,
Z¹ represents a direct bond, O or S and
Z² represents a direct bond, O or S,
where the group -Z¹-A*-Z² together with the carbon atoms, attached to the group, of the phenyl ring form a fused-on 5- or 6-membered ring,
(R²)ₙ represents n substituents R²,
where R², if n = 1, or each of the substituents R², if n is greater than 1, independently of the others represents halogen, cyano, nitro, hydroxy, (C₁-C₈)-alkyl, (C₂-C₆)-alkenyl, (C₂-C₆)-alkynyl, (C₁-C₈)-alkoxy, (C₁-C₈)-alkylthio, (C₁-C₈)-alkylsulphinyl, (C₁-C₈)-alkylsulphonyl, (C₁-C₆)-haloalkyl, (C₁-C₆)-haloalkoxy, (C₁-C₆)-haloalkylthio, (C₁-C₆)-haloalkylsulphinyl, (C₁-C₆)-haloalkylsulphonyl, (C₂-C₆)-haloalkenyl, (C₂-C₆)-haloalkynyl, (C₁-C₆) -alkoxy- (C₁-C₄) -alkyl, (C₁-C₆)-haloalkoxy-(C₁-C₄)-alkyl, (C₃-C₆)-cycloalkyl which is optionally substituted by one or more radicals from the group consisting of halogen and (C₁-C₄)-alkyl, (C₃-C₆)-cycloalkoxy which is optionally substituted by one or more radicals from the group consisting of halogen and (C₁-C₄)-alkyl, or a radical of the formula C(O)OR¹⁰, C(O)NR¹¹R¹², C(O)-Het³, NR¹³R¹⁴ or Het⁴
or where in each case two groups R² located ortho at the ring together are a group of the formula - Z³-A**-Z⁴ in which
A** represents an alkylene group having 1 to 4 carbon atoms which is optionally substituted by one or more radicals from the group consisting of halogen, (C₁-C₄)-alkyl, (C₁-C₄)-haloalkyl, (C₁**-**C₄)-alkoxy and (C₁**-**C₄)-haloalkoxy,
Z³ represents a direct bond, O or S and
Z⁴ represents a direct bond, O or S,
where the group -Z³-A**-Z⁴ together with the carbon atoms, attached to the group, of the phenyl ring form a fused-on 5- or 6-membered ring,
R³ represents halogen, cyano, nitro, hydroxy, (C₁-C₈)-alkyl, (C₂-C₆) -alkenyl, (C₂-C₆) -alkynyl, (C₁-C₈)-alkoxy, (C₁-C₈)-alkylthio, (C₁-C₈)-alkylsulphinyl, (C₁-C₈)-alkylsulphonyl, (C₁-C₆)-haloalkyl, (C₁-C₆)-haloalkoxy, (C₁-C₆)-haloalkylthio, (C₁-C₆)-haloalkylsulphinyl, (C₁-C₆)-haloalkylsulphonyl, (C₂-C₆)-haloalkenyl, (C₂-C₆)-haloalkynyl, (C₁-C₆)-alkoxy- (C₁-C₄)-alkyl, (C₁-C₆)-haloalkoxy- (C₁-C₄)-alkyl, (C₃-C₆)-cycloalkyl which is optionally substituted by one or more radicals from the group consisting of halogen and (C₁-C₄) -alkyl, (C₃-C₆)-cycloalkoxy which is optionally substituted by one or more radicals from the group consisting of halogen and (C₁-C₄)-alkyl, or a radical of the formula C(O)OR¹⁵, C(O)NR¹⁶R¹⁷, C(O)-Het⁵, NR¹⁸R¹⁹ or Het⁶
R⁴ represents hydrogen or a hydrolyzable radical, preferably
R⁴ represents hydrogen or an optionally substituted hydrocarbon radical or an optionally substituted heterocyclyl radical, where each of the two last-mentioned carbon-containing radicals including substituents has 1 to 30 carbon atoms, preferably 1 to 24 carbon atoms, in particular 1 to 20 carbon atoms,
or
R⁴ represents a radical of the formula SiR^{a}R^{b}R^{c}, -NR^{a}R^{b} or -N=CR^{c}R^{d},
where in the 3 last-mentioned formulae each of the radicals R^{a}, R^{b}, R^{c} and R^{d} independently of the others represents hydrogen or an optionally substituted hydrocarbon radical, where, however, SiH₃ for SiR^{a}R^{b}R^{c} is excluded, or R^{a} and R^{b} together with the nitrogen atom of the group -NR^{a}R^{b} represent a 3- to 9-membered heterocycle which, in addition to the nitrogen atom, may contain one or two further ring heteroatoms from the group consisting of N, O and S and which is unsubstituted or substituted, or R^{c} and R^{d} together with the carbon atom of the group -N=CR^{c}R^{d} represent a 3- to 9-membered carbocyclic radical or a heterocyclic radical which may contain 1 to 3 ring heteroatoms from the group consisting of N, O and S, where the carbocyclic or heterocyclic radical is unsubstituted or substituted,
where each of the radicals R^{a}, R^{b}, R^{c} and R^{d} including substituents has up to 30 carbon atoms, preferably up to 24 carbon atoms, in particular up to 20 carbon atoms, or
R⁴ represents a radical of the formula -C(=O)-R^{e} or -P(=O) (R^{f})₂, where R^{e} and the radicals R^{f} independently of one another each represent hydrogen, OH, (C₁-C₈)-alkyl, (C₁-C₄)-haloalkyl, (C₂-C₈) -alkenyl, (C₂-C₈) -alkynyl, (C₁-C₆)-alkoxy, (C₁-C₆)-alkoxy- (C₁-C₈)-alkyl, (C₁-C₄)-haloalkoxy, (C₁-C₄)-haloalkoxy-(C₁-C₈)-alkyl, (C₃-C₈) -alkenyloxy, (C₃-C₈) -alkenyloxy- (C₁-C₈)-alkyl, (C₃-C₈) -alkynyloxy, (C₃-C₈)-alkynyloxy-(C₁-C₈)-alkyl, -NR*R**, with R* and R** being defined below, tri-[(C₁-C₄)-alkyl]silyl, tri-[(C₁-C₄)-alkyl] silyl- (C₁-C₈)alkyl, (C₃-C₆)-cycloalkyl, (C₃-C₆)-cycloalkyl-(C₁-C₈)-alkyl, (C₅-C₆)-cycloalkenyl, (C₅-C₆)-cycloalkenyl-(C₁-C₈)-alkyl, (C₅-C₆) -cycloalkynyl, (C₅-C₆)-cycloalkynyl-(C₁-C₈)-alkyl, phenyl, phenyl-(C₁-C₈)-alkyl, phenoxy, phenoxy-(C₁-C₈)-alkyl, phenylamino, phenylamino-(C₁-C₈)-alkyl, a radical Het⁷, Het⁷- (C₁-C₆)-alkyl or Het⁷O-(C₁-C₆)-alkyl,
where each of the 15 last-mentioned radicals is unsubstituted in the acyclic moiety or substituted by one or more identical or different radicals R^{A} and is unsubstituted in the cyclic moiety or substituted by one or more identical or different radicals R^{B},
R⁵, R¹⁰ and R¹⁵ independently of one another each represent hydrogen, (C₁-C₆)-alkyl, (C₁-C₄)-haloalkyl, (C₃-C₆)-cycloalkyl, (C₃-C₆)-halocycloalkyl, (C₂-C₄)-alkenyl, (C₂-C₄)-haloalkenyl, (C₂-C₄)-alkynyl or the group M mentioned below, preferably (C₁-C₄)-alkyl,
R⁶, R⁷, R⁸, R⁹, R¹¹, R¹², R¹³, R¹⁴, R¹⁶, R¹⁷, R¹⁸ and R¹⁹ independently of one another each represent hydrogen, (C₁-C₆)-alkyl, (C₂-C₆)-alkenyl or (C₂-C₆)-alkynyl, where each of the 3 last-mentioned radicals independently of the others is unsubstituted or substituted by one or more radicals from the group consisting of halogen, nitro, cyano and phenyl which is optionally substituted, preferably unsubstituted or substituted by one or more radicals from the group consisting of halogen, nitro, cyano, (C₁-C₄)-alkyl and (C₁-C₄)-haloalkyl, or
(C₃-C₆)-cycloalkyl or phenyl, where each of the 2 last-mentioned radicals in each case independently of the other is unsubstituted or substituted by one or more radicals from the group consisting of halogen, nitro, cyano, (C₁-C₄)-alkyl, (C₁-C₄)-haloalkyl, phenyl and benzyl, where each of the 2 last-mentioned radicals is optionally substituted, preferably unsubstituted or substituted by one or more radicals from the group consisting of halogen, nitro, cyano, (C₁-C₄)-alkyl and (C₁-C₄)-haloalkyl,
Het¹, Het², Het³, Het⁴, Het⁵ and Het⁶ independently of one another each represent a saturated or partially unsaturated radical of a heterocycle having 3 to 9 ring atoms and at least one nitrogen atom as ring heteroatom at position 1 of the ring and optionally 1, 2 or 3 further ring heteroatoms from the group consisting of N, O and S, where the radical of the heterocycle at the nitrogen atom in position 1 of the ring is attached to the remainder of the molecule of the compound of the formula (I) and where the heterocycle is unsubstituted or substituted by one or more radicals from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio and oxo, preferably the radical of a saturated heterocycle of the type mentioned, in particular a morpholino, piperidino or pyrrolidino group,
Het⁷ independently of the others in each case represents a saturated, partially unsaturated or heteroaromatic monocyclic heterocyclyl radical having 3 to 9 ring atoms or a 9- or 10-membered bicyclic heterocycle, each containing 1, 2, 3 or 4 heteroatoms selected from the group consisting of O, N and S,
R*, R** independently of one another (and also independently of other radicals NR*R**) each represent H, (C₁-C₈)-alkyl, (C₂-C₈)-alkenyl, (C₂-C₈)-alkynyl, (C₁-C₄) -alkoxy- (C₁-C₄) -alkyl, (C₁-C₆)-alkanoyl, [(C₁-C₄)-haloalkyl]carbonyl, [(C₁-C₄)-alkoxy]carbonyl, [(C₁-C₄)-haloalkoxy]carbonyl, (C₃-C₆)-cycloalkyl, (C₃-C₆)-cycloalkyl- (C₁-C₄)-alkyl, phenyl, phenyl-(C₁-C₄)-alkyl, where each of the 4 last-mentioned radicals is optionally substituted in the cycle by one or more identical or different radicals from the group consisting of halogen, (C₁-C₄)-alkyl, (C₁**-**C₄)-haloalkyl, (C₁-C₄)-alkoxy and (C₁-C₄)-haloalkoxy or, in the case of saturated or partially unsaturated cyclic base groups, also oxo, or
R* and R** together with the nitrogen atom represent a 3- to 8-membered heterocycle which, in addition to the nitrogen atom, may contain one or two further ring heteroatoms from the group consisting of N, O and S and which may be unsubstituted or substituted by one or more radicals from the group consisting of (C₁-C₄) -alkyl, (C₁-C₄) -haloalkyl and oxo,
R^{A} represents halogen, cyano, hydroxy or (C₁-C₆)-alkoxy,
R^{B} represents halogen, cyano, hydroxy, oxo, nitro, (C₁-C₈)-alkyl, (C₁-C₆)-haloalkyl, cyano- (C₁-C₆)-alkyl, hydroxy-(C₁-C₆)-alkyl, nitro-(C₁-C₆)-alkyl, (C₂-C₈)-alkenyl, (C₂-C₈)-haloalkenyl, (C₂-C₈)-alkynyl, (C₂-C₈) -haloalkynyl, (C₁-C₈)-alkoxy, (C₂-C₈)-alkenyloxy, (C₂-C₈)-alkynyloxy, (C₁-C₈)-haloalkoxy, (C₁-C₆) -alkoxy- (C₁-C₆) -alkyl, (C₁-C₆)-alkoxy- (C₁-C₄)-alkoxy, (C₁-C₆)-haloalkoxy- (C₁-C₄)-alkyl, (C₁-C₆)-haloalkoxy-(C₁-C₄)-alkoxy, (C₁-C₈)-alkylthio, (C₂-C₆)-alkenylthio, (C₂-C₆)-alkynylthio, (C₁-C₈)-alkylsulphinyl, (C₁-C₆)-haloalkylsulphinyl, (C₁-C₈)-alkylsulphonyl, (C₁-C₆)-haloalkylsulphonyl, a radical of the formula R^{aa}-C(=O)-, R^{aa}-C(=O)-(C₁-C₆)alkyl, the radicals R^{aa} being defined below, - NR*R**, R* and R** being defined below, tri-[(C₁-C₄)-alkyl]silyl, tri-[(C₁-C₄)-alkyl]silyl- (C₁-C₆)-alkyl, (C₃-C₈)-cycloalkyl, (C₃-C₈)-cycloalkoxy, (C₃-C₈)-cycloalkyl-(C₁-C₈)-alkyl, (C₃-C₈)-cycloalkyl-(C₁-C₈)-alkoxy, phenyl, phenyl-(C₁-C₈)-alkyl, phenoxy, phenoxy-(C₁-C₈)-alkyl, phenylamino, phenylamino-(C₁-C₈)-alkyl or a 5- or 6-membered monocyclic or 9- or 10-membered bicyclic heterocycle which contains 1, 2, 3 or 4 heteroatoms selected from the group consisting of O, N and S, where each of the 11 last-mentioned radicals is optionally substituted in the cyclic moiety by one or more identical or different radicals R^{bb},
R^{aa} independently of one another each represent hydrogen, OH, (C₁-C₈)-alkyl, (C₁-C₆)-haloalkyl, (C₂-C₈)-alkenyl, (C₂-C₈)-alkynyl, (C₁-C₈)-alkoxy, (C₁-C₆)-alkoxy-(C₁-C₆)-alkyl, (C₁-C₆)-alkoxy-(C₁-C₆)-alkyloxy, (C₁-C₆)-haloalkoxy, (C₁-C₆)-haloalkoxy-(C₁-C₆)-alkyl, (C₁-C₆)-haloalkoxy- (C₁-C₆) -alkoxy, (C₃-C₈)-alkenyloxy, (C₃-C₈)-alkenyloxy- (C₁-C₆)-alkyl, (C₃-C₈)-alkenyloxy-(C₁-C₆)-alkoxy, (C₃-C₈)-alkynyloxy, (C₃-C₈)-alkynyloxy- (C₁-C₆) -alkyl, (C₃-C₈)-alkynyloxy-(C₁-C₆)-alkoxy, -NR*R*, where R* and R** are as defined above, tri-[(C₁-C₄)alkyl]silyl, tri- [(C₁-C₄)alkyl] Silyl- (C₁-C₆) -alkyl, tri-[(C₁-C₄) alkyl] silyl- (C₁-C₆)-alkoxy, (C₃-C₈)-cycloalkyl, (C₃-C₈)-cycloalkoxy, (C₃-C₈)-cycloalkyl- (C₁-C₈)-alkyl, (C₃-C₈)-cycloalkyl-(C₁-C₈)-alkoxy, (C₅-C₈)-cycloalkenyl, (C₅-C₈)-cycloalkenyl-(C₁-C₆) -alkyl, (C₅-C₈)-cycloalkenyloxy, (C₅-C₈)-cycloalkynyl, (C₅-C₈)-cycloalkynyl- (C₁-C₆)-alkyl, (C₅-C₈)-cycloalkynyl-(C₁-C₆)-alkoxy, phenyl, phenyl-(C₁-C₈)-alkyl, phenyl-(C₁-C₈)-alkoxy, phenoxy, phenoxy-(C₁-C₈)-alkyl, phenoxy-(C₁-C₈)-alkoxy, phenylamino, phenylamino-(C₁-C₈)-alkyl, phenylamino- (C₁-C₈)-alkoxy or a 5- or 6-membered monocyclic or 9- or 10-membered bicyclic heterocycle which is optionally attached via an alkylene group or an alkoxy group and contains 1, 2, 3 or 4 heteroatoms selected from the group consisting of O, N and S, where each of the 20 last-mentioned radicals is optionally substituted in the cyclic moiety by one or more identical or different radicals R^{cc}, and R^{bb} and R^{cc} independently of one another each represent halogen, (C₁-C₄)-alkyl, (C₁-C₄)-haloalkyl, (C₁-C₄)-alkoxy or (C₁-C₄)-haloalkoxy or in the case of saturated or partially unsaturated cyclic base groups also represent oxo and
M represents an equivalent of a cation,
preferably a metal ion equivalent, an ammonium ion which is optionally substituted by 1 to 4 identical or different radicals from the group consisting of (C₁-C₆)-alkyl, (C₂-C₆)-alkenyl, (C₂-C₆)-alkynyl, (C₃-C₆)-cycloalkyl, (C₅-C₆)-cycloalkenyl, (C₅-C₆)-cycloalkynyl, phenyl, (C₃-C₆)-cycloalkyl- (C₁-C₄) -alkyl, (C₅-C₆) -cycloalkenyl- (C₁-C₄)-alkyl, phenyl-(C₁-C₄)-alkyl, or a tertiary sulphonium ion which is preferably substituted by 3 identical or different radicals from the group consisting of (C₁-C₆)-alkyl, (C₂-C₆) -alkenyl, (C₂-C₆)-alkynyl, (C₃-C₆)-cycloalkyl, (C₅-C₆)-cycloalkenyl, (C₅-C₆)-cycloalkynyl, phenyl, (C₃-C₆)-cycloalkyl- (C₁-C₄)-alkyl, (C₅-C₆)-cycloalkenyl- (C₁-C₄)-alkyl, phenyl-(C₁-C₄)-alkyl, in particular (C₁-C₄)-alkyl,
m represents 0, 1, 2 or 3, preferably 0, 1 or 2, in particular 0 or 1, and
n represents 0, 1, 2, 3, 4 or 5, preferably 0, 1, 2 or 3.

2. Compounds or salts thereof according to Claim 1, **characterized in that**
R⁴ represents hydrogen or an optionally substituted hydrocarbon radical or an optionally substituted heterocyclyl radical, where each of the two last-mentioned carbon-containing radicals including substituents has 1 to 30 carbon atoms,
or
R⁴ represents a radical of the formula SiR^{a}R^{b}R^{c}, -NR^{a}R^{b} or -N=CR^{c}R^{d},
where in the 3 last-mentioned formulae each of the radicals R^{a}, R^{b}, R^{c} and R^{d} independently of the others represents hydrogen or an optionally substituted hydrocarbon radical, where, however, SiH₃ for SiR^{a}R^{b}R^{c} is excluded, or R^{a} and R^{b} together with the nitrogen atom of the group -NR^{a}R^{b} represent a 3- to 9-membered heterocycle which, in addition to the nitrogen atom, may contain one or two further ring heteroatoms from the group consisting of N, O and S and which is unsubstituted or substituted, or R^{c} and R^{d} together with the carbon atom of the group -N=CR^{c}R^{d} represent a 3- to 9-membered carbocyclic radical or a heterocyclic radical which may contain 1 to 3 ring heteroatoms from the group consisting of N, O and S, where the carbocyclic or heterocyclic radical is unsubstituted or substituted,
where each of the radicals R^{a}, R^{b}, R^{c} and R^{d} including substituents has up to 30 carbon atoms,
or
R⁴ represents a radical of the formula -C(=O)-R^{e} or - P(=O)(R^{f})₂, where R^{e} and the radicals R^{f} independently of one another each represent hydrogen, OH, (C₁-C₈)-alkyl, (C₁-C₄)-haloalkyl, (C₂-C₈)-alkenyl, (C₂-C₈)-alkynyl, (C₁-C₆)-alkoxy, (C₁-C₆) -alkoxy- (C₁-C₈)-alkyl, (C₁-C₄)-haloalkoxy, (C₁-C₄) -haloalkoxy- (C₁-C₈)-alkyl, (C₃-C₈)-alkenyloxy, (C₃-C₈)-alkenyloxy-(C₁-C₈)-alkyl, (C₃-C₈)-alkynyloxy, (C₃-C₈)-alkynyloxy-(C₁-C₈)-Alkyl, - NR*R**, with R* and R** being defined below, tri-[(C₁-C₄)-alkyl] silyl, tri- [(C₁-C₄)-alkyl] silyl- (C₁-C₈)alkyl, (C₃-C₆)-cycloalkyl, (C₃-C₆)-cycloalkyl-(C₁-C₈)-alkyl, (C₅-C₆)-cycloalkenyl, (C₅-C₆)-cycloalkenyl-(C₁-C₈)-alkyl, (C₅-C₆)-cycloalkynyl, (C₅-C₆)-cycloalkynyl-(C₁-C₈)-alkyl, phenyl, phenyl-(C₁-C₈)-Alkyl, phenoxy, phenoxy-(C₁-C₈)-alkyl, phenylamino, phenylamino-(C₁-C₈)-alkyl, a radical Het⁷, Het⁷-(C₁-C₆)-alkyl or Het⁷-O-(C₁-C₆)-alkyl,
where each of the 15 last-mentioned radicals is unsubstituted in the acyclic moiety or substituted by one or more identical or different radicals R^{A} and is unsubstituted in the cyclic moiety or substituted by one or more identical or different radicals R^{B},
Het⁷ independently of the others in each case represents a saturated, partially unsaturated or heteroaromatic monocyclic heterocyclyl radical having 3 to 9 ring atoms or a 9- or 10-membered bicyclic heterocycle, each containing 1, 2, 3 or 4 heteroatoms selected from the group consisting of O, N and S,
R*, R** independently of one another (and also independently of other radicals NR*R**) each represent H, (C₁-C₈)-alkyl, (C₂-C₈)-alkenyl, (C₂-C₈)-alkynyl, (C₁-C₄)-alkoxy-(C₁-C₄)-alkyl, (C₁-C₆)-alkanoyl, [(C₁-C₄)-haloalkyl]carbonyl, [(C₁-C₄)-alkoxy]carbonyl, [(C₁-C₄)-haloalkoxy]carbonyl, (C₃-C₆)-cycloalkyl, (C₃-C₆)-cycloalkyl-(C₁-C₄)-alkyl, phenyl, phenyl-(C₁-C₄)-alkyl, where each of the 4 last-mentioned radicals is optionally substituted in the cycle by one or more identical or different radicals from the group consisting of halogen, (C₁-C₄)-alkyl, (C₁-C₄)-haloalkyl, (C₁-C₄)-alkoxy and (C₁-C₄)-haloalkoxy or, in the case of saturated or partially unsaturated cyclic base groups, also oxo, or
R* and R** together with the nitrogen atom represent a 3- to 8-membered heterocycle which, in addition to the nitrogen atom, may contain one or two further ring heteroatoms from the group consisting of N, O and S and which may be unsubstituted or substituted by one or more radicals from the group consisting of (C₁-C₄)-alkyl, (C₁-C₄)-haloalkyl and oxo,
R^{A} represents halogen, cyano, hydroxy or (C₁-C₆)-alkoxy,
R^{B} represents halogen, cyano, hydroxy, oxo, nitro, (C₁-C₈)-alkyl, (C₁-C₆)-haloalkyl, cyano- (C₁-C₆)-alkyl, hydroxy-(C₁-C₆)-alkyl, nitro-(C₁-C₆)-alkyl, (C₂-C₈)-alkenyl, (C₂-C₈) -haloalkenyl, (C₂-C₈)-alkynyl, (C₂-C₈)-haloalkynyl, (C₁-C₈)-alkoxy, (C₂-C₈)-alkenyloxy, (C₂-C₈)-alkynyloxy, (C₁-C₈)-haloalkoxy, (C₁-C₆)-alkoxy-(C₁-C₆)-alkyl, (C₁-C₆)-alkoxy-(C₁-C₄)-alkoxy, (C₁-C₆)-haloalkoxy-(C₁-C₄)-alkyl, (C₁-C₆)-haloalkoxy-(C₁₋C₄)-alkoxy, (C₁-C₈)-alkylthio, (C₂-C₆)-alkenylthio, (C₂-C₆)-alkynylthio, (C₁-C₈)-alkylsulphinyl , (C₁-C₆)-haloalkylsulphinyl, (C₁-C₈)-alkylsulphonyl, (C₁-C₆) -haloalkylsulphonyl, a radical of the formula R^{aa}-C(=O)-, R^{aa}-C(=O)-(C₁-C₆)alkyl, the radicals R^{aa} being defined below, - NR*R**, R* and R** being defined below, tri-[(C₁-C₄)-alkyl] silyl, tri-[(C₁-C₄)-alkyl]silyl-(C₁-C₆)-alkyl, (C₃-C₈)-cycloalkyl, (C₃-C₈)-cycloalkoxy, (C₃-C₈)-cycloalkyl-(C₁-C₈)-alkyl, (C₃-C₈)-cycloalkyl-(C₁-C₈)-alkoxy, phenyl, phenyl-(C₁-C₈)-alkyl, phenoxy, phenoxy-(C₁-C₈)-alkyl, phenylamino, phenylamino-(C₁-C₈)-alkyl or a 5- or 6-membered monocyclic or 9- or 10-membered bicyclic heterocycle which contains 1, 2, 3 or 4 heteroatoms selected from the group consisting of O, N and S, where each of the 11 last-mentioned radicals is optionally substituted in the cyclic moiety by one or more identical or different radicals R^{bb},
R^{aa} independently of one another each represent hydrogen, OH, (C₁-C₈)-alkyl, (C₁-C₆) -haloalkyl, (C₂-C₈)-alkenyl, (C₂-C₈)-alkynyl, (C₁-C₈)-alkoxy, (C₁-C₆)-alkoxy-(C₁-C₆)-alkyl, (C₁-C₆)-alkoxy-(C₁-C₆)-alkyloxy, (C₁-C₆)-haloalkoxy, (C₁-C₆)-haloalkoxy-(C₁-C₆)-alkyl, (C₁-C₆)-haloalkoxy-(C₁-C₆)-alkoxy, (C₃-C₈)-alkenyloxy, (C₃-C₈)-alkenyloxy-(C₁-C₆)-alkyl, (C₃-C₈)-alkenyloxy-(C₁-C₆)-alkoxy, (C₃-C₈)-alkynyloxy, (C₃-C₈)-alkynyloxy-(C₁-C₆)-alkyl, (C₃-C₈)-alkynyloxy-(C₁-C₆)-alkoxy, -NR*R*, where R* and R** are as defined above, tri-[(C₁-C₄)alkyl]silyl, tri-[(C₁-C₄)alkyl]silyl-(C₁-C₆)-alkyl, tri-[(C₁-C₄)alkyl]silyl-(C₁-C₆)-alkoxy, (C₃-C₈)-cycloalkyl, (C₃-C₈)-cycloalkoxy, (C₃-C₈)-cycloalkyl-(C₁-C₈)-alkyl, (C₃-C₈)-cycloalkyl-(C₁-C₈)-alkoxy, (C₅-C₈)-cycloalkenyl, (C₅-C₈)-cycloalkenyl-(C₁-C₆)-alkyl, (C₅-C₈)-cycloalkenyloxy, (C₅-C₈)-cycloalkynyl, (C₅-C₈)-cycloalkynyl-(C₁-C₆)-alkyl, (C₅-C₈)-cycloalkynyl-(C₁-C₆)-alkoxy, phenyl, phenyl-(C₁-C₈)-alkyl, phenyl-(C₁-C₈)-alkoxy, phenoxy, phenoxy-(C₁-C₈)-alkyl, phenoxy-(C₁-C₈)-alkoxy, phenylamino, phenylamino-(C₁-C₈)-alkyl, phenylamino-(C₁-C₈)-alkoxy or a 5- or 6-membered monocyclic or 9- or 10-membered bicyclic heterocycle which is optionally attached via an alkylene group or an alkoxy group and contains 1, 2, 3 or 4 heteroatoms selected from the group consisting of O, N and S, where each of the 20 last-mentioned radicals is optionally substituted in the cyclic moiety by one or more identical or different radicals R^{cc}, and
R^{bb} and R^{cc} independently of one another each represent halogen, (C₁-C₄)-alkyl, (C₁-C₄)-haloalkyl, (C₁-C₄)-alkoxy or (C₁-C₄)-haloalkoxy or in the case of saturated or partially unsaturated cyclic base groups also represent oxo.

3. Compounds or salts thereof according to Claim 1 or 2, **characterized in that**
(R¹)ₘ represents m substituents R¹,
where R¹, if m = 1, or each of the substituents R¹, if m is greater than 1, independently of the others represents halogen, cyano, nitro, hydroxy, (C₁-C₆)-alkyl, (C₂-C₄)-alkenyl, (C₂-C₄)-alkynyl, (C₁-C₆)-alkoxy, (C₁-C₆)-alkylthio, (C₁-C₆)-alkylsulphinyl, (C₁-C₆)-alkylsulphonyl, (C₁-C₄)-haloalkyl, (C₁-C₄)-haloalkoxy, (C₁-C₄)-haloalkylthio, (C₁-C₄)-haloalkylsulphinyl, (C₁-C₄)-haloalkylsulphonyl, (C₂-C₄)-haloalkenyl, (C₂-C₄)-haloalkynyl, (C₁-C₄)-alkoxy- (C₁-C₄) -alkyl, (C₁-C₄)-haloalkoxy-(C₁-C₄)-alkyl, (C₃-C₆)-cycloalkyl which is optionally substituted by one or more radicals from the group consisting of halogen and (C₁-C₄)-alkyl, (C₃-C₆)-cycloalkoxy which is optionally substituted by one or more radicals from the group consisting of halogen and (C₁-C₄)-alkyl, or a radical of the formulaC(O)OR⁵, C(O)NR⁶R⁷, C(O)-Het¹, NR⁸R⁹ or Het²
or where in each case two groups R¹ located ortho at the ring or R¹ and R³ together represent a group of the formula -Z¹-A*-Z² in which
A* represents an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z¹ represents a direct bond, O or S and
Z² represents a direct bond, O or S,
where the group -Z¹-A*-Z² together with the carbon atoms, attached to the group, of the phenyl ring form a fused-on 5- or 6-membered ring,
R⁵ represents hydrogen, (C₁-C₄)-alkyl, (C₁-C₄)-haloalkyl, (C₃-C₆)-cycloalkyl, (C₃-C₆)-halocycloalkyl, (C₂-C₄)-alkenyl, (C₂-C₄)-haloalkenyl, (C₂-C₄)-alkynyl or the group M,
R⁶, R⁷, R⁸ and R⁹ independently of one another each represent hydrogen or (C₁-C₄)-alkyl which is unsubstituted or substituted by one or more radicals from the group consisting of halogen, nitro, cyano and phenyl, or
(C₃-C₆)-cycloalkyl or phenyl, where each of the 2 last-mentioned radicals in each case independently of the other is unsubstituted or substituted by one or more radicals from the group consisting of halogen, (C₁-C₄)-alkyl, (C₁-C₄)-haloalkyl, phenyl and benzyl,
Het¹ and Het² independently of one another each represent a saturated or partially unsaturated radical of a heterocycle having 3 to 6 ring atoms and at least one nitrogen atom as ring heteroatom at position 1 of the ring and optionally 1, 2 or 3 further ring heteroatoms from the group consisting of N, O and S, where the radical of the heterocycle is attached at the nitrogen atom in position 1 of the ring to the remainder of the molecule of the compound of the formula (I) and where the heterocycle is unsubstituted or substituted by one or more radicals from the group consisting of halogen, (C₁-C₄) -alkyl, (C₁-C₄)-haloalkyl and oxo,
M represents the equivalent of a cation and
m represents 0, 1, 2 or 3.

4. Compounds or salts thereof according to any of Claims 1 to 3, **characterized in that**
(R²)ₙ represents n substituents R²,
where R², if n = 1, or each of the substituents R², if n is greater than 1, independently of the others represents halogen, cyano, nitro, hydroxy, (C₁-C₆)-alkyl, (C₂-C₄)-alkenyl, (C₂-C₄)-alkynyl, (C₁-C₆)-alkoxy, (C₁-C₆)-alkylthio, (C₁-C₆)-alkylsulphinyl, (C₁-C₆)-alkylsulphonyl, (C₁-C₄)-haloalkyl, (C₁-C₄)-haloalkoxy, (C₁-C₄)-haloalkylthio, (C₁-C₄)-haloalkylsulphinyl, (C₁-C₄)-haloalkylsulphonyl, (C₂-C₄)-haloalkenyl, (C₂-C₄)-haloalkynyl, (C₁-C₄)-alkoxy-(C₁-C₄)-alkyl, (C₁-C₄)-haloalkoxy-(C₁-C₄)-alkyl, (C₃-C₆)-cycloalkyl which is optionally substituted by one or more radicals from the group consisting of halogen and (C₁-C₄)-alkyl, (C₃-C₆)-cycloalkoxy which is optionally substituted by one or more radicals from the group consisting of halogen and (C₁-C₄)-alkyl, or a radical of the formula C(O)OR¹⁰, C(O)NR¹¹R¹², C(O)-Het³, NR¹³R¹⁴ or Het⁴
or where in each case two groups R² located ortho at the ring together are a group of the formula - Z³-A**-Z⁴ in which
A** represents an alkylene group having 1 to 4 carbon atoms which is optionally substituted by one or more radicals from the group consisting of halogen, (C₁-C₄)-alkyl, (C₁-C₉)-haloalkyl, (C₁-C₄)-alkoxy and (C₁-C₄)-haloalkoxy,
Z³ represents a direct bond, O or S and
Z⁴ represents a direct bond, 0 or S,
where the group -Z³-A**-Z⁴ together with the carbon atoms, attached to the group, of the phenyl ring form a fused-on 5- or 6-membered ring,
R¹⁰ represents hydrogen, (C₁-C₄)-alkyl, (C₁-C₄)-haloalkyl, (C₃-C₆)-cycloalkyl, (C₃-C₆)-halocycloalkyl, (C₂-C₄)-alkenyl, (C₂-C₄)-haloalkenyl, (C₂-C₄)-alkynyl or the group M,
R¹¹, R¹², R¹³ and R¹⁴ independently of one another each represent hydrogen or (C₁-C₄)-alkyl which is unsubstituted or substituted by one or more radicals from the group consisting of halogen, nitro, cyano and phenyl, or
(C₃-C₆)-cycloalkyl or phenyl, where each of the 2 last-mentioned radicals in each case independently of the other is unsubstituted or substituted by one or more radicals from the group consisting of halogen, (C₁-C₄)-alkyl, (C₁-C₄) -haloalkyl, phenyl and benzyl,
Het³ and Het⁴ independently of one another each represent a saturated or partially unsaturated radical of a heterocycle having 3 to 6 ring atoms and at least one nitrogen atom as ring heteroatom at position 1 of the ring and optionally 1, 2 or 3 further ring heteroatoms from the group consisting of N, O and S, where the radical of the heterocycle is attached at the nitrogen atom in position 1 of the ring to the remainder of the molecule of the compound of the formula (I) and where the heterocycle is unsubstituted or substituted by one or more radicals from the group consisting of halogen, (C₁-C₄)-alkyl, (C₁-C₄)-haloalkyl and oxo,
M represents the equivalent of a cation and
n represents 0, 1, 2, 3, 4 or 5.

5. Compounds or salts thereof according to any of Claims 1 to 4, **characterized in that**
R³ represents halogen, cyano, nitro, hydroxy, (C₁-C₆)-alkyl, (C₂-C₄) -alkenyl, (C₂-C₄)-alkynyl, (C₁-C₆)-alkoxy, (C₁-C₆)-alkylthio, (C₁-C₆)-alkylsulphinyl, (C₁-C₆)-alkylsulphonyl, (C₁-C₄)-haloalkyl, (C₁-C₄)-haloalkoxy, (C₁-C₄)-haloalkylthio, (C₁-C₄)-haloalkylsulphinyl, (C₁-C₄)-haloalkylsulphonyl, (C₂-C₄)-haloalkenyl, (C₂-C₄) -haloalkynyl, (C₁-C₄)-alkoxy- (C₁-C₄) -alkyl, (C₁-C₄)-haloalkoxy- (C₁-C₄)-alkyl, (C₃-C₆)-cycloalkyl which is optionally substituted by one or more radicals from the group consisting of halogen and (C₁-C₄)-alkyl, (C₃-C₆)-cycloalkoxy which is optionally substituted by one or more radicals from the group consisting of halogen and (C₁-C₉)-alkyl, or a radical of the formula C(O)OR¹⁵, C(O)NR¹⁶R¹⁷, C(O)-Het⁵, NR¹⁸R¹⁹ or Het⁶,
R¹⁵ represents hydrogen, (C₁-C₄)-alkyl, (C₁-C₄)-haloalkyl, (C₃-C₆)-cycloalkyl, (C₃-C₆)-halocycloalkyl, (C₂-C₄)-alkenyl, (C₂-C₄)-haloalkenyl, (C₂-C₄)-alkynyl or the group M mentioned, preferably hydrogen, (C₁-C₄)-alkyl or the group M,
R¹⁶, R¹⁷, R¹⁸ and R¹⁹ independently of one another each represent hydrogen or (C₁-C₄)-alkyl which is unsubstituted or substituted by one or more radicals from the group consisting of halogen, nitro, cyano and phenyl, or
(C₃-C₆)-cycloalkyl or phenyl, where each of the 2 last-mentioned radicals in each case independently of the other is unsubstituted or substituted by one or more radicals from the group consisting of halogen, (C₁-C₄)-alkyl, (C₁-C₄)-haloalkyl, phenyl and benzyl,
Het⁵ and Het⁶ independently of one another each represent a saturated or partially unsaturated radical of a heterocycle having 3 to 6 ring atoms and at least one nitrogen atom as ring heteroatom at position 1 of the ring and optionally 1, 2 or 3 further ring heteroatoms from the group consisting of N, O and S, where the radical of the heterocycle is attached at the nitrogen atom in position 1 of the ring to the remainder of the molecule of the compound of the formula (I) and where the heterocycle is unsubstituted or substituted by one or more radicals from the group consisting of halogen, (C₁-C₄)-alkyl, (C₁-C₄)-haloalkyl and oxo, and
M represents the equivalent of a cation.

6. Compounds or salts thereof according to any of Claims 1 to 5, **characterized in that**
(R¹)ₘ represents m substituents R¹,
where R1, if m = 1, or each of the substituents R¹, if m is greater than 1, independently of the others represents halogen, cyano, nitro, hydroxy, (C₁-C₄)-alkyl, (C₁-C₄)-alkoxy, (C₁-C₄) -alkylthio, (C₁-C₄)-alkylsulphinyl, (C₁-C₄)-alkylsulphonyl, (C₁-C₄)-haloalkyl, (C₁-C₄)-haloalkoxy, (C₁-C₄)-haloalkylthio, (C₁-C₄)-haloalkylsulphinyl, (C₁-C₄)-haloalkylsulphonyl, (C₁-C₄)-alkoxy-(C₁-C₄)-alkyl, (C₃-C₆)-cycloalkyl, or a radical of the formula C(O)OR⁵, C(O)NR⁶R⁷, C(O)-Het¹, NR⁸R⁹ or Het² ,
or where in each case two groups R¹ located ortho at the ring or R¹ and R³ together represent a group of the formula -Z¹-A*-Z² in which
A* represents an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z¹ represents a direct bond, O or S and
Z² represents a direct bond, O or S,
where the group -Z¹-A*-Z² together with the carbon atoms, attached to the group, of the phenyl ring form a fused-on 5- or 6-membered ring,
R⁵ represents hydrogen, (C₁-C₄)-alkyl or the group M mentioned,
R⁶, R⁷, R⁸ and R⁹ independently of one another each represent hydrogen, (C₁-C₄)-alkyl, (C₁-C₄)-haloalkyl, benzyl, (C₃-C₆)-cycloalkyl or phenyl,
Het¹ and Het² independently of one another each represent a morpholino, piperidino or pyrrolidino group and
m represents 0, 1, 2 or 3,
(R²)ₙ represents n substituents R²,
where R², if n = 1, or each of the substituents R², if n is greater than 1, independently of one another represent halogen, cyano, nitro, hydroxy, (C₁-C₄)-alkyl, (C₁-C₄)-alkoxy, (C₁-C₄)-alkylthio, (C₁-C₄)-alkylsulphinyl, (C₁-C₄)-alkylsulphonyl, (C₁-C₄)-haloalkyl, (C₁-C₄)-haloalkoxy, (C₁-C₄)-haloalkylthio, (C₁-C₄)-haloalkylsulphinyl, (C₁-C₄)-haloalkylsulphonyl, (C₁-C₄)-alkoxy-(C₁-C₄)-alkyl, (C₃-C₆)-cycloalkyl, or a radical of the formula C(O)OR¹⁰, C(O)NR¹¹R¹², C(O)-Het³, NR¹³R¹⁴ or Het⁴,
or where in each case two groups R² located ortho at the ring together are a group of the formula - Z³-A**-Z⁴ in which
A** represents an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z³ represents a direct bond, O or S and
Z⁴ represents a direct bond, O or S,
where the group -Z³-A**-Z⁴ together with the carbon atoms, attached to the group, of the phenyl ring form a fused-on 5- or 6-membered ring,
R¹⁰ represents hydrogen, (C₁-C₄)-alkyl or the group M mentioned,
R¹¹, R¹², R¹³ and R¹⁴ independently of one another each represent hydrogen, (C₁-C₄)-alkyl, (C₁-C₄)-haloalkyl, benzyl, (C₃-C₆)-cycloalkyl or phenyl,
Het³ and Het⁴ independently of one another each represent a morpholino, piperidino or pyrrolidino group and
n represents 0, 1, 2, 3, 4 or 5,
R³ represents halogen, cyano, nitro, hydroxy, (C₁-C₄)-alkyl, (C₁-C₄) -alkoxy, (C₁-C₄)-alkylthio, (C₁-C₄)-alkylsulphinyl, (C₁-C₄)-alkylsulphonyl, (C₁-C₄)-haloalkyl, (C₁-C₄)-haloalkoxy, (C₁-C₄)-haloalkylthio, (C₁-C₄)-haloalkylsulphinyl, (C₁-C₄)-haloalkylsulphonyl, (C₁-C₄)-alkoxy-(C₁-C₄)-alkyl, (C₃-C₆)-cycloalkyl or a radical of the formula C(O)OR¹⁵, C(O)NR¹⁶R¹⁷, C(O)-Het⁵, NR¹⁸R¹⁹ or Het⁶,
R¹⁵ represents hydrogen, (C₁-C₄)-alkyl or the group M mentioned,
R¹⁶, R¹⁷, R¹⁸ and R¹⁹ independently of one another each represent hydrogen, (C₁-C₄)-alkyl, (C₁-C₄)-haloalkyl, benzyl, (C₃-C₆)-cycloalkyl or phenyl,
Het⁵ and Het⁶ independently of one another each represent a morpholino, piperidino or pyrrolidino group and
M represents the equivalent of a cation.

7. Process for preparing a compound of the formula (I) as defined in any of Claims 1 to 6 or a salt thereof, **characterized in that**, diastereomer mixtures of the compounds of the formula (I) comprising the compound (I) to be prepared are obtained, for example, **characterized in that**
(a) in the case of the preparation of diastereomer mixtures of the compounds of the formula (I), compounds of the formula (II) 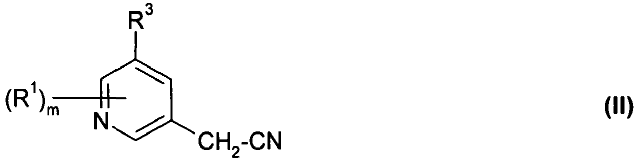 are reacted with compounds of the formula (III) or salts thereof 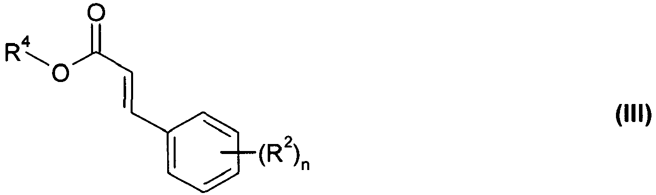 to give compounds of the formula (I) 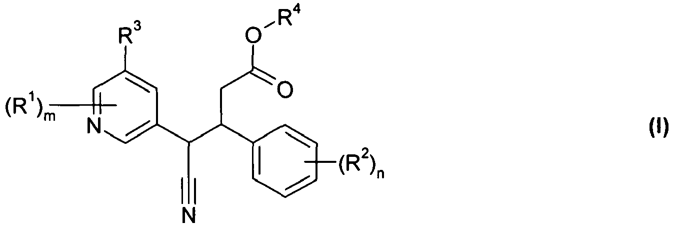 where R¹, R², R³, R⁴, m und n in the compounds (II) and (III) are as defined in the respective compound of the formula (I) to be prepared,
(b) compounds of the formula (I*) 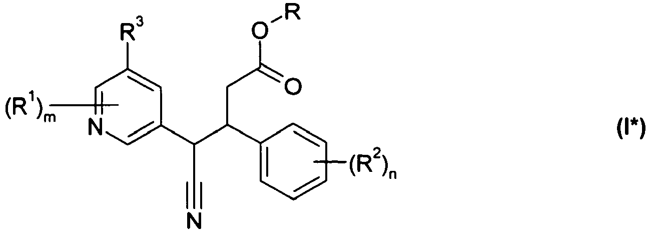 in which R is a radical from the group consisting of the radicals possible for R⁴, but different from the radical R⁴ in the compound (I) to be prepared, is reacted with a compound of the formula R⁴-OH in which R⁴ is defined as in formula (I), to give compound (I), where R¹, R², R³, m and n in the compound (I*) are as defined in the compound of the formula (I) to be prepared in ewach case.
(c) compounds of the formula (I*) (see b) in which R represents a hydrogen atom are reacted with a compound of the formula R⁴-OH in which R⁴ is as defined in formula (I), to give the compounds of the formula (I), or
(d) in the case in which optically active compounds of a diastereomeric form of the compound of the formula (I) are prepared, the racemic mixture of the diastereomeric form of the compound of the formula (I) is subjected to an optical resolution and the desired enantiomer is isolated in a stereochemical purity of from 60 to 100%, preferably from 70 to 100%, more preferably from 80 to 100%, in particular from 90 to 100%, based on the mixture of erythro or threo enantiomers present.

8. Herbicidal or plant growth-regulating composition, **characterized in that** it comprises one or more compounds of the formula (I) or salts thereof as defined in any of Claims 1 to 6 and formulation auxiliaries customary in crop protection.

9. Method for controlling harmful plants or for regulating the growth of plants, **characterized in that** an effective amount of one or more compounds of the formula (I) or salts thereof as defined in any of Claims 1 to 6 is applied onto the plants, plant seeds, the soil in which or on which the plants grow or the area under cultivation.

10. Method according to Claim 9, **characterized in that** the compounds of the formula (I) or salts thereof are employed for the selective control of harmful plants or for regulating the growth in crops of useful plants or ornamental plants.

11. Use of the compounds of the formula (I) or the salts thereof according to any of Claims 1 to 6 as herbicides or plant growth regulators.

## Revendications

1. Composés de formule (I) ou leurs sels 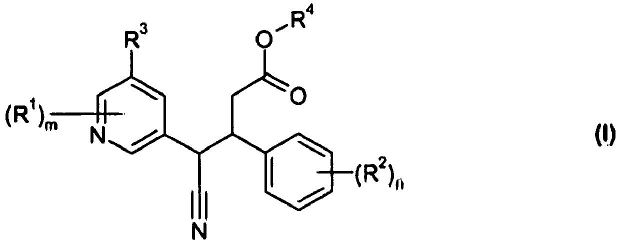 dans laquelle
(R¹)ₘ signifie m substituants R¹,
R¹, lorsque m = 1, ou chacun des substituants R¹, lorsque m est supérieur à 1, indépendamment, signifiant halogène, cyano, nitro, hydroxy, (C₁-C₈)alkyle, (C₂-C₆)alcényle, (C₂-C₆) alcynyle, (C₁-C₈)alcoxy, (C₁-C₈) alkylthio, (C₁-C₈) alkylsulfinyle, (C₁-C₈) alkylsulfonyle, (C₁-C₆) halogénoalkyle, (C₁-C₆)halogénoalcoxy, (C₁-C₆) halogénoalkylthio, (C₁-C₆)halogénoalkylsulfinyle, (C₁-C₆)halogénoalkylsulfonyle, (C₂-C₆)halogénoalcényle, (C₂-C₆) halogénoalcynyle, (C₁-C₆) alcoxy- (C₁-C₄)alkyle, (C₁-C₆)halogénoalcoxy-(C₁-C₄)alkyle, (C₃-C₆)cycloalkyle, qui est le cas échéant substitué par un ou plusieurs radicaux du groupe formé par halogène et (C₁-C₄)alkyle, (C₃-C₆)cycloalcoxy, qui est le cas échéant substitué par un ou plusieurs radicaux du groupe formé par halogène et (C₁-C₄)alkyle, ou un radical de formule C(O)OR⁵, C(O)NR⁶R⁷, C(O)-Het¹, NR⁸R⁹ ou Het² ou à chaque fois deux groupes R¹ ou R¹ et R³ en position ortho sur le cycle formant ensemble un groupe de formule -Z¹-A*-Z², dans laquelle
A* représente un groupe alkylène comprenant 1 à 4 atomes de carbone, qui est le cas échéant substitué par un ou plusieurs radicaux du groupe formé par halogène, (C₁-C₄)alkyle, (C₁-C₄)halogénoalkyle, (C₁-C₄) alcoxy et (C₁-C₄)halogénoalcoxy,
Z¹ représente une liaison directe, O ou S et
Z² représente une liaison directe, O ou S,
le groupe -Z¹-A*-Z² formant ensemble avec les atomes de carbone liés au groupe du cycle phényle un cycle condensé de 5 ou 6 chaînons,
(R²)ₙ signifie n substituants R²,
R², lorsque n = 1, ou chacun des substituants R², lorsque n est supérieur à 1, indépendamment, signifiant halogène, cyano, nitro, hydroxy, (C₁-C₈)alkyle, (C₂-C₆)alcényle, (C₂-C₆)alcynyle, (C₁-C₈)alcoxy, (C₁-C₈)alkylthio, (C₁-C₈) alkylsulfinyle, (C₁-C₈)alkylsulfonyle, (C₁-C₆)halogénoalkyle, (C₁-C₆)halogénoalcoxy, (C₁-C₆) halogénoalkylthio, (C₁-C₆)halogénoalkylsulfinyle, (C₁-C₆)halogénoalkylsulfonyle, (C₂-C₆)halogénoalcényle, (C₂-C₆)halogénoalcynyle, (C₁-C₆)alcoxy-(C₁-C₄)alkyle, (C₁-C₆)halogénoalcoxy-(C₁-C₄)alkyle, (C₃-C₆)cycloalkyle, qui est le cas échéant substitué par un ou plusieurs radicaux du groupe formé par halogène et (C₁-C₄)alkyle, (C₃-C₆)cycloalcoxy, qui est le cas échéant substitué par un ou plusieurs radicaux du groupe formé par halogène et (C₁-C₄)alkyle, ou un radical de formule C(O)OR¹⁰, C(O)NR¹¹R¹², C(O)-Het³, NR¹³R¹⁴ ou Het⁴ ou à chaque fois deux groupes R² en position ortho sur le cycle formant ensemble un groupe de formule -Z³-A**-Z⁴, dans laquelle
A** représente un groupe alkylène comprenant 1 à 4 atomes de carbone, qui est le cas échéant substitué par un ou plusieurs radicaux du groupe formé par halogène, (C₁-C₄)alkyle, (C₁-C₄)halogénoalkyle, (C₁-C₄)alcoxy et (C₁-C₄)halogénoalcoxy,
Z³ représente une liaison directe, O ou S et
Z⁴ représente une liaison directe, O ou S,
le groupe -Z³-A**-Z⁴ formant ensemble avec les atomes de carbone liés au groupe du cycle phényle un cycle condensé de 5 ou 6 chaînons,
R³ signifie halogène, cyano, nitro, hydroxy, (C₁-C₈)alkyle, (C₂-C₆)alcényle, (C₂-C₆)alcynyle, (C₁-C₈)alcoxy, (C₁-C₈)alkylthio, (C₁-C₈) alkylsulfinyle, (C₁-C₈)alkylsulfonyle, (C₁-C₆)halogénoalkyle, (C₁-C₆)halogénoalcoxy, (C₁-C₆)halogénoalkylthio, (C₁-C₆)halogénoalkylsulfinyle, (C₁-C₆)halogénoalkylsulfonyle, (C₂-C₆)halogénoalcényle, (C₂-C₆)halogénoalcynyle, (C₁-C₆)alcoxy- (C₁-C₄)alkyle, (C₁-C₆)halogénoalcoxy- (C₁-C₄)alkyle, (C₃-C₆)cycloalkyle, qui est le cas échéant substitué par un ou plusieurs radicaux du groupe formé par halogène et (C₁-C₄)alkyle, (C₃-C₆)cycloalcoxy, qui est le cas échéant substitué par un ou plusieurs radicaux du groupe formé par halogène et (C₁-C₄)alkyle, ou un radical de formule C(O)OR¹⁵, C(O)NR¹⁶R¹⁷, C(O)-Het⁵, NR¹⁸R¹⁹ ou Het⁶,
R⁴ signifie hydrogène ou un radical hydrolysable, de préférence
R⁴ signifie hydrogène ou un radical hydrocarboné le cas échéant substitué ou un radical hétérocyclyle le cas échéant substitué, chacun des deux derniers radicaux contenant du carbone mentionnés, y compris les substituants, comprenant 1 à 30 atomes de carbone, de préférence 1 à 24 atomes de carbone, en particulier 1 à 20 atomes de carbone, ou
R⁴ signifie un radical de formule SiR^{a}R^{b}R^{c}, -NR^{a}R^{b} ou -N=CR^{c}R^{d}, chacun des radicaux R^{a}, R^{b}, R^{c} et R^{d} dans les 3 dernières formules mentionnées, indépendamment, signifiant hydrogène ou un radical hydrocarboné le cas échéant substitué, cependant à l'exclusion de SiH₃ pour SiR^{a}R^{b}R^{c}, ou R^{a} et R^{b} formant ensemble avec l'atome de N du groupe -NR^{a}R^{b} un hétérocycle de 3 à 9 chaînons, qui peut contenir, en plus de l'atome N, un ou deux autres hétéroatomes de cycle du groupe formé par N, O et S et qui est non substitué ou substitué, ou R^{c} et R^{d} signifiant, ensemble avec l'atome C du groupe -N=CR^{c}R^{d}, un radical carbocyclique de 3 à 9 chaînons ou un radical hétérocyclique, qui peut contenir 1 à 3 hétéroatomes de cycle du groupe formé par N, O et S, le radical carbocyclique ou hétérocyclique étant non substitué ou substitué, chacun des radicaux R^{a}, R^{b}, R^{c} et R^{d}, y compris les substituants, comprenant jusqu'à 30 atomes de carbone, de préférence jusqu'à 24 atomes de carbone, en particulier jusqu'à 20 atomes de carbone, ou
R⁴ signifie un radical -C(=O)-R^{e} ou -P(=O) (R^{f})₂, R^{e} et les radicaux R^{f} signifiant, indépendamment, à chaque fois hydrogène, OH, (C₁-C₈)alkyle, (C₁-C₄)halogénoalkyle, (C₂-C₈)alcényle, (C₂-C₈)alcynyle, (C₁-C₆)alcoxy, (C₁-C₆) alcoxy- (C₁-C₈) alkyle, (C₁-C₄)halogénoalcoxy, (C₁-C₄)halogénoalcoxy-(C₁-C₈)alkyle, (C₃-C₈)alcényloxy, (C₃-C₈)alcényloxy- (C₁-C₈) alkyle, (C₃-C₈)alcynyloxy, (C₃-C₈)alcynyloxy-(C₁-C₈)alkyle, -NR*R**, R* et R** étant définis davantage ci-dessous, tri-[ (C₁-C₄)alkyl]-silyle, tri- [(C₁-C₄)alkyl]-silyl-(C₁-C₈)alkyle, (C₃-C₆)cycloalkyle, (C₃-C₆)cycloalkyl- (C₁-C₈)alkyle, (C₅-C₆)cycloalcényle, (C₅-C₆)cycloalcényl-(C₁-C₈)alkyle, (C₅-C₆)cycloalcynyle, (C₅-C₆)cycloalcynyl- (C₁-C₈)alkyle, phényle, phényl-(C₁-C₈)alkyle, phénoxy, phénoxy-(C₁-C₈)alkyle, phénylamino, phénylamino- (C₁-C₈)alkyle, un radical Het⁷, Het⁷-(C₁-C₆)alkyle ou Het⁷-O-(C₁-C₆)alkyle,
chacun des 15 derniers radicaux mentionnés étant non substitué ou substitué, dans la partie acyclique, par un ou plusieurs radicaux R^{A} identiques ou différents et étant non substitué ou substitué, dans la partie cyclique, par un ou plusieurs radicaux R^{B} identiques ou différents,
R⁵, R¹⁰ et R¹⁵ signifient à chaque fois, indépendamment, hydrogène, (C₁-C₆)alkyle, (C₁-C₄)halogénoalkyle, (C₃-C₆)cycloalkyle, (C₃-C₆)halogénocycloalkyle, (C₂-C₄)alcényle, (C₂-C₄)halogénoalcényle, (C₂-C₄)alcynyle ou le groupe M mentionné ci-dessous, de préférence (C₁-C₄)alkyle,
R⁶, R⁷, R⁸, R⁹, R¹¹, R¹², R¹³, R¹⁴, R¹⁶, R¹⁷, R¹⁸ et R¹⁹
signifient à chaque fois, indépendamment, hydrogène, (C₁-C₆)alkyle, (C₂-C₆)alcényle ou (C₂-C₆)alcynyle, chacun des 3 derniers radicaux mentionnés étant à chaque fois, indépendamment, non substitué ou substitué par un ou plusieurs radicaux du groupe formé par halogène, nitro, cyano et phényle, qui est le cas échéant substitué, de préférence non substitué ou substitué par un ou plusieurs radicaux du groupe formé par halogène, nitro, cyano, (C₁-C₄)alkyle et (C₁-C₄)halogénoalkyle, ou (C₃-C₆)cycloalkyle ou phényle, chacun des 2 derniers radicaux mentionnés étant à chaque fois, indépendamment, non substitué ou substitué par un ou plusieurs radicaux du groupe formé par halogène, nitro, cyano, (C₁-C₄)alkyle, (C₁-C₄)halogénoalkyle, phényle et benzyle, chacun des 2 derniers radicaux mentionnés étant le cas échéant substitué, de préférence non substitué ou substitué par un ou plusieurs radicaux du groupe formé par halogène, nitro, cyano, (C₁-C₄)alkyle et (C₁-C₄)halogénoalkyle,
Het¹, Het², Het³, Het⁴, Het⁵ et Het⁶ signifient à chaque fois, indépendamment, un radical saturé ou partiellement insaturé d'un hétérocycle comprenant 3 à 9 atomes de cycle et au moins un atome de N comme hétéroatome de cycle en position 1 du cycle et le cas échéant 1, 2 ou 3 autres hétéroatomes de cycle du groupe formé par N, O et S, le radical de l'hétérocycle étant lié au niveau de l'atome N en position 1 du cycle au reste de la molécule du composé de formule (I) et l'hétérocycle étant non substitué ou substitué par un ou plusieurs radicaux du groupe formé par halogène, (C₁-C₄)alkyle, (C₁-C₄)halogénoalkyle, (C₁-C₄)alcoxy, (C₁-C₄)halogénoalcoxy, (C₁-C₄)alkylthio et oxo,
de préférence le radical d'un hétérocycle saturé du type mentionné, en particulier un groupe morpholino, pipéridino ou pyrrolidino,
Het⁷ signifie à chaque fois, indépendamment, un radical hétérocyclyle monocyclique saturé, partiellement insaturé ou hétéroaromatique comprenant 3 à 9 atomes de cycle ou un hétérocycle bicyclique de 9 ou 10 chaînons, contenant à chaque fois 1, 2, 3 ou 4 hétéroatomes, choisis dans le groupe formé par O, N et S,
R*, R** signifient à chaque fois, indépendamment (également indépendamment d'autres radicaux NR*R**) H, (C₁-C₈)alkyle, (C₂-C₈)alcényle, (C₂-C₈)alcynyle, (C₁-C₄)alcoxy-(C₁-C₄)alkyle, (C₁-C₆)alcanoyle, [(C₁-C₄)halogénoalkyl]-carbonyle, [(C₁-C₄)alcoxy] -carbonyle, [(C₁-C₄)halogénoalcoxy]-carbonyle, (C₃-C₆)cycloalkyle, (C₃-C₆)cycloalkyl-(C₁-C₄)alkyle, phényle, phényl-(C₁-C₄)alkyle, chacun des 4 derniers radicaux mentionnés dans le cycle étant le cas échéant substitué par un ou plusieurs radicaux identiques ou différents du groupe formé par halogène, (C₁-C₄)alkyle, (C₁-C₄)halogénoalkyle, (C₁-C₄)alcoxy ou (C₁-C₄)halogénoalcoxy ou, dans le cas de groupes de base cycliques, saturés ou partiellement insaturés, également substitué par oxo, ou
R* et R** signifient, ensemble avec l'atome N, un hétérocycle de 3 à 8 chaînons, qui peut contenir en plus de l'atome N, un ou deux autres hétéroatomes de cycle du groupe formé par N, 0 et S et qui est non substitué ou substitué par un ou plusieurs radicaux du groupe formé par (C₁-C₄)alkyle, (C₁-C₄)halogénoalkyle et oxo,
R^{A} signifie halogène, cyano, hydroxy ou (C₁-C₆)alcoxy,
R^{B} signifie halogène, cyano, hydroxy, oxo, nitro, (C₁-C₈)alkyle, (C₁-C₆)halogénoalkyle, cyano-(C₁-C₆)alkyle, hydroxy-(C₁-C₆) alkyle, nitro-(C₁-C₆)alkyle, (C₂-C₈)alcényle, (C₂-C₈)halogénoalcényle, (C₂-C₈)alcynyle, (C₂-C₈)halogénoalcynyle, (C₁-C₈)alcoxy, (C₂-C₈) alcényloxy, (C₂-C₈) alcynyloxy, (C₁-C₈)halogénoalcoxy, (C₁-C₆)alcoxy- (C₁-C₆)alkyle, (C₁-C₆)alcoxy- (C₁-C₄)alcoxy, (C₁-C₆)halogénoalcoxy-(C₁-C₄)alkyle, (C₁-C₆)halogénoalcoxy- (C₁-C₄)alcoxy, (C₁-C₈)alkylthio, (C₂-C₆)alcénylthio, (C₂-C₆)alcynylthio, (C₁-C₈)alkylsulfinyle, (C₁-C₆)halogénoalkylsulfinyle, (C₁-C₈)alkylsulfonyle, (C₁-C₆)halogénoalkylsulfonyle, un radical de formule R^{aa}-C(=O)-, R^{aa}-C(=O)-(C₁-C₆)alkyle, les radicaux R^{aa} étant définis davantage ci-dessous, -NR*R**, les radicaux R* et R** étant définis davantage ci-dessous, tri-[(C₁-C₄)alkyl]-silyle, tri-[(C₁-C₄)alkyl]-silyl-(C₁-C₆)alkyle, (C₃-C₈)cycloalkyle, (C₃-C₈)cycloalcoxy, (C₃-C₈)cycloalkyl- (C₁-C₈)alkyle, (C₃-C₈)cycloalkyl- (C₁-C₈)alcoxy, phényle, phényl-(C₁-C₈)alkyle, phénoxy, phénoxy-(C₁-C₈)alkyle, phénylamino, phénylamino-(C₁-C₈)alkyle ou un hétérocycle monocyclique de 5 ou 6 chaînons ou bicyclique de 9 ou 10 chaînons, contenant 1, 2, 3 ou 4 hétéroatomes choisis dans le groupe constitué par O, N et S, chacun des 11 derniers radicaux mentionnés étant substitué, dans la partie cyclique, le cas échéant par un ou plusieurs radicaux R^{bb} identiques ou différents,
R^{aa} signifie à chaque fois, indépendamment, hydrogène, OH, (C₁-C₈)alkyle, (C₁-C₆)halogénoalkyle, (C₂-C₈)alcényle, (C₂-C₈)alcynyle, (C₁-C₈)alcoxy, (C₁-C₆)alcoxy- (C₁-C₆)alkyle, (C₁-C₆)alcoxy- (C₁-C₆)alkyloxy, (C₁-C₆)halogénoalcoxy, (C₁-C₆)halogénoalcoxy- (C₁-C₆)alkyle, (C₁-C₆)halogénoalcoxy-(C₁-C₆)alcoxy, (C₃-C₈)alcényloxy, (C₃-C₈)alcényloxy- (C₁-C₆)alkyle, (C₃-C₈)alcényloxy-(C₁-C₆)alcoxy, (C₃-C₈)alcynyloxy, (C₃-C₈)alcynyloxy-(C₁-C₆)alkyle, (C₃-C₈)alcynyloxy-(C₁-C₆)alcoxy, -NR*R*, R* et R** étant définis comme ci-dessus, tri-(C₁-C₄)alkyl]-silyle, tri-[(C₁-C₄)alkyl] -silyl- (C₁-C₆)alkyle, tri-[(C₁-C₄)alkyl]-silyl-(C₁-C₆)alcoxy, (C₃-C₈)cycloalkyle, (C₃-C₈)cycloalcoxy, (C₃-C₈)cycloalkyl-(C₁-C₈)alkyle, (C₃-C₈)cycloalkyl- (C₁-C₈)alcoxy, (C₅-C₈)cycloalcényle, (C₅-C₈)cycloalcényl-(C₁-C₆)alkyle, (C₅-C₈)cycloalcényloxy, (C₅-C₈)cycloalcynyle, (C₅-C₈)cycloalcynyl- (C₁-C₆) alkyle, (C₅-C₈)cycloalcynyl-(C₁-C₆)alcoxy, phényle, phényl-(C₁-C₈)alkyle, phényl-(C₁-C₈)alcoxy, phénoxy, phénoxy-(C₁-C₈)alkyle, phénoxy-(C₁-C₈)alcoxy, phénylamino, phénylamino-(C₁-C₈)alkyle, phénylamino-(C₁-C₈)alcoxy ou un hétérocycle, le cas échéant lié via un groupe alkylène ou un groupe alcoxy, monocyclique de 5 ou 6 chaînons ou bicyclique de 9 ou 10 chaînons, contenant 1, 2, 3 ou 4 hétéroatomes choisis dans le groupe formé par O, N et S, chacun des 20 derniers radicaux mentionnés étant le cas échéant substitué, dans la partie cyclique, par un ou plusieurs radicaux R^{cc} identiques ou différents et
R^{bb} et R^{cc} signifient à chaque fois, indépendamment, halogène, (C₁-C₄)alkyle, (C₁-C₄)halogénoalkyle, (C₁-C₄)alcoxy ou (C₁-C₄)halogénoalcoxy ou, dans le cas de groupes de base cycliques, saturés ou partiellement insaturés, également oxo et
M signifie un équivalent d'un cation, de préférence un équivalent d'ion métallique, un ion d'ammonium, qui est le cas échéant substitué par 1 à 4 radicaux identiques ou différents du groupe formé par (C₁-C₆)alkyle, (C₂-C₆)alcényle, (C₂-C₆)alcynyle, (C₃-C₆)cycloalkyle, (C₅-C₆)cycloalcényle, (C₅-C₆)cycloalcynyle, phényle, (C₃-C₆)cycloalkyl-(C₁-C₄)alkyle, (C₅-C₆)cycloalcényl-(C₁-C₄)alkyle, phényl(C₁-C₄)alkyle, ou un ion de sulfonium tertiaire, qui est de préférence substitué par 3 radicaux identiques ou différents du groupe formé par (C₁-C₆)alkyle, (C₂-C₆)alcényle, (C₂-C₆)alcynyle, (C₃-C₆) cycloalkyle, (C₅-C₆) cycloalcényle, (C₅-C₆)cycloalcynyle, phényle, (C₃-C₆)cycloalkyl-(C₁-C₄)alkyle, (C₅-C₆)cycloalcényl-(C₁-C₄)alkyle, phényl(C₁-C₄)alkyle, en particulier (C₁-C₄)alkyle,
m vaut 0, 1, 2 ou 3, de préférence 0, 1 ou 2, en particulier 0 ou 1, et
n vaut 0, 1, 2, 3, 4 ou 5, de préférence 0, 1, 2 ou 3.

2. Composés ou leurs sels selon la revendication 1, **caractérisés en ce que**
R⁴ signifie hydrogène ou un radical hydrocarboné le cas échéant substitué ou un radical hétérocyclyle le cas échéant substitué, chacun des deux derniers radicaux contenant du carbone mentionnés, y compris les substituants, comprenant 1 à 30 atomes de carbone ou
R⁴ signifie un radical de formule SiR^{a}R^{b}R^{c}, -NR^{a}R^{b} ou -N=CR^{c}R^{d}, chacun des radicaux R^{a}, R^{b}, R^{c} et R^{d} dans les 3 dernières formules mentionnées, indépendamment, signifiant hydrogène ou un radical hydrocarboné le cas échéant substitué, cependant à l'exclusion de SiH₃ pour SiR^{a}R^{b}R^{c}, ou R^{a} et R^{b} formant ensemble avec l'atome de N du groupe -NR^{a}R^{b} un hétérocycle de 3 à 9 chaînons, qui peut contenir, en plus de l'atome N, un ou deux autres hétéroatomes de cycle du groupe formé par N, O et S et qui est non substitué ou substitué, ou R^{c} et R^{d} signifiant, ensemble avec l'atome C du groupe -N=CR^{c}R^{d}, un radical carbocyclique de 3 à 9 chaînons ou un radical hétérocyclique, qui peut contenir 1 à 3 hétéroatomes de cycle du groupe formé par N, O et S, le radical carbocyclique ou hétérocyclique étant non substitué ou substitué, chacun des radicaux R^{a}, R^{b}, R^{c} et R^{d}, y compris les substituants, comprenant jusqu'à 30 atomes de carbone, ou
R⁴ signifie un radical -C(=O)-R^{e} ou -P(=O) (R^{f})₂, R^{e} et les radicaux R^{f} signifiant, indépendamment, à chaque fois hydrogène, OH, (C₁-C₈)alkyle, (C₁-C₄)halogénoalkyle, (C₂-C₈)alcényle, (C₂-C₈)alcynyle, (C₁-C₆)alcoxy, (C₁-C₆)alcoxy-(C₁-C₈)alkyle, (C₁-C₄)halogénoalcoxy, (C₁-C₄) halogénoalcoxy- (C₁-C₈)alkyle, (C₃-C₈)alcényloxy, (C₃-C₈)alcényloxy-(C₁-C₈)alkyle, (C₃-C₈)alcynyloxy, (C₃-C₈)alcynyloxy- (C₁-C₈)alkyle, -NR*R*^{*}, R* et R** étant définis davantage ci-dessous, tri-[(C₁-C₄)alkyl]-silyle, tri- [(C₁-C₄)alkyl] -silyl- (C₁-C₈)alkyle, (C₃-C₆)cycloalkyle, (C₃-C₆)cycloalkyl-(C₁-C₈)alkyle, (C₅-C₆)cycloalcényle, (C₅-C₆)cycloalcényl-(C₁-C₈)alkyle, (C₅-C₆)cycloalcynyle, (C₅-C₆)cycloalcynyl-(C₁-C₈)alkyle, phényle, phényl-(C₁-C₈)alkyle, phénoxy, phénoxy-(C₁-C₈)alkyle, phénylamino, phénylamino-(C₁-C₈)alkyle, un radical Het⁷, Het⁷-(C₁-C₆)alkyle ou Het⁷-O-(C₁-C₆)alkyle, chacun des 15 derniers radicaux mentionnés étant non substitué ou substitué, dans la partie acyclique, par un ou plusieurs radicaux R^{A} identiques ou différents et étant non substitué ou substitué, dans la partie cyclique, par un ou plusieurs radicaux R^{B} identiques ou différents,
Het⁷ signifie à chaque fois, indépendamment, un radical hétérocyclyle monocyclique saturé, partiellement insaturé ou hétéroaromatique comprenant 3 à 9 atomes de cycle ou un hétérocycle bicyclique de 9 ou 10 chaînons, contenant à chaque fois 1, 2, 3 ou 4 hétéroatomes, choisis dans le groupe formé par O, N et S,
R*, R** signifient à chaque fois, indépendamment (également indépendamment d'autres radicaux NR*R**) H, (C₁-C₈)alkyle, (C₂-C₈)alcényle, (C₂-C₈)alcynyle, (C₁-C₄)alcoxy- (C₁-C₄)alkyle, (C₁-C₆)alcanoyle, [(C₁-C₄)halogénoalkyl]-carbonyle, [(C₁-C₄)alcoxy]-carbonyle, [(C₁-C₄)halogénoalcoxy] - carbonyle, (C₃-C₆)cycloalkyle, (C₃-C₆)cycloalkyl-(C₁-C₄)alkyle, phényle, phényl-(C₁-C₄)alkyle, chacun des 4 derniers radicaux mentionnés dans le cycle étant le cas échéant substitué par un ou plusieurs radicaux identiques ou différents du groupe formé par halogène, (C₁-C₄)alkyle, (C₁-C₄)halogénoalkyle, (C₁-C₄)alcoxy ou (C₁-C₄)halogénoalcoxy ou, dans le cas de groupes de base cycliques, saturés ou partiellement insaturés, également substitué par oxo, ou
R* et R** signifient, ensemble avec l'atome N, un hétérocycle de 3 à 8 chaînons, qui peut contenir en plus de l'atome N, un ou deux autres hétéroatomes de cycle du groupe formé par N, O et S et qui est non substitué ou substitué par un ou plusieurs radicaux du groupe formé par (C₁-C₄)alkyle, (C₁-C₄)halogénoalkyle et oxo,
R^{A} signifie halogène, cyano, hydroxy ou (C₁-C₆)alcoxy,
R^{B} signifie halogène, cyano, hydroxy, oxo, nitro, (C₁-C₈)alkyle, (C₁-C₆)halogénoalkyle, cyano-(C₁-C₆)alkyle, hydroxy-(C₁-C₆)alkyle, nitro-(C₁-C₆)alkyle, (C₂-C₈)alcényle, (C₂-C₈)halogénoalcényle, (C₂-C₈)alcynyle, (C₂-C₈)halogénoalcynyle, (C₁-C₈)alcoxy, (C₂-C₈)alcényloxy, (C₂-C₈)alcynyloxy, (C₁-C₈)halogénoalcoxy, (C₁-C₆)alcoxy- (C₁-C₆)alkyle, (C₁-C₆)alcoxy- (C₁-C₄)alcoxy, (C₁-C₆)halogénoalcoxy-(C₁-C₄)alkyle, (C₁-C₆)halogénoalcoxy-(C₁-C₄)alcoxy, (C₁-C₈)alkylthio, (C₂-C₆)alcénylthio, (C₂-C₆)alcynylthio, (C₁-C₈)alkylsulfinyle, (C₁-C₆)halogénoalkylsulfinyle, (C₁-C₈)alkylsulfonyle, (C₁-C₆)halogénoalkylsulfonyle, un radical de formule R^{aa}-C(=O)-, R^{aa}-C(=O)-(C₁-C₆)alkyle, les radicaux R^{aa} étant définis davantage ci-dessous, -NR*R**, les radicaux R* et R** étant définis davantage ci-dessous, tri-[(C₁-C₄)alkyl]-silyle, tri-[(C₁-C₄)alkyl]-silyl-(C₁-C₆)alkyle, (C₃-C₈)cycloalkyle, (C₃-C₈)cycloalcoxy, (C₃-C₈)cycloalkyl-(C₁-C₈)alkyle, (C₃-C₈)cycloalkyl- (C₁-C₈)alcoxy, phényle, phényl-(C₁-C₈)alkyle, phénoxy, phénoxy-(C₁-C₈)alkyle, phénylamino, phénylamino-(C₁-C₈)alkyle ou un hétérocycle monocyclique de 5 ou 6 chaînons ou bicyclique de 9 ou 10 chaînons, contenant 1, 2, 3 ou 4 hétéroatomes choisis dans le groupe formé par O, N et S, chacun des 11 derniers radicaux mentionnés étant substitué, dans la partie cyclique, le cas échéant par un ou plusieurs radicaux R^{bb} identiques ou différents, R^{aa} signifie à chaque fois, indépendamment, hydrogène, OH, (C₁-C₈)alkyle, (C₁-C₆)halogénoalkyle, (C₂-C₈)alcényle, (C₂-C₈)alcynyle, (C₁-C₈)alcoxy, (C₁-C₆)alcoxy- (C₁-C₆)alkyle, (C₁-C₆)alcoxy-(C₁-C₆)alkyloxy, (C₁-C₆)halogénoalcoxy, (C₁-C₆)halogénoalcoxy- (C₁-C₆)alkyle, (C₁-C₆)halogénoalcoxy- (C₁-C₆)alcoxy, (C₃-C₈)alcényloxy, (C₃-C₈)alcényloxy- (C₁-C₆)alkyle, (C₃-C₈)alcényloxy-(C₁-C₆)alcoxy, (C₃-C₈)alcynyloxy, (C₃-C₈)alcynyloxy-(C₁-C₆)alkyle, (C₃-C₈)alcynyloxy- (C₁-C₆)alcoxy, -NR*R*, R* et R** étant définis comme ci-dessus, tri-[(C₁-C₄)alkyl]-silyle, tri-[(C₁-C₄)alkyl]-silyl- (C₁-C₆)alkyle, tri- [(C₁-C₄)alkyl] - silyl-(C₁-C₆)alcoxy, (C₃-C₈)cycloalkyle, (C₃-C₈)cycloalcoxy, (C₃-C₈)cycloalkyl-(C₁-C₈)alkyle, (C₃-C₈)cycloalkyl- (C₁-C₈)alcoxy, (C₅-C₈)cycloalcényle, (C₅-C₈)cycloalcényl-(C₁-C₆)alkyle, (C₅-C₈)cycloalcényloxy, (C₅-C₈)cycloalcynyle, (C₅-C₈)cycloalcynyl- (C₁-C₆)alkyle, (C₅-C₈)cycloalcynyl-(C₁-C₆)alcoxy, phényle, phényl-(C₁-C₈)alkyle, phényl-(C₁-C₈)alcoxy, phénoxy, phénoxy-(C₁-C₈)alkyle, phénoxy-(C₁-C₈)alcoxy, phénylamino, phénylamino-(C₁-C₈)alkyle, phénylamino-(C₁-C₈)alcoxy ou un hétérocycle, le cas échéant lié via un groupe alkylène ou un groupe alcoxy, monocyclique de 5 ou 6 chaînons ou bicyclique de 9 ou 10 chaînons, contenant 1, 2, 3 ou 4 hétéroatomes choisis dans le groupe formé par O, N et S, chacun des 20 derniers radicaux mentionnés étant le cas échéant substitué, dans la partie cyclique, par un ou plusieurs radicaux R^{cc} identiques ou différents et
R^{bb} et R^{cc} signifient à chaque fois, indépendamment, halogène, (C₁-C₄)alkyle, (C₁-C₄)halogénoalkyle, (C₁-C₄)alcoxy ou (C₁-C₄)halogénoalcoxy ou, dans le cas de groupes de base cycliques, saturés ou partiellement insaturés, également oxo.

3. Composés ou leurs sels selon la revendication 1 ou 2, **caractérisés en ce que**
(R¹)ₘ signifie m substituants R¹,
R¹, lorsque m = 1, ou chacun des substituants R¹, lorsque m est supérieur à 1, indépendamment, signifiant halogène, cyano, nitro, hydroxy, (C₁-C₆)alkyle, (C₂-C₄)alcényle, (C₂-C₄)alcynyle, (C₁-C₆)alcoxy, (C₁-C₆)alkylthio, (C₁-C₆)alkylsulfinyle, (C₁-C₆)alkylsulfonyle, (C₁-C₄)halogénoalkyle, (C₁-C₄)halogénoalcoxy, (C₁-C₄)halogénoalkylthio, (C₁-C₄)halogénoalkylsulfinyle, (C₁-C₅)halogénoalkylsulfonyle, (C₂-C₄)halogénoalcényle, (C₂-C₄)halogénoalcynyle, (C₁-C₄)alcoxy-(C₁-C₄)alkyle, (C₁-C₄)halogénoalcoxy- (C₁-C₄)alkyle, (C₃-C₆)cycloalkyle, qui est le cas échéant substitué par un ou plusieurs radicaux du groupe formé par halogène et (C₁-C₄)alkyle, (C₃-C₆)cycloalcoxy, qui est le cas échéant substitué par un ou plusieurs radicaux du groupe formé par halogène et (C₁-C₄)alkyle, ou un radical de formule C(O)OR⁵, C(O)NR⁶R⁷, C(O)-Het¹, NR⁸R⁹ ou Het² ou à chaque fois deux groupes R¹ ou R¹ et R³ en position ortho sur le cycle formant ensemble un groupe de formule -Z¹-A*-Z², dans laquelle
A* représente un groupe alkylène comprenant 1 à 4 atomes de carbone, qui est le cas échéant substitué par un ou plusieurs radicaux du groupe formé par halogène, (C₁-C₄)alkyle, (C₁-C₄)halogénoalkyle, (C₁-C₄)alcoxy et (C₁-C₄)halogénoalcoxy,
Z¹ représente une liaison directe, O ou S et
Z² représente une liaison directe, O ou S,
le groupe -Z¹-A*-Z² formant ensemble avec les atomes de carbone liés au groupe du cycle phényle un cycle condensé de 5 ou 6 chaînons,
R⁵ signifie hydrogène, (C₁-C₄)alkyle, (C₁-C₄)halogénoalkyle, (C₃-C₆)cycloalkyle, (C₃-C₆)halogénocycloalkyle, (C₂-C₄)alcényle, (C₂-C₄)halogénoalcényle, (C₂-C₄)alcynyle ou le groupe M,
R⁶, R⁷, R⁸ et R⁹ signifient à chaque fois, indépendamment, hydrogène ou (C₁-C₄)alkyle, qui est non substitué ou substitué par un ou plusieurs radicaux du groupe formé par halogène, nitro, cyano et phényle, ou (C₃-C₆)cycloalkyle ou phényle, chacun des 2 derniers radicaux mentionnés étant à chaque fois, indépendamment, non substitué ou substitué par un ou plusieurs radicaux du groupe formé par halogène, (C₁-C₄)alkyle, (C₁-C₄)halogénoalkyle, phényle et benzyle,
Het¹ et Het² signifient à chaque fois, indépendamment, un radical saturé ou partiellement insaturé d'un hétérocycle comprenant 3 à 6 atomes de cycle et au moins un atome de N comme hétéroatome de cycle en position 1 du cycle et le cas échéant 1, 2 ou 3 autres hétéroatomes de cycle du groupe formé par N, O et S, le radical de l'hétérocycle étant lié au niveau de l'atome N en position 1 du cycle au reste de la molécule du composé de formule (I) et l'hétérocycle étant non substitué ou substitué par un ou plusieurs radicaux du groupe formé par halogène, (C₁-C₄)alkyle, (C₁-C₄)halogénoalkyle et oxo,
M signifie l'équivalent d'un cation et
m vaut 0, 1, 2 ou 3.

4. Composés ou leurs sels selon l'une quelconque des revendications 1 à 3, **caractérisés en ce que**
(R²) signifie n substituants R²,
R², lorsque n = 1, ou chacun des substituants R², lorsque n est supérieur à 1, indépendamment, signifiant halogène, cyano, nitro, hydroxy, (C₁-C₆)alkyle, (C₂-C₄)alcényle, (C₂-C₄)alcynyle, (C₁-C₆)alcoxy, (C₁-C₆)alkylthio, (C₁-C₆)alkylsulfinyle, (C₁-C₆)alkylsulfonyle, (C₁-C₄)halogénoalkyle, (C₁-C₄)halogénoalcoxy, (C₁-C₄)halogénoalkylthio, (C₁-C₄)halogénoalkylsulfinyle, (C₁-C₄)halogénoalkylsulfonyle, (C₂-C₄)halogénoalcényle, (C₂-C₄)halogénoalcynyle, (C₁-C₄)alcoxy-(C₁-C₄)alkyle, (C₁-C₄)halogénoalcoxy- (C₁-C₄) alkyle, (C₃-C₆)cycloalkyle, qui est le cas échéant substitué par un ou plusieurs radicaux du groupe formé par halogène et (C₁-C₄)alkyle, (C₃-C₆)cycloalcoxy, qui est le cas échéant substitué par un ou plusieurs radicaux du groupe formé par halogène et (C₁-C₄) alkyle, ou un radical de formule C(O)OR¹⁰, C(O)NR¹¹R¹², C(O)-Het³, NR¹³R¹⁴ ou Het⁴ ou à chaque fois deux groupes R² en position ortho sur le cycle formant ensemble un groupe de formule -Z³-A**-Z⁴, dans laquelle
A** représente un groupe alkylène comprenant 1 à 4 atomes de carbone, qui est le cas échéant substitué par un ou plusieurs radicaux du groupe formé par halogène, (C₁-C₄)alkyle, (C₁-C₄)halogénoalkyle, (C₁-C₄)alcoxy et (C₁-C₄)halogénoalcoxy,
Z³ représente une liaison directe, O ou S et
Z⁴ représente une liaison directe, O ou S,
le groupe -Z³-A**-Z⁴ formant ensemble avec les atomes de carbone liés au groupe du cycle phényle un cycle condensé de 5 ou 6 chaînons,
R¹⁰ signifie hydrogène, (C₁-C₄)alkyle, (C₁-C₄)halogénoalkyle, (C₃-C₆)cycloalkyle, (C₃-C₆)halogénocycloalkyle, (C₂-C₄)alcényle, (C₂-C₄)halogénoalcényle, (C₂-C₄)alcynyle ou le groupe M,
R¹¹, R¹², R¹³ et R¹⁴ signifient à chaque fois, indépendamment, hydrogène ou (C₁-C₄)alkyle, qui est non substitué ou substitué par un ou plusieurs radicaux du groupe formé par halogène, nitro, cyano et phényle, ou (C₃-C₆)cycloalkyle ou phényle, chacun des 2 derniers radicaux mentionnés étant à chaque fois, indépendamment, non substitué ou substitué par un ou plusieurs radicaux du groupe formé par halogène, (C₁-C₄)alkyle, (C₁-C₄)halogénoalkyle, phényle et benzyle,
Het³ et Het⁴ signifient à chaque fois, indépendamment, un radical saturé ou partiellement insaturé d'un hétérocycle comprenant 3 à 6 atomes de cycle et au moins un atome de N comme hétéroatome de cycle en position 1 du cycle et le cas échéant 1, 2 ou 3 autres hétéroatomes de cycle du groupe formé par N, O et S, le radical de l'hétérocycle étant lié au niveau de l'atome N en position 1 du cycle au reste de la molécule du composé de formule (I) et l'hétérocycle étant non substitué ou substitué par un ou plusieurs radicaux du groupe formé par halogène, (C₁-C₄)alkyle, (C₁-C₄)halogénoalkyle et oxo,
M signifie l'équivalent d'un cation et
n vaut 0, 1, 2, 3, 4 ou 5 ;

5. Composés ou leurs sels selon l'une quelconque des revendications 1 à 4, **caractérisés en ce que**
R³ signifie halogène, cyano, nitro, hydroxy, (C₁-C₆)alkyle, (C₂-C₄)alcényle, (C₂-C₄)alcynyle, (C₁-C₆)alcoxy, (C₁-C₆)alkylthio, (C₁-C₆)alkylsulfinyle, (C₁-C₆)alkylsulfonyle, (C₁-C₄)halogénoalkyle, (C₁-C₄)halogénoalcoxy, (C₁-C₄)halogénoalkylthio, (C₁-C₄)halogénoalkylsulfinyle, (C₁-C₄)halogénoalkylsulfonyle, (C₂-C₄)halogénoalcényle, (C₂-C₄)halogénoalcynyle, (C₁-C₄)alcoxy-(C₁-C₄)alkyle, (C₁-C₄)halogénoalcoxy-(C₁-C₄)alkyle, (C₃-C₆)cycloalkyle, qui est le cas échéant substitué par un ou plusieurs radicaux du groupe formé par halogène et (C₁-C₄)alkyle, (C₃-C₆)cycloalcoxy, qui est le cas échéant substitué par un ou plusieurs radicaux du groupe formé par halogène et (C₁-C₄)alkyle, ou un radical de formule C(O)OR¹⁵, C(O)NR¹⁶R¹⁷, C(O)-Het⁵, NR¹⁸R¹⁹ ou Het⁶,
R¹⁵ signifie hydrogène, (C₁-C₄)alkyle, (C₁-C₄)halogénoalkyle, (C₃-C₆)cycloalkyle, (C₃-C₆)halogénocycloalkyle, (C₂-C₄)alcényle, (C₂-C₄)halogénoalcényle, (C₂-C₄) alcynyle ou le groupe M mentionné, de préférence hydrogène, (C₁-C₄)alkyle ou le groupe M,
R¹⁶, R¹⁷, R¹⁸ et R¹⁹ signifient à chaque fois, indépendamment, hydrogène ou (C₁-C₄)alkyle, qui est non substitué ou substitué par un ou plusieurs radicaux du groupe formé par halogène, nitro, cyano et phényle, ou (C₃-C₆)cycloalkyle ou phényle, chacun des 2 derniers radicaux mentionnés étant à chaque fois, indépendamment, non substitué ou substitué par un ou plusieurs radicaux du groupe formé par halogène, (C₁-C₄)alkyle, (C₁-C₄)halogénoalkyle, phényle et benzyle,
Het⁵ et Het⁶ signifient à chaque fois, indépendamment, un radical saturé ou partiellement insaturé d'un hétérocycle comprenant 3 à 6 atomes de cycle et au moins un atome de N comme hétéroatome de cycle en position 1 du cycle et le cas échéant 1, 2 ou 3 autres hétéroatomes de cycle du groupe formé par N, O et S, le radical de l'hétérocycle étant lié au niveau de l'atome N en position 1 du cycle au reste de la molécule du composé de formule (I) et l'hétérocycle étant non substitué ou substitué par un ou plusieurs radicaux du groupe formé par halogène, (C₁-C₄)alkyle, (C₁-C₄)halogénoalkyle et oxo,
M signifie l'équivalent d'un cation.

6. Composés ou leurs sels selon l'une quelconque des revendications 1 à 5, **caractérisés en ce que** (R¹)ₘ signifie m substituants R¹,
R¹, lorsque m = 1, ou chacun des substituants R¹, lorsque m est supérieur à 1, signifiant, indépendamment, halogène, cyano, nitro, hydroxy, (C₁-C₄)alkyle, (C₁-C₄)alcoxy, (C₁-C₄)alkylthio, (C₁-C₄)alkylsulfinyle, (C₁-C₄)alkylsulfonyle, (C₁-C₄)halogénoalkyle, (C₁-C₄)halogénoalcoxy, (C₁-C₄)halogénoalkylthio, (C₁-C₄)halogénoalkylsulfinyle, (C₁-C₄)halogénoalkylsulfonyle, (C₁-C₄)alcoxy-(C₁-C₄)alkyle, (C₃-C₆)cycloalkyle, ou un radical de formule C(O)OR⁵, C(O)NR⁶R⁷, C(O)-Het¹, NR⁸R⁹ ou Het², ou à chaque fois deux groupes R¹ ou R¹ et R³ en position ortho sur le cycle signifiant ensemble un groupe de formule -Z¹-A*-Z², dans laquelle
A* représente un groupe alkylène comprenant 1 à 4 atomes de carbone, qui est le cas échéant substitué par un ou plusieurs radicaux du groupe formé par halogène, (C₁-C₄)alkyle, (C₁-C₄)halogénoalkyle, (C₁-C₄)alcoxy et (C₁-C₄)halogénoalcoxy,
Z¹ représente une liaison directe, O ou S et
Z² représente une liaison directe, O ou S,
le groupe -Z¹-A*-Z² formant ensemble avec les atomes de carbone liés au groupe du cycle phényle un cycle condensé de 5 ou 6 chaînons,
R⁵ signifie hydrogène, (C₁-C₄)alkyle ou le groupe M mentionné,
R⁶, R⁷, R⁸ et R⁹ signifient à chaque fois, indépendamment, hydrogène, (C₁-C₄)alkyle, (C₁-C₉)halogénoalkyle, benzyle, (C₃-C₆)cycloalkyle ou phényle,
Het¹ et Het² signifient à chaque fois, indépendamment, un groupe morpholino, pipéridino ou pyrrolidino et
m vaut 0, 1, 2 ou 3,
(R²)ₙ signifie n substituants R²,
R², lorsque n = 1, ou chacun des substituants R², lorsque n est supérieur à 1, signifiant, indépendamment, halogène, cyano, nitro, hydroxy, (C₁-C₄)alkyle, (C₁-C₄)alcoxy, (C₁-C₄)alkylthio, (C₁-C₄)alkylsulfinyle, (C₁-C₄) alkylsulfonyle, (C₁-C₄)halogénoalkyle, (C₁-C₄) halogénoalcoxy, (C₁-C₄)halogénoalkylthio, (C₁-C₉)halogénoalkylsulfinyle, (C₁-C₉)halogénoalkylsulfonyle, (C₁-C₉)alcoxy-(C₁-C₄)alkyle, (C₃-C₆) cycloalkyle, ou un radical de formule C(O)OR¹⁰, C(O)NR¹¹R¹², C(O)-Het³, NR¹³R¹⁴ ou Het⁴, ou à chaque fois deux groupes R² en position ortho sur le cycle signifiant ensemble un groupe de formule -Z³-A*^{*}-Z⁴, dans laquelle
A** représente un groupe alkylène comprenant 1 à 4 atomes de carbone, qui est le cas échéant substitué par un ou plusieurs radicaux du groupe formé par halogène, (C₁-C₄)alkyle, (C₁-C₄)halogénoalkyle, (C₁-C₄)alcoxy et (C₁-C₄)halogénoalcoxy,
Z³ représente une liaison directe, 0 ou S et
Z⁴ représente une liaison directe, 0 ou S,
le groupe -Z³-A*^{*}-Z⁴ formant ensemble avec les atomes de carbone liés au groupe du cycle phényle un cycle condensé de 5 ou 6 chaînons,
R¹⁰ signifie hydrogène, (C₁-C₄)alkyle ou le groupe M mentionné,
R¹¹, R¹², R¹³ et R¹⁴ signifient à chaque fois, indépendamment, hydrogène ou (C₁-C₄)alkyle, (C₁-C₉)halogénoalkyle, benzyle, (C₃-C₆)cycloalkyle ou phényle,
Het³ et Het⁴ signifient à chaque fois, indépendamment, un groupe morpholino, pipéridino ou pyrrolidino et
n vaut 0, 1, 2, 3, 4 ou 5,
R³ signifie halogène, cyano, nitro, hydroxy, (C₁-C₄)alkyle, (C₁-C₄) alcoxy, (C₁-C₄) alkylthio, (C₁-C₄)alkylsulfinyle, (C₁-C4)alkylsulfonyle, (C₁-C₄)halogénoalkyle, (C₁-C₄)halogénoalcoxy, (C₁-C₄)halogénoalkylthio, (C₁-C₄)halogénoalkylsulfinyle, (C₁-C₄)halogénoalkylsulfonyle, (C₁-C₄)alcoxy-(C₁-C₄)alkyle, (C₃-C₆)cycloalkyle ou un radical de formule C(O)OR¹⁵, C(O)NR¹⁶R¹⁷, C(O)-Het⁵, NR¹⁸R¹⁹ ou Het⁶,
R¹⁵ signifie hydrogène, (C₁-C₄)alkyle ou le groupe M mentionné,
R¹⁶, R¹⁷, R¹⁸ et R¹⁹ signifient à chaque fois, indépendamment, hydrogène, (C₁-C₄) alkyle, (C₁-C₄)halogénoalkyle, benzyle, (C₃-C₆)cycloalkyle ou phényle,
Het⁵ et Het⁶ signifient à chaque fois, indépendamment, un groupe morpholino, pipéridino ou pyrrolidino et
M signifie l'équivalent d'un cation.

7. Procédé pour la préparation d'un composé de formule (I), tel qu'il est défini selon l'une quelconque des revendications 1 à 6, **caractérisé en ce qu'**on obtient par exemple des mélanges de diastéréo-isomères des composés de formule (I) présentant une teneur en composé (I) à préparer, **caractérisé en ce que**
(a) dans le cas de la préparation de mélanges de diastéréo-isomères des composés de formule (I), on transforme des composés de formule (II) 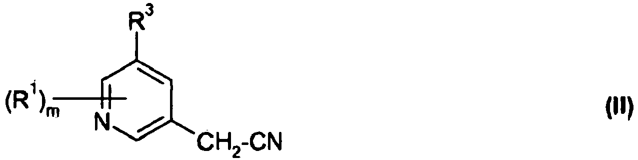 avec des composés de formule (III) ou leurs sels 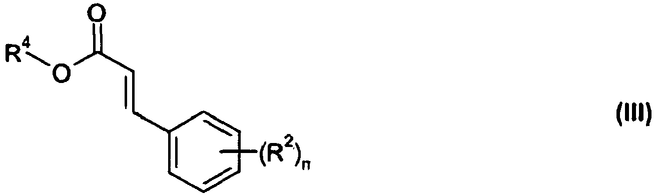 en composés de formule (I) 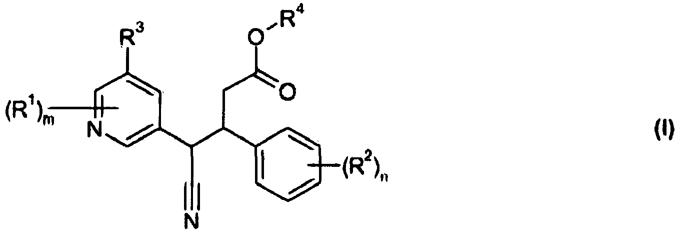 R¹, R², R³, R⁴, m et n dans les composés (II) et (III) étant définis comme dans le composé de formule (I) à chaque fois à préparer,
(b) on transforme des composés de formule (I*) 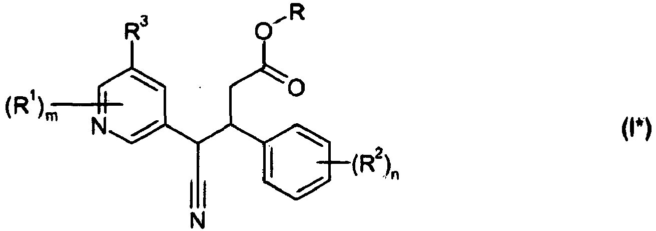 dans laquelle R signifie un radical du groupe des radicaux possibles pour R⁴, mais différent du radical R⁴ dans le composé (I) à préparer, avec un composé de formule R⁴-OH, dans laquelle R⁴ est défini comme dans la formule (I), en composé (I), R¹, R², R³, m et n dans le composé (I*) étant définis comme dans le composé de formule (I) à chaque fois à préparer,
(c) on transforme des composés de formule (I*) (cf. b), dans laquelle R représente un atome d'hydrogène, avec un composé de formule R⁴-OH, dans laquelle R⁴ est défini comme dans la formule (I), en composé (I), ou
(d) pour le cas où des composés optiquement actifs d'une forme diastéréo-isomère du composé de formule (I) sont préparés, on réalise, avec le mélange racémique de la forme diastéréo-isomère du composé de formule (I), une séparation de racémate et on isole l'énantiomère souhaité en une pureté stéréochimique de 60 à 100%, de préférence de 70 à 100%, plus préférablement de 80 à 100%, en particulier de 90 à 100%, par rapport au mélange des énantiomères érythro ou thréo existant.

8. Agent herbicide ou de régulation de la croissance de plantes, **caractérisé en ce qu'**il contient un ou plusieurs composés de formule (I) ou leurs sels, tels qu'ils sont définis selon l'une quelconque des revendications 1 à 6 et des adjuvants de formulation usuels dans la phytoprotection.

9. Procédé pour lutter contre des plantes nuisibles ou pour la régulation de la croissance de plantes, **caractérisé en ce qu'**on applique une quantité active d'un ou de plusieurs composés de formule générale (I) ou de leurs sels, tels qu'ils sont définis selon l'une quelconque des revendications 1 à 6, sur les plantes, les graines de plantes, le sol, dans ou sur lequel les plantes poussent, ou sur la surface de culture.

10. Procédé selon la revendication 9, **caractérisé en ce que** les composés de formule (I) ou leurs sels sont utilisés pour la lutte sélective contre des plantes nuisibles ou pour la régulation de la croissance dans les cultures de plantes utiles ou décoratives.

11. Utilisation des composés de formule (I) ou de leurs sels selon l'une quelconque des revendications 1 à 6 comme herbicides ou régulateurs de la croissance de plantes.
